# EUROPÄISCHE PATENTSCHRIFT

(11) **EP 1 307 446 B1**
(45) Veröffentlichungstag und Bekanntmachung des Hinweises auf die Patenterteilung: **20.04.2005**
(21) Anmeldenummer: 01969481.9
(22) Anmeldetag: 23.07.2001
(51) Int. Cl.: C07D 405/14, C07D 257/02, A61P 43/00, A61K 31/555

(54) **PERFLUORALKYLHALTIGE KOMPLEXE MIT ZUCKERRESTEN, VERFAHREN ZU DEREN HERSTELLUNG UND IHRE VERWENDUNG**
PERFLUOROALKYL-CONTAINING COMPLEXES COMPRISING SUGAR RESIDUES, METHOD FOR PRODUCING THE SAME AND USE THEREOF
COMPLEXES PERFLUOROALKYLES A RESIDUS SACCHARIDES, LEUR PROCEDE DE FABRICATION ET LEUR UTILISATION

(30) Priorität: 11.08.2000 DE 10040381
(43) Veröffentlichungstag der Anmeldung: 07.05.2003
(73) Patentinhaber: SCHERING AKTIENGESELLSCHAFT, 13342 Berlin (DE)
(72) Erfinder: PLATZEK, Johannes, 12621 Berlin (DE); MARESKI, Peter, 13359 Berlin (DE); NIEDBALLA, Ulrich, 14195 Berlin (DE); RADÜCHEL, Bernd, 13465 Berlin (DE); WEINMANN, Hanns-Joachim, 14129 Berlin (DE); MISSELWITZ, Bernd, 16548 Glienicke (DE)
(86) Internationale Anmeldenummer: PCT/EP2001/008499
(87) Internationale Veröffentlichungsnummer: WO 2002/014309

(56) Entgegenhaltungen:
- EP-A- 0 707 857
- WO-A-99/01161
- DE-A- 19 603 033
- US-A- 5 707 604
- US-A- 5 804 163

## Beschreibung

Die Erfindung betrifft die in den Patentansprüchen gekennzeichneten Gegenstände, nämlich perfluoralkylhaltige Metallkomplexe mit Zuckerresten der allgemeinen Formel I, Verfahren zu deren Herstellung und ihre Verwendung in der NMR- und Röntgendiagnostik, Radiodiagnostik und Radiotherapie, in der MRT-Lymphographie sowie als blood-pool-agents. Die erfindungsgemäßen Verbindungen sind in ganz besonderer Weise für die intravenöse Lymphographie, für die Tumor-Diagnostik und für das Infarkt- und Nekrose-Imaging geeignet.

In der kernmagnetischen Resonanz kommt nach dem Element Wasserstoff dem Element Fluor die größte Bedeutung zu.
1) Fluor hat eine hohe Empfindlichkeit von 83 % der des Wasserstoffs.
2) Fluor hat nur ein NMR-aktives Isotop.
3) Fluor hat eine dem Wasserstoff ähnliche Resonanzfrequenz - Fluor und Wasserstoff können mit der gleichen Anlage gemessen werden.
4) Fluor ist biologisch inert.
5) Fluor kommt in biologischem Material (Ausnahme: Zähne) nicht vor und kann deshalb als Sonde oder Kontrastmittel vor einem störsignalfreiem Untergrund eingesetzt werden.

Diese Eigenschaften führten dazu, daß Fluor einen breiten Raum in der diagnostischen Patentliteratur mit der magnetischen Kernresonanz als Grundlage einnimmt: Fluor-19-imaging, Funktionsdiagnose, Spektroskopie.

So werden in dem US Patent 4,639,364 (Mallinckrodt) Trifluormethansulfonamide als Kontrastmittel für das Fluor-19-imaging vorgeschlagen:

CF₃SO₂NH₂

CF₃SO₂NH-CH₂-(CHOH)₄-CH₂OH

Ebenfalls mit dem Fluor-19-imaging beschäftigt sich das deutsche Patent DE 4203254 (Max-Planck-Gesellschaft), in dem ein Anilinderivat vorgeschlagen wird:

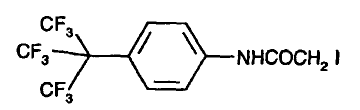

Das Fluor-19-imaging ist Gegenstand der Anmeldung WO 93/07907 (Mallinckrodt), in der ebenfalls Phenylderivate als Kontrastmittel beansprucht werden:

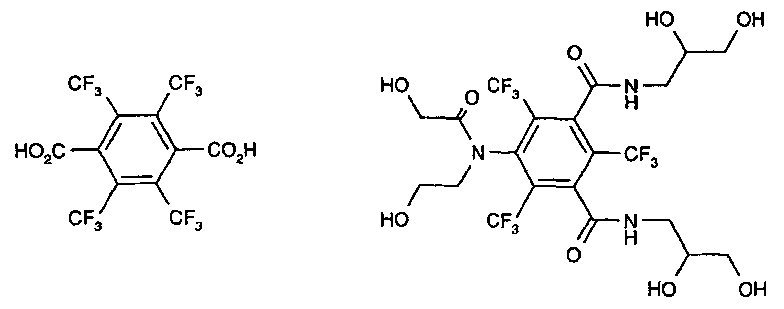

Für das Fluor-19-imaging werden auch erheblich einfacher gebaute Verbindungen beansprucht. So wird im Patent US 4,586,511 (Children's Hospital Medical Center) das Perfluoroctylbromid

CF₃(CF₂)₇-Br

genannt,
im Europäischen Patent EP 307863 (Air Products) der Perfluor-15-krone-5-ether 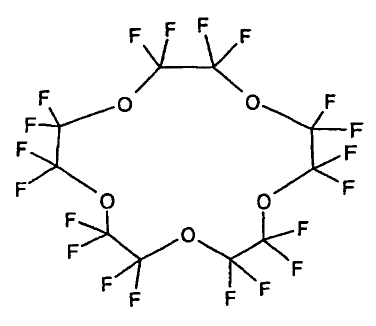 und im amerikanischen Patent US 4,588,279 (University of Cincinnati, Children's Hospital Research Foundation) Perfluorkohlenstoffverbindungen wie Perfluorcyclononan oder -octan, perfluorierte Ether wie das Tetrahydrofuran 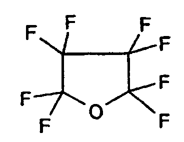 oder Diether wie der Perfluor-propylenglycol-diether 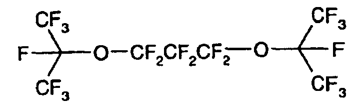 Ebenfalls für das Fluor-19-imaging dienen die in der Anmeldung WO 94/22368 (Molecular Biosystems) genannten Verbindungen z.B. 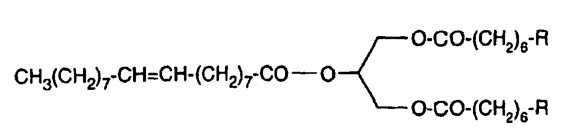 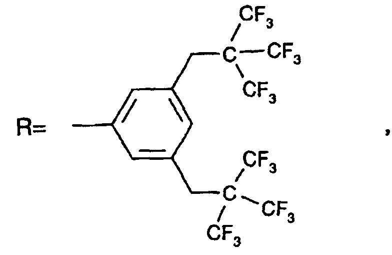 die als fluorhaltigen Rest die Perfluor-1H, 1H-neopentylgruppe besitzen.

Einen weiteren Strukturtyp mit erweiterter diagnostischer Nutzung zeigt das amerikanische Patent US 5,362,478 (VIVORX) auf, in dem die Kombination Fluorkohlenstoff / polymere Hülle für imaging-Zwecke beansprucht wird. Genannt werden Perfluornonan und Humanserumalbumin. Diese Kombination erweist sich darüberhinaus als geeignet, das Fluoratom als Sonde für lokale Temperaturmessung und zur Bestimmung des Sauerstoffpartialdruckes einzusetzen.

Perfluorkohlenstoffe werden auch im US Patent 4,586,511 zur Sauerstoffbestimmung beansprucht.

In der deutschen Patentschrift DE 4008179 (Schering) werden fluorhaltige Benzolsulfonamide als pH-Sonden beansprucht:

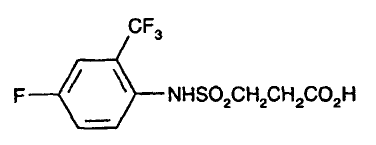

Für die NMR-Diagnostik werden auch Verbindungen, die Jod- und Fluoratome enthalten, als kontrastverstärkende Mittel beansprucht, so in WO 94/05335 und WO 94/22368 (beide Molecular Biosystems):

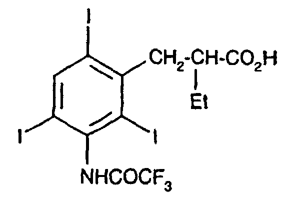

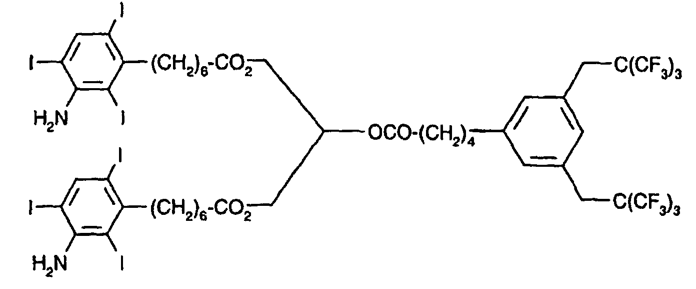

Auch die Kombination Fluor-paramagnetisches Metallion wird für das Fluor-19-imaging beansprucht, und zwar für offenkettige Komplexe in WO 94/22368 (Molecular Biosystems) mit z.B.: 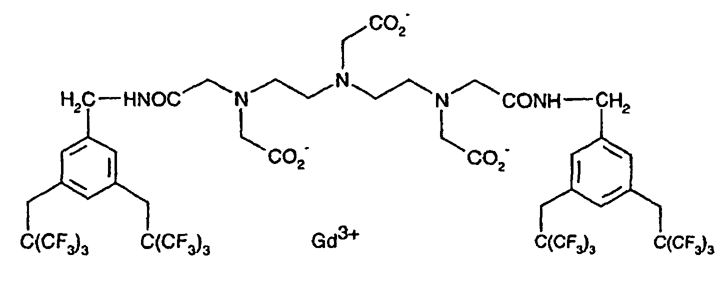 und in EP 292 306 (TERUMO Kabushiki Kaisha) mit z.B.: 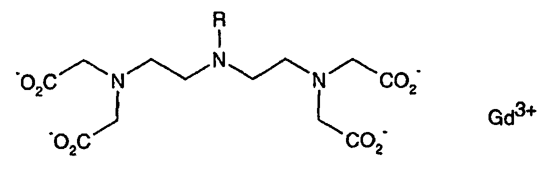 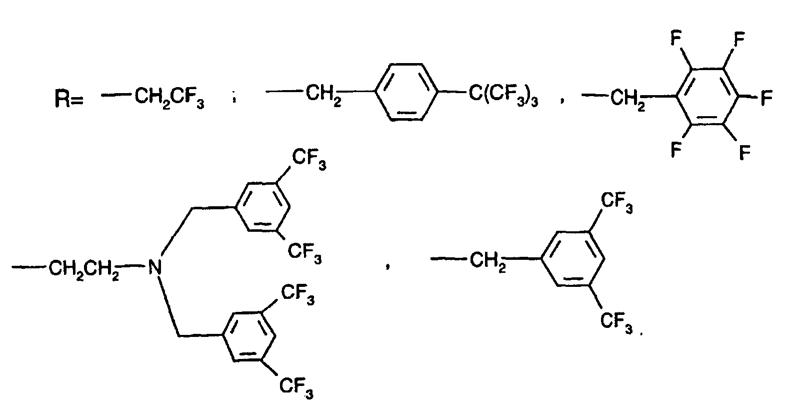 aber auch für cyclische Verbindungen, wie sie in EP 628 316 (TERUMO Kabushiki Kaisha) 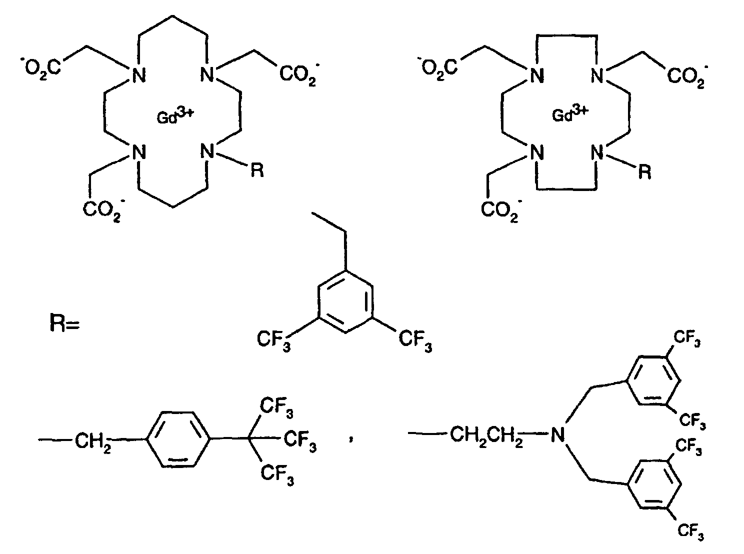 genannt werden.

Die Kombination Fluoratom-Seltenes Erdmetall wird auch für die NMR-spektroskopischen Temperaturmessungen in DE 4317588 (Schering) beansprucht:

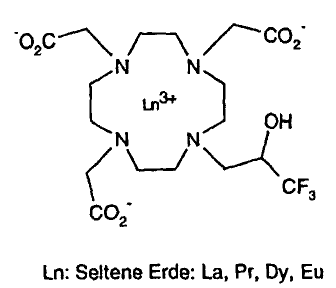

Während bei Verbindungen, die die Elemente Fluor und Jod enthalten, keine Wechselwirkungen zwischen den beiden Kernen stattfinden, findet in Verbindungen, die Fluor und paramagnetische Zentren (Radikale, Metallionen) enthalten, eine intensive Wechselwirkung statt, die sich in einer Verkürzung der Relaxationszeit des Fluorkernes äußern. Die Größe dieses Effekts hängt von der Anzahl der ungepaarten Elektronen des Metallions (Gd³⁺ > Mn²⁺ > Fe³⁺ > Cu²⁺) und von der Entfernung zwischen dem paramagnetischen Ion und dem ¹⁹F-Atom ab.

Je mehr ungepaarte Elektronen des Metallions vorhanden sind und je näher diese an das Fluor gebracht werden, desto größer ist die Verkürzung der Relaxationszeit des Fluorkernes.

Die Verkürzung der Relaxationszeit als Funktion des Abstandes vom paramagnetischen Ion macht sich bei allen Kernen mit ungerader Spinzahl bemerkbar, so auch beim Proton, und Gadoliniumverbindungen finden deshalb. breite Anwendung als Kontrastmittel in der Kernspintomographie (Magnevist® , Prohance®, Omniscan®, Dotarem®).

Beim ¹H-MR-imaging (¹H-MRI) wird jedoch die Relaxationszeit T¹ oder T² der Protonen, das heißt vor allem der Protonen des Wassers, und nicht die Relaxationszeit der Fluorkerne gemessen und für die Bildgebung verwendet. Das quantitative Maß für die Verkürzung der Reläxationszeit ist die Relaxivity [L/mmol^{·}s].

Zur Verkürzung der Relaxationszeiten werden mit Erfolg Komplexe paramagnetischer Ionen eingesetzt. In der folgenden Tabelle ist die Relaxivity einiger Handelspräparate angegeben:

| | **T**^{**1**}**-relaxivity in Wasser** [L/mmol^{·}s ,39°C, 0.47 T] | **T**^{**1**}**-relaxivity in Plasma** [L/mmol^{·}s,39°C, 0.47 T] |
|---|---|---|
| **MAGNEVIST ®** | 3,8 | 4,8 |
| **DOTAREM ®** | 3,5 | 4,3 |
| **OMNISCAN ®** | 3,8 | 4 , 4 |
| **PRO HANCE ®** | 3,7 | 4,9 |

In diesen Verbindungen finden nur Wechselwirkungen zwischen Protonen und dem Gadoliniumion statt. Für diese Kontrastmittel in Wasser wird also eine Relaxivity von ca. 4 [L/mmol·s] beobachtet.

Es werden also erfolgreich für das MR-imaging sowohl Fluor-Verbindungen für Fluor-19-imaging, bei dem die verkürzte Relaxationszeit des Fluor-Kernes ausgenutzt wird, als auch nicht Fluor-haltige Verbindungen, bei denen die Relaxationszeit der Protonen des Wassers gemessen wird, verwendet.

Bei der Einführung eines perfluorkohlenstoffhaltigen Restes in ein paramagnetisches Kontrastmittel, das heißt bei der Kombination von Eigenschaften, die bisher nur für Fluorimaging-Verbindungen als geeignet bekannt waren, mit Verbindungen, die für Protonen-imaging verwendet wurden, steigt überraschenderweise auch die Relaxivity betreffend die Protonen des Wassers rapide an. Sie erreicht nun Werte von 10-50 [L/mmol·s] im Vergleich zu Werten zwischen 3,5 und 3,8 [L/mmol·s] wie sie für einige Handelsprodukte in obiger Tabelle bereits aufgeführt wurden.

Aus DE 196 03 033.1 sind bereits perfluoralkylhaltige Metallkomplexe bekannt. Diese Verbindungen sind jedoch nicht für alle Anwendungen befriedigend einsetzbar. So besteht nach wie vor ein Bedarf an Kontrastmitteln für die Darstellung von malignen Tumoren, von Lymphknoten und von nekrotischem Gewebe.

Maligne Tumoren metastasieren gehäuft in regionale Lymphknoten, wobei auch mehrere Lymphknotenstationen beteiligt sein können. So werden Lymphknotenmetastasen in etwa 50 - 69% aller Patienten mit malignen Tumoren gefunden (Elke, Lymphographie, in: Frommhold, Stender, Thurn (eds.), Radiologische Diagnostik in Klinik und Praxis, Band IV, Thieme Verlag Stuttgart, 7th ed., 434-496, 1984). ). Die Diagnose eines metastatischen Befalls von Lymphknoten ist im Hinblick auf die Therapie und Prognose maligner Erkrankungen von großer Bedeutung. Mit den modernen bildgebenden Methoden (CT, US und MRI) werden lymphogene Absiedlungen von malignen Tumoren nur unzureichend erkannt, da zumeist nur die Größe des Lymphknotens als Diagnosekriterium herangezogen werden kann. Damit sind kleine Metastasen in nicht-vergrößerten Lymphknoten (< 2 cm) nicht von Lymphknotenhyperplasien ohne malignen Befall zu unterscheiden (Steinkamp et al., Sonographie und Kernspintomographie: Differentialdiagnostik von reaktiver Lymphknoten-vergrößerung und Lymphknotenmetastasen am Hals, Radiol.diagn. 33:158, 1992).

Wünschenswert wäre es, daß bei Einsatz von spezifischen Kontrastmitteln Lymphknoten mit metastatischem Befall und hyperplastische Lymphknoten unterschieden werden können.

Bekannt ist die direkte Röntgen-Lymphographie (Injektion einer öligen Kontrastmittelsuspension in ein präpariertes Lymphgefäß) als eine nur noch selten benutzte invasive Methode, die nur wenige Lymphabflußstationen darstellen kann.

Experimentell werden in Tierexperimenten auch Fluoreszenzmarkierte Dextrane verwendet, um nach deren interstitieller Applikation den Lymphabfluß beobachten zu können. Allen gebräuchlichen Markern für die Darstellung von Lymphwegen und Lymphknoten nach interstitieller/intrakutaner Applikation ist also gemein, daß es sich um Substanzen mit partikulärem Charakter ("particulates", z.B. Emulsionen und Nanokristallsuspensionen) oder große Polymere handelt (s.a. WO 90/14846).

Die bisher beschriebenen Zubereitungen erwiesen sich jedoch aufgrund ihrer mangelnden lokalen und systemischen Verträglichkeit sowie ihrer geringen Lymphgängigkeit, die eine unzureichende diagnostischen Effizienz bedingt, als noch nicht optimal für die indirekte Lymphographie geeignet.

Da die Darstellung von Lymphknoten von zentraler Bedeutung für die frühe Erkennung des metastatischen Befalls bei Krebspatienten ist, besteht ein großer Bedarf an lymphspezifischen Kontrastmittelzubereitungen zur Diagnose entsprechender Veränderungen des Lymphsystems.

Möglichst hohe Kontrastmittelbeladung und hohe Stabilität sind ebenso wünschenswert wie diagnostisch relevante, möglichst gleichmäßige Lymphanreicherung über mehrere Lymphstationen hinweg. Die Belastung des Gesamtorganismus sollte durch rasche und vollständige Ausscheidung des Kontrastmittels gering gehalten werden. Ein rascher Wirkungseintritt möglichst bereits innerhalb weniger Stunden nach Kontrastmittelapplikation ist für die radiologische Praxis von Bedeutung. Eine gute Verträglichkeit ist notwendig.

Nicht zuletzt ist es wünschenswert, lymphspezifische Kontrastmittel zur Verfügung zu haben, die es erlauben, in einer diagnostischen Sitzung sowohl den Primärtumor als auch eine mögliche Lymphknotenmetastasierung zur Darstellung zu bringen.

Ein anderer wichtiger Bereich in der Medizin ist die Detektion, Lokalisierung und Überwachung von Nekrosen oder Infarkten. So ist der Myokardinfarkt nicht ein stationärer Vorgang, sondern ein dynamischer Prozeß, der sich über einen längeren Zeitraum (Wochen bis Monate) erstreckt. Die Erkrankung verläuft in etwa drei Phasen, die nicht scharf voneinander getrennt, sondern überlappend sind. Die erste Phase, die Entwicklung des Myokardinfarktes, umfaßt die 24 Stunden nach dem Infarkt, in denen die Zerstörung wie eine Stoßwelle (Wellenfrontphänomen) vom Subendokard zum Myokard fortschreitet. Die zweite Phase, der bereits bestehende Infarkt, umfaßt die Stabilisierung des Bereiches, in dem Faserbildung (Fibrose) als Heilprozeß erfolgt. Die dritte Phase, der ausgeheilte Infarkt, beginnt, nachdem alles zerstörte Gewebe durch fibröses Narbengewebe ersetzt ist. Während dieser Periode findet eine umfangreiche Restrukturierung statt.

Bis heute ist kein präzises und zuverlässiges Verfahren bekannt, das die aktuelle Phase eines Myokardinfarktes am lebenden Patienten diagnostizierbar macht. Für die Beurteilung eines Myokardinfarktes ist es von entscheidender Bedeutung, zu wissen, wie groß der Anteil des bei dem Infarkt verlorenen Gewebes ist und an welcher Stelle der Verlust erfolgte, denn von dieser Kenntnis hängt die Art der Therapie ab.

Infarkte erfolgen nicht nur im Myokard, sondern auch in anderen Geweben, besonders im Hirn.

Während der Infarkt in gewissem Umfang heilbar ist, können bei einer Nekrose, dem lokal begrenzten Gewebetod, nur die schädlichen Folgen für den Restorganismus verhindert oder wenigstens gemildert werden. Nekrosen können auf vielfache Weise entstehen: durch Verletzungen, Chemikalien, Sauerstoffdefizit oder duch Strahlung. Wie beim Infarkt ist die Kenntnis von Umfang und Art einer Nekrose wichtig für das weitere ärztliche Vorgehen.

Schon früh erfolgten daher Versuche, die Lokalisation von Infarkten und Nekrosen durch Einsatz von Kontrastmitteln bei nichtinvasiven Verfahren wie Szintigraphie oder Kernspintomographie zu verbessern. In der Literatur nehmen die Versuche, Porphyrine für das Nekroseimaging einzusetzen, einen großen Raum ein. Die erzielten Resultate ergeben jedoch ein widersprüchliches Bild. So beschreiben Winkelman und Hoyes in Nature, 200, 903 (1967), daß sich Mangan-5,10,15,20-Tetrakis(4-sulfonatophenyl)-porphyrin (TPPS) selektiv im nekrotischen Teil eines Tumors anreichert.

Lyon et al. (Magn. Res. Med. 4, 24 (1987)) dagegen beobachteten, daß sich Mangan-TPPS im Körper verteilt, und zwar in Niere, Leber, Tumor und nur zu einem geringen Teil in den Muskeln. Interessant ist dabei, daß die Konzentration im Tumor erst am 4. Tag ihr Maximum erreicht und das auch nur, nachdem die Autoren die Dosis von 0.12 mmol/kg auf 0.2 mmol/kg gesteigert hatten. Die Autoren sprechen daher auch von einer nichtspezifischen Aufnahme des TPPS in den Tumor. Bockhurst et al. wiederum berichten in Acta Neurochir 60, 347 (1994, Suppl.), daß MnTPPS selektiv an Tumorzellen bindet.

Foster et al. (J. Nucl. Med. 26, 756 (1985)) ihrerseits fanden, daß sich ¹¹¹ In-5,10,15,20-Tetrakis-(4-N-methylpyridinium)-Porphyrin (TMPyP) nicht im nekrotischen Teil, sondern in den lebenden Randschichten anreichert. Daraus zu folgern, daß eine Porphyrin-Gewebe-Wechselwirkung besteht, ist naheliegend, aber nicht zwingend.

In Circulation Vol. 90, No. 4, Teil 2, Seite 1468, Abstract No. 2512 (1994) berichten Ni et al., daß sie mit einem Mangan-Tetraphenyl-Porphyrin (Mn-TPP) und einem Gadolinium-Mesoporphyrin (Gd-MP) Infarktbereiche darstellen können. In der internationalen Patentanmeldung WO 95/31219 wurden beide Substanzen zum Infarkt- und Nekroseimaging eingesetzt. Die Autoren Marchal und Ni schreiben (siehe Beispiel 3), daß für die Verbindung Gd-MP der Metallgehalt der Infarkt-Niere ähnlich hoch war wie der des nichtinfarzierten Organs, daß er jedoch für das Myokard beim infarzierten Gewebe (Beispiel 1) neunmal so groß war. Erstaunlich war, daß das Verhältnis der Signalintensitäten beim MRI für infarziertes im Vergleich zum gesunden Gewebe in beiden Fällen mit 2.10 bzw. 2.19 vergleichbar hoch war. Weitere Metalloporphyrine sind in der Anmeldung DE 19835082 (Schering AG) beschrieben werden.

Porphyrine neigen dazu, sich in der Haut abzulagern, was zu einer Photosensibilisierung führt. Die Sensibilisierung kann Tage, ja sogar Wochen andauern. Dies ist ein unerwünschter Nebeneffekt bei der Verwendung von Porphyrinen als Diagnostika. Außerdem ist der therapeutische Index für die Porphyrine nur sehr klein, da z.B. für Mn-TPPS eine Wirkung erst bei einer Dosis von 0.2 mmol/kg einsetzt, die LD₅₀ aber bereits bei 0.5 mmol/kg liegt.
Nicht vom Porphyringerüst abgeleitete Kontrastmittel für das Nekrose- und Infarkt-Imaging sind in DE 19744003 (Schering AG), DE 19744004 (Schering AG) und WO 99/17809 (EPIX) beschrieben. Bisher gibt es aber noch keine Verbindungen, die befriedigend als Kontrastmittel beim Infarkt- und Nekroseimaging eingesetzt werden können.

Aufgabe der Erfindung war es deshalb, Kontrastmittel zur verfügung zu stellen, die insbesondere für die MRT-Lymphographie, aber auch für die Tumor-Diagnostik und das Nekroseund Infarkt-Imaging einsetzbar sind.

Die Aufgabe der Erfindung wird durch die perfluoralkylhaltigen Komplexe mit Zuckerresten der allgemeinen Formel I 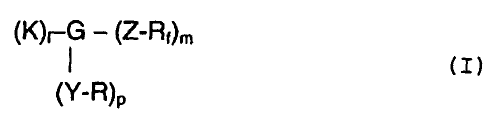 in der
- R: einen über die 1-OH- oder 1-SH-Position gebundenen Mono- oder Oligosaccharidrest darstellt,
- R_{f}: eine perfluorierte, geradkettige oder verzweigte Kohlenstoffkette mit der Formel -CₙF₂ₙE ist, in der E ein endständiges Fluor-, Chlor-, Brom-, Jod- oder Wasserstoffatom darstellt und n für die Zahlen 4-30 steht,
- K: für einen Metallkomplex der allgemeinen Formel II steht, 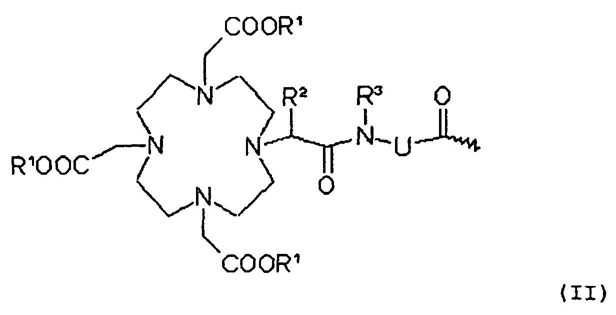
in der
- R¹: ein Wasserstoffatom oder ein Metallionenäquivalent der Ordnungszahlen 21-29, 31-33, 37-39, 42-44, 49 oder 57-83 bedeutet,
mit der Maßgabe, daß mindestens zwei R¹ für Metallionenäquivalente stehen
- R² und R³: unabhängig voneinander Wasserstoff, C₁-C₇-Alkyl, Benzyl, Phenyl, -CH₂OH oder -CH₂OCH₃ darstellen und
- U: -C₆H₄-O-CH₂-ω-, - (CH₂)₁₋₅-ω, eine Phenylengruppe, -CH₂-NHCO-CH₂-CH (CH₂COOH) -C₆H₄-ω-, -C₆H₄- (OCH₂CH₂)₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3-(CH₂)₀₋₅ COOH-Gruppen substituierte C₁-C₁₂-Alkylen- oder C₇-C₁₂-C₆H₄-O-Gruppe darstellt, wobei ω für die Bindungsstelle an -CO- steht,
oder
der allgemeinen Formel III 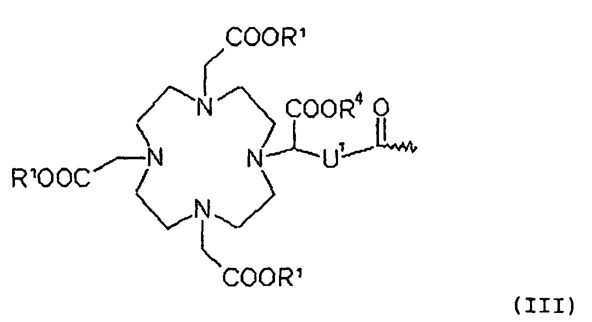 in der R¹ die oben genannte Bedeutung hat, R⁴ Wasserstoff oder ein unter R¹ genanntes Metallionenäquivalent darstellt und U¹ - C₆H₄-O-CH₂-ω- darstellt, wobei ω die Bindungsstelle an -CObedeutet
oder der allgemeinen Formel IV 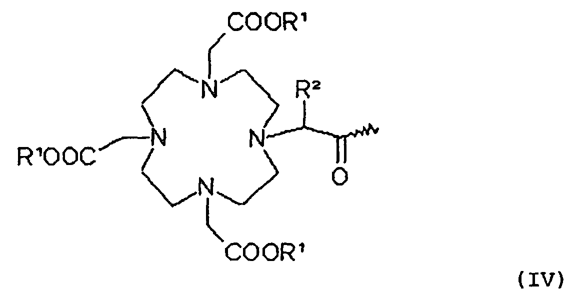 in der R¹ und R² die oben genannte Bedeutung haben
oder der allgemeinen Formel V A oder V B 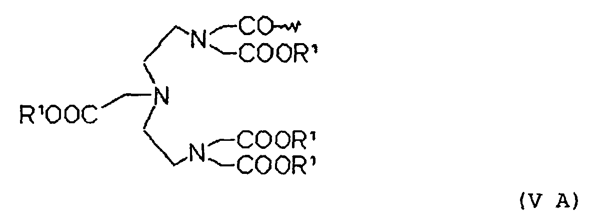 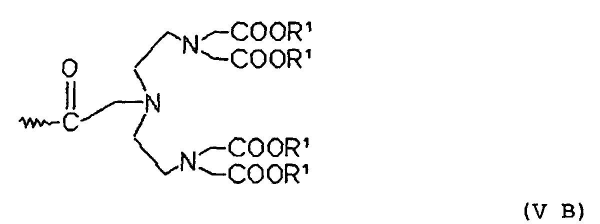 in der R¹ die oben genannte Bedeutung hat,
oder der allgemeinen Formel VI 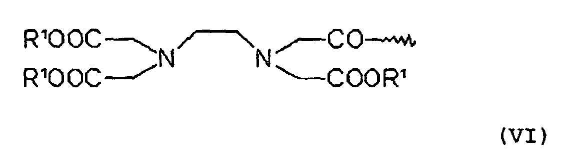 in der R¹ die oben genannte Bedeutung hat,
oder der allgemeinen Formel VII 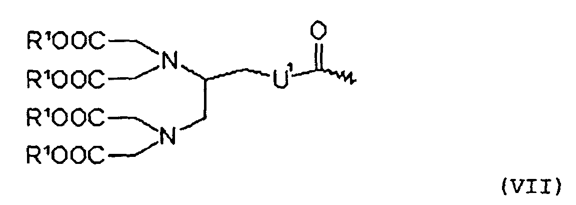 in der R¹ die oben genannte Bedeutung hat und
- U¹: -C₆H₄-O-CH₂-ω- darstellt, wobei ω die Bindungsstelle an -CO- bedeutet
oder der allgemeinen Formel VIII 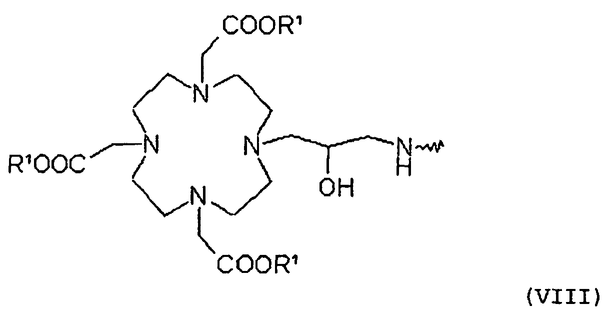 in der R¹ die oben genannte Bedeutung hat,
und im Rest K gegebenenfalls vorhandene freie Säuregruppen gegebenenfalls als Salze organischer und/oder anorganischer Basen oder Aminosäuren oder Aminosäureamide vorliegen können,
- G: für den Fall, daß K die Metallkomplexe II bis VII bedeutet, einen mindestens dreifach funktionalisierten Rest ausgewählt aus den nachfolgenden Resten a) bis j) darstellt 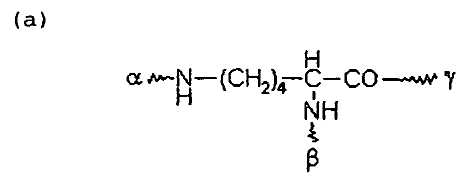 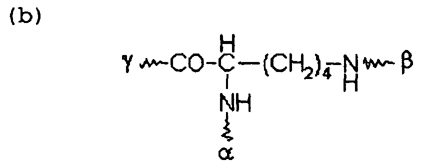 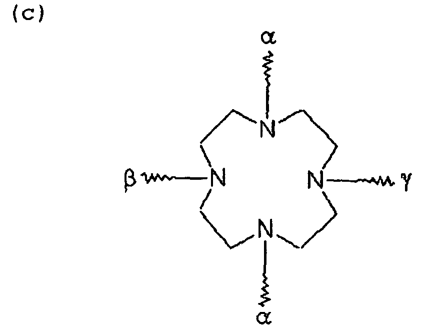 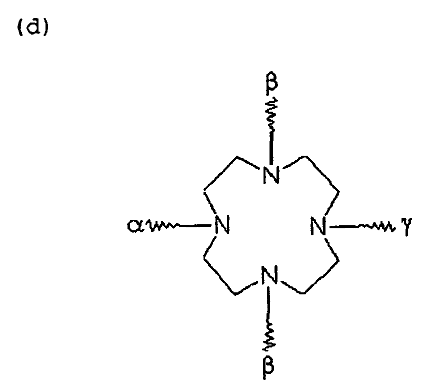 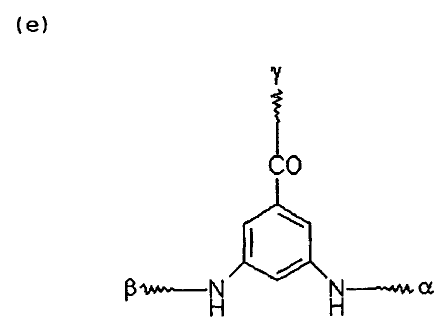 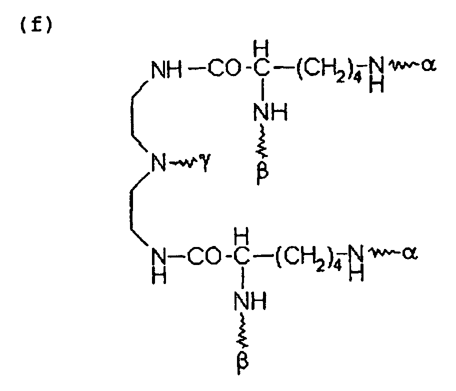 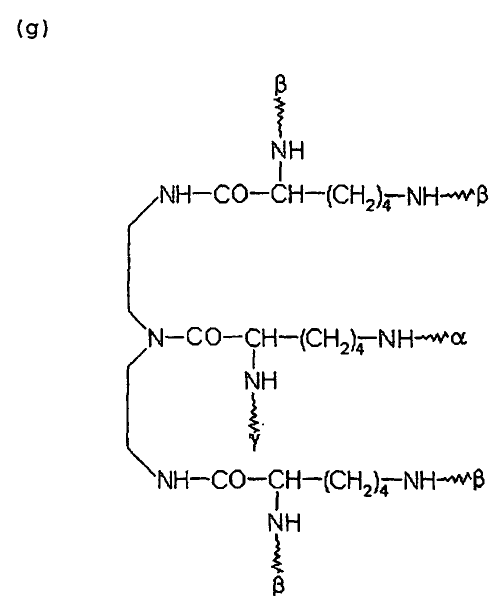 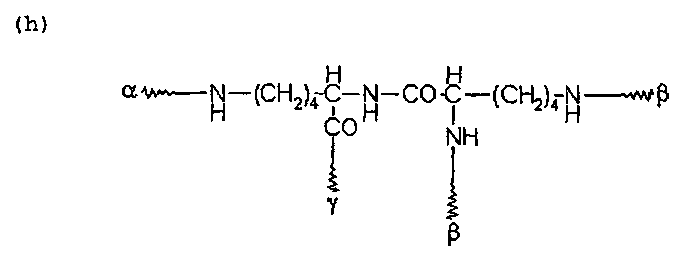 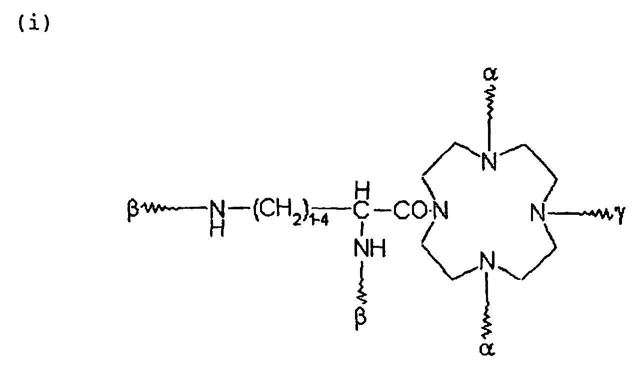 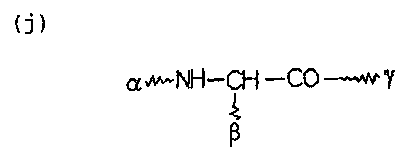
und
- G: für den Fall, daß K den Metallkomplex VIII bedeutet, einen mindestens dreifach funktionalisierten Rest ausgewählt aus. k) oder 1) darstellt, 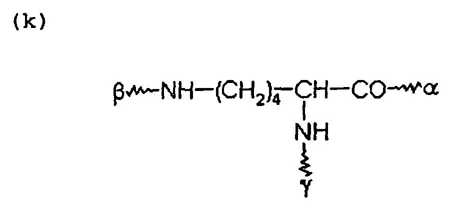 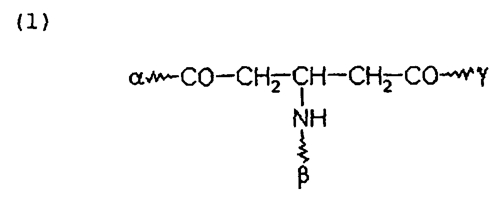
wobei α die Bindungsstelle von G an den Komplex K bedeutet, β die Bindungsstelle von G zum Rest Y ist und γ die Bindungsstelle von G zum Rest Z darstellt
- Y: -CH₂-, δ-(CH₂)ₙCO-β (wobei n=1-5 ist), δ-CH₂-CHOH-CO-β oder δ-CH(CHOH-CH₂OH)-CHOH-CHOH-CO-β bedeutet, wobei δ die Bindungsstelle zum Zuckerrest R darstellt und β die Bindungsstelle zum Rest G ist
- Z: für 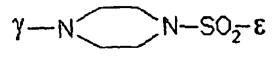 γ-COCH₂-N(C₂H₅)-SO₂-ε,
γ-COCH₂-O-(CH₂)₂-SO₂-ε, 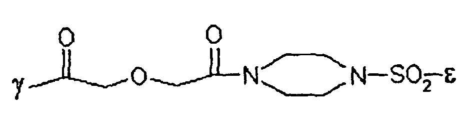 oder
γ - NHCH₂CH₂-O-CH₂CH₂ - ε
steht, wobei γ die Bindungsstelle von Z zum Rest G darstellt und ε die Bindungsstelle von Z an den
perfluorierten Rest R_{f} bedeutet
und
- l, m: unabhängig voneinander die ganzen Zahlen 1 oder 2 bedeuten und
- p: die ganzen Zahlen 1 bis 4 bedeutet,
gelöst.

Ist die erfindungsgemäße Verbindung zur Anwendung in der NMR-Diagnostik bestimmt, so muß das Metallion der signalgebenden Gruppe paramagnetisch sein. Dies sind insbesondere die zweiund dreiwertigen Ionen der Elemente der Ordnungszahlen 21-29, 42, 44 und 58-70. Geeignete Ionen sind beispielsweise das Chrom(III)-, Eisen(II)-, Kobalt(II)-, Nickel(II)-, Kupfer(II)-, Praseodym(III)-, Neodym(III)-, Samarium(III)- und Ytterbium(III)-ion. Wegen ihres starken magnetischen Moments sind besonders bevorzugt Gadolinium(III)-, Terbium(III)-, Dysprosium(III)-, Holmium(III)-, Erbium(III)-, Eisen(III)- und Mangan(II)-ionen.

Für die Verwendung der erfindungsgemäßen Verbindungen in der Nuklearmedizin (Radiodiagnostik und Radiotherapie) muß das Metallion radioaktiv sein. Geeignet sind zum Beispiel Radioisotope der Elemente mit den Ordnungszahlen 27, 29, 31-33, 37-39, 43, 49, 62, 64, 70, 75 und 77. Bevorzugt sind Technetium, Gallium, Indium, Rhenium und Yttrium.

Ist die erfindungsgemäße Verbindung zur Anwendung in der Röntgen-Diagnostik bestimmt, so leitet sich das Metallion vorzugsweise von einem Element höherer Ordnungszahl ab, um eine ausreichende Absorption der Röntgenstraheln zu erzielen. Es wurde gefunden, daß zu diesem Zweck diagnostische Mittel, die ein physiologisch verträgliches Komplexsalz mit Metallionen von Elementen der Ordnungszahlen 25, 26 und 39 sowie 57-83 enthalten, geeignet sind.

Bevorzugt sind Mangan(II)-, Eisen(II)-, Eisen(III)-, Praseodym(III)-, Neodym(III)-, Samarium(III)-, Gadolinium(III)-, Ytterbium(III)- oder Bismut(III)-ionen, insbesondere Dysprosium(III)-ionen und Yttrium(III)-ionen.

In R¹ gegebenenfalls vorhandene acide Wasserstoffatome, das heißt diejenigen, die nicht durch das Zentralion substituiert worden sind, können gegebenenfalls ganz oder teilweise durch Kationen anorganischer und/oder organischer Basen oder Aminosäuren oder Aminosäureamide ersetzt sein.

Geeignete anorganische Kationen sind beispielsweise das Lithiumion, das Kaliumion, das Calciumion und insbesondere das Natriumion. Geeignete Kationen organischer Basen sind unter anderem solche von primären, sekundären oder tertiären Aminen, wie zum Beispiel Ethanolamin, Diethanolamin, Morpholin, Glucamin, N,N-Dimethylglucamin und insbesondere N-Methylglucamin. Geeignete Kationen von Aminosäuren sind beispielsweise die des Lysins, des Arginins und des Ornithins sowie die Amide ansonsten saurer oder neutraler Aminosäuren.

Besonders bevorzugte Verbindungen der allgemeinen Formel I sind solche mit dem Makrocyclus K der allgemeinen Formel II.

Der Rest U im Metallkomplex K bedeutet vorzugsweise -CH₂- oder C₆H₄-O-CH₂-ω, wobei ω für die Bindungsstelle an -CO- steht.

Die Alkylgruppen R² und R³ im Makrocyclus der allgemeinen Formel II können geradkettig oder verzweigt sein. Beispielhaft seien Methyl, Ethyl, Propyl, Isopropyl, n-Butyl, 1-Methylpropyl, 2-Methylpropyl, n-Pentyl, 1-Methylbutyl, 2-Methylbutyl, 3-Methylbutyl, 1,2-Dimethylpropyl genannt. Vorzugsweise bedeuten R² und R³ unabhängig voneinander Wasserstoff oder C₁-C₄-Alkyl.
In einer ganz besonders bevorzugten Ausführungsform steht R² für Methyl und R³ für Wasserstoff.

Der Rest R in der allgemeinen Formel I bedeutet einen über die 1-OH- oder 1-SH-Position gebundenen Monosaccharidrest oder Thiozuckerrest, wobei es sich hierbei erfindungsgemäß auch um Desoxyzucker handeln kann, die anstelle einer oder mehrerer OH-Gruppen ein H-atom enthalten. In einer bevorzugten Ausführungsform der Erfindung bedeutet R einen Monosaccharidrest mit 5 oder 6 C-Atomen, vorzugsweise Glucose, Mannose, Galactose, Ribose, Arabinose oder Xylose oder deren Desoxyzucker wie beispielsweise 6-Desoxygalactose (Fucose) oder 6-Desoxymannose (Rhamnose) oder deren Thiozucker, wobei Glucose, Mannose und Galactose besonders bevorzugt sind.

Von den erfindungsgemäßen Verbindungen der allgemeinen Formel I sind weiterhin solche bevorzugt, in denen R_{f} -CₙF₂ₙ₊₁ bedeutet. n steht vorzugsweise für die Zahlen 4-15. Ganz besonders bevorzugt sind die Reste -C₄F₉, -C₆F₁₃, -C₈F₁₇, -C₁₂F₂₅ und -C₁₄F₂₉ sowie die Reste der in den Beispielen genannten Verbindungen.

Der mindestens dreifach funktionalisierte Rest G in der allgemeinen Formel I, der das "Gerüst" darstellt, bedeutet in einer bevorzugten Ausführungsform der Erfindung den Lysinrest (a) oder (b).

Y und Z bedeuten die in der allgemeinen Formel I angegebenen Linker, wobei unabhängig voneinander für Z der Rest 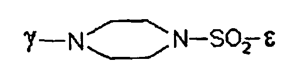 und für Y der Rest δ-CH₂CO-β bevorzugt sind.

Die perfluoralkylhaltigen Metallkomplexe mit Zuckerresten der allgemeinen Formel I 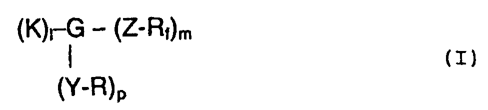 mit K in der Bedeutung eines Metallkomplexes der allgemeinen Formeln II bis VII und G in der Bedeutung der Formeln a) bis j), wobei Y, Z, R, R_{f}, m, p und 1 die oben genannte Bedeutung haben, werden hergestellt, indem man in an sich bekannter Weise eine Carbonsäure der allgemeinen Formel IIa 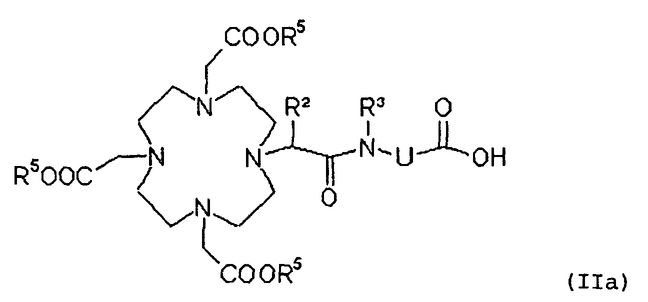 worin R⁵ ein Metallionenäquivalent der Ordnungszahlen 21-29, 31-33, 37-39, 42-44, 49 oder 57-83 oder eine Carboxylschutzgruppe bedeutet, und R², R³ und U die genannte Bedeutung haben
oder eine Carbonsäure der allgemeinen Formel IIIa 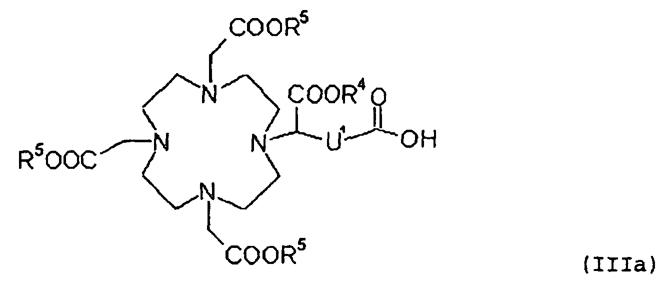 worin R⁴, R⁵ und U¹ die genannte Bedeutung haben
oder eine Carbonsäure der allgemeinen Formel IVa 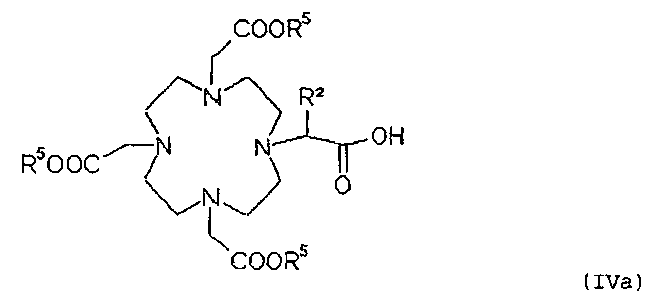 worin R⁵ und R² die genannte Bedeutung haben
oder eine Carbonsäure der allgemeinen Formel Va oder Vb 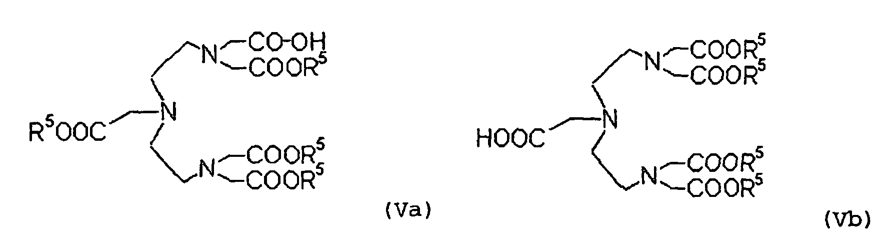 worin R⁵ die genannte Bedeutung hat
oder eine Carbonsäure der allgemeinen Formel VIa 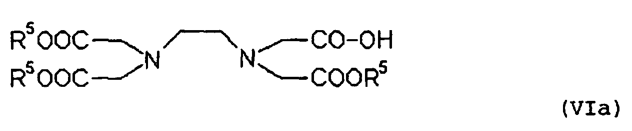 worin R⁵ die genannte Bedeutung hat
oder eine Carbonsäure der allgemeinen Formel VIIa 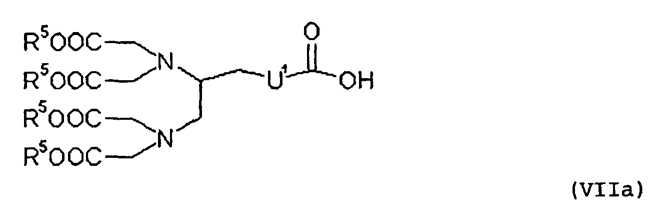 worin R⁵ und U¹ die genannten Bedeutungen haben,
in gegebenenfalls aktivierter Form mit einem Amin der allgemeinen Formel IX 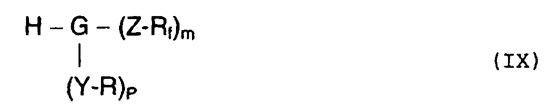 in der G die Bedeutung der Formeln a) bis j) hat und R, R_{f}, Y, Z, m und p die angegebene Bedeutung haben, in einer Kupplungsreaktion und gegebenenfalls nachfolgender Abspaltung gegebenenfalls vorhandener Schutzgruppen zu einem Metallkomplex der allgemeinen Formel I umsetzt oder
wenn R⁵ die Bedeutung einer Schutzgruppe hat, nach Abspaltung dieser Schutzgruppen in einem Folgeschritt in an sich bekannter Weise mit mindestens einem Metalloxid oder Metallsalz eines Elementes der Ordnungszahlen 21-29, 31-33, 37-39, 42-44, 49 oder 57-83 umsetzt, und anschließend, falls gewünscht, gegebenenfalls vorhandene acide Wasserstoffatome durch Kationen anorganischer und/oder organischer Basen, Aminosäuren oder Aminosäureamide substituiert.

Die erfindungsgemäßen Verbindungen der allgemeinen Formel I mit K in der Bedeutung eines Metallkomplexes der allgemeinen Formel VIII und G in der Bedeutung der Formeln k) oder 1) werden hergestellt, indem man in an sich bekannter Weise ein Amin der allgemeinen Formel VIIIa 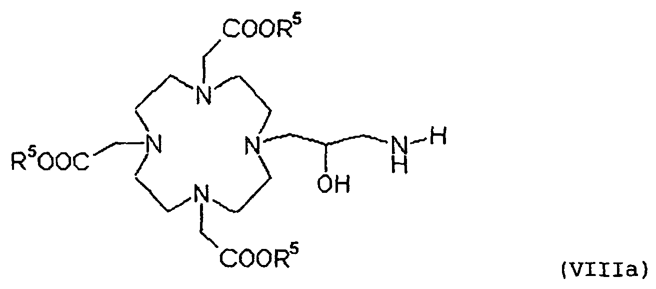 worin R⁵ ein Metallionenäquivalent der Ordnungszahlen 21-29, 31-33, 37-39, 42-44, 49 oder 57-83 oder eine Carboxylschutzgruppe bedeutet,
mit einer, gegebenenfalls aktivierten, Carbonsäure der allgemeinen Formel X 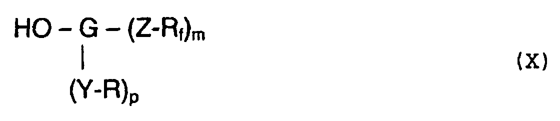 worin G die Bedeutung der Formeln k) oder 1) hat und R, R_{f}, Y, Z, m und p die angegebenen Bedeutungen haben, in einer Kupplungsreaktion und gegebenenfalls nachfolgender Abspaltung gegebenenfalls vorhandener Schutzgruppen zu einem Metallkomplex der allgemeinen Formel I umsetzt
oder
wenn R⁵ die Bedeutung einer Schutzgruppe hat, nach Abspaltung dieser Schutzgruppen in einem Folgeschritt in an sich bekannter Weise mit mindestens einem Metalloxid oder Metallsalz eines Elementes der Ordnungszahlen 21-29, 31-33, 37-39, 42-44, 49 oder 57-83 umsetzt, und anschließend, falls gewünscht, gegebenenfalls vorhandene acide Wasserstoffatome durch Kationen anorganischer und/oder organischer Basen, Aminosäuren oder Aminosäureamide substituiert.

Die eingesetzten Carbonsäuren der allgemeinen Formeln IIa bis VIIa sind entweder bekannte Verbindungen oder werden nach den in den Beispielen beschriebenen Verfahren hergestellt. So ist die Herstellung der Carbonsäuren der allgemeinen Formel IIa aus DE 196 52 386 bekannt. Die Herstellung von Carbonsäuren der allgemeinen Formel IIIa kann in Analogie zu Beispiel 3 der vorliegenden Anmeldung erfolgen. Die Herstellung der Carbonsäuren der allgemeinen Formel IVa ist DE 197 28 954 entnehmbar.

Vorstufe für Verbindungen der allgemeinen Formel VA ist die N³-(2,6-Dioxomorpholinoethyl)-N⁶-(ethoxycarbonylmethyl)-3,6-diazaoctandisäure, die in EP 263 059 beschrieben ist.

Die Verbindungen der allgemeinen Formel VB leiten sich von der isomeren Diethylentriamin-pentaessigsäure ab, die über die am mittleren N-Atom stehende Essigsäure bindet. Diese DTPA ist in den Patenten DE 195 07 819 und DE 195 08 058 beschrieben.

Verbindungen der allgemeinen Formel VI leiten sich vom N-(Carboxymethyl)-N-[2-(2,6-dioxo-4-morpholinyl)-ethyl]-glycin ab, dessen Herstellung in J. Am. Oil. Chem. Soc. (1982), 59 (2), 104-107, beschrieben ist.

Verbindungen der allgemeinen Formel VII leiten sich von der 1-(4-Carboxymethoxybenzyl)-ethylendiamin-tetraessigsäure ab, die im Patent US 4,622,420 beschrieben ist.

Die Herstellung der Amine der allgemeinen Formel IX und der Carbonsäuren der allgemeinen Formel X ist in den Beispielen der vorliegenden Anmeldung detalliert beschrieben und kann analog den in den Beispielen beschriebenen Verfahren vorgenommen werden. Das Amin der allgemeinen Formel VIIIa ist eine bekannte Ausgangsverbindung.

Die als Ausgangsstoffe eingesetzten perbenzylierten Zuckersäuren können analog Lockhoff, Angew. Chem. 1998, 110 Nr. 24, S. 3634ff. Hergestellt werden. So erfolgt z.B. die Herstellung der 1-O-Essigsäure von Perbenzyl-Glucose über 2 Stufen, via Trichloracetimidat und Umsetzung mit Hydroxyessigsäureethylester, BF₃-Katalyse in THF und anschließender Verseifung mit NaOH in MeOH/THF.

In einem vorteilhafteren Verfahren können die als Ausgangsstoffe eingesetzten perbenzylierten Zuckersäuren auch hergestellt werden, indem die perbenzylierten 1-OH-Zucker in einem mit Wasser nicht mischbaren organischen Lösungsmittel gelöst und mit einem Alkylierungsreagenz der allgemeinen Formel XI

Nu-L-COO-Sg (XI),

worin Nu ein Nucleofug bedeutet, L -(CH₂)-ₙ, (wobei n=1-5 ist), -CH₂-CHOH-, -CH(CHOH-CH₂OH)-CHOH-CHOH- ist, und Sg eine Schutzgruppe darstellt,

in Gegenwart einer Base und gegebenenfalls eines Phasentransfer-Katalysators umgesetzt werden. Als Nucleofug können im Alkylierungsreagenz der allgemeinen Formel XI beispielsweise die Reste -Cl, -Br, -J, -OTs, -OMs, -OSO₂CF₃, -OSO₂C₄F₉ oder -OSO₂C₈F₁₇ enthalten sein.
Bei der Schutzgruppe handelt es sich um eine übliche Säureschutzgruppe. Diese Schutzgruppen sind dem Fachmann gut vertraut (Protective Groups in Organic Syntheses, second Edition, T.W.Greene and P.G.M. Wuts, John Wiley & Sons Inc., New York 1991).

Die erfindungsgemäße Umsetzung kann bei Temperaturen von 0-50°C, vorzugsweise von 0°C bis Raumtemperatur erfolgen. Die Reaktionszeiten betragen von 10 Minuten bis 24 Stunden, vorzugsweise von 20 Minuten bis 12 Stunden.

Die Base wird entweder in fester Form, vorzugsweise fein gepulvert, oder als 10-70%ige, vorzugsweise 30-50%ige, wäßrige Lösung zugesetzt. Als bevorzugte Basen dienen NaOH und KOH.

Als organische, nicht mit Wasser mischbare Lösungsmittel können im erfindungsgemäßen Alkylierungsverfahren beispielsweise Toluol, Benzol, CF₃-Benzol, Hexan, Cyclohexan, Diethylether, Tetrahydrofuran, Dichlormethan, MTB oder deren Gemische eingesetzt werden.

Als Phasentransfer-Katalysatoren dienen im erfindungsgemäßen Verfahren die für diesen Zweck bekannten quartären Ammoniumoder Phosphoniumsalze oder auch Kronenether wie z. B. [15]-Krone-5 oder [18]-Krone-6. Vorzugsweise kommen quartäre Ammoniumsalze mit vier gleichen oder verschiedenen Kohlenwasserstoffgruppen am Kation, ausgewählt aus Methyl, Ethyl, Propyl, Isopropyl, Butyl oder Isobutyl in Frage. Die Kohlenwasserstoffgruppen am Kation müssen groß genug sein, um eine gute Löslichkeit des Alkylierungsreagenzes im organischen Lösungsmittel zu gewährleisten. Erfindungsgemäß besonders bevorzugt wird N(Butyl)₄⁺-Cl⁻, N(Butyl)₄⁺-HSO₄⁻, aber auch N(Methyl)₄⁺-Cl⁻ eingesetzt.

Es hat sich gezeigt, daß die erfindungsgemäßen Metallkomplexe insbesondere für die NMR- und Röntgen-Diagnostik, aber auch und für die Radiodiagnostik und Radiotherapie geeignet sind. Gegenstand der Erfindung ist deshalb auch die Verwendung der erfindungsgemäßen perfluoralkylhaltigen Metallkomplexe mit Zuckerresten zur Herstellung von Kontrastmitteln zur Anwendung in der NMR- und Röntgendiagnostik, insbesondere zur Lymphographie, zur Tumordiagnostik und zum Infarkt- und Nekrose-Imaging sowie in der Radiodiagnostik und Radiotherapie. Hervorragend geeignet sind die erfindungsgemäßen Verbindungen zur Anwendung in der interstitiellen und besonders in der intravenösen Lymphographie. Daneben können sie auch zur Darstellung des Vasalraumses (blood-pool-agents) dienen.

Gegenstand der Erfindung sind auch pharmazeutische Mittel, die mindestens eine physiologisch verträgliche erfindungsgemäße Verbindung enthalten, gegebenenfalls mit den in der Galenik üblichen Zusätzen.

Die Verbindungen der vorliegenden Erfindung zeichnen sich durch eine sehr gute systemische Verträglichkeit und eine hohe Lymphknotenanreicherung in drei aufeinanderfolgenden Lymphknotenstationen aus (was besonders für die i.v. Lymphographie wichtig ist). Sie sind damit besonders gut geeignet für die Anwendung in der MRT-Lymphographie.

Die erfindungsgemäßen Verbindungen sind auch in hervorragender Weise zur Erkennung und Lokalisierung von Gefäßkrankheiten geeignet, da sie sich bei der Applikation in den Intravasalraum ausschließlich in diesem verteilen. Die erfindungsgemäßen Verbindungen ermöglichen es, mit Hilfe der Kernspintomographie gut durchblutetes von schlecht durchblutetem Gewebe abzugrenzen und somit eine Ischämie zu diagnostizieren. Auch infarziertes Gewebe läßt sich aufgrund seiner Anämie vom umliegenden gesunden oder ischämischen Gewebe abgrenzen, wenn die erfindungsgemäßen Kontrastmittel angewandt werden. Dies ist von besonderer Bedeutung, wenn es z.B. darum geht, einen Herzinfarkt von einer Ischämie zu unterscheiden.

Gegenüber den bisher als blood-pool-agents eingesetzten makromolekularen Verbindungen, wie beispielsweise Gd-DTPA-Polylysin, zeigen die erfindungsgemäßen Verbindungen ebenfalls eine höhere T¹-Relaxivity und zeichnen sich somit durch eine Steigerung der Signalintensität bei der NMR-Bildgebung aus. Da sie daneben eine verlängerte Retention im Blutraum haben, können sie auch in relativ kleinen Dosierungen (von z.B. ≤ 50 µmol Gd/l Körpergewicht) appliziert werden. Vor allem aber werden die erfindungsgemäßen Verbindungen rasch und weitgehend vollständig aus dem Körper eliminiert.

Ferner zeigte sich, daß sich die erfindungsgemäßen Verbindungen in Gebieten mit erhöhter Gefäßpermeabilität, wie z.B. in Tumoren, anreichern; sie erlauben Aussagen über die Perfusion von Geweben, geben die Möglichkeit, das Blutvolumen in Geweben zu bestimmen, die Realaxationszeiten bzw. Densitäten des Blutes selektiv zu verkürzen, und die Permeabilität der Blutgefäße bildlich darzustellen. Solche physiologischen Informationen sind nicht durch den Einsatz von extrazellulären Kontrastmitteln, wie z.B. Gd-DTPA (Magnevist®) zu erhalten. Aus diesen Gesichtspunkten ergeben sich auch die Einsatzgebiete bei den modernen bildgebenden Verfahren Kernspintomographie und Computertomographie: spezifische Diagnose von malignen Tumoren, frühe Therapiekontrolle bei zytostatischer, antiphlogistischer oder vaso-dilatativer Therapie, frühe Erkennung von minderperfundierten Gebieten (z.B. im Myokard), Angiographie bei Gefäßerkrankungen, und Erkennung und Diagnose von sterilen oder infektiösen Entzündungen.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die zu den erfindungsgemäßen Metallkomplexen korrespondierenden Ca-Komplexe) oder - falls erforderlich - Elektrolyte wie zum Beispiel Natriumchlorid oder - falls erforderlich - Antioxidantien wie zum Beispiel Ascorbinsäure.

Sind für die enterale bzw. parenterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methyl-cellulose, Lactose, Mannit] und/oder Tensid(en) [zum Beispiel Lecithine, Tween®, Myrj®] und/oder Aromastoff(en) zur Geschmackskorrektur [zum Beispiel ätherischen Ölen] gemischt.

Prinzipiell ist es auch möglich, die erfindungsgemäßen pharmazeutischen Mittel ohne Isolierung der Komplexe herzustellen. In jedem Fall muß besondere Sorgfalt darauf verwendet werden, die Chelatbildung so vorzunehmen, daß die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Humanserumalbumin (HSA) verabreicht werden.

Die erfindungsgemäßen Mittel werden üblicherweise parenteral, vorzugsweise i.v., appliziert. Sie können auch intravasal oder interstitiell/intrakutan appliziert werden, je nachdem, ob Körpergefäße oder -gewebe untersucht werden sollen.

Die erfindungsgemäßen pharmazeutischen Mittel enthalten vorzugsweise 0,1µMol - 2 Mol/l des Komplexes und werden in der Regel in Mengen von 0,0001 - 5 mMol/kg dosiert.

Die erfindungsgemäßen Mittel erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Mittel erlaubt es, hochkonzentrierte Lösungen herzustellen, damit die Volumenbelastung des Kreislaufs in vertretbaren Grenzen zu halten und die Verdünnung durch die Körperflüssigkeit auszugleichen. Weiterhin weisen die erfindungsgemäßen Mittel nicht nur eine hohe Stabilität invitro auf, sondern auch eine überraschend hohe Stabilität invivo, so daß eine Freigabe oder ein Austausch der in den Komplexen gebundenen - an sich giftigen - Ionen innerhalb der Zeit, in der die neuen Kontrastmittel vollständig wieder ausgeschieden werden, nur äußerst langsam erfolgt.

Im allgemeinen werden die erfindungsgemäßen Mittel für die Anwendung als NMR-Diagnostika in Mengen von 0,0001-5 mMol/kg, vorzugsweise 0,005 - 0,5 mMol/kg, dosiert.

Ferner können die erfindungsgemäßen Komplexverbindungen vorteilhaft als Suszeptibilitäts-Reagenzien und als shift-Reagenzien für die in-vivo-NMR-Spektroskopie verwendet werden.

Die erfindungsgemäßen Mittel sind aufgrund ihrer günstigen radioaktiven Eigenschaften und der guten Stabilität der in ihnen enthaltenen Komplexverbindungen auch als Radiodiagnostika geeignet. Details einer solchen Anwendung und Dosierung werden z.B. in "Radiotracers for Medical Applications", CRC-Press, Boca Raton, Florida, beschrieben.

Die erfindungsgemäßen Verbindungen und Mittel können auch in der Positronen-Emissions-Tomographie, die positronenemittierende Isotope wie z.B. ⁴³Sc, ⁴⁴Sc, ⁵²Fe, ⁵⁵Co, ⁶⁸Ga und ⁸⁶Y verwendet (Heiss, W.D.; Phelps, M.E.; Positron Emission Tomography of Brain, Springer Verlag Berlin, Heidelberg, New York 1983), eingesetzt werden.

Die erfindungsgemäßen Verbindungen sind überraschenderweise auch zur Differenzierung von malignen und benignen Tumoren in Bereichen ohne Blut-Hirn-Schranke geeignet.

Die erfindungsgemäßen Kontrastmittel können nach intravenöser Applikation auch zur quantitativen Bestimmung von Änderungen der Kapillar-Integrität, hervorgerufen durch Hyperoxie (einschließlich "akuter kapillarer Löcher" und Wiederherstellung der normalen endothelialen Integrität nach hyperoxischer Schädigung)verwendet werden.
Histologische Untersuchungen bestätigen eine regionale mikrovaskulare Hyperpermeabilität.
Man kann daher die erfindungsgemäßen Kontrastmittel auch zur Darstellung abnormer Kapillar-Permeabilität verwenden.
Sie zeichnen sich auch dadurch aus, daß sie vollständig aus dem Körper eliminiert werden und somit gut verträglich sind.

Da sich die erfindungsgemäßen Substanzen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei Strahlentherapie von malignen Tumoren (z.B. Ausnutzen von Mössbauer-Effekten oder bei Neutroneneinfangtherapie). Geeignete β-emittierende Ionen sind zum Beispiel ⁴⁶Sc, ⁴⁷Sc, ⁴⁸Sc, ⁷²Ga, ⁷³Ga und ⁹⁰Y. Geeignete geringe Halbwertzeiten aufweisende α-emittierende Ionen sind zum Beispiel ²¹¹Bi, ²¹²Bi, ²¹³Bi und ²¹⁴Bi, wobei ²¹²Bi bevorzugt ist. Ein geeignetes Photonen- und Elektronen-emittierendes Ion ist 158_{Gd}, das aus ¹⁵⁷Gd durch Neutroneneinfang erhalten werden kann.

Ist das erfindungsgemäße Mittel zur Anwendung in der von R.L. Mills et al. (Nature Vol. 336, (1988), S. 787] vorgeschlagenen Variante der Strahlentherapie bestimmt, so muß sich das Zentralion von einem Mößbauer-Isotop wie beispielsweise ⁵⁷Fe oder ¹⁵¹Eu ableiten.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Human Serum Albumin verabreicht werden. Die Dosierung ist dabei abhängig von der Art der zellulären Störung, dem benutzten Metallion und der Art der bildgebenden Methode.

Die erfindungsgemäßen Mittel werden üblicherweise parenteral, vorzugsweise i.v., appliziert. Sie können auch - wie bereits erörtert - intravasal oder interstitiell/intrakutan appliziert werden, je nachdem, ob Körpergefäße oder -gewebe untersucht werden sollen.

Die erfindungsgemäßen Mittel sind hervorragend als Röntgenkontrastmittel geeignet, wobei besonders hervorzuheben ist, daß sich mit ihnen keine Anzeichen der von den jodhaltigen Kontrastmitteln bekannten anaphylaxieartigen Reaktionen in biochemisch-pharmakologischen Untersuchungen erkennen lassen. Besonders wertvoll sind sie wegen der günstigen Absorptionseigenschaften in Bereichen höherer Röhrenspannungen für digitale Substraktionstechniken.

Im allgemeinen werden die erfindungsgemäßen Mittel für die Anwendung als Röntgenkontrastmittel in Analogie zum Beispiel Meglumin-Diatrizoat in Mengen von 0,1 - 5 mMol/kg, vorzugsweise 0,25 - 1 mMol/kg, dosiert.

Insbesondere werden mit den erfindungsgemäßen Verbindungen höhere Blutkonzentrationen erreicht als mit extrazellulären Kontrastmitteln. Sie verteilen sich nach i.v. Applikation nur im Intravasalraum und haben damit einen entscheidenden Vorteil gegenüber den extrazellulären Kontrastmitteln.

### Beispiel 1

### a) 2-N-Trifluoracetyl -6-N- benzyloxycarbonyl-L-lysin

100,0 g (356,7mmol) 6-N-Benzyloxycarbonyl-L-lysin werden in einer Mischung aus 1000 ml Trifluoressigsäureethylester und 500 ml Ethanol gelöst und 24 Stunden bei Raumtemperatur gerührt. Man dampft zur Trockne ein und kristallisiert den Rückstand aus Diisopropylether.
Ausbeute: 128,9 g (96 % der Theorie) eines farblosen kristallinen Pulvers.
Schmelzpunkt: 98,5 °C.

| Elementaranalyse : | | | | |
|---|---|---|---|---|
| ber. | C 51,07 | H 5,09 | N 7,44 | F 15,14 |
| gef. | C 51,25 | H 5,18 | N 7,58 | F 15,03 |

### b) 2-N-Trifluoracetyl -6-N- benzyloxycarbonyl-L-lysin [1-(4-perfluoroctylsulfonyl)-piperazin]-amid

Zu 125,0 g (332,0mmol) der Titelverbindung aus Beispiel 1a) und 188,7 g (332,0 mmol) 1-Perfluoroctylsulfonylpiperazin (hergestellt nach DE 19603033 ) in 750 ml Tetrahydrofuran ,gibt man bei 0°C 164,2 g (0,664 mmol) EEDQ ( 2-Ethoxy-1,2-dihydrochinolin -1-carbonsäureethylester) zu und rührt über Nacht bei Raumtemperatur. Man dampft im Vakuum zur Trockene ein und chromatographiert an Kieselgel ( Laufmittel: Dichlormethan / Methanol = 20:1 ).
Ausbeute : 286,0 g (93% der Theorie ) eines farblosen Feststoffs.
Schmelzpunkt: 92 °C.

| Elementaranalyse: | | | | | |
|---|---|---|---|---|---|
| ber. | C 36,30 | H 2,83 | N 6,05 | F 41,01 | S 3,46 |
| gef. | C 36,18 | H 2,94 | N 5,98 | F 40,87 | S 3,40 |

### c) 6-N- Benzyloxycarbonyl-L-lysin-[1-(4-perfluoroctylsulfonyl)-piperazin]-amid

In eine Lösung aus 280,0 g (302,2mol ) der Titelverbindung aus Beispiel 1b) in 2000ml Ethanol, leitet man bei 0°C für eine Stunde Ammoniak-Gas ein. Man rührt anschließend 4 Stunden bei 0°C. Es wird zur Trockene eingedampft und der Rückstand aus Wasser ausgerührt. Man filtriert den Feststoff ab und trocknet im Vakuum bei 50 °C.
Ausbeute: 243,5 g (97 % der Theorie ) eines amorphen Feststoffs.

| Elementaranalyse: | | | | | |
|---|---|---|---|---|---|
| ber. | C 37,60 | H 3,28 | N 6,75 | F 38,89 | S 3,86 |
| gef. | C 37,55 | H 3,33 | N 6,68 | F 38,78 | S 3,81 |

### d) 6-N-Benzyloxycarbonyl - 2-N-[1-0-α-D -carbonylmethyl-(2,3,4,6-tetra-O-benzylmannopyranose]-L-lysin-[1-(4-perfluoroctylsulfonyl)-piperazin]-amid

Zu einer Lösung aus 100,0 g (120,4 mol) der Titelverbindung aus Beispiel 1c), 72,1 g (120,4 mol) 1-O-α-D-Carboxymethyl - 2,3,4,6-tetra-O-benzyl-mannopyranose und 13,86 g (120,4 mol) N-Hydroxysuccinimid, gelöst in 500 ml Dimethylformamid , gibt man bei 0°C 41,27g (200,0 mmol ) N,N - Dicyclohexylcarbodiimid hinzu. Man rührt 3 Stunden bei 0°C und anschließend über Nacht bei Raumtemperatur. Man filtriert vom ausgefallenen Harnstoff ab, dampft das Filtrat im Vakuum zur Trockene ein und chromatographiert an Kieselgel. (Laufmittel : Dichlormethan/Ethanol = 20 : 1).
Ausbeute: 136,1 g (87 % der Theorie) eines zähen Öls.

| Elementaranalyse: | | | | | |
|---|---|---|---|---|---|
| ber. | C 57,32 | H 4,89 | N 4,31 | F 24,86 | S 2,47 |
| gef. | C 57,38 | H 5,07 | N 4,22 | F 24,78 | S 2,39 |

### e) 2-N-[1-0-α-D-Carbonylmethyl-mannopyranose]-L-lysin-1-[(4-perfluoroctylsulfonyl)-piperazin)-amid

130,0 g (100,0 mmol) der Titelverbindung aus Beispiel 1d) werden in 2000 ml Ethanol gelöst und es werden 10,0 g Palladium Katalysator (10% Pd / C) hinzu gegeben. Man hydriert 12 Stunden bei Raumtemperatur. Man filtriert vom Katalysator ab und dampft das Filtrat im Vakuum zur Trockne ein.
Ausbeute : 91,7 g (quantitativ) eines farblosen Feststoffs.

| Elementaranalyse: | | | | | |
|---|---|---|---|---|---|
| ber. | C 34,07 | H 3,63 | N 6,11 | S 3,50 | F 35,24 |
| gef. | C 33,91 | H 3,72 | N 6,04 | S 3,40 | F 35,31 |

### f) 6-N-[1,4,7-Tris (carboxylatomethyl) ]-1,4,7,10-tetraazacyclododecan-10-N-(pentanoyl-3-aza-4-oxo-5-methyl-5-yl)]-2-N-[1-O-α-D-carbonylmethyl-mannopyranose]-L-lysin-[1-(4-perfluoroctylsulfonyl)-piperazin)-amid,Gd-Kornplex

50,0 g (54,55 mmol) der Titelverbindung aus Beispiel 1e), 6,28 g (54,55 mmol) N-Hydroxysuccinimid , 4,62 g (109,0 mol ) Lithiumchlorid und 34,35 g (54,55 mol) 1,4,7-Tris (carboxylatomethyl )-10- (carboxy - 3-aza-4-oxo-5-methyl-pent-5-yl )-1,4,7,10-tetraazacyclododecan, Gd-Komplex, werden unter leichter Erwärmung in 400 ml Dimethylsulfoxid gelöst. Bei 10°C gibt man 16,88 g (81,8 mol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18 Laufmittel: Gradient aus Wasser / Ethanol / Acetonitril).
Ausbeute: 75,9 g (91,0 % der Theorie) eines farblosen Feststoffs.
Wassergehalt : 8,6 %.

| Elementaranalyse (auf wasserfreie Substanz berechnet): | | | | | | |
|---|---|---|---|---|---|---|
| ber. | C 35,34 | H 4,09 | N 8,24 | S 2,10 | F 21,12 | Gd 10,28 |
| gef. | C 35,28 | H 4,15 | N 8,19 | S 2,15 | F 21.03 | Gd 10,14 |

### Beispiel 2

### a) 6-N-[1,4,7-Tris(carboxylatomethyl]-1,4,7,10-tetraazacyclododecan-10-N-(pentanoyl-3-aza-4-oxo-5-yl) ]-2-N-[1-O-α-D-carbonylmethyl-mannopyranose]-L-lysin-[1-(4-perfluoroctylsulfonyl)-piperazin]-amid, Gd-Komplex

50,0 g (54,55 mmol) der Titelverbindung aus Beispiel 1e), 6,28g (54,55mmol) N-Hydroxysuccinimid, 4,62g (109,0 mol ) Lithiumchlorid und 34,35g (54,55 mol) 1,4,7-Tris (carboxylatomethyl)-10- (carboxy-3-aza-4-oxo-5-methyl-pent-5-yl)-1,4,7,10-tetraazacyclododecan,Gd-Komplex , werden unter leichter Erwärmung in 400 ml Dimethylsulfoxid gelöst. Bei 10 °C gibt man 16,88g (81,8 mmol) N,N-Dicyclohexylcarbodiimid zu und rührt anschließend über Nacht bei Raumtemperatur. Man gießt die Lösung in 3000 ml Aceton und rürt 10 Minuten. Der ausgefallene Feststoff wird abfiltriert und anschließend durch Chromatographie gereinigt (RP-18 Laufmittel: Gradient aus Wasser /Ethanol / Acetonitril.
Ausbeute: 76,0 g (92,0 % der Theorie) eines farblosen Feststoffs.
Wassergehalt: 6,88 %.

| Elementaranalyse ( berechnet auf wasserfreie Substanz ): | | | | | | |
|---|---|---|---|---|---|---|
| ber. | C 34,90 | H 3,93 | N 8,32 | S 2,12 | F 21,33 | Gd 10,38 |
| gef. | C 34,81 | H 4,02 | N 8,27 | S 2,09 | F 21,22 | Gd 10,19 |

### Beispiel 3

### a) 2-[4-3-Oxapropionsäureethylester ]-phenylessigsäuremethylester

Zu 200,0 g (1204,0 mmol) 4-Hydroxyphenylessigsäuremethylester , 212,0 g (2000,0 mmol) Natriumcarbonat in 2000 ml Aceton gibt man 233,8 g (1400,0 mmol ) 2-Bromessigsäureethylester und kocht 5 Stunden unter Rückfluß. Man filtriert den Feststoff ab und dampft im Vakuum zur Trockene ein. Der Rückstand wird an Kieselgel chromatographiert. (Laufmittel : n-Hexan / Essigsäureethyester = 15:1).
Ausbeute: 288,5 g (95,0 % der Theorie) eines farblosen Öls.

| Elementaranalyse: | | |
|---|---|---|
| ber. | C 61,90 | H 6,39 |
| gef. | C 61,75 | H 6,51 |

### b) 2-[4-3-Oxapropionsäureethylester )]-phenyl-2-bromessigsäuremethylester

Zu 285,0 g (1130,0 mmol) der Titelverbindung aus Beispiel 3a) , gelöst in 2000 ml Tetrachlorkohlenstoff, gibt man 201,0 g (1130,0 mmol) N-Bromsuccinimid und 100,0 mg Dibenzoylperoxid und kocht acht Stunden unter Rückfluß. Man kühlt im Eisbad , filtriert das ausgefallene Succinimid ab und dampft das Filtrat im Vakuum zur Trockene ein. Der Rückstand wird an Kieselgel gereinigt (Laufmittel: n-Hexan / Aceton = 15 : 1).
Ausbeute: 359,2 g (96,0 % der Theorie) eines farblosen zähen Öls.

| Elementaranalyse: | | | |
|---|---|---|---|
| ber. | C 47,28 | H 4,57 | Br 24,16 |
| gef. | C 47,19 | H 4,71 | Br 24,05 |

### c) 2-[4-(3-Oxapropionsäureethylester)]-phenyl-2-[1-(1,4,7,10-tetraazacyclododecan -1-yl]-essigsäuremethylester

Zu 603,0 g (3500,0 mmol) 1,4,7,10- Tetraazacyclododecan, in 6000 ml Chloroform gibt man 350,0 g (1057,0 mmol) der Titelverbindung aus Beispiel 3b) und rührt über Nacht bei Raumtemperatur. Man extrahiert 3 mal mit jeweils 3000 ml Wasser, trocknet die organische Phase über Magnesiumsulfat und dampft im Vakuum zur Trockene ein. Der Rückstand wird ohne weitere Aufreinigung in die nächste Reaktion (Beispiel 3d) eingesetzt.
Ausbeute : 448,0 g (quantitativ) eines zähen Öls.

| Elementaranalyse: | | | |
|---|---|---|---|
| ber. | C 59,70 | H 8,11 | N 13,26 |
| gef. | C 59,58 | H 8,20 | N 13,18 |

### d) 2-[4-(3-Oxapropionsäure)]-phenyl-2-[1,4,7-tris(carboxmethyl)-1,4,7,10-tetraazacyclododecan-10-yl]-essigsäure

445,0 g (1053,0 mmol) der Titelverbindung aus Beispiel 3c) und 496,0 g (5270,0 mmol) Chloressigsäure werden in 4000 ml Wasser gelöst. Man stellt mit 30 %iger wässriger Natronlauge auf einen pH-Wert von 10 und rührt 8 Stunden bei 70°C. Anschließend stellt man den pH-Wert der Reaktionslösung durch Versetzen mit 30 %iger wässriger Natronlauge auf 13 ein und kocht 30 Minuten unter Rückfluß. Die Lösung wird im Eisbad gekühlt und durch Zugabe von konz. Salzsäure auf einen pH-Wert von 1 gestellt. Man dampft im Vakuum zur Trockene ein. Der Rückstand wird in 4000 ml Methanol aufgenommen und eine Stunde bei Raumtemperatur ausgerührt. Man filtriert vom ausgefallenen Kochsalz ab, dampft das Filtrat zur Trockene ein und reinigt den Rückstand an RP-18 C (Laufmittel: Gradient aus Wasser / Ethanol / Acetonitril).
Ausbeute : 403,0 g (69,0 % der Theorie ) eines farblosen Feststoffs.
Wassergehalt: 10,2 %.

| Elementaranalyse ( berechnet auf wasserfreie Substanz): | | | |
|---|---|---|---|
| ber. | C 51,98 | H 6,18 | N 10,10 |
| gef. | C 51,80 | H 6,31 | N 10,01 |

### e) 2-[4-(3-Oxapropionsäure)]-phenyl-2-[1,4,7-tris(carboxylatometltyl)-1,4,7,10-tetraazacyclododecan-10-yl]-essigsäure, Gd-Komplex

Zu 400 g (721,3 mmol) der Titelverbindung aus Beispiel 3d) in 2000 ml Wasser gibt man 130,73 g (360,65 mmol) Gadoliniumoxid und rührt 5 Stunden bei 80°C. Die Lösung wird filtriert und das Filtrat gefriergetrocknet.
Ausbeute: 511 g (quantitativ) eines amorphen Feststoffs.
Wassergehalt : 11,0%.

| Elementaranalyse ( berechnet auf wasserfreie Substanz): | | | | |
|---|---|---|---|---|
| ber. | C 40,67 | H 4,41 | N 7,98 | Gd 22,19 |
| gef. | C 40,51 | H 4,52 | N 8,03 | Gd 22,05 |

### f) 6-N-[2-[4-(3-Oxapropionyl )-phenyl]-2-[1,4,7-tris (carboxylatomethyl )-1,4,7,10-tetraazacyclododecan-10-yl]-essigsäure)]-2-N-(1-O-α-D-carbonylmethyl-mannopyranose)-L-lysin-[1-(4-perfluoroctylsulfonyl )-piperazin]-amid, Gd-Komplex, Natiumsalz

50,0 g (54,55 mmol) der Titelverbindung aus Beispiel 1e), 6,28 g (54,55 mmol ) N-Hydroxysuccinimid , 4,62 g (109,0 mmol) Lithiumchlorid und 38,66 g (54,55 mmol). der Titelverbindung aus Beispiel 3e) werden unter leichter Erwärmung in 400 ml Dimethylsulfoxid gelöst. Bei 10 °C gibt man 16,88 g (81,8 mmol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18; Laufmittel: Gradient aus Wasser/Ethanol/Acetonitril). Man löst das erhaltene Produkt in wenig Wasser und stellt mit wässriger Natronlauge den pH-Wert der Lösung auf 7,4 ein. Anschließend wird die Produktlösung gefriertrocknet.
Ausbeute: 79,1 g (89% der Theorie) eines farblosen Feststoffs.
Wassergehalt: 10,3 %.

| Elementaranalyse (berechnet auf wasserfreie Substanz ): | | | | | | |
|---|---|---|---|---|---|---|
| ber. | C 36,86 | H 3,77 | N 6,88 | S 1,97 | F 19,82 | Gd 9,65 |
| gef. | C 36,75 | H 3,84 | N 6,80 | S 2,03 | F 19,75 | Gd 9,57 |

### Beispiel 4

### a) 6-N-[1,4,7-Tris(t butyloxycarbonylmethyl)-1,4,7,10-tetraazacyclododecan-10-carbonylmethyl]-2-N-(1-0-α-D-carbonylmethyl-mannopyranose)-L-lysin-[1-(4-perfluoroctylsulfonyl)-piperazin]-amid

15,0 g (26,19 mmol) 1,4,7-Tris(t-butyloxycarbonylmethyl)-10-carboxymethyl-1,4,7,10-tetraazacyclododecan (hergestellt nach: WO91/05762), 24,0 g (26,19 mmol) der Titelverbindung aus Beispiel 1e) und 3,01 g (26,19 mmol) N-Hydroxysuccinimid werden in 150 ml Dimethylformamid gelöst und bei 0°C 8,25 g (40,0 mmol ) N,N-Dicyclohexyicarbodiimid zugegeben. Man rührt über Nacht bei Raumtemperatur. Der ausgefallene Harnstoff wird abfiltriert und das Filtrat im Vakuum zur Trockene eingedampft. Der Rückstand wird an Kieselgel chromatographiert. (Laufmittel: Dichlormethan / Methanol = 20:1).
Ausbeute: 35,45 g (92,0 % der Theorie) eines farblosen Feststoffs.

| Elementaranalyse: | | | | | |
|---|---|---|---|---|---|
| ber. | C 44,08 | H 5,69 | N 7,62 | F 21,95 | S 2,18 |
| gef. | C 44,01 | H 5,81 | N 7,53 | F 21.87 | S 2,03 |

### b) 6-N-[1,4,7-Tris(carboxylatomethyl]-1,4,7,10-tetraazacyclododecan-10-carbonylmethyl-] -2-N -[1-O-α-D-carbonylmethyl-mannopyranose]-L-lysin-[1-(4-pertluoroctylsulfonyl)-piperazin]-amid, Gd-Komplex

30,0 g (20,39 mmol) der Titelverbindung aus Beispiel 4a) werden in 50 ml Chloroform gelöst und 300 ml Trifluoressigsäure zugegeben. Man rührt 10 Minuten bei Raumtemperatur. Es wird im Vakuum zur Trockene eingedampft und der Rückstand in 300 ml Wasser gelöst. Man gibt 3,69 g (10,19 mmol) Gadoliniumoxid zu und rührt 5 Stunden bei 80 °C. Die Lösung wird im Vakuum zur Trockene eingedampft und an Kieselgel gereinigt (RP-18; Laufmittel: Gradient aus Wasser / Ethanol / Acetonitril).
Ausbeute: 11,0 g (37,0 % der Theorie) eines farblosen und amorphen Feststoffs.
Wassergehalt : 11,3 %.

| Elementaranalyse ( berechnet auf wasserfreie Substanz): | | | | | | |
|---|---|---|---|---|---|---|
| ber. | C 34,62 | H 3,87 | N 7,69 | F 22,16 | S 2,20 | Gd 10,97 |
| gef. | C 34,57 | H 3,95 | N 7,60 | F 22,05 | S 2,13 | Gd 10,90 |

### Beispiel 5

### a) 6-N-[3,6,9-Tris (carboxymethyl)-3,6,9-triazaundecandisäure-1-carboxy-11-oyl]-2-N-[1-O-α-D-carbonylmethyl-mannopyranose]-L-lysin-[1-(4-perfluoroctylsulfonyl)-piperazin]-amid

Zu 24,0 g (26.19 mmol) der Titelverbindung aus Beispiel 1e), gelöst in 100 ml Dimethylformaid / 30ml Pyridin, gibt man 12,10 g (30,0 mmol) 3-N-(2,6-Dioxomorpholinoethyl)-6-N-(ethoxycarbonylmethyl)-3,6-diazaoctandisäure und rührt 5 Stunden bei 50 °C. Man dampft im Vakuum zur Trockene ein. Der Rückstand wird in 200 ml Wasser gelöst und der pH-Wert der resultierenden Lösung durch Zugabe von 20% iger wässriger Natronlauge auf 13 gestellt. Man rührt 8 Stunden bei 22 °C und einem pH-Wert von 13. Die Lösung wird durch Zugabe von konz.Salzsäure auf einen pH-Wert von 7,2 gebracht und anschließend im Vakuum zur Trockene eingedampft. Der Rückstand wird an Kieselgel RP-18 chromatographiert ( Laufmittel: Gradient aus Wasser / Ethanol / Acetonitril). Ausbeute : 17,26 g (51,0 % der Theorie) eines farblosen Feststoffs.
Wassergehalt: 9,3 %.

| Elementaranalyse (berechnet auf wasserfreie Substanz ): | | | | | |
|---|---|---|---|---|---|
| ber. | C 37,19 | H 4,21 | N 7,59 | F 25,00 | S 2,48 |
| gef. | C 37,10 | H 4,30 | N 7,48 | F 25,07 | S 2,42 |

### b) 6-N-[3,6,9-Tris(carboxylatomethyl) -3,6,9-triazaundecandisäure-1-carboxy-11-oyl]-2-N-[1-O-α-D-carbonylmethyl-mannopyranose]-L-lysin-[1-(4-perfluoroctylsulfonyl)-piperazin]-amid, Gd-Komplex, Natriumsalz

Zu 10,0 g (7,74 mmol) der Titelverbindung aus Beispiel 5a) in 100 ml Wasser gibt man 1,40 g (3,87 mmol) Gadoliniumoxid und rührt 2 Stunden bei 70 °C. Die Lösung wird filtriert. Das Filtrat mit 2N Natronlauge auf einen pH-Wert von 7,4 gestellt und gefriergetrocknet. Ausbeute: 11,36 g (quantitativ) eines amorphen Feststoffs.
Wassergehalt : 10,5 %.

| Elementaranalyse ( berechnet auf wasserfreie Substanz ): | | | | | | | |
|---|---|---|---|---|---|---|---|
| ber. | C 32,72 | H 3,43 | N 6,68 | S 2,18 | Gd 10,71 | Na 1,57 | F 22,00 |
| gef. | C 32,65 | H 3,51 | N 6,71 | S 2,08 | Gd 10,61 | Na 1,68 | F 21,87 |

### Beispiel 6

### a) 6-N-Benzyloxycarbonyl-2-N-[1,4,7-tris(carboxylatomethyl)-1,4,7,10-tetraazacyclododecan]-10-(pentanoyl-3aza 4-oxo-5-methyl-5yl)]-L-lysin-[1-(4-perfluoroctylsulfonyl)-piperazin]-amid, Gd-Komplex

50,0 g (60,20 mmol) der Titelverbindung aus Beispiel 1c), 6,93 g (60,20 mmol) N-Hydroxysuccinimid, 5,09 g ( 120,0 mmol ) Lithiumchlorid und 37,91 g (60,20 mmol) 1,4,7-Tris[ carboxylatomethyl )-1,4,7,10-tetraazacyclododecan-10-(pentanoyl-3-aza-4-oxo-5-methyl-5-yl), Gd-Komplex werden unter leichter Erwärmung in 400 ml Dimethylsulfoxid gelöst. Bei 10 °C gibt man 20,63 g (100,0 mmol) N,N- Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt ( Kieselgel RP-18; Laufmittel: Gradient aus Wasser / Ethanol /Acetonitril ).
Ausbeute: 75,53 g (87,0 % der Theorie) eines farblosen Feststoffs.
Wassergehalt : 10,1 %.

| Elementaranalyse ( berechnet auf wasserfreie Substanz ): | | | | | | |
|---|---|---|---|---|---|---|
| ber. | C 37,48 | H 3,84 | N 8,74 | S 2,22 | F 22,39 | Gd 10,90 |
| gef. | C 37,39 | H 4,02 | N 8,70 | S 2,16 | F 22,29 | Gd 10,75 |

### b) 2-N-[1,4,7-Tris(carboxylatomethyl]-1,4,7,10-tetraazacyclododecan-Gd-Komplex,10-(pentanoyl-3-aza-4-oxo-5-methyl-5-yl)-L-lysin-[1-(4-perfluoroctylsulfonyl)-piperazin]-amid

70,0 g (48,53 mmol ) der Titelverbindung aus Beispiel 1d) werden in 500 ml Wasser / 100 ml Ethanol gelöst, mit 5,0 g Palladium Katalysator ( 10% Pd / C ) versetzt und solange bei Raumtemperatur unter einer Wasserstoffatmosphäre (1 atm) hydriert, bis keine Wasserstoffaufnahme mehr zu beobachten ist. Anschließend saugt man vom Katalysator ab, wäscht gründlich mit Ethanol nach (zweimal mit jeweils 75 ml) und engt im Vakuum zur Trockne ein. Die Titelverbindung wird als stark viskoses und farbloses Öl erhalten.
Ausbeute: 63,5 g (quantitativ ).
Wassergehalt: 9,8 %.

| Elementaranalyse (berechnet auf wasserfreie Substanz ): | | | | | | |
|---|---|---|---|---|---|---|
| ber. | C 37,48 | H 3,84 | N 8,74 | S 2,22 | F 22,39 | Gd 10,90 |
| gef. | C 37,39 | H 4,03 | N 8,65 | S 2,20 | F 22,31 | Gd 10,78 |

### c) 6-N-(1-O-α-D-Carbonylmethyl-2,3,4,6-tetra-O-benzyl-mannopyranose)-2-N-[1,4,7-tris(carboxylatomethyl)-1,4,7,10-tetraazacyclododecan, Gd-Komplex-10-(pentanoyl-3-aza-4oxo-5-methyl-5yl)]-L-lysin-[1-(4-perfluoroctylsulfonyl)-piperazin]-amid

50,0 g (38,22 mmol ) der Titelverbindung aus Beispiel 6b), 4,40 g (38,22 mmol) N-Hydroxysuccinimid, 3,39 g (80,0 mmol) Lithiumchlorid und 22,88 g (38,22 mmol) 1-O-α-D-Carboxymethyl-2,3,4,6-tetra-O-benzyl-mannopyranose werden unter leichter Erwärmung ( 30 bis 40 °C ) in 400 ml Dimethylsulfoxid gelöst. Bei 10°C gibt man 10,32 g (50,0 mmol) N,N-Dicyclohexylcarbodiimid zu und rührt anschließend über Nacht bei Raumtemperatur. Man giesst die Lösung in 3000 ml Aceton und rührt 10 Minuten. Der ausgefallene Feststoff wird abfiltriert und anschließend durch Chromatographie gereinigt (Kieselgel RP-18, Laufmittel: Gradient aus Wasser /Ethanol / Acetonitril).
Ausbeute: 64,25 g (89,0 % der Theorie ) eines farblosen Feststoffs.
Wassergehalt: 10,9 %.

| Elementaranalyse ( berechnet auf wasserfreie Substanz ): | | | | | | |
|---|---|---|---|---|---|---|
| ber. | C 46,42 | H 4,54 | N 6,67 | S 1,70 | F 17,10 | Gd 8,33 |
| gef. | C 46,36 | H 4,71 | N 6,60 | S 1,61 | F 17,19 | Gd 8,21 |

### d) 6-N-(1-O-α-D-Carbonylmethyl-2,3,4,6-tetra-O-benzyl-mannopyranose)-2-N-[1,4,7-tris(carboxylatomethyl)-1,4,8,10-tetraazacyclododecan, Gd-Komplex-10-(pentanoyl-3-aza-4oxo-5-methyl-5yl)]-L-lysin-[ 1-(4-perfluoroctylsulfonyl )-piperazin]-amid

60,0 g (31,77 mmol ) der Titelverbindung aus Beispiel 6c) werden in 500 ml Ethanol gelöst und mit 6,0 g Palladium Katalysator (10% Pd/C ) versetzt. Es wird solange bei Raumtemperatur unter einer Wasserstoffatmosphäre (1 atm) hydriert, bis keine Wasserstoffaufnahme mehr zu beobachten ist. Anschließend saugt man vom Katalysator ab, wäscht gründlich mit Ethanol nach (zweimal mit jeweils 150 ml) und engt im Vakuum zur Trockne ein.
Ausbeute: 48,55 g ( quantitativ) eines farblosen Feststoffs.
Wassergehalt : 3,9 %.

| Elementaranalyse (berechnet auf wasserfreie Substanz ): | | | | | | |
|---|---|---|---|---|---|---|
| ber. | C 35,37 | H 4,02 | N 8,25 | S 2,10 | F 21,13 | Gd 10,29 |
| gef. | C 35,28 | H 4,13 | N 8,17 | S 2,03 | F 21,05 | Gd 10,20 |

### Beispiel 7

### a) 1,7-Bis-(benzyloxycarbonyl)- 4-[2-(N-ethyl-N-pertluoroctylsulfonyl]-amino] -acetyl]-1,4,7,10 tetraazacyclododecan)

Zu 50,0 g (113,5 mmol ) 1,7-Bis(benzyloxycarbonyl)-1,4,7,10-tetraazacyclodedecan und 66,42 g ( 113,5 mmol ) 2-(N-Ethyl-N- perfluoroctylsulfonyl)-aminoessigsäure (hergestellt nach DE 196 03 033 ) in 300 ml Tetrahydrofuran, gibt man bei 0°C 49,46 g (200,0 mmol ) EEDQ (2-Ethoxy-1,2-dihydrochinolin-1-carbonsäureethylester) hinzu und rührt über Nacht bei Raumtemperatur. Man dampft im Vakuum zur Trockene ein und chromatographiert an Kieselgel (Laufmittel :Dichlormethan /Methanol =20:1).
Ausbeute: 65,2 g (57 % der Theorie ) eines farblosen Feststoffs.

| Elementaranalyse: | | | | | |
|---|---|---|---|---|---|
| ber. | C 42,91 | H 3,80 | N 6,95 | F 32,05 | S 3,18 |
| gef. | C 42,85 | H 3,90 | N 6,87 | F 31,98 | S 3,15 |

### b) 1,7-Bis-( benzyloxy )-4-[2-(N-ethyl-N-pertluoroctylsulfonyl)-amino)- acetyl-10-[1-O-α-D - carbonylmethyl - 2,3,4,6-tetra-O-benzyl -mannopyranose ] - 1,4,7,10-tetraazacyclododecan

Zu 60,0 g (59,53 mmol ) der Titelverbindung aus Beispiel 7a) und 35,64 g (59,53 mmol) 1-O-α-D- Carboxymethyl -2,3,4,6-tetra-O-benzyl-mannopyranose, hergestellt nach..DE 19728954., in 300ml Tetrahydrofuran, gibt man bei 0°C 24,73 g (100 mmol EEDQ (2-Ethoxy-1,2-dihydrochinolin -1- carbonsäureethylester) hinzu und rührt über Nacht bei Raumtemperatur. Man dampft im Vakuum zur Trockene ein und chromatographiert an Kieselgel (Laufmittel : Dichlormethan /Methanol = 20:1).
Ausbeute: 76,6 g (81,0 % der Theorie ) eines farblosen Feststoffs.

| Elementaranalyse: | | | | | |
|---|---|---|---|---|---|
| ber. | C 54,44 | H 4,70 | N 4,41 | F 20,33 | S 2,02 |
| gef. | C 54,37 | H 4,81 | N 4,35 | F 20,27 | S 1,96 |

### c) 1-[2-(N-Ethyl-N-perfluoroctylsulfonyl )-amino]-acetyl-7-(1-O-α-D- carbonylmethyl-mannopyranose )-1,4,7,10- tetraazacyclododecan

70 g (44,07 mmol ) der Titelverbindung aus Beispiel 7b werden in 800 ml Ethanol gelöst und 8 g Palladium Katalysator ( 10% Pd/C ) zugegeben. Man hydriert bei Raumtemperatur. Man filtriert vom Katalysator ab und dampft das Filtrat im Vakuum zur Trockene ein.
Ausbeute: 42,3 g (quantitativ) eines farblosen Feststoffs.

| Elementaranalyse: | | | | | |
|---|---|---|---|---|---|
| ber. | C 35,04 | H 3,99 | N 7,30 | F 33,65 | S 3,34 |
| gef. | C 35,15 | H 4,13 | N 7,13 | F 33,48 | S 3,26 |

### d) 1,7-Bis-[1,4,7-tris(carboxylatomethyl)-1,4,7,10-tetraazacyclododecan-Gd-Komplex-10-(pentanoyl-3-aza-4-oxo-5-methyl-5yl)-4-[2-(N-ethyl-N-perfluoroctylsulfonyl)-amino]-acetyl-10-( 1-O-α-D - carbonylmethyl-mannopyranose )- 1,4,7,10- tetraazacyclododecan

20 g (20,84 mmol) der Titelverbindung aus Beispiel 7c, 5,09g (120 mmol) Litbiumchlorid und 37,78 g (60 mmol) 1,4,7-Tris ( carboxylatomethyl ) - 10-pentanoyl-3-aza-4-oxo-5-methyl-5yl )- 1,4,7,10- tetraazacyclododecan ,Gd-Komplex werden unter leichter Erwärmung in 400 ml Dimethylsulfoxid gelöst. Bei 10°C gibt man 29,67 g (120 mmol) EEDQ hinzu und rührt anschließend über Nacht bei Raumtemperatur.Man gießt die Lösung in 3000 ml Aceton und rührt 10 Minuten. Der ausgefallene Feststoff wird abfiltriert und anschließend durch Chromatographie gereinigt ( Kieselgel RP-18 , Laufmittel: Gradient aus Wasser / Ethanol / Acetonitril ).
Ausbeute: 13,2 g (29,0 % der Theorie ) eines farblosen Feststoffs.
Wassergehalt: 11,8%.

| Elementaranalyse ( berechnet auf wasserfreie Substanz ): | | | | | | |
|---|---|---|---|---|---|---|
| ber. | C 36,31 | H 4,34 | N 9,62 | S 1,47 | F 14,79 | Gd 14,41 |
| gef. | C 36,24 | H 4,27 | N 9,58 | S 1,51 | F 14,85 | Gd 14,25 |

### Beispiel 8

### a) 1,7-Bis (benzyloxycarbonyl)-4-[2-(N-Ethyl-N-perfluoroctylsulfonyl)-amino]-acetyl-10-[pentanoyl-3-aza-4-oxo-5-methyl-5yl-[1,4,7-tris (carboxylatomethyl)-Gd-Komplex. 1,4,7,10-tetraazacyclododecan-10-yl] -1,4,7,10-tetraazacyclododecan

50,0 g (49,61 mmol )der Titelverbindung aus Beispiel 7a) , 5,71 g ( 49,61 mmol) N-Hydroxysuccinimid, 4,24 g (100 mmol ) Lithiumchlorid und 31,24 g ( 49,61 mmol) 1,4,7-Tris(carboxylatomethyl)-10(pentanoyl-3-aza-4-oxo-5-methyl-5-yl)-1,4,7,10-tetraazacylododecan, Gd-Komplex werden unter leichter Erwärmung in 350 ml Dimethylsulfoxid gelöst. Bei 10°C gibt man 15,47 g (75 mmol ) N,N-Dicyclohexylcarbodiimid zu und rührt anschließend über Nacht bei Raumtemperatur. Man gießt die Lösung in 2000 ml Aceton und rührt 10 Minuten.Der ausgefallene Feststoff wird abfiltriert und anschließend durch Chromatographie gereinigt ( Kieselgel RP-18, Laufmittel : Gradient aus Wasser / Ethanol / Acetonitril ).
Ausbeute : 65,1 g (81,0 % der Theorie ) eines farblosen Feststoffs.
Wassergehalt : 7,9 %.

| Elementaranalyse ( berechnet auf wasserfreie Substanz ): | | | | | | |
|---|---|---|---|---|---|---|
| ber. | C 40,79 | H 4,11 | N 8,65 | S 1,98 | F 19,94 | Gd 9,72 |
| gef. | C 40,71 | H 4,20 | N 8,58 | S 2,03 | F 19,87 | Gd 9,68 |

### b) 1-[2-(N-Ethyl-N-perfluoroctylsulfonyl)-amino]- acetyl - 7{-pentanoyl-3-aza-4-oxo-5-methyl-5yl-[ tris ( carboxylatomethyl -1,4,7,10 - tetraazacyclododecan - 10-yl ] -Gd-Komplex }-1,4,7,10 tetraazacyclododecan

60,0 g ( 37,05 mmol ) der Titelverbindung aus Beispiel 8a) werden in 600 ml Ethanol gelöst und 6,0 g Palladium Katalysator (10% Pd / C) zugegeben. Man hydriert bei Raumtemperatur. Man filtriert vom Katalysator ab und dampft das Filtrat im Vakuum zur Trockene ein. Ausbeute: 50,06 g (quantitativ ) eines farblosen Feststoffs.
Wassergehalt : 3,9 %.

| Elementaranalyse ( berechnet auf wasserfreie Substanz ): | | | | | | |
|---|---|---|---|---|---|---|
| ber. | C 34,67 | H 4,03 | N 10,37 | S 2,37 | F 23,90 | Gd 11,64 |
| gef. | C 34,58 | H 4,15 | N 10,28 | S 2,30 | F 23,84 | Gd 11,57 |

### c) 1-[2-(N-Ethyl-N-perfluoroctylsulfonyl)-amino]-acetyl-4,10 - bis [1-O-α-D-carbonylmethyl- 2,3,4,6-tetra - O - benzyl - mannopyranose] -7-{pentanoyl-3-aza-4-oxo-5-methyl-5-yl-[1,4,7-tris(carboxylatomethyl)-1,4,7,10-tetraazacyclododecan-10-yl]-Gd-Komplex}-1,4,7,10- tetraazacyclododecan

40,0 g (29,60 m mol) der Titelverbindung aus Beispiel 8b) , 2,54 g (60,0 mmol) Lithiumchlorid und 44,9 g ( 75,0 mmol ) 1-O-α-D-Carboxymethyl-2,3,4,6-tetra-O-benzylmannopyranose werden unter leichter Erwärmung in 300 ml Dimethylsulfoxid gelöst. Bei 10°C gibt man 24,73 g ( 100,0 mmol) EEDQ hinzu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt ( Kieselgel RP-18, Laufmittel : Gradient aus Wasser / Ethanol / Acetonitril).
Ausbeute: 31,98 g (43,0 % der Theorie ) eines farblosen Feststoffs.
Wassergehalt : 3,5 %.

| Elementaranalyse ( berechnet auf wasserfreie Substanz): | | | | | | |
|---|---|---|---|---|---|---|
| ber. | C 53,06 | H 5,05 | N 5,57 | S 1,28 | F 12,85 | Gd 6,26 |
| gef. | C 52,95 | H 5,19 | N 5,48 S | 1,23 | F 12,77 | Gd 6,14 |

### d) 1-[2-(N-Ethyl-N-perfluoroctylsulfonyl)-amino]-acetyl-4,10 - bis [1-O-α-Dcarbonylmethyl- 2,3,4,6-tetra - O - benzyl - mannopyranose]-7-{pentanoyl-3-aza-4-oxo-5-methyl-5-yl-[1,4,7-tris(carboxylatomethyl)-1,4,7,10-tetraazacyclododecan-10-yl]-Gd-Komplex}-1,4,7,10- tetraazacyclododecan

30,0 g (11,94 mmol ) der Titelverbindung aus Beispiel 8c) werden in 300 ml Ethanol / 30 ml Wasser gelöst und 4,0 g Palladium Katalysator (10% Pd / C ) zugegeben. Man hydriert bei Raumtemperatur, filtriert vom Katalysator ab und dampft das Filtrat im Vakuum zur Trockene ein.
Ausbeute: 21,39 g ( quantitativ ) eines farblosen Feststoffs.
Wassergehalt: 3,4 %.

| Elementaranalyse ( berechnet auf wasserfreie Substanz ): | | | | | | |
|---|---|---|---|---|---|---|
| ber. | C 36,87 | H 4,39 | N 7,82 | S 1,79 | F 18,03 | Gd 8,78 |
| gef. | C 36,80 | H 4,50 | N 7,85 | S 1,68 | F 17,91 | Gd 8,70 |

### Beispiel 9

### a) 6-N-[3,6-Bis(carboxymethyl) -octan-1,8-dicarbonsäure- 1-carboxy-8-oyl]-2-N- (1-O-α-Dcarboxymethyl - mannopyranose )-lysin - [1-(4-perfluoroctylsulfonyl ) -piperazin ] - amid

Zu 27,5 g (30,0 mmol ) der Titelverbindung aus Beispiel 1e) , gelöst in 300 ml Dimethylformamid / 100ml Pyridin,gibt man 25,62 g (100,0 mmol ) Ethylendiamin-N, N, N', N' - tetraessigsäuredianhydrid und rührt 5 Stunden bei 50°C. Man dampft im Vakuum zur Trockene ein. Der Rückstand wird in 300 ml Wasser gelöst, durch Zugabe von 20% iger wässriger Natronlauge auf einen pH Wert von 10 gestellt und anschließend wird die basische Produktlösung durch Zugabe von. konz. Salzsäure auf einen pH Wert von 3 gebracht und im Vakuum zur Trockene eingedampft. Der Rückstand wird an Kieselgel RP-18 chromatographiert ( Laufmittel : Gradient aus Wasser / Ethanol / Acetonitril ).
Ausbeute : 18,22 g (51,0 % der Theorie ) eines farblosen Feststoffs.
Wassergehalt : 7,9 %.

| Elementaranalyse ( berechnet auf wasserfreie Substanz ): | | | | | |
|---|---|---|---|---|---|
| ber. | C 36,31 | H 3,98 | N 7,06 | F 27,12 | S 2,69 |
| gef. | C 36,23 | H 4,07 | N 6,98 | F 27,05 | S 2,62 |

### b) 6-N-[3,6-Bis (carboxylatomethyl )-octan-1,8-dicarbonsäure-1-carboxylato-8-oyl- Mn-Komplex, Natriumsalz] - 2-N-(1-O-α-D-carboxymethyl-mannopyranose)-L-lysin-[1-(4-perfluoroctylsulfonyl)-piperazin ] - amid

10 g ( 8,397 mmol ) der Titelverbindung aus Beispiel 9a) werden in 200 ml Wasser gelöst. Man gibt 965 mg ( 8,397 mmol) Mangan ( II ) carbonat zu und rührt 3 Stunden auf 60°C. Die Lösung wird mit 5% iger wässriger Natronlauge auf einen pH Wert von 7,4 gestellt, filtriert und anschließend gefriergetrocknet.
Ausbeute : 10,52 g ( 99,0 % der Theorie ) eines farblosen Feststoffs.
Wassergehalt : 7,8 %.

| Elementaranalyse ( berechnet auf wasserfreie Substanz ): | | | | | | | |
|---|---|---|---|---|---|---|---|
| ber. | C 34,16 | H 3,50 | N 6,64 | S 2,53 | F 25,52 | Mn 4,34 | Na 1,82 |
| gef. | C 34,06 | H 3,61 | N 6,58 | S 2,47 | F 25,47 | Mn 4,30 | Na 1,97 |

### Beispiel 10

### a) 1,2,3,4,6-Penta-O-acetyl-α,β-D-mannopyranose

Auf analoge Weise, wie in der Literatur beschrieben [M.L.Wolfrom und A.Thompson in Methods in Carbohydrate Chemistry (R.L. Whistler, M.L. Wolfrom and J.N. BeMiller, Eds.), Academic Press, New York, Vol.II, 53 , pp. 211-215, (1963)] liefert die Umsetzung von 150 g (832.5 mmol) α,β-D-Mannopyranose mit einem Gemisch aus 1500 ml absolutem Pyridin und 1500 ml Essigsäureanhydrid nach Aufarbeitung 315 g (96,7 %) der oben genannten Titelverbindung als Rohprodukt in Form eines viskosen und farblosen Öls. Durch ¹H-NMRspektroskopische Untersuchung der so erhaltenen Titelverbindung konnte das α zu β-Verhältnis beider Anomeren mit 4:1 bestimmt werden. Auf eine Trennung der α,β-Anomeren der oben genannten Titelverbindung kann zur Durchführung der nachfolgenden Reaktionsschritte verzichtet werden.

| Elementaranalyse: | | |
|---|---|---|
| ber. | C 49,21 | H 5,68 |
| gef. | C 49,12 | H 5,78 |

### b) 1-O-α-D-(5-Ethoxycarbonyl)-pentyl-2,3,4,6-tetra-O-acetyl-mannopyranose

Auf analoge Weise, wie in der Literatur für die Synthese von Aryl Glycopyranosiden beschrieben [J. Conchie und G.A. Levvy in Methods in Carbohydrate Chemistry (R.L. Whistler, M.L. Wolfrom and J.N. BeMiller, Eds.), Academic Press, New York, Vol.II , 90, pp. 345-347, ( 1963 )] führt die Umsetzung von 156,2 g (400 mmol) der Titelverbindung aus Beispiel 10a) als α, β-Anomerengemisch mit 67 ml ( 400 mmol ) 6-Hydroxy-hexansäureethylester und 60,8 ml (520 mmol) Zinn-IV-chlorid in insgesamt 600 ml 1,2-Dichlorethan nach säulenchromatographischer Aufreinigung (Eluent: Hexan/ Essigsäureethylester 2:1) zur Bildung von 100,05 g (51 % d. Th.) der oben genannten Titelverbindung als farbloses und viskoses Öl. Durch ¹H-NMR-spektroskopische Untersuchung der so erhaltenen Titelverbindung konnte gezeigt werden, daß es sich bei der oben genannten Titelverbindung ausschließlich um das reine α-Anomere handelt.

| Elementaranalyse: | | |
|---|---|---|
| ber. | C 52,94 | H 6,77 |
| gef. | C 52,80 | H 6,78 |

### c) 1-O-α-D-(5-Carboxy)-pentyl-2,3,4,6-tetra-O-benzyl-mannopyranose

Eine gerührte Suspension von 141,0 g ( 289 mmol ) der Titelverbindung aus Beispiel 10b) in 200 ml Dioxan wird bei Raumtemperatur und unter gleichzeitigem kräftigen Rühren portionsweise mit insgesamt 238,5 g ( 4,26mol ) fein gepulvertem Kaliumhydroxydpulver versetzt. Zur Erhöhung der Rührfähigkeit wird das Reaktionsgemisch mit weiteren 200 ml Dioxan versetzt und die so erhaltene Suspension im Anschluß zur Siedehitze erhitzt und bei dieser Temperatur mit insgesamt 372 ml ( 3,128 mol ) Benzylbromid über einen Zeitraum von zwei Stunden tropfenweise versetzt. Nach einer Reaktionszeit von 4 Stunden bei 110 °C gefolgt von 12 Stunden bei Raumtemperatur wird das Reaktionsgemisch zum Zwecke der Aufarbeitung in insgesamt 2,5 Liter Eiswasser langsam eingegossen und die Wasserphase im Anschluß vollständig mit Diethylether extrahiert. Nach dem Waschen der so erhaltenen Etherphase und dem anschließenden Trocknen der selbigen über Natriumsulfat wird vom Salz abgesaugt und der Diethylether im Vakuum abgezogen. Überschüssiges Benzylbromid wird anschließend im Ölpumpenvakuum quantitativ bei einer Ölbadtemperatur von 180 °C aus dem Reaktionsgemisch abdestilliert. Der so erhaltene, harzig-ölige Rückstand wird an Kieselgel unter Verwendung von Essigsäureethylester/Hexan (1:10) als Eluent gereinigt.
Ausbeute: 172,2, g (91,0 % d. Th.) der oben genannten Titelverbindung in Form eines farblosen und äußerst viskosen Öls.

| Elementaranalyse: | | |
|---|---|---|
| ber. | C 75,68 | H 7,16 |
| gef. | C 75,79 | H 7,04 |

### d) 6-N-Benzyloxycarbonyl-2-N-[1-0-α-D-(5-carbonyl)-pentyl-2,3,4,6-tetra-O-benzylmannopyranose]-L-lysin-[1-(4-perfluoroctylsulfonyl)-piperazin]-amid

100,0 g (134,0 mmol) der unter Beispiel 10e) hergestellten Carbonsäure und 32,4 g (281,4 mmol) N-Hydroxysuccinimid werden in 500 ml Dimethylformamid gelöst und bei 0°C mit insgesamt 58,0 g (281,4 mmol) N,N'-Dicyclohexylcarbodiimid portionsweise versetzt und es wird 3 Stunden bei dieser Temperatur nachgerührt. Zu der so hergestellten Aktivester-Lösung gibt man eine auf 0°C gekühlte Lösung von 111,3 g (134,0 mmol) der Titelverbindung aus Beispiel 1c) , gelöst in 300 ml Dimethylformamid tropfenweise hinzu und rührt 2 Stunden bei 0°C sowie 12 h bei Raumtemperatur. Zur Aufarbeitung filtriert man vom ausgefallenen Dicyclohexylharnstoff ab und zieht das Lösungsmittel anschließend bis zur Trockne ab. Der so erhaltene Rückstand wird anschließend an Kieselgel chromatographiert (Laufmittel: Dichlormethan / Ethanol 20:1 ; die Durchführung der Chromatographie erfolgt unter Verwendung eines Solvensgradienten mit kontinuirlicher Zunahme des Ethanolanteils).
Ausbeute: 132,5 g (67,4 % d.Th.) der Titelverbindung in Form eines farblosen und stark viskosen Öls.

| Elementaranalyse: | | | | | |
|---|---|---|---|---|---|
| ber. | C 54.02 | H 4.88 | N 3.82 | F 22.01 | S 2.19 |
| gef. | C 53.87 | H 4.85 | N 4.02 | F 22.55 | S 2.06 |

### e) 2-N-[1-0-α-D-(5-Carbonyl)pentyl-mannopyranose]-L-lysin-[1-(4-perfluoroctylsulfonyl)-piperazin]-amid

In 800 ml Ethanol werden 120,0 g (81,77 mmol) der unter 10d) hergestellten Verbindung gelöst, mit 4,5 g Pearlman-Katalysator (Pd 20 %, C) versetzt und solange bei Raumtemperatur unter einer Wasserstoffatmosphäre (1 atm) hydriert, bis keine Wasserstoffaufnahme mehr zu beobachten ist (ca. 8 Stunden). Man saugt vom Katalysator ab, wäscht gründlich mit Ethanol nach (ca. 200 ml) und engt im Vakuum zur Trockne ein. Die Titelverbindung wird als stark viskoses und farbloses Öl erhalten.
Ausbeute: 78,5 g (98,7 % d. Th.).

| Elementaranalyse: | | | | | |
|---|---|---|---|---|---|
| ber. | C 37.04 | H 4.25 | N 5.76 | F 33.20 | S 3.30 |
| gef. | C 36.96 | H 4.85 | N 5.41 | F 34.13 | S 3.22 |

### f) 2-N-[1-O-α-D-(5-Carbonyl)pentyl-mannopyranose]-6-N-[1,4,7-tris-(carboxylatomethyl)-10-(-3-aza-4-oxo-5-methyl-5-yl-pentanoyl)-1,4,7,10-tetraazacyclododecan]-L-lysin-[1-(4-perfluoroctylsulfonyl)-piperazin]-amid, Gd-Komplex

99,8 g (158,4 mmol; 2.2 Molequivalente bezogen auf die eingesetzte Aminkomponente aus Beispiel 10e) des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 6,7 g wasserfreies Lithiumchlorid (158,4 mmol) werden bei 40 C° in 800 ml absolutem Dimethylsulfoxid unter Rühren gelöst. Bei dieser Temperatur wird im Anschluss mit insgesamt 18,2 g (158,4 mmol) N-Hydroxysuccinimid und 70,0 g (71,96 mmol) der Titelverbindung aus Beispiel 10e), gelöst in 250 ml absolutem Dimethylsulfoxid, versetzt. Nach dem Abkühlen auf Raumtemperatur wird die Reaktionslösung mit 32,7 g ( 158,4 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off : 3000 Da) entsalzt und von niedermolekularen Bestandteilen gereinigt. Das Retentat wird anschließend gefriergetrocknet.
Ausbeute: 93,0 g (81,6 % d. Th.) als farbloses Lyophilisat.
H₂O-Gehalt (Karl-Fischer): 9,53 %.

| Elementaranalyse (berechnet auf wasserfreie Substanz): | | | | | | |
|---|---|---|---|---|---|---|
| ber. | C 37.15 | H 4.39 | N 7.96 | F 20.38 | S 2.02 | Gd 9.92 |
| gef. | C 36.92 | H 4.50 | N 7.68 | F 19.77 | S 1.91 | Gd 10.08 |

### Beispiel 11

### a) 2-N-[1-O-α-D-(5-Carbonyl)pentyl-mannopyranose]-6-N-{2-[4-(3-oxapropionyl)-phenyl]-2-[1,4,7-tris-(carboxylatomethyl)-1,4,7,10-tetraazacyclododecan-10-yl]-essigsäure}-L-lysin-[1-(4-perfluoroctylsulfonyl)-piperazin]-amid, Gd-Komplex, Natiumsalz

Eine gerührte Suspension von 5,0 g (9.06 mmol) der Titelverbindung aus Beispiel 3e) in 15 ml absolutem Dimethylsulfoxid wird bei 70°C mit 0,68 g (15,9 mmol) Lithiumchlorid versetzt. Nach 30 minütigem Rühren bei 70°C wird die nun klare Reaktionslösung portionsweise mit insgesamt 1,83 g (15,9 mmol) N-Hydroxysuccinimid versetzt und das Reaktionsgemisch noch 1 Stunde bei dieser Temperatur gehalten. Nach dem Abkühlen auf 0°C wird mit 4,52 g (23,85 mmol) Dicyclohexylcarbodiimid versetzt und die Reaktionslösung noch 1 weitere Stunde bei 0°C, gefolgt von 12 Stunden bei 22°C, gerührt. Die so erhaltene Reaktionslösung des N-Hydroxysuccinimidesters der Titelverbindung aus Beispiel 3e) wird nun bei 22°C tropfenweise mit einer Lösung von 4,0 g (4,12 mmol) der Titelverbindung aus Beispiel 10e) in 15 ml absolutem Dimethylsulfoxid versetzt und weitere 12 Stunden bei Raumtemperatur nachgerührt. Zur Aufarbeitung wird die Reaktionslösung bei 22°C in 900 ml Aceton eingetropft, wobei die Titelverbindung als farbloser Niederschlag ausfällt. Der Niederschlag wird abgesaugt, in 200 ml destilliertem Wasser gelöst und anschließend mit 1 molarer Natronlauge der pH-Wert dieser Lösung auf genau 7,2 eingestellt. Die so erhaltene wässrige Produktlösung wird über eine YM3-Ultrafiltrationsmembran (AMICON ® :cut off : 3000 Da) zum Zwecke des Entsalzens und der Abtrennung von niedermolekularen Bestandteilen dreimal ultrafiltriert. Das so erhaltene Retentat wird anschließend gefriergetrocknet.
Ausbeute: 6,33g (92,4 % d. Th., bezogen auf die eingesetzte Aminkomponente) als farbloses Lyophilisat mit einem Wassergehalt von 7,38 %.

| Elementaranalyse (berechnet auf wasserfreie Substanz): | | | | | | | |
|---|---|---|---|---|---|---|---|
| ber. | C 38.48 | H 4.13 | N 6.65 | F 19.16 | S 1.90 | Gd 9.33 | Na 1.36 |
| gef. | C 39.52 | H 4.12 | N 6.67 | F 19.70 | S 1.89 | Gd 9.30 | Na 1.41 |

### Beispiel 12

### a) 3,5-Bis-benzyloxycarbonylamino-benzoesäure-N-(3-oxa-1H,1H,2H,2H,4H,4H, 5H,5H-perfluortridecyl)-amid

In einem Lösungsmittelgemisch aus 125 ml trockenem Tetrahydrofuran und 125 ml trockenem Dioxan werden 20g (47,5 mmol) 3,5-Bis-benzyloxycarbonylamino-benzoesäure (Synthese gemäß nachfolgender Literaturstelle: Skulnick, Harvey I.; Johnson, Paul D.; Aristoff, Paul A.; Morris, Jeanette K.; Lovasz, Kristine D.; et al.; J.Med.Chem.; 40; 7; 1997; 1149-1164) sowie 4,78 g (47,5 mmol) Triethylamin gelöst. Nach dem Abkühlen auf -15°C tropft man unter Rühren eine Lösung von 6,56g (48 mmol) Chlorameisensäureisobutylester in 30 ml trockenem Tetrahydrofuran langsam hinzu, wobei die Innentemperatur unterhalb von -10°C zu halten ist. Nach einer Reaktionszeit von 15 Minuten bei -15°C tropft man eine Lösung von 58,6 g (47,5 mmol) 1-Amino-1H,1H,2H,2H,4H,4H,5H,5H-3-oxa-perfluortride-can und 4,78 g (47,5 mmol) Triethylamin in 100ml trockenem Tetrahydrofuran bei -20°C hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 300 ml Essigsäureethylester aufgenommen und zweimal mit je 200 ml gesättigter Natriumhydrogencarbonatlösung sowie einmal mit 300 ml Wasser gewaschen. Nach dem Trocknen der organischen Phase über Natriumsulfat wird vom Salz abgesaugt und der Essigsäureethylester im Vakuum abgezogen. Der verbleibende ölige -Rückstand wird an Kieselgel unter Verwendung von Dichlormethan/Hexan/2-Propanol (10:5:1) als Eluent gereinigt.
Ausbeute: 36,2 g (82,5% d. Th.) der Titelverbindung als farbloses Öl..

| Elementaranalyse: | | | | |
|---|---|---|---|---|
| ber. | C 46,82 | H 3,27 | N 4,55 | F 34,97 |
| gef. | C 47,21 | H 3,31 | N 4,61 | F 34,48 |

### b) 3,5-Di-anvno-benzoesäure-N-(3-oxa-1H,1H,2H,2H,4H,4H, 5H,5H-perfluortridecyl)]-amid

In 300 ml Ethanol werden 30,0 g ( 32,4 mmol) des unter 12a) hergestellten Amids gelöst und mit 1,2 g Pearlman-Katalysator (Pd 20 %, C) versetzt. Man hydriert solange bei Raumtemperatur unter einer Wasserstoffatmosphäre (1 atm), bis keine Wasserstoffaufnahme mehr zu beobachten ist. Man saugt vom Katalysator ab, wäscht gründlich mit Ethanol nach (ca. 300 ml) und engt im Vakuum zur Trockne ein. Die Titelverbindung wird als stark viskoses , gelbliches Öl erhalten.
Ausbeute: 20,12 g (94,8 % d. Th.).

| Elementaranalyse: | | | | |
|---|---|---|---|---|
| ber. | C 36,66 | H 2,77 | N 6,41 | F 49,28 |
| gef. | C 36,07 | H 2,87 | N 6,23 | F 49,43 |

### c) 3-N-[-(1-O-α-D-Carbonylmethyl-2,3,4,6-tetra-O-benzyl-mannopyranose)-5-aminobenzoesäure-N-(3-oxa-1H,1H,2H,2H,4H,4H, 5H,5H-perfluortridecyl)-amid

10,95 g (18,30 mmol) 1-Carboxymethyloxy-2,3,4,-tetra-O-benzyl-α-Dmannopyranosid [ Darstellung wie in der Patentschrift DE 197 28 954 C1 beschrieben] werden in 150 ml Dimethylformamid gelöst und mit insgesamt 2,09 g (18,3 mmol) N-Hydroxysuccinimid versetzt.. Man kühlt auf 0°C ab und gibt 3,78 g (18,3 mmol) Dicyclohexylcarbodiimid zu. Es wird eine Stunde bei 0°C und anschließend 4 Stunden bei Raumtemperatur gerührt. Man kühlt auf 0°C ab und tropft innerhalb von 3 h eine Lösung aus 24,0 g (36,6 mmol, 2 Molequivalente bezogen auf eingesetzte Carbonsäure) der unter Beispiel 12b) beschriebenen Diaminoverbindung, gelöst in 350 ml Dimethylformamid langsam hinzu. Anschließend rührt man noch eine Stunde bei 0°C, dann über Nacht bei Raumtemperatur. Es wird im Vakuum zur Trockne eingedampft und der Rückstand in 300 ml Essigsäureethylester aufgenommen. Man filtriert vom ausgefallenem Harnstoff ab und wäscht das Filtrat 2 mal mit je 100 ml 5 %iger wässriger Soda-Lösung. Die organische Phase wird über Magnesiumsulfat getrocknet und im Vakuum zur Trockne eingedampft. Der Rückstand wird an Kieselgel chromatographiert (Laufmittel: n-Hexan/Isopropanol 13:1). Man erhält 16,8 g (74,3 % d. Th., bezogen auf eingesetzte Carbonsäure) der Titelverbindung in Form eines farblosen Öls. Durch Erhöhung der Polarität der Eluentzusammensetzung auf n-Hexan/Isopropanol 5:1 werden in den nachfolgenden Chromatographiefraktionen insgesamt 10,15 g an nicht umgesetzter Diaminoverbindung 12b) wiedergewonnen, welche erneut nach oben genannter Reaktionsvorschrift umgesetzt werden können.

| Elementaranalyse: | | | | |
|---|---|---|---|---|
| ber. | C 54,42 | H 4,40 | N 3,40 | F 26,13 |
| gef. | C 54,32 | H 4,49 | N 3,48 | F 25,94 |

### d) 3-N-[-(1-O-α-D-Carbonylmethyl-mannopyranose)]-5-amino-benzoesäure-N-(3-oxa-1H,1H,2H,2H,4H,4H, 5H,5H-perfluortridecyl)-amid

In analoger Weise wie für die Synthese der Titelverbindung aus Beispiel 12b) beschrieben, liefert die Hydrogenolyse von 12,0 g (9,70 mmol) der Titelverbindung aus Beispiel 12c), unter Verwendung von 0,5 g Pearlman-Katalysator (Pd 20 %, C) in einem Ethanol/Wasser (9:1) -Gemisch nach Aufarbeitung 8,08 g (96,7 % d. Th.) der oben genannten Titelverbindung in Form eines gelblich gefärbten und viskosen Öls.

| Elementaranalyse: | | | | |
|---|---|---|---|---|
| ber. | C 37,64 | H 3,28 | N 4,88 | F 37,49 |
| gef. | C 37,32 | H 3,17 | N 4,97 | F 37,55 |

### e) 3-N-(1-O-α-D-Carbonylmethyl-mannopyranose)-5-N-{2-[4-(3-oxapropionyl )-phenyl]-2-[1,4,7-tris-(carboxylatomethyl)-1,4,7,10-tetraazacyclododecan-10-yl]-essigsäure}benzoesäure-N-(3-oxa-1H,1H,2H,2H,4H,4H, 5H,5H-perfluortridecyl)-amid,Gd-Komplex, Natiumsalz

13,6 g (19,2 mmol; 2.2 Molequivalente bezogen auf die eingesetzte Aminkomponente aus Beispiel 12d) ) des unter Beispiel 3e) beschriebenen Gd-Komplexes und 0,81 g wasserfreies Lithiumchlorid (19,2 mmol) werden bei 40 C° in 100 ml absolutem Dimethylsulfoxid unter Rühren gelöst und bei dieser Temperatur mit insgesamt 2,2 g (19,2 mmol) N-Hydroxysuccinimid und 7,5g (8,7 mmol) der Titelverbindung aus Beispiel 12d), gelöst in 50 ml absolutem Dimethylsulfoxid, versetzt. Nach dem Abkühlen auf Raumtemperatur wird die Reaktionslösung mit 3,96 g ( 19,2 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 11,51 g (84,5 % d. Th.) als farbloses Lyophilisat.
H₂O-Gehalt (Karl-Fischer): 6,77 %.

| Elementaranalyse (berechnet auf wasserfreie Substanz): | | | | | | |
|---|---|---|---|---|---|---|
| ber. | C 40,05 | H 3,94 | N 6,29 | F 20,71 | Gd 10,08 | Na 1,47 |
| gef. | C 39.98 | H 4.00 | N 6,31 | F 20,73 | Gd 10.11 | Na 1,42 |

### Beispiel 13

### a) 3,5-Bis-(benzyloxycarbonylamino)-1-{N-[1-(4-perfluoroctylsulfonyl)-piperazin]}-benzamid

In einem Lösungsmittelgemisch aus 60 ml trockenem Tetrahydrofuran und 70 ml trockenem Dioxan werden 10g (23,75 mmol) 3,5-Bis-benzyloxycarbonylamino-benzoesäure (Synthese gemäß nachfolgender Literaturstelle: Skulnick, Harvey I.; Johnson, Paul D.; Aristoff, Paul A.; Morris, Jeanette K.; Lovasz, Kristine D.; et al.; J.Med.Chem.; 40; 7; 1997; 1149-1164 ) sowie 2,39 g (23,75 mmol) Triethylamin gelöst. Nach dem Abkühlen auf -15°C tropft man unter Rühren eine Lösung von 3,28g (24 mmol) Chlorameisensäureisobutylester in 20 ml trockenem Tetrahydrofuran langsam hinzu, wobei die Innentemperatur -10°C nicht überschreitet. Nach einer Reaktionszeit von 15 Minuten bei -15°C tropft man eine Lösung von 23,0 g (23,75 mmol) Perfluoroctylsulfonylpiperazin und 2,39 g (23,75 mmol) Triethylamin in 50 ml trockenem Tetrahydrofuran bei -20°C hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 200 ml Essigsäureethylester aufgenommen und zweimal mit je 100 ml gesättigter Natriumhydrogencarbonatlösung sowie einmal mit 300 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Hexan/2-Propanol (15:5:1) als Eluent gereinigt.
Ausbeute: 18,35 g (79,6 % d. Th.) der Titelverbindung als farbloses Öl..

| Elementaranalyse: | | | | | |
|---|---|---|---|---|---|
| ber. | C 43,31 | H 2,80 | N 5,77 | F 33,27 | S 3,30 |
| gef. | C 43,21 | H 2,75 | N 5,61 | F 33,38 | S 3,22 |

### b) 3,5-Di-amino-1-{N-[1-(4-perfluoroctylsulfonyl)-piperazin]}-benzamid

In 100 ml Ethanol werden 9,70 g ( 10,0 mmol) des unter 13a) hergestellten Amids gelöst und mit 0,4 g Pearlman-Katalysator (Pd 20 %, C) versetzt. Man hydriert solange bei Raumtemperatur unter einer Wasserstoffatmosphäre (1 atm), bis keine Wasserstoffaufnahme mehr zu beobachten ist. Man saugt vom Katalysator ab, wäscht gründlich mit Ethanol nach (ca. 150 ml) und engt im Vakuum zur Trockne ein. Die Titelverbindung wird als stark viskoses , gelbliches Öl erhalten.
Ausbeute: 6,9 g (98,2 % d. Th.).

| Elementaranalyse: | | | | | |
|---|---|---|---|---|---|
| ber. | C 32,49 | H 2,15 | N 7,98 | F 45,98 | S 4,56 |
| gef. | C 32,56 | H 2,17 | N 8,09 | F 45,63 | S 4,61 |

### c) 5-Amino-3-N-(1-0-α-D-Carbonylmethyl-2,3,4,6-tetra-O-benzyl-mannopyranose)-benzoesäure-N-[1-(4-pertluoroctylsulfonyl)- piperazin]-amid

5,48 g (9,15 mmol) 1-Carboxymethyloxy-2,3,4,-tetra-O-benzyl-α-D-mannopyranosid [Darstellung wie in der Patentschrift DE 197 28 954 C1 beschrieben] werden in 100 ml Dimethylformamid gelöst und mit insgesamt 1,04 g (9,15 mmol) N-Hydroxysuccinimid versetzt.. Man kühlt auf 0°C ab und gibt 1,89 g (9,15 mmol) Dicyclohexylcarbodiimid zu. Es wird eine Stunde bei 0°C und anschließend 4 Stunden bei Raumtemperatur gerührt. Nach dem erneuten Abkühlen auf 0°C tropft man innerhalb von 3 h eine Lösung aus 12,85 g (18,30 mmol, 2 molequivalente bezogen auf eingesetzte Carbonsäure) der unter Beispiel 13b) beschriebenen Diaminoverbindung, gelöst in 250 ml Dimethylformamid langsam hinzu. Anschließend rührt man noch eine Stunde bei 0°C, dann über Nacht bei Raumtemperatur. Es wird im Vakuum zur Trockne eingedampft und der Rückstand in 100 ml Essigsäureethylester aufgenommen. Man filtriert vom ausgefallenem Harnstoff ab und wäscht das Filtrat zweimal mit je 100 ml 5 %iger wässriger Soda-Lösung. Die organische Phase wird über Magnesiumsulfat getrocknet und im Vakuum zur Trockne eingedampft. Der Rückstand wird an Kieselgel chromatographiert (Laufmittel: n-Hexan/Isopropanol 13:1). Man erhält 8,14 g (69,4 % d. Th., bezogen auf eingesetzte Carbonsäure) der Titelverbindung in Form eines farblosen Öls. Durch Erhöhung der Polarität der Eluentzusammensetzung während der Chromatographie auf 6:1 (n-Hexan/Isopropanol) werden in den nachfolgenden Chromatographiefraktionen noch insgesamt 4,36 g an nicht umgesetzter Diaminoverbindung 13b) wiedergewonnen, welche erneut nach oben genannter Reaktionsvorschrift umgesetzt werden können.

| Elementaranalyse: | | | | | |
|---|---|---|---|---|---|
| ber. | C 51,49 | H 4,01 | N 4,37 | F 25,17 | S 2,50 |
| gef. | C 51,60 | H 4,19 | N 4,28 | F 25,14 | S 2,44 |

### d) 5-Amino-3-N-(1-0-α-D-Carbonylmethyl-mannopyranose)-benzoesäure-N-[1-(4-perfluoroctylsulfonyl)-piperazin]-amid

In analoger Weise wie für die Synthese der Titelverbindung aus Beispiel 13b) beschrieben, liefert die Hydrogenolyse von 6,4 g (5,0 mmol) der Titelverbindung aus Beispiel 13c), unter Verwendung von 0,3 g Pearlman-Katalysator (Pd 20 %, C) in einem Ethanol/Wasser (8:1)-Gemisch nach Aufarbeitung 4,43 g (96,2 % d. Th.) der oben genannten Titelverbindung in Form eines gelblich gefärbten und viskosen Öls.

| Elementaranalyse: | | | | | |
|---|---|---|---|---|---|
| ber. | C 35,15 | H 2,95 | N 6,07 | F 35,01 | S 3,48 |
| gef. | C 35,32 | H 3,02 | N 5,89 | F 35,05 | S 3,58 |

### e) 3-N-(1-0-α-D-Carbonylmethyl-mannopyranose)-5-N-[1,4,7-tris(carboxylatomethyl)-10-(3-aza-4-oxo-5-methyl-5-yl-pentanoyl)-1,4,7,10-tetraazacyclododecan]-benzoesäure-N-[1-(4-perfluoroctylsulfonyl)- piperazin]-amid, Gd-Komplex

5,54 g (8,8 mmol; 2.2 Molequivalente bezogen auf die eingesetzte Aminkomponente aus Beispiel 13d) ) des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,37 g wasserfreies Lithiumchlorid (8,8 mmol) werden bei 40 C° in 60 ml absolutem Dimethylsulfoxid unter Rühren gelöst und bei dieser Temperatur mit insgesamt 1,01 g (8.8 mmol) N-Hydroxysuccinimid und 3,7 g (4,0 mmol) der Titelverbindung aus Beispiel 13d), gelöst in 40 ml absolutem Dimethylsulfoxid, versetzt. Nach dem Abkühlen auf Raumtemperatur wird die Reaktionslösung mit 1,82 g ( 8,8 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 5,36 g (87,4 % d. Th.) als farbloses Lyophilisat.
H₂O-Gehalt (Karl-Fischer): 6,77 %.

| Elementaranalyse (berechnet auf wasserfreie Substanz): | | | | | | |
|---|---|---|---|---|---|---|
| ber. | C 36,01 | H 3,61 | N 8,22 | F 21,05 | Gd 10,25 | S 2,09 |
| gef. | C 35,87 | H 3,70 | N 8,22 | F 20,91 | Gd 10.18 | S 2,16 |

### Beispiel 14

### a) 1,4,7-Triazaheptan-1,7-Bis-(2-N-trifluoracetyl-6-N- benzyloxycarbonyl-L-lysin)-diamid

100 g (107,9 mmol) der unter Beispiel 1a) hergestellten Carbonsäure und 26,1 g (226,59 mmol) N-Hydroxysuccinimid werden in 500 ml Dimethylformamid gelöst und bei 0°C mit insgesamt 46,7 g (226,59 mmol) N,N'-Dicyclohexylcarbodiimid portionsweise versetzt und es wird 3 Stunden bei dieser Temperatur nachgerührt. Zu der so hergestellten Aktivester-Lösung gibt man eine auf 0°C gekühlte Lösung von 5,57 g (53,95 mmol) Diethylentriamin , gelöst in 60 ml Dimethylformamid tropfenweise hinzu und rührt 2 Stunden bei 0°C sowie 12 h bei Raumtemperatur. Zur Aufarbeitung filtriert man vom ausgefallenen Dicyclohexylharnstoff ab und zieht das Lösungsmittel anschließend bis zur Trockne ab. Der so erhaltene Rückstand wird anschließend an Kieselgel chromatographiert (Laufmittel: Dichlormethan / Ethanol 15:1 ; die Durchführung der Chromatographie erfolgte unter Verwendung eines Solvensgradienten mit kontinuierlicher Zunahme des Ethanolanteils). Ausbeute: 26,0 g ( 58,8 % d.Th., bezogen auf die eingesetzte Aminkomponente) der Titelverbindung in Form eines farblosen und stark viskosen Öls.

| Elementaranalyse: | | | | |
|---|---|---|---|---|
| ber. | C 52,74 | H 5,78 | N 11,96 | F 13,90 |
| gef. | C 52,66 | H 5,89 | N 11,88 | F 14,02 |

### b) 1,4,7-Triazaheptan-1,7-Bis-(2-N-trifluoracetyl-6-N-benzyloxycarbonyl-L-lysin)-diamid-4-[2-(N-ethyl-N-perfluoroctylsulfonyl)-amino]-acetyl

In eine Lösung aus 20 g (24,4 mmol) des unter 14a) hergestellten Diamids, gelöst in einem Gemisch aus 150 ml Tetrahydrofuran und 15 ml Chloroform, werden bei 0°C und unter Stickstoffatmosphäre 16,18 g (27,0 mmol) 2-[N-Ethyl-N-perfluoroctylsulfonyl)-aminoessigsäure (Darstellung gemäß: DE 196 03 033 ), gelöst in 50 ml Tetrahydrofuran, hinzugegeben. Anschließend gibt man bei 0°C insgesamt 18,0 g (36,6 mmol) EEDQ [2-Ethoxy-1-ethoxycarbonyl-1,2-dihydrochinolin] portionsweise hinzu und läßt über Nacht bei Raumtemperatur rühren und engt anschließend im Vakuum ein. Das verbleibende Öl wird wird an Kieselgel chromatographiert (Laufmittel: n-Hexan/Isopropanol 15:1). Man erhält 24,74 g (72,4 % d. Th., bezogen auf eingesetztes sec.- Amin) der Titelverbindung in Form eines farblosen Öls.

| Elementaranalyse: | | | | | |
|---|---|---|---|---|---|
| ber. | C 42,01 | H 3,96 | F 31,19 | N 8,00 | S 2,29 |
| gef. | C 41,92 | H 4,07 | F 31,22 | N 7,87 | S 2,34 |

### c) 1,7-Bis-(6-N- benzyloxycarbonyl-L-lysin)-diamid-4-[2-(N-ethyl-N-perfluoroctylsulfonyl)-amino]-acetyl-1,4,7-triazaheptan

In 100 ml Ethanol werden 22,0 g (15,7 mmol) der unter Beispiel 14b) hergestellten Titelverbindung gelöst und man leitet bei 0°C in diese Lösung für 40 Minuten Ammoniakgas ein. Anschließend wird weitere 4 Stunden bei 0°C gerührt sowie 3 Stunden bei Raumtemperatur und engt bei 40°C Badtemperatur im Vakuum zur Trockne ein. Der verbleibende ölige Rückstand wird an Kieselgel unter Verwendung von Dichlormethan/Hexan/2-Propanol (20:10:1) als Eluent gereinigt.
Ausbeute: 12.92 g (98,4 % d. Th.) der oben genannten Titelverbindung als farbloses und stark viskoses Öl.

| Elementaranalyse: | | | | | |
|---|---|---|---|---|---|
| ber. | C 44,22 | H 4,64 | N 9,38 | S 2,68 | F 27,03 |
| gef. | C 44,31 | H 4,72 | N 9,30 | S 2,74 | F 26,99 |

### d) 1,7-Bis-[6-N- benzyloxycarbonyl-2-N-(1-O-α-D-carbonylmethyl-2,3,4,6-tetra-O-benzylmannopyranose)-L-lysin]-diamid-4-[2-(N-ethyl-N-perfluoroctylsulfonyl)-amino]-acetyl-1,4,7-triazaheptan

5,47 g (9,15 mmol) 1-Carboxymethyloxy-2,3,4,-tetra-O-benzyl-α-D-mannopyranosid [ Darstellung wie in der Patentschrift DE 197 28 954 C1 beschrieben] werden in 80 ml Dimethylformamid gelöst und mit insgesamt 1,05 g (9,15 mmol) N-Hydroxysuccinimid versetzt.. Man kühlt auf 0°C ab und gibt 1,9 g (9,15 mmol) Dicyclohexylcarbodiimid zu. Es wird eine Stunde bei 0°C und anschließend 4 Stunden bei Raumtemperatur gerührt. Man kühlt auf 0°C ab und tropft innerhalb von 3 h eine Lösung aus 7,65g (9,15 mmol) der unter Beispiel 14c) beschriebenen Aminoverbindung, gelöst in 50 ml Dimethylformamid langsam hinzu. Anschließend rührt man noch eine Stunde bei 0°C, dann über Nacht bei Raumtemperatur. Es wird im Vakuum zur Trockne eingedampft und der Rückstand in 100 ml Essigsäureethylester aufgenommen. Man filtriert vom ausgefallenem Harnstoff ab und wäscht das Filtrat zweimal mit je 50 ml 5 %iger wässriger Soda-Lösung. Die organische Phase wird über Magnesiumsulfat getrocknet und im Vakuum zur Trockne eingedampft. Der Rückstand wird an Kieselgel chromatographiert (Laufinittel: n-Hexan/Isopropanol 20:1). Man erhält 17,01 g (78,9 % d. Th., bezogen auf eingesetzte Carbonsäure) der Titelverbindung in Form eines farblosen Öls.

| Elementaranalyse: | | | | | |
|---|---|---|---|---|---|
| ber. | C 59,13 | H 5,43 | N 4,76 | F 13,71 | S 1,36 |
| gef. | C 59,22 | H 5,39 | N 4,85 | F 13,70 | S 1,40 |

### e) 1,7-Bis-[2-N-(1-O-α-D-carbonylmethyl-mannopyranose)-L-lysin]-diamid-4-[2-(N-ethyl-N-periluoroctylsulfonyl)-amino]-acetyl-1,4,7-triazaheptan

In 150 ml Ethanol werden 15,0 g ( 6,36 mmol) des unter 14d) hergestellten Amids gelöst und mit 0,5 g Pearlman-Katalysator (Pd 20 %, C) versetzt. Man hydriert solange bei Raumtemperatur unter einer Wasserstoffatmosphäre (1 atm), bis keine Wasserstoffaufnahme mehr zu beobachten ist. Man saugt vom Katalysator ab, wäscht gründlich mit Ethanol nach (ca. 100 ml) und engt im Vakuum zur Trockne ein. Die Titelverbindung wird als stark viskoses , gelbliches Öl erhalten.
Ausbeute: 8,54 g (97,2% d. Th.).

| Elementaranalyse: | | | | | |
|---|---|---|---|---|---|
| ber. | C 39,13 | H 5,04 | N 8,11 | F 23,38 | S 2,32 |
| gef. | C 39,07 | H 4,98 | N 8,18 | F 23,40 | S 2,30 |

### f) 1,7-Bis-[2-N-(1-O-α-D-carbonylmethyl-mannopyranose)-6-N-[1,4,7-tris(carboxylatomethyl)-10-(3-aza-4-oxo-5-methyl-5-yl-pentanoyl)-1,4,7,10-tetraazacyclododecan]-L-lysin]-diamid-4-[2-(N-ethyl-N-perfluoroctylsulfonyl)-amino]-acetyl-1,4,7-triazaheptan , Digadolinium-Komplex

Eine gerührte Suspension von 5,7 g (9.06 mmol) des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure in 75 ml absolutem Dimethylsulfoxid wird bei 70°C mit 0,68 g (15,9 mmol) Lithiumchlorid versetzt. Nach 30 minütigem Rühren bei 70°C wird die nun klare Reaktionslösung portionsweise mit insgesamt 1,83 g (15,9 mmol) N-Hydroxysuccinimid versetzt und das Reaktionsgemisch noch 1 Stunde bei dieser Temperatur gehalten. Nach dem Abkühlen auf 0°C wird mit 4,52 g (23,85 mmol) Dicyciohexylcarbodiimid versetzt und die Reaktionslösung noch 1 weitere Stunde bei 0°C, gefolgt von 12 Stunden bei 22°C, gerührt. Die so erhaltene Reaktionslösung des N-Hydroxysuccinimidesters des Gd-Komplexes der 10-(4-Carboxy-1-methyl-2-oxo-3-azabutyl)-1,4,7,10-tetraazacyclododecan-1,4,7-triessigsäure wird nun bei 22°C tropfenweise mit einer Lösung von 2,84 g (2,06 mmol) der Titelverbindung aus Beispiel 14e) in 15 ml absolutem Dimethylsulfoxid versetzt und weitere 12 Stunden bei Raumtemperatur nachgerührt. Zur Aufarbeitung wird die Reaktionslösung bei 22°C in 500 ml Aceton eingetropft, wobei die Titelverbindung als farbloser Niederschlag ausfällt. Der Niederschlag wird abgesaugt, in 200 ml destilliertem Wasser gelöst und über eine YM3-Ultrafiltrationsmembran (AMICON ® : cut off : 3000 Da) zum Zwecke des Entsalzens und der Abtrennung von niedermolekularen Bestandteilen dreimal ultrafiltriert. Das so erhaltene Retentat wird anschließend gefriergetrocknet.
Ausbeute: 4,80g (89,6 % d. Th., bezogen auf die eingesetzte Aminkomponente) als farbloses Lyophilisat mit einem Wassergehalt von 8,98 %.

| Elementaranalyse (berechnet auf wasserfreie Substanz): | | | | | | |
|---|---|---|---|---|---|---|
| ber. | C 38.28 | H 4.84 | N 9,68 | F 12,40 | S 1.23 | Gd 12,07 |
| gef. | C 38,20 | H 4.91 | N 9,77 | F 12,45 | S 1.19 | Gd 12,10 |

### Beispiel 15

### a) 1,7-Bis(benzyloxycarbonyl)-4-{3-oxa-pentan-1,5-dicarbonsäure-1-oyl-5-[1-(4-perfluoroctylsulfonyl)-piperazin]-amid}-1,4,7,10-tetraazacyclododecan

In eine Lösung von 10,75 g (24,4 mmol) 1,7-Bis-[benzyloxycarbonyl]-1,4,7,10-tetraazacyclododecan, gelöst in einem Gemisch aus 150 ml Tetrahydrofuran und 15 ml Chloroform, werden bei 0°C und unter Stickstoffatmosphäre 16,56 g (24,4 mmol) der Titelverbindung aus Beispiel 15e) , gelöst in 150 ml Tetrahydrofuran hinzugegeben. Anschließend gibt man bei 0°C insgesamt 18,0 g (36,6 mmol) EEDQ [2-Ethoxy-1-ethoxycarbonyl-1,2-dihydrochinolin] portionsweise hinzu und läßt über Nacht bei Raumtemperatur rühren und engt anschließend im Vakuum ein. Das verbleibende Öl wird wird an Kieselgel chromatographiert (Laufmittel: n-Hexan/Isopropanol 12:1). Man erhält 17,22 g (64,3 % d. Th., bezogen auf eingesetztes sec.- Amin) des Monoamids sowie 3,8 g (8,8 % d. Th.) des Diamids als Nebenprodukt. Die Titelverbindung wird in Form eines farblosen Öls isoliert.

| Elementaranalyse: | | | | | |
|---|---|---|---|---|---|
| ber. | C43,41 | H 3,92 | F 29,18 | N 7,59 | S 2,60 |
| gef. | C 43,52 | H 4,07 | F 29,24 | N 7,67 | S 2,55 |

### b) 1,7-Bis (benzyloxycarbonyl)- 4-{3-oxa-pentan-1,5-dicarbonsäure-1-oyl-5-[1-(4-perfluoroctylsulfonyl)-piperazin]-amid}-10-[1-O-α-D-(5-carbonyl)-pentyl-2,3,4,6-tetra-Obenzyl-mannopyranose]-1,4,7,10-tetraazacyclododecan

10,0 g (13,4 mmol) der unter Beispiel 10c) hergestellten Carbonsäure und 3,24 g (28,1 mmol) N-Hydroxysuccinimid werden in 100 ml Dimethylformamid gelöst und bei 0°C mit insgesamt 5,8 g (28,1 mmol) N,N'-Dicyclohexylcarbodiimid portionsweise versetzt und es wird 3 Stunden bei dieser Temperatur nachgerührt.Zu der so hergestellten Aktivester-Lösung gibt man eine auf 0°C gekühlte Lösung von 14,83 g (13,4 mmol) der Titelverbindung aus Beispiel 15 a) , gelöst in 100 ml Dimethylformamid tropfenweise hinzu und rührt 2 Stunden bei 0°C sowie 12 h bei Raumtemperatur. Zur Aufarbeitung filtriert man vom ausgefallenen Dicyclohexylhamstoff ab und zieht das Lösungsmittel anschließend bis zur Trockne ab. Der so erhaltene Rückstand wird anschließend an Kieselgel chromatographiert (Laufmittel: Dichlormethan / Essigsäureethylester 20:1 ; die Durchführung der Chromatographie erfolgte unter Verwendung eines Solvensgradienten mit kontinuierlicher Zunahme des Essigsäureethylesteranteils).
Ausbeute: 18,3 g ( 78,2 % d.Th.) der Titelverbindung in Form eines farblosen und stark viskosen Öls.

| Elementaranalyse: | | | | | |
|---|---|---|---|---|---|
| ber. | C 55,11 | H 5,03 | N 4.82 | F 18,52 | S 1,84 |
| gef. | C 54.87 | H 4.85 | N 4.92 | F 18.55 | S 1,86 |

### c) 1-{3-oxa-pentan-1,5-dicarbonsäure-1-oyl-5-[1-(4-perfluoroctylsulfonyl)-piperazin]-amid}-7-[1-O-α-D-(5-carbonyl)-pentyl-mannopyranose]-1,4,7,10-tetraazacyclododecan

In 150 ml Ethanol werden 17,0 g (9,75 mmol) der unter 14b) hergestellten Verbindung gelöst, mit 1,0 g Pearlman-Katalysator (Pd 20 %, C) versetzt und solange bei Raumtemperatur unter einer Wasserstoffatmosphäre (1 atm) hydriert, bis keine Wasserstoffaufnahme mehr zu beobachten ist. Man saugt vom Katalysator ab, wäscht gründlich mit Ethanol nach (zweimal mit jeweils 75 ml) und engt im Vakuum zur Trockne ein. Die Titelverbindung wird als stark viskoses und farbloses Öl erhalten.
Ausbeute: 10,76 g (99,0 % d. Th.).

| Elementaranalyse: | | | | | |
|---|---|---|---|---|---|
| ber. | C 38,78 | H 4,61 | N 7,54 | F 28,97 | S 2,88 |
| gef. | C 38,86 | H 4.65 | N 7.41 | F 29,02 | S 2,92 |

### d) 1,7-Bis-[1,4,7-tris(carboxylatomethyl)-1,4,7,10- tetraazacyclododecan-Gd-Komplex-10-(pentanoyl-3-aza-4-oxo-5-methyl-5yl)-4-[2-(N-ethyl-N-perfluoroctylsulfonyl]-amino]-acetyl-2-oxa-acetyl]-10-[1-O-α-D-6-carbonylpentyl-mannopyranose]-1,4,7,10 tetraazacyclododecan

24,86 g (39,46 mmol; 4,4 Molequivalente bezogen auf die eingesetzte Aminkomponente 15c) des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 1,67 g wasserfreies Lithiumchlorid (39,46 mmol) werden bei 40 C° in 200 ml absolutem Dimethylsulfoxid unter Rühren gelöst und bei dieser Temperatur mit insgesamt 4,53g (39,46 mmol) N-Hydroxysuccinimid und 10,0 g (8,97 mmol) der Titelverbindung aus Beispiel 14c), gelöst in 100 ml absolutem Dimethylsulfoxid, versetzt. Nach dem Abkühlen auf Raumtemperatur wird die Reaktionslösung mit 8,14 g ( 39,46 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off :3000 Da) entsalzt und von niedermolekularen Bestandteilen gereinigt. Das Retentat wird anschließend gefriergetrocknet. Ausbeute: 16,37 g (79,3 % d. Th.) als farbloses Lyophilisat.
H₂O-Gehalt (Karl-Fischer): 7,65 %.

| Elementaranalyse (berechnet auf wasserfreie Substanz): | | | | | | |
|---|---|---|---|---|---|---|
| ber. | C 38,01 | H 4.61 | N 9,58 | F 13,81 | S 1,37 | Gd 13,45 |
| gef. | C 37.92 | H 4.55 | N 9.58 | F 13.77 | S 1.31 | Gd 13.48 |

### e) 3-Oxa-pentan-1,5-dicarbonsäure-mono-[1-(4-perfluoroctylsulfonyl)-piperazin]-amid

25 g (44,0 mmol) 1-Perfluoroctylsulfonylpiperazin werden in 150 ml Tetrahydrofuran gelöst und bei Raumtemperatur mit insgesamt 5,1 g (44,0 mmol) Diglycolsäureanhydrid versetzt und die so erhaltene Reaktionslösung für 12 h am Rückfluss gekocht. Nach dem Abkühlen auf Raumtemperatur wird bis zur Trockne eingeengt und der verbleibende ölige Rückstand wird an Kieselgel unter Verwendung von Dichlormethan /2-Propanol (16:1) als Eluent gereinigt
Ausbeute: 27,94 g (92,8 % d. Th.) der oben genannten Titelverbindung in Form eines farblosen und viskosen Öls.

| Elementaranalyse: | | | | | |
|---|---|---|---|---|---|
| ber. | C 58,52 | H 4,27 | N 1,98 | S 2,26 | F 22,80 |
| gef. | C 58,42 | H 4,41 | N 1,80 | S 2.28 | F 23,02 |

### Beispiel 16

### a) 1,7-Bis (benzyloxycarbonyl)-4-{3-oxa-pentan-1,5-dicarbonsäure-1-oyl-5-[1-(4-gerfluorooctylsulfonyl)-piperazin]-amid}-[10-[1-O-β-D - 6-carbonylpentyl - 2,3,4,6-tetra-O-benzyl -glucopyranose ] -1,4,7,10-tetraazacyclododecan)

In 750 ml trockenem Tetrahydrofuran werden 68,5 g ( 91,79 mmol) 1-Carboxymethyloxy-2,3,4,-tetra-O-benzyl-α-D-mannopyranosid [ Darstellung wie in der Patentschrift DE 197 28 954 C1 beschrieben] gelöst und anschließend 9,25 g (91,79 mmol) Triethylamin hinzugegeben. Nach dem Abkühlen der Reaktionslösung auf -15°C bis -20°C tropft man bei dieser Temperatur unter Rühren eine Lösung von 12,64 g (92,5 mmol) Chlorameisensäureisobutylester in 150 ml trockenem Tetrahydrofuran langsam hinzu, wobei die Zutropfgeschwindigkeit so zu wählen ist, □ass eine Innentemperatur von -10°C nicht überschritten wird. Nach einer Reaktionszeit von 15 Minuten bei -15°C tropft man anschließend eine Lösung von 101,6 g (91,79 mmol) der Titelverbindung aus Beispiel 15a) und 9,25 g (91,79 mmol) Triethylamin , als Lösung in 500 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 450 ml Essigsäureethylester aufgenommen und zweimal mit je 300 ml gesättigter Natriumhydrogencarbonatlösung sowie einmal mit 400 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Dichlormethan/Hexan/2-Propanol (10:20: 1) als Eluent gereinigt.
Ausbeute: 130,6 g ( 81,6 % d. Th.) der oben genannten Titelverbindung als farbloses und stark viskoses Öl.

| Elementaranalyse: | | | | | |
|---|---|---|---|---|---|
| ber. | C 55,11 | H 5,03 | N 4,82 | F 18,52 | S 1,84 |
| gef. | C 55,20 | H 5,09 | N 4,91 | F 18,48 | S 1,80 |

### b) 1-{3-Oxa-pentan-1,5-dicarbonsäure-1-oyl-5-[1-(4-perfluoroctylsulfonyl)-piperazin]-amid}-7-[1-O-α-D-(5-carbonyl)-pentyl-mannopyranose]-1,4,7,10-tetraazacyclododecan

In 1000 ml Ethanol werden 110,0 g (63,08 mmol) der unter 16a) hergestellten Verbindung gelöst, mit 5,0 g Pearlman-Katalysator (Pd 20 %, C) versetzt und bis zur quantitativen Aufnahme an Wasserstoff hydriert. Man saugt vom Katalysator ab, wäscht mit Ethanol nach und engt im Vakuum zur Trockne ein. Die Titelverbindung wird als zähes und farbloses Öl erhalten.
Ausbeute: 92,61 g (99,5 % d. Th.).

| Elementaranalyse: | | | | | |
|---|---|---|---|---|---|
| ber. | C 52,10 | H 5,12 | N 5,70 | F 21,89 | S 2,17 |
| gef. | C 52,20 | H 5,09 | N 5,71 | F 21,87 | S 2,20 |

### b) 1,7-Bis-[1,4,7-tris(carboxylatomethyl)-10-(3-aza-4-oxo-5-methyl-5-yl-pentanoyl)-4- {3-oxa-pentan-1,5-dicarbonsäure-1-oyl-5-[1-(4-perfluoroctylsulfonyl)-piperazin]-amid}-10-[1-O-α-D-(5-carbonyl)-pentyl-mannopyranose]-1,4,7,10-tetraazacyclododecan, Digadolinium-Komplex

55,4 g [ 88,0 mmol; 4,4 Molequivalente bezogen auf die eingesetzte Diaminkomponente aus Beispiel 13d) ] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 3,7 g wasserfreies Lithiumchlorid (88,0 mmol) werden bei 40 C° in 500 ml absolutem Dimethylsulfoxid unter Rühren gelöst und bei dieser Temperatur mit insgesamt 10,1 g (88,0 mmol) N-Hydroxysuccinimid und 29,5 g (20,0 mmol) der Titelverbindung aus Beispiel 16b), gelöst in 200 ml absolutem Dimethylsulfoxid, versetzt. Nach dem Abkühlen auf Raumtemperatur wird die Reaktionslösung mit 18,2 g ( 88,0 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Ausbeute: 35,96 g (76,9 % d. Th.) als farbloses Lyophilisat.
H₂O-Gehalt (Karl-Fischer): 5,98 %

| Elementaranalyse (berechnet auf wasserfreie Substanz): | | | | | | |
|---|---|---|---|---|---|---|
| ber. | C 38,01 | H 4,61 | N 8,22 | F 13,81 | Gd 13,45 | S 1,37 |
| gef. | C 37,87 | H 4,70 | N 8,22 | F 13,90 | Gd 13.48 | S 1,36 |

### Beispiel 17

### a) 5-(Ethoxycarbonyl)pentyl-2,3,4,6-tetra-O-acetyl-α-D-mannopyranosid

Auf analoge Weise, wie in der Literatur für die Synthese von Arylglycopyranosiden beschrieben [J. Conchie und G.A. Levvy in Methods in Carbohydrate Chemistry (R.L. Whistler, M.L. Wolfrom and J.N. BeMiller, Eds.), Academic Press, New York, Vol.II , 90, pp. 345-347, ( 1963 )] führt die Umsetzung von 156,2 g (400 mmol) D-Mannosepentaacetat als α, β-(α, β-Verhältnis = 4:1) -Anomerengemisch [ zur Synthese von 1,2,3,4,6-Penta-Oacetyl-α,β-D-mannopyranose vgl.: M.L.Wolfrom und A.Thompson in Methods in Carbohydrate Chemistry (R.L. Whistler, M.L. Wolfrom and J.N. BeMiller, Eds.), Academic Press, New York, Vol.II, 53 , pp. 211-215, (1963)] mit 67 ml (400 mmol ) 6-Hydroxy-hexansäureethylester und 60,8 ml (520 mmol) Zinn-IV-chlorid in insgesamt 600 ml 1,2-Dichlorethan nach säulenchromatographischer Aufreinigung (Eluent: Hexan/ Essigsäureethylester 2:1) zur Bildung von 100,05 g (51 % d. Th.) der oben genannten Titelverbindung als farbloses und viskoses Öl. Durch ¹H-NMR-spektroskopische Untersuchung der so erhaltenen Titelverbindung konnte gezeigt werden, □ass es sich bei der oben genannten Titelverbindung ausschließlich um das reine α-Anomere handelt.

| Elementaranalyse: | | |
|---|---|---|
| ber. | C 52,94 | H 6,77 |
| gef. | C 52,80 | H 6,78 |

### b) 5-(Carboxy)pentyl-2,3,4,6-tetra-O-benzyl-α-D-mannopyranosid

Eine gerührte Suspension von 141,0 g ( 289 mmol ) der Titelverbindung aus Beispiel 17a) in 200 ml Dioxan wird bei Raumtemperatur und unter gleichzeitigem kräftigen Rühren portionsweise mit insgesamt 238,5 g ( 4,26mol ) fein gepulvertem Kaliumhydroxydpulver versetzt. Zur Erhöhung der Rührfähigkeit wird das Reaktionsgemisch mit weiteren 200 ml Dioxan versetzt und die so erhaltene Suspension im Anschluß zur Siedehitze erhitzt und bei dieser Temperatur mit insgesamt 372 ml ( 3,128 mol ) Benzylbromid ,über einen Zeitraum von zwei Stunden ,tropfenweise versetzt. Nach einer Reaktionszeit von 4 Stunden bei 110 °C gefolgt von 12 Stunden bei Raumtemperatur wird das Reaktionsgemisch zum Zwecke der Aufarbeitung in insgesamt 2,5 Liter Eiswasser langsam eingegossen und die Wasserphase im Anschluß vollständig mit Diethylether extrahiert. Nach dem Waschen der so erhaltenen Etherphase und dem anschließenden Trocknen der Etherphase über Natriumsulfat wird vom Salz abgesaugt und der Diethylether im Vakuum abgezogen. Überschüssiges Benzylbromid wird anschließend im Ölpumpenvakuum quantitativ bei einer Ölbadtemperatur von 180 °C aus dem Reaktionsgemisch abdestilliert. Der so erhaltene, harzig-ölige Rückstand wird an Kieselgel unter Verwendung von Essigsäureethylester/Hexan (1:10) als Eluent gereinigt.
Ausbeute: 172,2, g (91,0 % d. Th.) der oben genannten Titelverbindung in Form eines farblosen und äußerst viskosen Öls

| Elementaranalyse: | | |
|---|---|---|
| ber. | C 75,68 | H 7,16 |
| gef. | C 75,79 | H 7,04 |

### c) 5-[(Carboxy)-pentyl-2,3,4,6-tetra-O-benzyl-α-D-mannopyranosid-]Nhydroxysuccinimidester

60,0 g (91,5 mmol) der Titelverbindung aus Beispiel 17b) werden in 750 ml Dimethylformamid gelöst und mit insgesamt 10,4 g (91,5 mmol) N-Hydroxysuccinimid versetzt.. Man kühlt auf 0°C ab und gibt 18,9 g (91,5 mmol) Dicyclohexylcarbodiimid zu. Es wird eine Stunde bei 0°C und anschließend 4 Stunden bei Raumtemperatur gerührt. Das Lösungsmittel im Vakuum abgezogen und der verbleibende Rückstand mit 100 ml Essigsäureethylester versetzt und auf 0°C abgekühlt. Es wird vom ausgefallenem Harnstoff abfiltriert und das erhaltene Filtrat im Vakuum bis zur Trockne eingeengt. Der so erhaltene, harzig-ölige Rückstand wird an Kieselgel unter Verwendung von Essigsäureethylester/Hexan (1:20) als Eluent gereinigt.
Ausbeute: 61,23 g (89,0 % d. Th.) der oben genannten Titelverbindung in Form eines farblosen und viskosen Öls.

| Elementaranalyse: | | | |
|---|---|---|---|
| ber. | C 70,29 | H 6,57 | N 1,86 |
| gef. | C 70,39 | H 5,64 | N 1,91 |

### d) 2,6-Bis-{ 6-N_{ε}-2-N_{α}-[-[1-0-α-D-6-carbony-pentyl-(2,3,4,6-tetra-O-benzyl)-mannopyranose}-L-lysin}-methylester

In eine auf 0°C gekühlte Lösung aus 4,26 g (18,30 mmol; 0,5 Molequivalente bezogen auf eingesetzte Carbonsäure) L-Lysinmethylester-dihydrochlorid (kommerziell erhältlich bei der Firma Bachem) und 4,05 g (40,26 mmol) Triethylamin in 100 ml Dimethylformamid tropft man eine Lösung von 27,51 g (36,6 mmol) der Titelverbindung aus Beispiel 17c) in 150 ml Dimethylformamid hinzu. Nach beendeter Zugabe rührt man noch eine Stunde bei 0°C nach und dann über Nacht bei Raumtemperatur. Es wird im Vakuum zur Trockne eingedampft und der Rückstand in 300 ml Essigsäureethylester aufgenommen. Man filtriert vom ausgefallenem Harnstoff ab und wäscht das Filtrat zweimal mit je 100 ml 5 %iger wässriger Soda-Lösung. Die organische Phase wird über Magnesiumsulfat getrocknet und im Vakuum zur Trockne eingedampft. Der Rückstand wird an Kieselgel chromatographiert (Laufmittel: n-Hexan/Isopropanol 25:1). Man erhält 39,56 g (75,4 % d. Th.) der Titelverbindung in Form eines farblosen Öls.

| Elementaranalyse: | | | |
|---|---|---|---|
| ber. | C 72,88 | H 7,31 | N 1,95 |
| gef. | C 72,90 | H 7,29 | N 2,02 |

### e) 2,6-Bis-[ 6-N_{ε}-2-N_{α}-[1-0-α-D-6-carbony-pentyl-(2,3,4,6-tetra-O-benzyl)-mannopyranose]]-L-lysin

In 150 ml Ethanol werden 30,0 g (20,92 mmol) der unter Beispiel 17d) hergestellten Verbindung gelöst. Man gibt dann die Lösung von 4 g (100,0 mmol) Natriumhydroxid in 10 ml destilliertem Wasser dazu und rührt 3 Stunden bei 50°C. Nach dem Dünnschichtchromatogramm ist die Verseifung quantitativ. Man engt im Vakuum zur Trockne ein und nimmt den verbliebenen Rückstand in 300 ml Essigsäureethylester auf und extrahiert die organische Phase zweimal mit je 100 ml verdünnter, wässriger Citronensäurelösung.Nach dem Trocknen über Natriumsulfat wird filtriert und man engt im Vakuum zur Trockne ein. Der Rückstand wird an Kieselgel chromatographiert (Laufmittel: n-Hexan/Isopropanol 13:1). Man erhält 25,56 g (88,5 % d. Th.,) der Titelverbindung in Form eines farblosen Öls.

| Elementaranalyse: | | | |
|---|---|---|---|
| ber. | C 72,88 | H 7,31 | N 1,95 |
| gef. | C 72,78 | H 7,33 | N 1,96 |

### f) 2,6-Bis-[ 6-N_{ε}-2-N_{α}-[1-0-α-D-6-Carbony-pentyl-(2,3,4,6-tetra-O-benzyl)-mannopyranose]-L-lysin]-N-hydroxysuccinimidester

14,0 g (9,15 mmol) der Titelverbindung aus Beispiel 17e) werden in 100 ml Dimethylformamid gelöst und mit insgesamt 1,04 g (9,15 mmol) N-Hydroxysuccinimid versetzt. Man kühlt auf 0°C ab und gibt 1,89 g (9,15 mmol) Dicyclohexylcarbodiimid zu. Es wird eine Stunde bei 0°C und anschließend 4 Stunden bei Raumtemperatur gerührt. Das Lösungsmittel wird anschließend im Vakuum abgezogen und der verbleibende Rückstand mit 100 ml Essigsäureethylester versetzt und auf 0°C abgekühlt. Es wird vom ausgefallenem Harnstoff abfiltriert und das erhaltene Filtrat im Vakuum bis zur Trockne eingeengt. Der so erhaltene, harzig-ölige Rückstand wird an Kieselgel unter Verwendung von Essigsäureethylester/n-Hexan (1:20) als Eluent gereinigt.
Ausbeute: 12,94 g (92,4 % d. Th.) der oben genannten Titelverbindung in Form eines farblosen und viskosen Öls.

| Elementaranalyse: | | | |
|---|---|---|---|
| ber. | C 71,40 | H 7,05 | N 2,74 |
| gef. | C 71,39 | H 7,14 | N 2,81 |

### g) 2,6-N,N'-Bis[1-0-α-D-(6-carbonyl)-pentyl-2,3,4,6-tetra-O-benzyl-mannopyranose]-Llysin-1,7-(1,4,7-triazaheptan)-diamid

In eine auf 0°C gekühlte Lösung von 0,47 g (4,57 mmol) Diethylentriamin in 25 ml Dimethylformamid tropft man eine Lösung aus 14,0 g (9,15 mmol; 2 Molequivalente bezogen auf das eingesetzte Amin) der Titel verbindung aus Beispiel 17f) in 100 ml Dimethylformamid langsam hinzu. Nach beendeter Zugabe rührt man noch eine Stunde bei 0°C nach und dann über Nacht bei Raumtemperatur. Es wird im Vakuum zur Trockne eingedampft und der Rückstand in 200 ml Essigsäureethylester aufgenommen. Man filtriert vom ausgefallenem Harnstoff ab und wäscht das Filtrat zweimal mit je 50 ml 5 %iger wässriger Soda-Lösung. Die organische Phase wird über Magnesiumsulfat getrocknet und im Vakuum zur Trockne eingedampft. Der Rückstand wird an Kieselgel chromatographiert (Laufmittel: n-Hexan/Isopropanol 25:1). Man erhält 9,53 g (71,4 % d. Th.) der Titelverbindung in Form eines farblosen Öls.

| Elementaranalyse: | | | |
|---|---|---|---|
| ber. | C 72,79 | H 7,42 | N 3,36 |
| gef. | C 72,90 | H 7,39 | N 3,32 |

### h) 2-N-[2-(N-ethyl-N-perfluoroctylsulfonyl)-amino]-acetyl-6-N-(benzyloxycarbonyl)-L-lysinmethylester

20,8 g (35,6 mmol) der 2-[N-Ethyl-N-perfluoroctylsulfonyl)-aminoessigsäure sowie 3,60 g (35,6 mmol) Triethylamin werden in 200 ml Dimethylformamid gelöst und es werden 4,09 g (35,6 mol) N-Hydroxysuccinimid hinzugegeben. Man kühlt auf 0°C ab und gibt 7,34 g (35,6 mmol) Dicyclohexylcarbodiimid zu. Es wird eine Stunde bei 0°C und anschließend 4 Stunden bei Raumtemperatur gerührt. Man kühlt auf 0°C ab und tropft innerhalb von 10 Minuten eine Lösung aus 11,77g (35,6 mmol) 6-N-Benzyloxycarbonyl-L-lysin-methylester-hydrochlorid und 4,0 g (40,0 mmol) Triethylamin in 100 ml Dimethylformamid hinzu. Man rührt eine Stunde bei 0°C, dann über Nacht bei Raumtemperatur. Es wird im Vakuum zur Trockne eingedampft und der Rückstand in 100 ml Essigsäureethylester aufgenommen. Man filtriert vom ausgefallenem Harnstoff ab und wäscht das Filtrat 2 mal mit je 100 ml 5 %iger aqu. Soda-Lösung. Die organische Phase wird über Magnesiumsulfat getrocknet und im Vakuum zur Trockne eingedampft. Der Rückstand wird an Kieselgel chromatographiert (Laufmittel: n-Hexan/Essigsäureethylester 20:1). Man erhält 27,43 g (88,0 % d. Th.) eines farblosen Öls.

| Elementaranalyse: | | | | | |
|---|---|---|---|---|---|
| ber. | C 38,41 | H 3,45 | N 4,80 | F 36,89 | S 3,66 |
| gef. | C 38,45 | H 3,38 | N 4,88 | F 37,02 | S 3,71 |

### b) 2-Nα- {[2-(N-ethyl-N-perfluoroctylsulfonyl]-amino-acetyl}-6-Nε-(benzyloxycarbonyl)-L-lysin

In 150 ml Ethanol werden 25,0 g (28,55 mmol) der unter Beispiel 17h) hergestellten Verbindung gelöst. Man gibt dann die Lösung von 4 g (100,0 mmol) Natriumhydroxid in 10 ml destilliertem Wasser dazu und rührt 3 Stunden bei 50°C. Nach dem Dünnschichtchromatogramm ist die Verseifung quantitativ. Man engt im Vakuum zur Trockne ein und nimmt den verbliebenen Rückstand in 300 ml Essigsäureethylester auf und extrahiert die organische Phase zweimal mit je 100 ml verdünnter, wässriger Citronensäurelösung.Nach dem Trocknen über Natriumsulfat wird filtriert und man engt im Vakuum zur Trockne ein. Der Rückstand wird an Kieselgel chromatographiert (Laufmittel: n-Hexan/Isopropanol 10:1). Man erhält 22,73g (92,4 % d. Th.,) der Titelverbindung in Form eines farblosen Öls.

| Elementaranalyse: | | | | | |
|---|---|---|---|---|---|
| ber. | C 37,64 | H 3,28 | N 4,88 | F 37,49 | S 3,72 |
| gef. | C 37,65 | H 3,38 | N 4,88 | F 37,52 | S 3,73 |

### j) 1,4,7-Triazaheptan-4-{2-N-(2-(N-ethyl-N-perfluoroctylsulfonyl)-amino]-acetyl-6-N-benzyloxycarbonyl}-L-lysin-amid-1,7-bis{2,6-N,N'-bis[1-O-α-D-(5-carbonyl)-pentyl-2,3,4,6-tetra-O-benzylmannopyranose]-L-lysin-diamid}

In 250 ml trockenem Tetrahydrofuran werden 15,33 g (17,8 mmol) der Titelverbindung aus Beispiel 17i) sowie 1,80 g (17,8 mmol) Triethylamin gelöst. Nach dem Abkühlen der Reaktionslösung auf -15°C bis -20°C tropft man bei dieser Temperatur unter Rühren eine Lösung von 4,92 g (35,6 mmol) Chlorameisensäureisobutylester , gelöst in 50 ml trockenem Tetrahydrofuran , langsam hinzu, wobei die Zutropfgeschwindigkeit so zu wählen ist, ass eine Innentemperatur von -10°C nicht überschritten wird. Nach einer Reaktionszeit von 15 Minuten bei -15°C tropft man anschließend eine Lösung von 52,0 g (17,8 mmol) der Titelverbindung aus Beispiel 17g) und 1,80g (17,8 mmol) Triethylamin, in 300 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 500 ml Essigsäureethylester aufgenommen und zweimal mit je 200 ml gesättigter Natriumhydrogencarbonatlösung sowie einmal mit 200 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Essigsäureethylester/ n-Hexan (1:20) als Eluent gereinigt.
Ausbeute: 54,6 g ( 81,6% d. Th.) der oben genannten Titelverbindung als farbloses und stark viskoses Öl.

| Elementaranalyse: | | | | | |
|---|---|---|---|---|---|
| ber. | C 65,09 | H 6,45 | N 3,72 | F 8,58 | S 0,85 |
| gef. | C 65,13 | H 4,41 | N 3,69 | F 8,52 | S 0,90 |

### k) 1,4,7-Triazaheptan-4-{2-N-[2-(N-ethyl-N-perfluoroctylsulfonyl)-amino]-acetyl}-L-lysinamid-1,7-bis(2,6-N,N'-bis[1-O-α-D-(5-carbonyl)-pentyl-mannopyranose]-L-lysin-diamid}

In 500 ml Ethanol werden 50,0 g (13,28 mmol) der unter 17j) hergestellten Verbindung gelöst, mit 4,0 g Pearlman-Katalysator (Pd 20 %, C) versetzt und solange bei Raumtemperatur unter einer Wasserstoffatmosphäre (1 atm) hydriert, bis keine Wasserstoffaufnahme mehr zu beobachten ist. Man saugt vom Katalysator ab, wäscht gründlich mit Ethanol nach (ca. 400 ml) und engt im Vakuum zur Trockne ein. Die Titelverbindung wird als stark viskoses und farbloses Öl erhalten.
Ausbeute: 26,85 g (93,0 % d. Th.).

| Elementaranalyse: | | | | | |
|---|---|---|---|---|---|
| ber. | C 45,85 | H 6,35 | N 6,44 | F 14,86 | S 1,47 |
| gef. | C 45.76 | H 6.35 | N 6.41 | F 14,92 | S 1,39 |

### 1) 1,4,7-Triazaheptan-4-{2-N-[2-(N-ethyl-N-perfluoroctylsulfonyl)-amino]-acetyl-6-N-[1,4,7-tris(carboxylatomethyl)-10-(3-aza-4-oxo-5-methyl-5-yl-pentanoyl)-1,4,7,10-tetraazacyclododecan}-L-lysin-amid-1,7-bis{2,6-N,N'-bis[1-O-α-D(5-carbonyl)-pentylmannopyranose]-L-lysin-diamid},Gadolinium-Komplex

5,54 g (8,8 mmol; 2.2 Molequivalente bezogen auf die eingesetzte Aminkomponente aus Beispiel 17k)) des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,37 g wasserfreies Lithiumchlorid (8,8 mmol) werden bei 40 C° in 60 ml absolutem Dimethylsulfoxid unter Rühren gelöst und bei dieser Temperatur mit insgesamt 1,01 g (8.8 mmol) N-Hydroxysuccinimid und 1,84 g (4,0 mmol) der Titelverbindung aus Beispiel 17k), gelöst in 40 ml absolutem Dimethylsulfoxid, versetzt. Nach dem Abkühlen auf Raumtemperatur wird die Reaktionslösung mit 1,82 g (8,8 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Ausbeute: 8,77g (78,7 % d. Th.) als farbloses Lyophilisat.
H₂O-Gehalt (Karl-Fischer): 4,43 %.

| Elementaranalyse (berechnet auf wasserfreie Substanz): | | | | | | |
|---|---|---|---|---|---|---|
| ber. | C 43,98 | H 5,97 | N 7,54 | F 11,59 | Gd 5,64 | S 1,15 |
| gef. | C 43,97 | H 6,02 | N 7,62 | F 11,61 | Gd 10.18 | S 1,15 |

### Beispiel 18

### a) 2-Nα-6-Nε-Bis-[1-O-α-D-Carbonylmethyl-2,3,4,6-tetra-O-benzyl-mannopyranose]-L-lysin]-methylester

10,95 g (18,30 mmol) 1-Carboxymethyloxy-2,3,4,-tetra-O-benzyl-α-D-mannopyranosid [ Darstellung wie in der Patentschrift DE 197 28 954 C1 beschrieben] werden in 150 ml Dimethylformamid gelöst und mit insgesamt 2,09 g (18,3 mmol) N-Hydroxysuccinimid versetzt. Man kühlt auf 0°C ab und gibt 3,78 g (18,3 mmol) Dicyclohexylcarbodiimid zu. Es wird eine Stunde bei 0°C und anschließend 4 Stunden bei Raumtemperatur gerührt. Man kühlt auf 0°C ab und tropft innerhalb von einer Stunde eine Lösung aus 2,13 g (9,15 mmol; 0,5 Molequivalente bezogen auf die eingesetzte Carbonsäure) L-Lysinmethylester-dihydrochlorid (kommerziell erhältlich bei der Firma Bachem) und 2,02 g (20,13 mmol) Triethylamin in 70 ml Dimethylformamid hinzu. Nach beendeter Zugabe rührt man noch eine Stunde bei 0°C nach und dann über Nacht bei Raumtemperatur. Es wird im Vakuum zur Trockne eingedampft und der Rückstand in 300 ml Essigsäureethylester aufgenommen. Man filtriert vom ausgefallenem Harnstoff ab und wäscht das Filtrat zweimal mit je 100 ml 5 %iger wässriger Soda-Lösung. Die organische Phase wird über Magnesiumsulfat getrocknet und im Vakuum zur Trockne eingedampft. Der Rückstand wird an Kieselgel chromatographiert (Laufmittel: n-Hexan/Isopropanol 25:1). Man erhält 10,05 g (82,3 % d. Th.) der Titelverbindung in Form eines farblosen Öls.

| Elementaranalyse: | | | |
|---|---|---|---|
| ber. | C 71,94 | H 6,79 | N 2,10 |
| gef. | C 71,90 | H 6,79 | N 2,09 |

### b) 2-Nα-6-Nε-Bis-[1-O-α-D-Carbonylmethyl-2,3,4,6-tetra-O-benzylmannopyranose]-L-lysin

Auf analogem Wege, wie im Beispiel 17e) für die Synthese der dort relevanten Titelverbindung beschrieben, führt die Methylesterverseifung von 15 g (11,23 mmol) der Titelverbindung aus Beispiel 18a) zur Bildung von 13,89 g (93,6 % d. Th.) an oben genannter Titelverbindung in Form eines farblosen und viskosen Öls.

| Elementaranalyse: | | | |
|---|---|---|---|
| ber. | C 71,80 | H 6,71 | N 2,12 |
| gef. | C 71,84 | H 6,69 | N 2,15 |

### c) 2-Nα-6-Nε-Bis-[1-O-α-D-carbonylmethyl-2,3,4,6-tetra-O-benzylmannopyranose]-L-lysin-N-hydroxysuccinimidester

12,09 g (9,15 mmol) der Titelverbindung aus Beispiel 18b) werden in 100 ml Dimethylformamid gelöst und mit insgesamt 1,04 g (9,15 mmol) N-Hydroxysuccinimid versetzt.. Man kühlt auf 0°C ab und gibt 1,89 g (9,15 mmol) Dicyclohexylcarbodiimid zu. Es wird eine Stunde bei 0°C und anschließend 4 Stunden bei Raumtemperatur gerührt. Das Lösungsmittel wird anschließend im Vakuum abgezogen und der verbleibende Rückstand mit 100 ml Essigsäureethylester versetzt und auf 0°C abgekühlt. Es wird vom ausgefallenem Harnstoff abfiltriert und das erhaltene Filtrat im Vakuum bis zur Trockne eingeengt. Der so erhaltene, harzig-ölige Rückstand wird an Kieselgel unter Verwendung von Essigsäureethylester/ n-Hexan (1:20) als Eluent gereinigt.
Ausbeute: 12,24 g (94,4 % d. Th.) der oben genannten Titelverbindung in Form eines farblosen und viskosen Öls.

| Elementaranalyse: | | | |
|---|---|---|---|
| ber. | C 70,27 | H 6,47 | N 2,96 |
| gef. | C 70,31 | H 6,44 | N 3,01 |

### d) 6-N-Benzyloxycarbonyl-2-N-{[2,6-N,N'-bis(1-O-α-D-carbonylmethyl-2,3,4,6-tetra-O-benzyl-mannopyranose)]-L-lysyl-}-L-lysin-[1-(4-perfluoroctylsulfonyl)-piperazin]-amid

19,0 g (13,4 mmol) des unter Beispiel 18c) hergestellten Carbonsäure-N-Hydroxysuccinimidesters werden in 75 ml Dimethylformamid gelöst und bei 0°C mit einer auf 0°C gekühlten Lösung von 11,13 g (13,4 mmol) der Titelverbindung aus Beispiel 1c) , gelöst in 50,0 ml Dimethylformamid , tropfenweise versetzt. Man rührt die resultierende Reaktionslösung noch 2 Stunden bei 0°C sowie 12 h bei Raumtemperatur. Zur Aufarbeitung filtriert man vom ausgefallenen Dicyclohexylharnstoff ab und zieht das Lösungsmittel anschließend bis zur Trockne im Vakuum ab. Der so erhaltene Rückstand wird an Kieselgel chromatographiert [Laufmittel: Dichlormethan / Ethanol 28:1 ; die Durchführung der Chromatographie erfolgt hier unter Verwendung eines Solvensgradienten mit einem ,im Chromatographieverlauf kontinuierlich zunehmenden , Anteil an der eingesetzten polaren Eluentkomponente (hier: Ethanol) ].
Ausbeute: 25,28 g ( 88,4 % d.Th.) der Titelverbindung in Form eines farblosen und stark viskosen Öls.

| Elementaranalyse: | | | | | |
|---|---|---|---|---|---|
| ber. | C 59,10 | H 5,34 | N 3.94 | F 15,13 | S 1,50 |
| gef. | C59,18 | H 5.35 | N 4.02 | F 15.15 | S 1.56 |

### e) 2-N-{[2,6-N,N'-bis(1-O-α-D-carbonylmethyl-mannopyranose)]-L-lysyl-L-lysin-[1-(4-perfluoroctylsulfonyl)-piperazin]-amid

In 200 ml Ethanol werden 20,0 g (9,37 mmol) der unter 18d) hergestellten Verbindung gelöst, mit 1,5 g Pearlman-Katalysator (Pd 20 %, C) versetzt und solange bei Raumtemperatur unter einer Wasserstoffatmosphäre (1 atm) hydriert, bis keine Wasserstoffaufnahme mehr zu beobachten ist. Man saugt vom Katalysator ab, wäscht gründlich mit Ethanol nach (zweimal mit je ca. 100 ml) und engt im Vakuum zur Trockne ein. Die Titelverbindung wird als stark viskoses und farbloses Öl erhalten.
Ausbeute: 11,62 g (97,0 % d. Th.).

| Elementaranalyse: | | | | | |
|---|---|---|---|---|---|
| ber. | C 38,50 | H 4.65 | N 6,57 | F 25,25 | S 2,51 |
| gef. | C 38.46 | H 4.65 | N 6.51 | F 25.23 | S 2,52 |

### f) 6-N-[1,4,7-tris(carboxylatomethyl)-10-(3-aza-4-oxo-5-methyl-5-yl-pentanoyl)-1,4,7,10-tetraazacyclododecan)-2-N-{[2,6-N,N'-bis(1-O-α-D-carbonylmethyl-mannopyranose)]-Llysyl}-L-lysin-[1-(4-perfluoroctylsulfonyl)-piperazin]-amid, Gd-Komplex

9,98 g (15,84 mmol; 2.2 Molequivalente bezogen auf die eingesetzte Aminkomponente aus Beispiel 18e) des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,67 g wasserfreies Lithiumchlorid (15,84 mmol) werden bei 40 C° in 100 ml absolutem Dimethylsulfoxid unter Rühren gelöst und bei dieser Temperatur mit insgesamt 1,82 g (15,84 mmol) N-Hydroxysuccinimid und 9,19 g (7,19 mmol) der Titelverbindung aus Beispiel 18e), gelöst in 50 ml absolutem Dimethylsulfoxid, versetzt. Nach dem Abkühlen auf Raumtemperatur wird die Reaktionslösung mit 3,27 g ( 15,84 mmol) N.N'-Dicyclohexyicarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und und dabei gleichzeitig von möglichen, noch vorhandenen niedermolekularen Bestandteilen , gereinigt. Das Retentat wird anschließend gefriergetrocknet.
Ausbeute: 11,85 g (87,2 % d. Th.) als farbloses Lyophilisat.
H₂O-Gehalt (Karl-Fischer): 5,54 %

| Elementaranalyse (berechnet auf wasserfreie Substanz): | | | | | | |
|---|---|---|---|---|---|---|
| ber. | C 38,12 | H 4,64 | N 8,15 | F 20.38 | S 1,70 | Gd 8,32 |
| gef. | C 38,16 | H 4.59 | N 8,18 | F 20,37 | S 1.68 | Gd 8,28 |

### Beispiel 19

### a)1,7-Bis(benzyloxycarbonyl)-4-(3-Oxa-2H,2H,4H,4H,5H,5H-perfluortridecanoyl)-1,4,7,10-tetraazacyclododecan

In eine Lösung von 10,75 g (24,4 mmol) 1,7-Bis-[benzyloxycarbonyl]-1,4,7,10-tetraazacyclododecan ,gelöst in einem Gemisch aus 150 ml Tetrahydrofuran und 15 ml Chloroform, werden bei 0°C und unter Stickstoffatmosphäre 12,74 g (24,4 mmol) der Titelverbindung aus Beispiel 19g) , gelöst in 150 ml Tetrahydrofuran hinzugegeben. Anschließend gibt man bei 0°C insgesamt 18,0 g (36,6 mmol) EEDQ [2-Ethoxy-1-ethoxycarbonyl-1,2-dihydrochinolin] portionsweise hinzu und läßt über Nacht bei Raumtemperatur rühren und engt anschließend im Vakuum ein. Das verbleibende Öl wird wird an Kieselgel chromatographiert (Laufmittel: n-Hexan/Isopropanol 16:1). Man erhält 15,89 g (69,0 % d. Th., bezogen auf eingesetztes sec.- Amin) des Monoamids sowie 3,8 g (8,8 % d. Th.) des Diamids als Nebenprodukt. Die Titelverbindung wird in Form eines farblosen Öls isoliert.

| Elementaranalyse: | | | | |
|---|---|---|---|---|
| ber. | C 45,77 | H 3,95 | F 34,19 | N 5,93 |
| gef. | C 45,72 | H 4,01 | F 34,22 | N 5,88 |

### b) 1,7-Bis(benzyloxycarbonyl)-4-(3-Oxa-2H,2H,4H,4H,5H,5H-perfluortridecanoyl)-10-[1-S-α-D-(2-carbonyl)-ethyl-2,3,4,6-tetra-O-acetyl-mannopyranose]-1,4,7,10-tetraazacyclododecan

7,09 g (13,4 mmol) vom 3-(2,3,4,6-Tetra-O-acetyl-1-thio-α-D-mannopyranosyl)-propionsäure-N-hydroxysuccinimidester (Darstellung gemäß: J. Haensler et al., Bioconjugate Chem. 4, 85, (1993); Chipowsky, S., and Lee,Y.C (1973) , Synthesis of 1-thio-aldosides; Carbohydrate Research 31, 339-346 ) werden in 100 ml Dimethylformamid gelöst und bei 0°C mit einer auf 0°C vorgekühlten Lösung von 12,65 g (13,4 mmol) der Titelverbindung aus Beispiel 19 a) , gelöst in 100 ml Dimethylformamid, tropfenweise versetzt. Man rührt 2 Stunden bei 0°C sowie 12 h bei Raumtemperatur. Zur Aufarbeitung zieht man das Lösungsmittel im Vakuum bis zur Trockne ab und chromatographiert den so erhaltenen Rückstand anschließend an Kieselgel (Laufmittel: Dichlormethan / Essigsäureethylester 20:1 ; die Durchführung der Chromatographie erfolgte unter Verwendung eines Solvensgradienten mit kontinuierlicher Zunahme des Essigsäureethylesteranteils).
Ausbeute: 16,23 g ( 88,9 % d.Th.) der Titelverbindung in Form eines farblosen und stark viskosen Öls.

| Elementaranalyse: | | | | | |
|---|---|---|---|---|---|
| ber. | C 46.70 | H 4,36 | N 4,11 | F 23,69 | S 2,35 |
| gef. | C 46,66 | H 4.35 | N 4.12 | F 23,65 | S 2,30 |

### c) 1-(3-Oxa-2H,2H,4H,4H,5H,5H-perfluartridecanoyl)-7-[1-S-α-D-(2-carbonyl)-ethyl-2,3,4,6-tetra-O-acetyl-mannopyranose]-1,4,7,10-tetraazacyclododecan

In 150 ml Ethanol werden 15,0 g (11,0 mmol) der unter 19b) hergestellten Verbindung gelöst, mit 1,0 g Pearlman-Katalysator (Pd 20 %, C) versetzt und solange bei Raumtemperatur unter einer Wasserstoffatmosphäre (1 atm) hydriert, bis keine Wasserstoffaufnahme mehr zu beobachten ist. Man saugt vom Katalysator ab, wäscht gründlich mit Ethanol nach (zweimal mit jeweils 75 ml) und engt im Vakuum zur Trockne ein. Die Titelverbindung wird als stark viskoses und farbloses Öl erhalten.
Ausbeute: 11,56 g (96,0 % d. Th.).

| Elementaranalyse: | | | | | |
|---|---|---|---|---|---|
| ber. | C 40,59 | H 4,33 | N 5,12 | F 29,50 | S 2,93 |
| gef. | C 40,63 | H 4.35 | N 5,11 | F 29,52 | S 2,92 |

### d) 1-(3-Oxa-2H,2H,4H,4H,5H,5H-perfluortridecanoyl)-7-[1-S-α-D-(2-carbonyl)-ethylmannopyranose]-1,4,7,10-tetraazacyclododecan

10,0 g (9,13 mmol) der Titelverbindung aus Beispiel 19c) werden 100 ml absolutem Methanol suspendiert und bei 5 °C mit einer katalytischen Menge Natriummethanolat versetzt. Nach einer Reaktionszeit von 3h bei Raumtemperatur zeigt die Dünnschichtchromatographische Kontrolle (Eluent: Chloroform / Methanol 4:1) des Reaktionsverlaufs bereits quantitative Umsetzung an. Zum Zwecke der Aufarbeitung wird die nun klare Reaktionslösung durch Versetzen mit Amberlite IR 120 ( H⁺-Form )-Kationenaustauscherharz neutralisiert, vom Austauscher abgesaugt, mit Methanol nachgewaschen und das so erhaltene methanolische Filtrat im Vakuum bis zur Trockne abgezogen. Der erhaltene ölige Rückstand wird durch Säulenchromatographie an Kieselgel (Laufmittel: Dichlormethan / n-Hexan/Essigsäureethylester 15:20:1 ; die Durchführung der Chromatographie erfolgte unter Verwendung eines Solvensgradienten mit kontinuierlicher Zunahme des Essigsäureethylesteranteils) gereinigt.Nach ¹H-NMR-spektroskopischer Untersuchung der Titelverbindung, konnte anhand der Größe der Kopplungskonstanten von J_{1,2} = 0,9 Hz eindeutig auf das Vorliegen der α-Konfiguration am anomeren Zentrum der D-Mannopyranose geschlossen werden. Die vorliegende α -Konfiguration ist die am Anomeriezentrum ausschließlich vorliegende Konfiguration, d.h. die Menge an möglicherweise gebildetem und β-konfiguriertem Anomeren der Titelverbindung liegt somit unterhalb der ¹H-NMR-spektroskopischen Nachweisgrenze. Die oben genannte Titelverbindung wurde demnach nur in Form des reinen α-konfigurierten Anomeren dargestellt.
Ausbeute: 8,28 g (98,0 % d.Th.) der Titelverbindung in Form eines farblosen und stark viskosen Öls.

| Elementaranalyse : | | | | | |
|---|---|---|---|---|---|
| ber. | C 37,59 | H 4,24 | N 6,05 | F 34,85 | S 3,46 |
| gef. | C 37,57 | H 4,28 | N 6,02 | F 34,85 | S 3,44 |

### e) 1-(3-Oxa-2H,2H,4H,4H,5H,5H-perfluortridecanoyl)-7-[1-S-α-D-(2-carbonyl)-ethylmannopyranose]-4,10-bis[1,4,7-tris(carboxylatomethyl)-10-(3-aza-4-oxo-5-methyl-5-ylpentanoyl)]-1,4,7,10-tetraazacyclododecan, Digadolinium-Komplex

2,48 g [(3,94 mmol); 4,4 Molequivalente bezogen auf die eingesetzte Diaminkomponente 19d)] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazäcyclododecan-1,4,7-triessigsäure und 167 mg wasserfreies Lithiumchlorid (3,94 mmol) werden bei 40 C° in 40 ml absolutem Dimethylsulfoxid unter Rühren gelöst und bei dieser Temperatur mit insgesamt 453 mg (3,94 mmol) N-Hydroxysuccinimid und 980 mg (0,895 mmol) der Titelverbindung aus Beispiel 19d), gelöst in 10 ml absolutem Dimethylsulfoxid, versetzt. Nach dem Abkühlen auf Raumtemperatur wird die Reaktionslösung mit 814 mg ( 3,946 mmol) N.N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Ausbeute: 1,32 g (69,1 % d. Th.) als farbloses Lyophilisat.
H₂O-Gehalt (Karl-Fischer): 7,65 %

| Elementaranalyse (berechnet auf wasserfreie Substanz): | | | | | | |
|---|---|---|---|---|---|---|
| ber. | C 37,43 | H 4,45 | N 9,12 | F 15,02 | S 1,49 | Gd 14,63 |
| gef. | C 37,42 | H 4,50 | N 9,18 | F 15,07 | S 1,51 | Gd 14,58 |

### f) 3-Oxa-2H,2H,4H,4H,5H,5H-perfluortridecansäure-t-butylester

25,0 g (53,8 mmol) 1H,1H,2H,2H-Perfluoro-1-decanol [kommerziell erhältlich bei der Firma Lancaster] werden in 250 ml absolutem Toluol gelöst und bei Raumtemperatur mit einer katalytischen Menge ( ca. 0,75 g ) Tetra-n-butyl-ammoniumhydrogensulfat versetzt. Anschließend gibt man bei 0°C insgesamt 7,55 g (134,6 mmol; 2,5 equ. bezogen auf die eingesetzte Alkoholkomponente) fein gepulvertes Kaliumhydroxidpulver hinzu, gefolgt von 15,73 g (80,7 mmol; 1,5 equ. bezogen auf die eingesetzte Alkoholkomponente) Bromessigsäure-tert.-butylester und lässt noch 2 Stunden bei 0°C nachrühren..Die so erhaltene Reaktionslösung wird für 12 h bei Raumtemperatur gerührt und zum Zwecke der Aufarbeitung wird mit insgesamt 500 ml Essigsäureethylester und 250 ml Wasser versetzt. Die org. Phase wird abgetrennt und zweimal mit Wasser gewaschen. Nach dem Trocknen der organischen Phase über Natriumsulfat wird vom Salz abgesaugt und das Solvens im Vakuum abgezogen. Der verbleibende ölige Rückstand wird an Kieselgel unter Verwendung von Essigsäureethylester/Hexan (1:10) als Eluent gereinigt.
Ausbeute: 26,3 g ( 84,6% d. Th.) der oben genannten Titelverbindung als farbloses und stark viskoses Öl

| Elementaranalyse: | | | |
|---|---|---|---|
| ber. | C 33,23 | H 2,61 | F 55,85 |
| gef. | C 33,29 | H 2,61 | F 55,90 |

### g) 3-Oxa-2H,2H,4H,4H,5H,5H-perfluortridecancarbonsäure

20,0 g (34,58 mmol) der Titelverbindung aus Beispiel 19f) werden in 200 ml eines Gemisches, bestehend aus Methanol und 0,5 molarer Natronlauge im Verhältnis von 2:1 unter Rühren bei Raumtemperatur suspendiert und anschließend auf 60 °C erwärmt. Nach einer Reaktionszeit von 12 h bei 60 °C wird das nun klare Reaktionsgemisch zur Aufarbeitung durch Versetzen mit Amberlite IR 120 (H⁺-Form)-Kationenaustauscherharz neutralisiert, vom Austauscher abgesaugt und das so erhaltene methanolisch-wässrige Filtrat im Vakuum bis zur Trockne abgezogen. Der erhaltene amorph-ölige Rückstand wird an Kieselgel unter Verwendung von Essigsäureethylester/n-Hexan (1:3) als Eluent gereinigt.
Ausbeute: 16,0 g ( 88,6% d. Th.) der oben genannten Titelverbindung als farbloses und stark viskoses Öl

| Elementaranalyse: | | | |
|---|---|---|---|
| ber. | C 27,60 | H 1,35 | F 61,85 |
| gef. | C 27,58 | H 1,36 | F 61,90 |

### Beispiel 20

### a)6-Benzyloxycarbonyl-2-[2-(N-ethyl-N-perfluoroctylsulfonyl)-amino]-acetyl-L-lysinmethylester

Zu 8,0 g (24,4 mmol) ε-Carbonyloxybenzyl-L-lysinmethylester Hydrochlorid (kommerziell erhältlich bei der Firma Bachem), gelöst in einem Gemisch aus 150 ml Tetrahydrofuran ,15 ml Chloroform und 2,62 g (26,0 mmol) Triethylamin , werden bei 0°C und unter Stickstoffatmosphäre 16,18 g (27,0 mmol) 2-[N-Ethyl-N-perfluoroctylsulfonyl)-aminoessigsäure (Darstellung gemäß: DE 196 03 033) , gelöst in 50 ml Tetrahydrofuran , tropfenweise hinzugegeben. Anschließend gibt man bei 0°C insgesamt 18,0 g (36,6 mmol) EEDQ [2-Ethoxy-1-ethoxycarbonyl-1,2-dihydrochinolin] portionsweise hinzu und läßt über Nacht bei Raumtemperatur rühren. Man engt anschließend im Vakuum ein und chromatographiert das verbleibende Öl an Kieselgel (Laufmittel: n-Hexan / Isopropanol 15: 1). Man erhält 17,0 g (79,6 % d. Th., bezogen auf eingesetztes prim.- Amin) der Titelverbindung in Form eines farblosen Öls.

| Elementaranalyse: | | | | | |
|---|---|---|---|---|---|
| ber. | C 38,41 | H 3,45 | F 36,89 | N 4,80 | S 3,66 |
| gef. | C 38,42 | H 3,47 | F 36,92 | N 4,87 | S 3,64 |

### b) 2-[2-(N-ethyl-N-gerfluoroctylsulfonyl)-amino]-acetyl-L-lysin-methylester

In 200 ml Ethanol werden 15,0 g (20,23 mmol) der unter Beispiel 20a) hergestellten Verbindung gelöst, mit 800 mg Pearlman-Katalysator (Pd 20 % auf Aktivkohle) versetzt und bis zur Aufnahme der berechneten Menge Wasserstoff hydriert. Man saugt vom Katalysator ab, wäscht gründlich mit Ethanol nach und engt im Vakuum zur Trockne ein. Die Titelverbindung wird als farbloses Öl erhalten.
Ausbeute: 14,68 g (97,9 % d.Th.)

| Elementaranalyse: | | | | | |
|---|---|---|---|---|---|
| ber. | C 32,40 | H 3,26 | F 43,56 | N 5,67 | S 4,32 |
| gef. | C 32,42 | H 3,27 | F 43,60 | N 5,67 | S 4,34 |

### c) 6-(1-O-α-D-carbonylmethyl-2,3,4,6-tetra-O-benzyl-mannopyranose) 2-[2-(N-ethyl-N-perfluoroctylsulfonyl)-amino]-acetyl-L-lysin-methylester

In 500 ml trockenem Tetrahydrofuran werden 21,31 g (35,6 mmol) 1-Carboxymethyloxy-2,3,4,-tetra-O-benzyl-α-D-mannopyranosid [ Darstellung wie in der Patentschrift DE 197 28 954 C1 beschrieben] sowie 3,60 g (35,6 mmol) Triethylamin gelöst. Nach dem Abkühlen der Reaktionslösung auf -15°C bis -20°C tropft man bei dieser Temperatur unter Rühren eine Lösung von 4,92 g (35,6 mmol) Chlorameisensäureisobutylester in 75 ml trockenem Tetrahydrofuran langsam hinzu, wobei die Zutropfgeschwindigkeit so zu wählen ist, daß eine Innentemperatur von -10°C nicht überschritten wird. Nach einer Reaktionszeit von 15 Minuten bei -15°C tropft man anschließend eine Lösung von 26,39 g (35,6 mmol) der Titelverbindung aus Beispiel 20b) und 3,60g ( 35,6 mmol) Triethylamin, in 100 ml trockenem Tetrahydrofuran bei -20°C langsam hinzu. Nach einer Reaktionszeit von einer Stunde bei -15°C sowie zwei Stunden bei Raumtemperatur wird die Reaktionslösung im Vakuum bis zur Trockne eingeengt. Der verbleibende Rückstand wird in 250 ml Essigsäureethylester aufgenommen und zweimal mit je 100 ml gesättigter Natriumhydrogencarbonatlösung sowie einmal mit 200 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Essigsäureethylester/n-Hexan (1:10) als Eluent gereinigt.
Ausbeute: 38,12 g ( 81,0 % d. Th.) der oben genannten Titelverbindung als farbloses und stark viskoses Öl

| Elementaranalyse: | | | | | |
|---|---|---|---|---|---|
| ber. | C 49,92 | H 3,92 | N 2,53 | F 29,18 | S 2,90 |
| gef. | C 49,99 | H 4,11 | N 2,69 | F 29,22 | S 3,01 |

### d) 6-(1-O-α-D-carbonylmethyl-2,3,4,6-tetra-O-benzyl-mannopyranose) 2-[2-(N-ethyl-N-perfluoroctylsulfonyl)-amino]-acetyl-L-lysin

In 250 ml Methanol werden 27,65 g (20,92 mmol) der unter Beispiel 20c) hergestellten Verbindung gelöst. Man gibt dann die Lösung von 4,0 g (100,0 mmol) Natriumhydroxid in 10 ml destilliertem Wasser dazu und rührt 3 Stunden bei 50°C. Nach Kontrolle des Reaktionsverlaufes mittels Dünnschichtchromatographie ist die Methylesterverseifung bereits quantitativ erfolgt. Man engt im Vakuum zur Trockne ein und nimmt den verbliebenen Rückstand in 300 ml Essigsäureethylester auf und extrahiert die organische Phase zweimal mit je 100 ml verdünnter, wässriger Citronensäurelösung.Nach dem Trocknen über Natriumsulfat wird filtriert und im Vakuum zur Trockne eingeengt. Der erhaltene Rückstand wird an Kieselgel chromatographiert (Laufmittel: n-Hexan/Chloroform/Isopropanol 15:10:1). Man erhält 24,31g (88,9 % d. Th.,) der Titelverbindung in Form eines farblosen und viskosen Öls.

| Elementaranalyse: | | | | | |
|---|---|---|---|---|---|
| ber. | C 51,46 | H 4,70 | N 3,21 | F 24,71 | S 2,45 |
| gef. | C 51,49 | H 4,71 | N 3,19 | F 24,72 | S 2,41 |

### e) 6-(1-O-α-D-carbonylmethyl-mannopyranose) 2-[2-(N-ethyl-N-perfluoroctylsulfonyl)-amino]-acetyl-L-lysin

20,0 g (15,30 mmol) der Titelverbindung aus Beispiel 20d) werden in einer Mischung ,bestehend aus 250 ml 2-Propanol und 25 ml Wasser, gelöst und mit 1,0 g Palladiumkatalysator (10 % Pd auf Aktivkohle) versetzt. Man hydriert für 12 Stunden bei Raumtemperatur und einem Wasserstoffdruck von einer Atmosphäre. Es wird vom Katalysator abfiltriert und das Filtrat im Vakuum zur Trockne eingedampft. Der Rückstand wird in 200 ml Methanol gelöst und das Reaktionsprodukt durch Versetzen mit insgesamt 800 ml Diethylether zur Fällung gebracht. Nach dem Absaugen des so erhaltenen Feststoffs wird dieser im Vakuum bei 50°C getrocknet.
Ausbeute: 14,32 g (99,0 % d. Th.) eines amorphen Feststoffes.

| Elementaranalyse: | | | | | |
|---|---|---|---|---|---|
| ber. | C 35,56 | H 3,84 | N 4,44 | S 3,39 | F 34,15 |
| gef. | C 35,58 | H 3,81 | N 4,45 | S 3,40 | F 34,17 |

### f) 6-(1-O-α-D-carbonylmethyl-mannopyranose) 2-[2-(N-ethyl-N-perfluoroctylsulfonyl)-amino]-acetyl-L-lysin-N-{2-hydroxy-prop-3-yl-[1,4,7-tris(carboxylatomethyl)-1,4,7,10-tetraazacyclododecan-10-yl]}-amid, Gd-Komplex

7,48 g (7,91 mmol) der Titelverbindung aus Beispiel 20e) werden bei 40 °C in 50 ml Dimethylsulfoxid gelöst und es wird 1,00 g (8,70 mol) N-Hydroxysuccinimid hinzugegeben. Man kühlt auf 20°C ab und gibt 1,795 g (8,7 mmol) Dicyclohexylcarbodiimid hinzu. Es wird eine Stunde bei 20°C und anschließend 4 Stunden bei 40°C gerührt. Anschließend tropft man bei dieser Temperatur innerhalb von 10 Minuten eine Lösung aus 4,53 g (7,91 mmol) des Gadolinium-Komplexes von 10-(2-Hydroxy-3-aminopropyl)-4,7,10-tris(carboxymethyl)-1,4,7,10-tetraazacyclododecanin [zur Darstellung vergleiche: WO 97/02051] in 20 ml Dimethylsulfoxid hinzu. Man rührt eine Stunde bei 40°C, dann über Nacht bei Raumtemperatur. Die so erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 9,71 g (81,7 % d. Th.) als farbloses Lyophilisat.
H₂O-Gehalt (Karl-Fischer): 3,97 %

| Elementaranalyse (berechnet auf wasserfreie Substanz): | | | | | | |
|---|---|---|---|---|---|---|
| ber. | C 35,16 | H 4,16 | N 7,45 | F 21,48 | Gd 10,46 | S 2,13 |
| gef. | C 35,17 | H 4,20 | N 7,42 | F 21,49 | Gd 10.48 | S 2,09 |

### Beispiel 21

### a) 6-N-[1-O-α-D-(5-carbonyl)-pentyl-2,3,4,6-tetra-O-benzyl-mannopyranose]- 2N-[2-(Nethyl-N-perfluoroctylsulfonyl)-amino]-acetyl-L-lysin-methylester

5,23 g (8,0 mmol) des in Beispiel 10c) beschriebenen 5-(Carboxy)pentyl-2,3,4,6-tetra-Obenzyl-α-D-mannopyranosids,1,3 g (8,0 mmol) 1-Hydroxybenzotriazol und 2,6 g (8,0 mmol) 2-(1H-Benzotriazol-1-yl)-1,1,3,3-tetramethyluronium tetrafluorborat (TBTU; Peboc Limited, UK) werden in 75 ml DMF gelöst und 15 Minuten gerührt. Diese Lösung wird anschließend mit 5,16 ml (30 mmol) N-Ethyldiisopropylamin und mit 5,93 g (8,0 mmol) des unter Beispiel 20b) beschriebenen Amins versetzt und 1,5 Tage bei Raumtemperatur gerührt. Zur Aufarbeitung zieht man das Lösungsmittel im Vakuum bis zur Trockne ab und chromatographiert den so erhaltenen Rückstand anschließend an Kieselgel (Laufmittel: Dichlormethan / Essigsäureethylester 30:1 ; die Durchführung der Chromatographie erfolgte unter Verwendung eines Solvensgradienten mit kontinuierlicher Zunahme des Essigsäüreethylesteranteils).
Ausbeute: 9,70 g ( 88,0 % d.Th.) der Titelverbindung in Form eines farblosen und stark viskosen Öls.

| Elementaranalyse: | | | | | |
|---|---|---|---|---|---|
| ber. | C 52,29 | H 4,97 | N 3,05 | F 23,43 | S 2,33 |
| gef. | C 52,33 | H 4.95 | N 3,12 | F 23,50 | S 2,30 |

### b) 6-N-[1-O-α-D-(5-carbonyl)-pentyl-2,3,4,6-tetra-O-benzyl-mannopyranose]-2N-[2-(Nethyl-N-perfluoroctylsulfonyl)-amino]-acetyl-L-lysin

In 150 ml Methanol werden 9,0 g (12,40 mmol) der unter Beispiel 21a) hergestellten Verbindung gelöst. Man gibt dann die Lösung von 2,48 g (62,0 mmol) Natriumhydroxid in 15 ml destilliertem Wasser dazu und rührt 3 Stunden bei 50°C. Nach Kontrolle des Reaktionsverlaufes mittels Dünnschichtchromatographie ist die Methylesterverseifung nach oben genannter Reaktionszeit bereits quantitativ erfolgt. Man engt im Vakuum zur Trockne ein und nimmt den verbliebenen Rückstand in 300 ml Essigsäureethylester auf und extrahiert die organische Phase zweimal mit je 100 ml verdünnter, wässriger Citronensäurelösung.Nach dem Trocknen über Natriumsulfat wird filtriert und im Vakuum zur Trockne eingeengt. Der erhaltene Rückstand wird an Kieselgel chromatographiert (Laufmittel: n-Hexan/Chloroform/Isopropanol 25:10:1). Man erhält 15,88 g (93,9 % d. Th.,) der Titelverbindung in Form eines farblosen und stark viskosen Öls.

| Elementaranalyse: | | | | | |
|---|---|---|---|---|---|
| ber. | C 51,95 | H 4,88 | N 3,08 | F 23,67 | S 2,35 |
| gef. | C 51,99 | H 4,91 | N 3,09 | F 23,70 | S 2,33 |

### c) 6-N-[1-O-α-D-(5-carbonyl)-pentyl-mannopyranose]- 2N-[2-(N-ethyl-N-perfluoroctylsulfonyl)-amino]-acetyl-L-lysin

13,0 g (9,52 mmol) der Titelverbindung aus Beispiel 21b) werden in einer Mischung bestehend aus 150 ml 2-Propanol und 25 ml Wasser gelöst und es werden 1,0 g des Palladiumkatalysators (10 % Pd auf Aktivkohle) hinzugegeben. Man hydriert für 12 Stunden bei 1 Atmosphäre Wasserstoffdruck und Raumtemperatur. Es wird vom Katalysator abfiltriert und das Filtrat im Vakuum zur Trockne eingedampft. Der erhaltene Rückstand wird an Kieselgel chromatographiert (Laufmittel: n-Hexan / Chloroform / Isopropanol 15:10:1). Man erhält 9,09 g (95,1 % d. Th.,) der Titelverbindung in Form eines farblosen und stark viskosen Öls.

| Elementaranalyse: | | | | | |
|---|---|---|---|---|---|
| ber. | C 37,10 | H 4,22 | N 4,19 | F 32,18 | S 3,10 |
| gef. | C 37,09 | H 4,21 | N 4,19 | F 32,20 | S 3,13 |

### d) 6-N-[1-O-α-D-(5-carbonyl)-pentyl-mannopyranose]-2N-[2-(N-ethyl-N-perfluoroctylsulfonyl)-amino]-acetyl-L-lysin-N-{2-hydroxy-prop-3-yl-[1,4,7-tris(carboxylatomethyl)-1,4,7,10-tetraazacyclododecan-10-yl]}-amid, Gd-Komplex

7,93 g (7,91 mmol) der Titelverbindung aus Beispiel 21c) werden bei 40 °C in 75 ml Dimethylsulfoxid gelöst und es wird mit 1,00 g (8,70 mol) N-Hydroxysuccinimid versetzt. Man kühlt auf Raumtemperatur ab und gibt insgesamt 1,795 g (8,7 mmol) Dicyclohexylcarbodiimid hinzu. Es wird eine Stunde bei 20°C und anschließend 4 Stunden bei 40°C gerührt. Zu dieser Lösung des Aktivesters der Titelverbindung aus Beispiel 21c) tropft man anschließend bei 40°C innerhalb von 10 Minuten eine Lösung, bestehend aus 4,53 g (7,91 mmol) des Gadolinium-Komplexes von 10-(2-Hydroxy-3-aminopropyl)-4,7,10-tris(carboxymethyl)-1,4,7,10-tetraazacyclododecanin [ zur Darstellung vergleiche: WO 97/02051], in 20 ml Dimethylsulfoxid hinzu. Man rührt eine Stunde bei 40°C, dann über Nacht bei Raumtemperatur. Die so erhaltene Suspension wird anschließend mit der ausreichenden Menge eines Gemisches aus Aceton / 2-Propanol ( 2:1) bis zur vollständigen Fällung der oben genannten Titelverbindung versetzt, der Niederschlag abgesaugt, mit Essigsäureethylester nachgewaschen, getrocknet, in Wasser aufgenommen, vom unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 9,71 g (78,8 % d. Th.) als farbloses Lyophilisat.
H₂O-Gehalt (Karl-Fischer): 6,65 %

| Elementaranalyse (berechnet auf wasserfreie Substanz): | | | | | | |
|---|---|---|---|---|---|---|
| ber. | C 36,97 | H 4,52 | N 7,19 | F 20,71 | Gd 10,08 | S 2,06 |
| gef. | C 37,02 | H 4,50 | N 7,22 | F 20,69 | Gd 10.08 | S 2,09 |

### Beispiel 22

### a) 6-N-{4-[2,3-Bis-(N,N-bis(t-butyloxycarbonylmethyl)-amino)-propyl]-phenyl}-3-oxapropionyl-2-N-(1-α-D-carbonylmethyl-mannopyranose) L-lysin-[1-(4-perfluoroctylsulfonyl)-piperazin]-amid

5,25 g (7,72 mmol) des Tetra-t.bu-esters der 1-(4-Carboxymethoxybenzyl)-EDTA ( Lit.: Patent, US 4622420 ) und 781 mg (7,72 mmol) Triethylamin werden in 50 ml Methylenchlorid gelöst. Bei -15°C tropft man eine Lösung aus 1,16 g (8,5 mmol) Chlorameisensäureisobutylester in 10 ml Methylchlorid innerhalb 5 Minuten hinzu und rührt noch weitere 20 Minuten bei -15°C. Anschließend kühlt man die Lösung auf -25°C ab und tropft eine Lösung , bestehend aus 7,07 g (7,72 mmol) der Titelverbindung aus Beispiel 10e) und 2,12 g (21,0 mmol) Triethylamin, in 70 ml Tetrahydrofuran innerhalb von 30 Minuten hinzu und rührt im Anschluß noch 30 Minuten bei -15° C und anschließend noch über Nacht bei Raumtemperatur nach. Zur Aufarbeitung wird das Lösungsmittel im Vakuum abgezogen und der verbleibende ölige Rückstand in 250 ml Chloroform aufgenommen. Man extrahiert die Chloroformphase zweimal mit je 100 ml einer 10 %igen wässrigen AmmoniumchloridLösung, trocknet die organische Phase über Magnesiumsulfat und dampft im Vakuum zur Trockne ein. Der Rückstand wird an Kieselgel chromatographiert (Laufmittel: Methylenchlorid/Ethanol= 20:1).
Ausbeute: 9,60 g (79,0 % d. Th.) eines farblosen und sehr zähen Öls.

| Elementaranalyse: | | | | | |
|---|---|---|---|---|---|
| ber. | C 46,39 | H 5,55 | N 5,32 | F 20,45 | S 2,03 |
| gef. | C 46,42 | H 5,51 | N 5,29 | F 20,49 | S 2,09 |

### b) 6-N-{4-[2,3-Bis-(N,N-bis(caboxymethyl)-amino)-propyl]-phenyl}-3-oxa-propionyl-2-N-(1-α-D-carbonylmethyl-mannopyranose) L-lysin-[1-(4-periluoroctylsulfonyl)-piperazin]-amid

In 150 ml Methanol werden 9,0 g (5,70 mmol) der unter Beispiel 22a) hergestellten Verbindung gelöst. Man gibt dann die Lösung von 4,0 g (100,0 mmol) Natriumhydroxid in 25 ml destilliertem Wasser dazu und rührt 6 Stunden bei 60°C. Nach Kontrolle des Reaktionsverlaufes mittels Dünnschichtchromatographie ist die Verseifung des tetra-t.butylesters nach oben genannter Reaktionszeit bereits quantitativ erfolgt. Man engt im Vakuum zur Trockne ein und nimmt den verbliebenen Rückstand in 50 ml Dimethylsulfoxid in der Wärme auf und anschließend wird mit der ausreichenden Menge eines Gemisches aus Aceton / Essigsäureethylester (1:1) bis zur vollständigen Fällung der oben genannten Titelverbindung versetzt, der so erhaltene Niederschlag abgesaugt, mit Essigsäureethylester gut nachgewaschen, getrocknet, in Wasser aufgenommen, der pH-Wert der Produktlösung mittels 1 molarer Salzsäure auf 3,5 eingestellt, von möglicherweise vorliegenden unlöslichen Bestandteilen abfiltriert und das Filtrat über eine AMICON® YM-3 Ultrafiltrationsmembran (cut off: 3000 Da) entsalzt und von niedermolekularen Bestandteilen gereinigt. Das Retentat wird anschließend gefriergetrocknet.
Ausbeute: 6,76 g (87,6 % d. Th.) als farbloses Lyophilisat.
H₂O-Gehalt (Karl-Fischer): 3,30 %.

| Elementaranalyse (berechnet auf wasserfreie Substanz): | | | | | |
|---|---|---|---|---|---|
| ber. | C 39,89 | H 4,09 | N 6,20 | F 23,84 | S 2,37 |
| gef. | C 39,92 | H 4,15 | N 6,22 | F 23,92 | S 2,29 |

### c) 6-N-{4-[2,3-Bis-(N,N-bis(caboxylatomethyl)-amino)-propyl]-phenyl}-3-oxa-propionyl-2-N-(1-α-D-carbonylmethyl-mannopyranose) L-lysin-[1-(4-perfluoroctylsulfonyl)-piperazin]-amid, Mn-Komplex, Dinatriumsalz

3,0 g (2,22 mmol) der Titelverbindung aus Beispiel 22b) werden in 150 ml eines Wasser/Ethanol (3:1)-Gemisch in der Siedehitze gelöst und bei 80°C portionweise mit 0,25 g (2,22 mmol) Mangan-II-carbonat versetzt. Anschließend wird die so erhaltene Reaktionslösung für 5 Stunden unter Rückfluß gekocht. Nach dem Abkühlen auf Raumtemperatur wird das Lösungsmittelgemisch im Vakuum vollständig abgezogen und der verbleibende Rückstand in einem Gemisch aus 200 ml destilliertem Wasser /n-Butanol (1:1) gelöst. Unter kräftigem Rühren wird durch Versetzen mit 1 N Natronlauge ein pH-Wert von 7,2 eingestellt. Nach dem vollständigen Abziehen des n-Butanols im Vakuum wird die verbleibende wässrige Phase über eine AMICON® YM-3 Ultrafiltrationsmembran (cut off 3000 Da) entsalzt und von niedermolekularen Bestandteilen gereinigt. Das Retentat wird anschließend gefriergetrocknet.
Ausbeute: 3,19 g (99,0 % d. Th.) als farbloses Lyophilisat.
H₂O-Gehalt (Karl-Fischer): 5,08 %.

| Elementaranalyse (berechnet auf wasserfreie Substanz): | | | | | | | |
|---|---|---|---|---|---|---|---|
| ber. | C 37,23 | H 3,54 | F 22,25 | Mn 3,78 | N 5,79 | Na 3,17 | S 2,21 |
| gef. | C 37,30 | H 3,49 | F 22,29 | Mn 3,81 | N 5,76 | Na 3,19 | S 2,18 |

### Beispiel 23

### a) 3-Benzyloxycarbonylamino-glutarsäure-[1-(4-perfluoroctylsulfonyl)-piperazin]-monoamid

Eine gerührte Lösung von 25,0 g (94,96 mmol) 3 -N-(Benzy]oxcarbonyl)-giutarsäureanhydrid [ Synthese gemäß: Hatanaka, Minoru; Yamamoto, Yu-ichi; Nitta, Hajime; Ishimaru, Toshiyasu; TELEAY; Tetrahedron Lett.; EN; 22; 39; 1981; 3883-3886;] in 150 ml absolutem Tetrahydrofuran wird unter Rührem mit einer Lösung von 53,97 g (95,0 mmol) 1-Perfluoroctylsulfonylpiperazin in 150 ml Tetrahydrofuran tropfenweise versetzt und die so erhaltene Reaktionslösung für 12 h am Rückfluss gekocht. Nach dem Abkühlen auf Raumtemperatur wird bis zur Trockne eingeengt und der verbleibende ölige Rückstand wird an Kieselgel unter Verwendung von Dichlormethan /2-Propanol (20:1) als Eluent gereinigt. Ausbeute: 75,80 g (96,0% d. Th.) der oben genannten Titelverbindung in Form eines farblosen und viskosen Öls.

| Elementaranalyse: | | | | | |
|---|---|---|---|---|---|
| ber. | C 36,11 | H 2,67 | N 5,05 | S 3,86 | F 38,84 |
| gef. | C 36,12 | H 2,61 | N 5,08 | S 3,88 | F 38,82 |

### b) 3-Amino-glutarsäure-[1-(4-perfluoroctylsulfonyl)-piperazin]-monoamid

In 300 ml Ethanol werden 31,50 g (37,88 mmol) der unter 23b) hergestellten Verbindung gelöst, mit 2,5g Pearlman-Katalysator (Pd 20 %, C) versetzt und bis zur quantitativen Aufnahme von Wasserstoff bei 1 Atmosphäre Wasserstoffdruck hydriert. Man saugt vom Katalysator ab, wäscht mit Ethanol nach und engt im Vakuum zur Trockne ein. Die Titelverbindung wird als weißlich-gelbes, viskoses Öl erhalten.
Ausbeute: 25,22 g (95,5 % d. Th.)

| Elementaranalyse: | | | | | |
|---|---|---|---|---|---|
| ber. | C 29,28 | H 2,31 | N 6,03 | S 4,06 | F 46,31 |
| gef. | C 29,32 | H 2,29 | N 6,08 | S 4,08 | F 46,28 |

### c) 3-N-(1-α-D-carbonylmethyl-2,3,4,6-tetra-O-benzyl-mannopyranose)-glutarsäure-[1-(4-perfluoroctylsulfonyl)-piperazin]-monoamid

21,52 g (18,96 mmol) 1-Carboxymethyloxy-2,3,4,-tetra-O-benzyl-α-D-mannopyranosid [ Darstellung wie in der Patentschrift DE 197 28 954 C1 beschrieben] werden bei Raumtemperatur in 100 ml absolutem Dimethylformamid gelöst und bei 0 °C mit 2,56 g (22,2 mmol) N-Hydroxysuccinimid, gefolgt von 4,55 g (22,2 mmol) Dicyclohexylcarbodimid versetzt. Nach einer Reaktionszeit von 60 Minuten bei 0 °C und 3 Stunden bei 22 °C filtriert man vom unlöslichen Dicyclohexylharnstoff ab und tropft die so erhaltene klare Aktivesterlösung der oben genannten Titelverbindung bei 0 °C langsam zu einer gerührten Lösung von 13,22 g (18,96 mmol) der Verbindung aus Beispiel 23b) , gelöst in 100 ml Dimethylformamid. Nach einer Reaktionszeit von 12 Stunden bei Raumtemperatur wird das Lösungsmittel im Vakuum abgezogen und der der verbleibende Rückstand in 300 ml Essigsäureethylester aufgenommen, es wird vom Harnstoff abfiltriert und das organische Filtrat zweimal mit je 100 ml gesättigter Natriumhydrogencarbonatlösung sowie einmal mit 100 ml 10%iger wässriger Citronensäurelösung und einmal mit 200 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Essigsäureethylester / n-Hexan (1:15) als Eluent gereinigt. Ausbeute: 21,39 g ( 88,3 % d. Th.) der oben genannten Titelverbindung als farbloses und stark viskoses Öl.

| Elementaranalyse: | | | | | |
|---|---|---|---|---|---|
| ber. | C 49,81 | H 4,10 | N 3,29 | F 25,27 | S 2,51 |
| gef. | C 49,89 | H 4,11 | N 3,32 | F 25,22 | S 2,51 |

### d) 3-N-(1-α-D-carbonylmethyl-mannopyranose)-glutarsäure-[1-(4-perfluoroctylsulfonyl)-piperazin]-monoamid

19,55 g (15,30 mmol) der Titelverbindung aus Beispiel 23c) werden in einer Mischung ,bestehend aus 250 ml 2-Propanol und 25 ml Wasser, gelöst und mit 1,5 g Palladiumkatalysator (10 % Pd auf Aktivkohle) versetzt. Man hydriert für 12 Stunden bei Raumtemperatur und einem Wasserstoffdruck von einer Atmosphäre. Es wird vom Katalysator abfiltriert und das Filtrat im Vakuum zur Trockne eingedampft. Der Rückstand wird in 200 ml Methanol gelöst und das Reaktionsprodukt durch Versetzen mit insgesamt 800 ml Diethylether zur Fällung gebracht. Nach dem Absaugen des so erhaltenen Feststoffs wird dieser im Vakuum bei 40°C getrocknet.
Ausbeute: 17,49 g (97,5 % d. Th.) eines amorphen Feststoffes.

| Elementaranalyse: | | | | | |
|---|---|---|---|---|---|
| ber. | C 32,73 | H 3,08 | N 4,58 | S 3,49 | F 35,20 |
| gef. | C 32,68 | H 3,15 | N 4,55 | S 3,50 | F 35,17 |

### e) 3-N-(1-α-D-carbonylmethyl-mannopyranose)-glutarsäure-[1-(4-perfluoroctylsulfonyl)-piperazin]-amid-5-N-{2-hydroxy-prop-3-yl-[1,4,7-tris(carboxylatomethyl)-1,4,7,10-tetraazacyclododecan-10-yl]}-amid, Gd-Komplex

14,43 g (15,84 mmol) der Titelverbindung aus Beispiel 23d) und 0,67 g wasserfreies Lithiumchlorid (15,84 mmol) werden bei 40 C° in 100 ml absolutem Dimethylsulfoxid unter Rühren gelöst und bei dieser Temperatur mit insgesamt 1,82 g (15,84 mmol) N-Hydroxysuccinimid und einer Lösung von 9,08 g (15,84 mmol) des Gadolinium-Komplexes von 10-(2-Hydroxy-3-arninopropyl)-4,7,10-tris(carboxymethyl)-1,4,7,10-tetraazacyclododecanin [ zur Darstellung vergleiche: WO 97/02051], in 50 ml Dimethylsulfoxid, versetzt. Nach dem Abkühlen auf Raumtemperatur wird die Reaktionslösung mit 3,27 g ( 15,84 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und und dabei gleichzeitig von möglichen , noch vorhandenen niedermolekularen Bestandteilen , gereinigt. Das Retentat wird anschließend gefriergetrocknet.
Ausbeute: 18,71g (80,2 % d. Th.) als farbloses Lyophilisat.
H₂O-Gehalt (Karl-Fischer): 4,87 %.

| Elementaranalyse (berechnet auf wasserfreie Substanz): | | | | | | |
|---|---|---|---|---|---|---|
| ber. | C 34,24 | H 3,83 | N 7,61 | F 21,92 | S 2,18 | Gd 10,67 |
| gef. | C 34,26 | H 3.79 | N 7,58 | F 21,87 | S 2,18 | Gd 10,68 |

### Beispiel 24

### 1,7-Bis(benzyloxycarbonyl)-4-{3-oxa-pentan-1,5-dicarbonsäure-1-oyl-5-[1-(4-perfluoroctylsulfonyl)-piperazin]-amid}-10-[2,6-N,N'-bis(1-O-α-D-carbonylmethyl-2,3,4,6-tetra-O-benzyl-mannopyranose)]-L-lysyl-1,4,7,10-tetraazacyclododecan

In eine Lösung aus 27,0 g (24,4 mmol) des unter Beispiel 15a) hergestellten sec. Amins, in einem Gemisch aus 150 ml Tetrahydrofuran und 15 ml Chloroform, werden bei 0°C und unter Stickstoffatmosphäre 33,04g (25,0 mmol) der Titelverbindung aus Beispiel 18c) , gelöst in 250 ml Tetrahydrofuran hinzugegeben.Anschließend gibt man bei 0°C insgesamt 18,0 g (36,6 mmol) EEDQ [2-Ethoxy-1-ethoxycarbonyl-1,2-dihydrochinolin] portionsweise hinzu und läßt über Nacht bei Raumtemperatur rühren. Man engt anschließend im Vakuum zur Trockne ein und das verbleibende Öl wird wird an Kieselgel chromatographiert (Laufmittel: n-Hexan/Isopropanol 25:1). Man erhält 45,87 g (78,0 % d. Th., bezogen auf eingesetztes sec.-Amin) der Titelverbindung in Form eines farblosen Öls.

| Elementaranalyse: | | | | | |
|---|---|---|---|---|---|
| ber. | C 59,30 | H 5,39 | F 13,40 | N 4,65 | S 1,33 |
| gef. | C 59,32 | H 5,37 | F 13,37 | N 4,70 | S 1,34 |

### b) 1-{3-oxa-pentan-1,5-dicarbonsäure-1-oyl-5-[1-(4-perfluoroctylsulfonyl)-piperazin]-amid}-7-[2,6-N,N'-bis(1-O-α-D-carbonylmethyl-mannopyranose)]-L-lysyl-1,4,7,10-tetraazacyclododecan

In 250 ml Ethanol werden 24,1 g (10,0 mmol) der unter Beispiel 24a) hergestellten Titelverbindung gelöst und mit 1,4 g Pearlman-Katalysator (Pd 20 %, C) versetzt. Man hydriert bis zur quantitativen Aufnahme an Wasserstoff, saugt dann vom Katalysator ab, wäscht mit Ethanol gut nach und engt im Vakuum zur Trockne ein. Das Produkt wird als gelblich gefärbtes und äusserst viskoses Öl erhalten.
Ausbeute: 12,80 g (90,1 % d. Th.).

| Elementaranalyse: | | | | | |
|---|---|---|---|---|---|
| ber. | C 39,72 | H 4,89 | F 22,73 | N 7,88 | S 2,26 |
| gef. | C 39,72 | H 4,87 | F 22,77 | N 7,90 | S 2,24 |

### c) 1-{3-oxa-pentan-1,5-dicarbonsäure-1-oyl-5-[1-(4-perfluoroctylsulfonyl)-piperazin]-amid}-7-[2,6-N,N'-bis(1-O-α-D-carbonylmethyl-mannopyranose)]-L-lysyl-4,10-bis[1,4,7-tris(carboxylatomethyl)-10-(3-aza-4-oxo-5-methyl-5-yl-pentanoyl)]-1,4,7,10-tetraazacyclododecan,Digadolinium-Komplex

5,54 g [8,8 mmol; 2.2 Molequivalente bezogen auf die eingesetzte Aminkomponente aus Beispiel 24b) ] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und wasserfreies Lithiumchlorid (0,37 g, 8,8 mmol) werden bei 40 C° in 60 ml absolutem Dimethylsulfoxid unter Rühren gelöst und bei dieser Temperatur mit insgesamt 1,01 g (8.8 mmol) N-Hydroxysuccinimid und 5,68 g (4,0 mmol) der Titelverbindung aus Beispiel 24b), gelöst in 40 ml absolutem Dimethylsulfoxid, versetzt. Nach dem Abkühlen auf Raumtemperatur wird die Reaktionslösung mit 1,82 g (8,8 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON@ YM-3 Ultrafiltrationsmembran (cut off 3000 Da) entsalzt und von niedermolekularen Bestandteilen gereinigt. Das Retentat wird anschließend gefriergetrocknet. Ausbeute: 8,52g (80,6 % d. Th.; bezogen auf die eingesetzte Diaminkomponente) als farbloses Lyophilisat.
H₂O-Gehalt (Karl-Fischer): 6.09 %.

| Elementaranalyse (berechnet auf wasserfreie Substanz): | | | | | | |
|---|---|---|---|---|---|---|
| ber. | C 38,61 | H 4,76 | N 9,53 | F 12,21 | Gd 11.89 | S 1,12 |
| gef. | C 38,57 | H 4,82 | N 9,52 | F 12,21 | Gd 11,93 | S 1,15 |

### Beispiel 25

### a) 1,7-Bis(benzyloxycarbonyl)-4-{3-oxa-pentan-1,5-dicarbonsäure-1-oyl-5-[1-(4-perfluoroctylsulfonyl)-piperazin]-amid}-10-{2,6-N,N'-bis(1-O-α-D-(5-carbonyl)-pentyl-2,3,4,6-tetra-O-benzyl-mannopyranose)}-L-lysyl-1,4,7,10-tetraazacyclododecan

In eine Lösung aus 27,0 g (24,4 mmol) des unter Beispiel 15a) hergestellten sec. Amins, in einem Gemisch aus 150 ml Tetrahydrofuran und 15 ml Chloroform, werden bei 0°C und unter Stickstoffatmosphäre 35,80 g (25,0 mmol) der Titelverbindung aus Beispiel 17e) , gelöst in 250 ml Tetrahydrofuran hinzugegeben.Anschließend gibt man bei 0°C insgesamt 18,0 g (36,6 mmol) EEDQ [2-Ethoxy-1-ethoxycarbonyl-1,2-dihydrochinolin] portionsweise hinzu und läßt über Nacht bei Raumtemperatur rühren. Man engt anschließend im Vakuum zur Trockne ein und das verbleibende Öl wird wird an Kieselgel chromatographiert (Laufmittel: n-Hexan/Isopropanol 20: 1). Man erhält 49,48 g (80,4 % d. Th., bezogen auf eingesetztes sec.-Amin) der Titelverbindung in Form eines farblosen Öls.

| Elementaranalyse: | | | | | |
|---|---|---|---|---|---|
| ber. | C 60,47 | H 5,79 | F 12,80 | N 4,44 | S 1,27 |
| gef. | C 60,52 | H 5,77 | F 12,77 | N 4,50 | S 1,30 |

### b) 1-{3-oxa-pentan-1,5-dicarbonsäure-1-oyl-5-[1-(4-perfluoroctylsulfonyl)-piperazin]-amid}-7-[2,6-N,N'-bis(1-O-α-D-(5-carbonyl)-pentyl-mannopyranose)]-L-lysyl-1,4,7,10-tetraazacyclododecan

In 250ml Ethanol werden 25,2 g (10,0 mmol) der unter Beispiel 25a) hergestellten Titelverbindung gelöst und mit 1,8 g Pearlman-Katalysator (Pd 20 %, C) versetzt. Man hydriert bis zur quantitativen Aufnahme an Wasserstoff, saugt dann vom Katalysator ab, wäscht mit Ethanol gut nach und engt im Vakuum zur Trockne ein. Das Produkt wird als gelblich gefärbtes und äusserst viskoses Öl erhalten.
Ausbeute: 14,11 g (92,5 % d. Th.)

| Elementaranalyse: | | | | | |
|---|---|---|---|---|---|
| ber.: | C 49,60 | H 7,20 | F 21,17 | N 7,34 | S 2,10 |
| gef.: | C 49,62 | H 7,17 | F 21,20 | N 7,30 | S 2,14 |

### c) 1-{3-oxa-pentan-1,5-dicarbonsäure-1-oyl-5-[1-(4-perfluoroctylsulfonyl)-piperazin]-amid}-7-[2,6-N,N'-bis(1-O-α-D-(5-carbonyl)-pentyl-mannopyranose)]-L-lysyl-4,10-bis[1,4,7-tris(carboxylatomethyl)-10-(3-aza-4-oxo-5-methyl-5-yl-pentanoyl)]-1,4,7,10-tetraazacyclododecan,Digadolinium-Komplex

5,54 g [8,8 mmol; 2.2 Molequivalente bezogen auf die eingesetzte Aminkomponente aus Beispiel 25b)] des in der Patentanmeldung DE 197 28 954 C1 unter Beispiel 31h) beschriebenen Gd-Komplexes der 10-(4-Carboxy-1-raethyl-2-oxo-3-azabutyl)-1,4,7,10-tetraazacyclododecan-1,4,7-triessigsäure und wasserfreies Lithiumchlorid ( 0,37 g , 8,8 mmol) werden bei 40 C° in 60 ml absolutem Dimethylsulfoxid unter Rühren gelöst und bei dieser Temperatur mit insgesamt 1,01 g (8.8 mmol) N-Hydroxysuccinimid und 6,10 g (4,0 mmol) der Titelverbindung aus Beispiel 25b), gelöst in 40 ml absolutem Dimethylsulfoxid, versetzt. Nach dem Abkühlen auf Raumtemperatur wird die Reaktionslösung mit 1,82 g ( 8,8 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Ausbeute: 9,26 g (84,0 % d. Th.; bezogen auf die eingesetzte Diaminkomponente) als farbloses Lyophilisat.
H₂O-Gehalt (Karl-Fischer): 5,89 %.

| Elementaranalyse (berechnet auf wasserfreie Substanz): | | | | | | |
|---|---|---|---|---|---|---|
| ber. | C 40,52 | H 5,16 | N 9,15 | F 11,72 | Gd 11.41 | S 1,16 |
| gef. | C 40,57 | H 5,20 | N 9,12 | F 11,69 | Gd 11,43 | S 1,18 |

### Beispiel 26

### a)6-N-t-Butyloxycarbonyl-2-N-benzyloxycarbonyl-L-lysin-[1-(4-perfluoroctylsulfonyl)-piperazin]-amid

19,02 g (50,0 mmol) α-N-(Benzyloxycarbonyl)-ε-N'-(tert.butyloxycarbonyl)-L-lysin (kommerziell erhältlich bei der Firma Bachern) werden in 150ml absolutem Tetrahydrofuran gelöst. Man gibt 8,31 g (50,0 mmol) Carbonyldiimidazol und 5,o3g (50,0 mmol) Triethylamin, gelöst in 75 ml trockenem Tetrahydrofuran,bei 0°C tropfenweise hinzu und lässt 10 Minuten bei dieser Temperatur nachrühren. Anschließend tropft man eine Lösung von 48,42 g (50,0 mmol ) Perfluoroctylsulfonylpiperazin und 5,03 g (50,0 mmol) Triethylamin in 250 ml trockenem Tetrahydrofuran bei 0°C hinzu. Nach Rühren über Nacht zieht man das Tetrahydrofuran im Vakuum ab und das verbleibenden Öl wird wird an Kieselgel chromatographiert (Laufmittel: n-Hexan / Isopropanol 15: 1). Man erhält 49,48 g (80,4 % d. Th., bezogen auf eingesetztes sec.- Amin) der Titelverbindung in Form eines farblosen Öls.

| Elementaranalyse (berechnet auf wasserfreie Substanz): | | | | | |
|---|---|---|---|---|---|
| ber. | C 40,01 | H 3,79 | N 6,02 | F 34,70 | S 3,45 |
| gef. | C 40,07 | H 3,82 | N 6,02 | F 34,67 | S 3,48 |

### b) 6-N-t-Butyloxycarbonyl-L-lysin-[1-(4-perfluoroctylsulfonyl)-piperazin]-amid

30,0g (32,2 mmol) der Titelverbindung aus Beispiel 26a) werden in 300ml Isopropanol gelöst und mit 1,5 g Pearlman-Katalysator (20%Palladiumhydroxid auf Kohle) versetzt.Man hydriert für 10 Stunden bei Raumtemperatur, wobei nach Kontrolle des Reaktionsverlaufes mittels Dünnschichtchromatographie die Hydrogenolytische Abspaltung der Benzyloxycarbonyl-Schutzgruppe nach der oben genannten Reaktionszeit bereits quantitativ erfolgt ist. Man filtriert vom Katalysator ab und engt das Filtrat im Vakuum zur Trockne ein. Der verbliebene Rückstand wird an Kieselgel chromatographiert.
(Laufmittel:n-Hexan/Isopropanol 25:1). Man erhält 25,13g (98,0%d.Th.) der Titelverbindung in Form eines farblosen Öls.

| Elementaranalyse: | | | | | |
|---|---|---|---|---|---|
| ber. | C 34,68 | H 3,67 | F 40,55 | N 7,03 | S 4,03 |
| gef. | C 34,72 | H 3,70 | F 40,60 | N 7,01 | S 3,98 |

### c) 6-N-t-Butyloxycarbonyl-2-N-[1-S-α-D-(2-carbonyl)-ethyl-2,3,4,6-tetra-O-acetylmannopyranose]-L-lysin-[1-(4-perfluoroctylsulfonyl)-piperazin]-amid

In 300ml trockenem Tetrahydrofuran werden 15,53 g (35,60 mmol) der 3-(2,3,4,6-Tetra-Oacetyl-1-thio-α-D-mannopyranosyl)-propionsäure (Darstellung gemäß: J. Haensler etal., Bioconjugate Chem. 4,85 , (1993); Chipowsky,S.,and Lee,Y.C (1973),Synthesis of 1-thio-aldosides; Carbohydrate Research 31,339-346 , sowie 3,60 g (35,60 mmol) Triethylamin gelöst. Nach dem Abkühlen der Reaktionslösung auf -15°C bis -20°C tropft man bei dieser Temperatur unter Rühren eine Lösung von 4,92g (35,60 mmol) Chlorameisensäureisobutylester in 75 ml trockenem Tetrahydrofuran langsam hinzu, wobei die Zutropfgeschwindigkeit so zu wählen ist, daß eine Innentemperatur von -10°C nicht überschritten wird.Nach einer Reaktionszeit von 15 Minuten bei -15°C tropft man anschließend eine Lösung von 28,35 g (35,60 mmol) der Titelverbindung aus Beispiel 22b) und 3,60 g (35,60 mmol) Triethylamin,in 200ml trockenem Tetrahydrofuran bei 20°C langsam hinzu. Nach einer Reaktionszeit von einer Stunde bei -15°C sowie zwei Stunden bei Raumtemperatur wird die Reaktionslösung im Vakuum bis zur Trockene eingeengt. Der verbleibende Rückstand wird in 250 ml Essigsäureethylester aufgenommen und zweimal mit je 100 ml gesättigter Natriumhydrogencarbonatlösung sowie einmal mit 200 ml Wasser gewaschen. Nach dem trocknen der organischen Phase über Natriumsulfat wird vom Salz abgesaugt und der Essigsäureethylester im Vakuum abgezogen. Der verbleibende ölige Rückstand wird an Kieselgel unter Verwendung von Essigsäureethylester / n-Hexan (1:25) als Eluent gereinigt.
Ausbeute: 34,21 g (79,1 % d.Th.) der oben genannten Titelverbindung als farbloses und stark viskoses Öl.

| Elementaranalyse: | | | | | |
|---|---|---|---|---|---|
| ber. | C 39,54 | H 4,23 | N 4,61 | F 26,58 | S 5,28 |
| gef. | C 39,49 | H 4,21 | N 4,59 | F 26,52 | S 5,31 |

### d) 6-N-t-Butyloxycarbonyl-2-N-[1-S-α-D-(2-carbonyl)-ethyl-mannopyranose]-L-lysin-[1-(4-perfluoroctylsulfonyl)-piperazin]-amid

29,93 g (24,64 mmol) der Titelverbindung aus Beispiel 26c) werden in 400 ml absolutem Methanol suspendiert und bei 5 °C mit einer katalytischen Menge Natriummethanolat versetzt. Nach einer Reaktionszeit von 3h bei Raumtemperatur zeigt die dünnschichtchromatographische Kontrolle (Eluent: Chloroform/Methanol = 9:1) des Reaktionsverlaufs bereits quantitative Umsetzung an. Zum Zwecke der Aufarbeitung wird die nun klare Reaktionslösung durch Versetzen mit Amberlite® IR 120 (H⁺-Form)-Kationenaustauscherharz neutralisiert,vom Austauscher abgesaugt und das so erhaltene methanolische Filtrat im Vakuum bis zur Trockne abgezogen. Der erhaltene amorphe Rückstand wird durch Chromatographie an Kieselgel unter Verwendung von 2-Propanol/Essigsäureethylester / n-Hexan (1:1:15) als Eluent gereinigt.
Ausbeute: 23,42 g (90,8 % d.Th.) eines farblosen und viskosen Öls.

| Elementaranalyse : | | | | | |
|---|---|---|---|---|---|
| ber. | C 36,72 | H 4,14 | N 5,35 | F 30,85 | S 6,13 |
| gef. | C 36,69 | H 4,11 | N 5,35 | F 30,82 | S 6,11 |

### e) 2-N-[ 1-S-α-D-(2-carbonyl)-ethyl-mannopyranose]-L-lysin-[1-(4-perfluoroctylsulfonyl)-piperazin]-amid

20,93 g (20,0 mmol) Titelverbindung aus Beispiel 26d) werden in einem Gemisch aus 50 ml Trifluoressigsäure und 100 ml Dichlormethan bei 0°C unter kräftigem Rühren gelöst und für 10 Minuten bei dieser Temperatur gerührt. Anschließend dampft man im Vakuum zur Trockne ein und nimmt den Rückstand in 150 ml Wasser auf. Der pH-Wert dieser wässrigen Produktlösung wird durch die tropfenweise Zugabe von 2 molarer wässriger Natronlauge auf 9,5 eingestellt. Die wässrige Produktlösung wird über eine AMICON® YM-3 Ultrafiltrationsmembran (cut off : 3000 Da) entsalzt und dabei gleichzeitig von möglichen, noch vorhandenen , niedermolekularen Bestandteilen gereinigt. Das Retentat wird anschließend gefriergetrocknet.
Ausbeute: 17,79 g (94,2 % d.Th.) des freien Amins als farbloses Lyophilisat.
H₂O-Gehalt (Karl-Fischer): 3,09 %.

| Elementaranalyse (berechnet auf wasserfreie Substanz): | | | | | |
|---|---|---|---|---|---|
| ber. | C 34,26 | H 3,73 | N 5,92 | F 34,12 | S 6,77 |
| gef. | C 34,26 | H 3,79 | N 5,88 | F 34,07 | S 6,80 |

### f) 2-N-[1-S-α-D-(2-carbonyl)-ethyl-mannopyranose]-6-N-[1,4,7-tris(carboxylatomethyl)-10-(3-aza-4-oxo-5-methyl-5-yl-pentanoyl)-1,4,7,10-tetraazacyclododecan]-L-lysin-[1-(4-perfluoroctylsulfonyl)-piperazin]-amid, Gadolinium-Komplex

5,54 g [(8,8 mmol, 2,2 Molequivalente bezogen auf die eingesetzte Aminkomponente aus Beispiel 26e)] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,37 g wasserfreies Lithiumchlorid (8,8 mmol) werden bei 40°C in 60 ml absolutem Dimethylsulfoxid unter Rühren gelöst und bei dieser Temperatur mit insgesamt 1,01 g (8,8 mmol) N-Hydroxysuccinimid und 3,78 g (4,0 mmol) der Titelverbindung aus Beispiel 26e), gelöst in 40 ml absolutem Dimethylsulfoxid, versetzt. Nach dem Abkühlen auf Raumtemperatur wird die Reaktionslösung mit 1,82 g (8,8 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, in Wasser aufgenommen, von unlöslichem Dicyclohexylharnstoff abfiltriert und das Filtrat über eine AMICON® YM-3 Ultrafiltrationsmembran (cut off 3000 Da) entsalzt und von niedermolekularen Bestandteilen gereinigt. Das Retentat wird anschließend gefriergetrocknet. Ausbeute: 5,17 g (83,0 % d.Th.) als farbloses Lyophilisat.
H₂O-Gehalt (Karl-Fischer): 4,43 %.

| Elementaranalyse (berechnet auf wasserfreie Substanz): | | | | | | |
|---|---|---|---|---|---|---|
| ber. | C 35,45 | H 4,07 | N 8,09 | F 20,72 | Gd 10,09 | S 4,11 |
| gef. | C 35,50 | H 4,01 | N 8,12 | F 20,68 | Gd 10,13 | S 4,14 |

### Beispiel 27

### a) 6-N-Benzyloxycarbonylr-2-N-(1-O-β-D-carbonylmethyl-2,3,4,6-tetra-Obenzylglucopyranose)- L-lysin-[1-(4-perfluoroetylsulfonyl)-piperazin]-amid

8,02 g (13,4 mmol) der in der Patentanmeldung DE 197 28 954 C1 unter Beispiel 46a) beschriebenen Titelverbindung [1-Carboxymethyloxy-2,3,4,6-tetra-O-benzyl-β-Dglucopyranosid] und 3,24 g (28,14 mmol) N-Hydroxysuccinimid werden in 100 ml Dimethylformamid gelöst und bei 0°C mit insgesamt 5,80 g (28,14 mmol) N,N'-Dicyclohexylcarbodiimid portionsweise versetzt. Es wird 3 Stunden bei dieser Temperatur nachgerührt. Zu der so hergestellten Aktivesterlösung gibt man eine auf 0°C gekühlte Lösung von 11,13 g (13,4 mmol) der Titelverbindung aus Beispiel 1c), gelöst in 50 ml Dimethylformamid, tropfenweise hinzu und rührt 2 Stunden bei 0°C sowie 12 Stunden bei Raumtemperatur. Zur Aufarbeitung filtriert man vom ausgefallenen Dicyclohexylharnstoff ab und zieht das Lösungsmittel anschließend bis zur Trockne ab. Der so erhaltene Rückstand wird anschließend an Kieselgel chromatographiert (Laufmittel: Dichlormethan/Ethanol, 20:1; die Durchführung der Chromatographie erfolgte unter Verwendung eines Solvensgradienten mit kontinuierlicher Zunahme des Ethanolgehalts).
Ausbeute: 12,67 g (67,0 % d.Th.) der Titelverbindung in Form eines farblosen und stark viskosen Öls.

| Elementaranalyse: | | | | | |
|---|---|---|---|---|---|
| ber. | C 52,77 | H 4,50 | N 3,97 | F 22,89 | S 2,27 |
| gef. | C 52,75 | H 4,61 | N 3,98 | F 22,94 | S 2,26 |

### b) 2-N-(1-O-β-D-carbonylmethyl-glucopyranose)- L-lysin-[1-(4-perfluoroctylsulfonyl)-piperazin]-amid

In 100 ml Ethanol werden 11,52 g (8,17 mmol) der unter 27a) hergestellten Verbindung gelöst, mit 0,5 g Pearlman-Katalysator (Pd 20% , C) versetzt und solange bei Raumtemperatur unter einer Wasserstoffatmosphäre (1atm) hydriert, bis keine Wasserstoffaufnahme mehr zu beobachten ist. Man saugt vom Katalysator ab,wäscht gründlich mit Ethanol nach (dreimal mit jeweils ca. 40ml) und engt im Vakuum zur Trockne ein. Die Titelverbindung wird als stark viskoses und farbloses Öl erhalten.
Ausbeute: 7,36 g (98,4 % d.Th.).

| Elementaranalyse: | | | | | |
|---|---|---|---|---|---|
| ber. | C 34,07 | H 3,63 | N 6,11 | F 35,24 | S 3,50 |
| gef. | C 34,11 | H 3,59 | N 6,08 | F 35,23 | S 3,52 |

### c) 2-N-(1-O-β-D-carbonylmethyl-glucopyranose)-6-N-[1,4,7-tris(carboxylatomethyl)-10-(aza-4-oxo-5-methyl-5-yl-pentanoyl)-1,4,7,10-tetraazacyclododecan]- L-lysin-[1-(4-perfluoroctylsulfonyl)-piperazin]-amid, Gd-Komplex ,

9,98 g [(15,84 mmol; 2,2 Molequivalente bezogen auf die eingesetzte Aminkomponente aus Beispiel 27b)] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,67 g (15,84 mmol) wasserfreies Lithiumchlorid werden bei 40°C in 80 ml absolutem Dimethylsulfoxid unter Rühren gelöst und bei dieser Temperatur mit insgesamt 1,82 g (15,84 mmol) N-Hydroxysuccinimid und 7,25 g (7,19 mmol) der Titelverbindung aus Beispiel 27b), gelöst in 30 ml absolutem Dimethylsulfoxid,versetzt. Nach dem Abkühlen auf Raumtemperatur wird die Reaktionslösung mit 3,27 g (15,84 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, in Wasser aufgenommen, von unlöslichem Dicyclohexylharnstoff abfiltriert und das Filtrat über eine AMICON® YM-3 Ultrafiltrationsmembran (cut off :3000Da) entsalzt und von niedermolekularen Bestandteilen gereinigt. Das Retentat wird anschließend gefriergetrocknet.
Ausbeute: 9,11 g (83,0 %d.Th.) als farbloses Lyophilisat.
H₂O-Gehalt (nach Karl-Fischer) : 4,02 %.

| Elementaranalyse (berechnet auf wasserfreie Substanz): | | | | | | |
|---|---|---|---|---|---|---|
| ber. | C 35,37 | H 4,02 | N 8,25 | F 21,13 | S 2,10 | Gd 10,29 |
| gef. | C 35,42 | H 4,07 | N 8,18 | F 21,09 | S 2,06 | Gd 10,34 |

### Beispiel 28

### a) 2-N-Trifluoracetyl-L-lysin-[1-(4-perfluoroctylsulfonyl)-piperazin]-amid

In 100 ml Ethanol werden 10,0 g (11,46 mmol) der unter 1b) hergestellten Verbindung gelöst, mit 1,0 g Pearlman- Katalysator (Pd 20% / C) versetzt und bis zur quantitativen Aufnahme an Wasserstoff hydriert.Man saugt vom Katalysator ab, wäscht mit Ethanol nach und engt im Vakuum zur Trockne ein. Die Titelverbindung wird als zähes und farbloses Öl erhalten. Ausbeute: 8,85 g (97,5 %d.Th.).

| Elementaranalyse: | | | | | |
|---|---|---|---|---|---|
| ber. | C 30,31 | H 2,54 | N 7,07 | F 47,95 | S 4,05 |
| gef. | C 30,36 | H 2,50 | N 7,11 | F 47,99 | S 4,00 |

### b) 2-N-Trifluoracetyl-6-N-[1-O-α-D-(5-carbonyl)-pentyl-2,3,4,6-tetra-O-benzylmannopyranose]-L-lysin-[1-(4-perfluoroctylsulfonyl)-piperazin]-amid

In eine auf 0°C gekühlte Lösung aus 29,0 g (36,6 mmol) der Titelverbindung aus Beispiel 28a) und 4,05 g (40,26 mmol) Triethylamin in 100 ml Dimethylformamid tropft man eine Lösung von 27,51 g (36,6 mmol) der Titelverbindung aus Beispiel 17c) in 150ml Dimethylformamid hinzu. Nach beendeter Zugabe rührt man noch eine Stunde bei 0°C nach und dann über Nacht bei Raumtemperatur. Es wird im Vakuum zur Trockne eingedampft und der Rückstand in 300 ml Essigsäureethylester aufgenommen. Man filtriert von unlöslichen Bestandteilen ab und wäscht das Filtrat zweimal mit je 100 ml 5%iger wässriger Soda-Lösung. Die organische Phase wird über Magnesiumsulfat getrocknet und im Vakuum zur Trockne eingedampft. Der Rückstand wird an Kieselgel chromatographiert (Laufmittel: n-Hexan/ Isopropanol 25:1). Man erhält 42,05 g (80,4 % d.Th.) der Titelverbindung in Form eines farblosen Öls.

| Elementaranalyse: | | | | | |
|---|---|---|---|---|---|
| ber. | C 50,42 | H 4,51 | N 7,96 | F 26,59 | S 2,24 |
| gef. | C 50,38 | H 4,50 | N 7,91 | F 26,62 | S 2,20 |

### c) 6-N-[1-O-α-D-(5-Carbonyl)-pentyl-2,3,4,6-tetra-O-benzyl-mannopyranose]-L-lysin-[1-(4-perfluoroctylsulfonyl)-piperazin]-amid

In 150ml Ethanol werden 20,0 g (14,0 mmol) der unter Beispiel 28b) hergestellten Verbindung gelöst. Man gibt dann die Lösung von 2,8 g (70,0 mmol) Natriumhydroxid in 25 ml destilliertem Wasser dazu und rührt 0,5 Stunden bei 50°C. Nach dem Dünnschichtchromatogramm ist die Schutzgruppenabspaltung zu diesem Zeitpunkt bereits quantitativ erfolgt. Man engt im Vakuum zur Trockene ein und entfernt Spuren von Wasser durch mehrmaliges Kodestillieren mit Ethanol. Der Rückstand wird an Kieselgel chromatographiert (Laufmittel: n-Hexan/ Isopropanol 20:1). Man erhält 16,66 g (89,3% d.Th.) der Titelverbindung in Form eines farblosen Öls.

| Elementaranalyse: | | | | | |
|---|---|---|---|---|---|
| ber.: | C 52,25 | H 4,91 | N 4,20 | F 24,22 | S 2,41 |
| gef.: | C 52,30 | H 4,90 | N 4,18 | F 24,22 | S 2,38 |

### d) 6-N-[1-O-α-D-(5-Carbonyl)-pentyl-mannopyranose]-L-lysin-[1-(4-perfluoroctylsulfonyl)-piperazin]-amid

In 150 ml eines 10:1 Gemisches aus Ethanol und Wasser werden 15,0 g (11,25 mmol) der unter 28c) hergestellten Verbindung gelöst und mit 1,0 g Pearlman- Katalysator (Pd 20% / C) versetzt. Anschließend wird bis zur quantitativen Aufnahme an Wasserstoff bei Raumtemperatur und unter einer Atmosphäre Wasserstoffdruck hydriert. Man saugt vom Katalysator ab, wäscht mit Ethanol/Wasser (10:1) nach und engt im Vakuum zur Trockne ein. Die Titelverbindung wird als zähes und farbloses Öl erhalten.
Ausbeute: 10,77 g (98,4 %d.Th.).

| Elementaranalyse: | | | | | |
|---|---|---|---|---|---|
| ber. | C 37,04 | H 4,25 | N 5,76 | F 33,20 | S 3,30 |
| gef. | C 37,06 | H 4,20 | N 5,81 | F 33,19 | S 3,30 |

### e) 6-N-[1-O-α-D-(5-Carbonyl)-pentyl-mannopyranose]-2-N-[1,4,7-tris(carboxylatomethyl)-10-(3-aza-4-oxo-5-methyl-5-yl-pentanoyl)-1,4,7,10-tetraazacyclododecan]-L-lysin-[1-(4-perfluoroctylsulfonyl)-piperazin]-amid, Gd-Komplex

5,54 g [(8,8 mmol; 2,2 Molequivalente bezogen auf die eingesetzte Aminkomponente aus Beispiel 28d)] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,37 g (8,8 mmol) wasserfreies Lithiumchlorid werden bei 40° C in 60 ml absolutem Dimethylsulfoxid unter Rühren gelöst und bei dieser Temperatur mit insgesamt 1,01 g (8,8 mmol) N-Hydroxysuccinimid und 3,89 g (4,0 mmol) der Titelverbindung aus Beispiel 28d), gelöst in 60ml absolutem Dimethylsulfoxid, versetzt. Nach dem Abkühlen auf Raumtemperatur wird die Reaktionslösung mit 1,82 g (8,8 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, in Wasser aufgenommen, von unlöslichem Dicyclohexylharnstoff abfiltriert und das Filtrat über eine AMICON® YM-3 Utrafiltrationsmembran (cut off : 3000Da) entsalzt und von niedermolekularen Bestandteilen gereinigt. Das Retentat wird anschließend gefriergetrocknet. Ausbeute: 4,81 g (75,9 %d.Th.) als farbloses Lyophilisat.
H₂O-Gehalt (Karl-Fischer): 8,98 %.

| Elementaranalyse (berechnet auf wasserfreie Substanz): | | | | | | |
|---|---|---|---|---|---|---|
| ber. | C 37,15 | H 4,39 | N 7,96 | F 20,38 | Gd 9,92 | S 2,02 |
| gef. | C 37,27 | H 4,40 | N 8,02 | F 20,31 | Gd 10.00 | S 1,98 |

### Beispiel 29

### a) 1,7-Bis(benzyloxycarbonyl)-4-(1-O-β-D-carbonylmethyl-2,3,4,6-tetra-O-benzylgalactopyranose)-10-{3-oxa-pentan-1,5-dicarbonsäure-1-oyl-5-[1-(4-perfluoroctylsulfonyl)-piperazin]-amid}-1,4,7,10-tetraazacyclododecan

In eine Lösung aus 27,0 g (24,4 mmol) des unter Beispiel 15a) hergestellten sec. Amins, in einem Gemisch aus 150 ml Tetrahydrofuran und 15 ml Chloroform, werden bei 0°C und unter Stickstoffatmosphäre 35,80 g (25,0 mmol) der Titelverbindung aus Beispiel 17e), gelöst in 250 ml Tetrahydrofuran, hinzugegeben.Anschließend gibt man bei 0°C insgesamt 18,0 g (36,6 mmol) EEDQ [2-Ethoxy-1-ethoxycarbonyl-1,2-dihydrochinolin] portionsweise hinzu und läßt über Nacht bei Raumtemperatur rühren. Man engt anschließend im Vakuum zur Trockene ein und das verbleibende Öl wird an Kieselgel chromatographiert (Laufmittel: n-Hexan/Isopropanol 20:1). Man erhält 32,11 g (78,0 % d. Th. , bezogen auf eingesetztes sec.- Amin) der Titelverbindung in Form eines farblosen Öls.

| Elementaranalyse: | | | | | |
|---|---|---|---|---|---|
| ber. | C 54,09 | H 4,72 | F 19,14 | N 4,98 | S 1,90 |
| gef. | C 54,12 | H 4,77 | F 19,17 | N 5,03 | S 1,90 |

### b) 1-(1-O-β-D-carbonylmethyl-galactopyranose)-7-{3-oxa-pentan-1,5-dicarbonsäure-1-oyl-5-[1-(4-perfluoroctylsulfonyl)-piperazin]-amid}-1,4,7,10-tetraazacyclododecan

In 250 ml Ethanol werden 30,0 g (17,77 mmol) der unter Beispiel 29a) hergestellten Titelverbindung gelöst und mit 3,0 g Pearlman-Katalysator (Pd 20 %, / C) versetzt. Man hydriert bis zur quantitativen Aufnahme an Wasserstoff, saugt dann vom Katalysator ab, wäscht mit Ethanol gut nach und engt im Vakuum zur Trockene ein. Das Produkt wird als gelblich gefärbtes und äusserst viskoses Öl erhalten.
Ausbeute: 17,89 g (95,1 % d. Th.).

| Elementaranalyse: | | | | | |
|---|---|---|---|---|---|
| ber. | C 36,30 | H 4,09 | F 30,50 | N 7,94 | S 3,03 |
| gef. | C 36,26 | H 4,12 | F 30,46 | N 7,90 | S 3,04 |

### c) 1-(1-O-β-D-carbonylmethyl-galactopyranose)-7-{3-oxa-pentan-1,5-dicarbonsäure-1-oyl-5-[1-(4-perfluoroctylsulfonyl)-piperazin]-amid}-4,10-bis[1,4,7-tris(carboxylatomethyl)-10-(3-aza-4-oxo-5-methyl-5-yl-pentanoyl)-1,4,7,10-tetraazacyclododecan]-1,4,7,10-tetraazacyclododecan, di-Gadolinium-Komplex

5,54 g [8,8 mmol; 4,4 Molequivalente bezogen auf die eingesetzte Aminkomponente aus Beispiel 29b) ] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,37 g (8,8 mmol) wasserfreies Lithiumchlorid werden bei 40 C° in 60 ml absolutem Dimethylsulfoxid unter Rühren gelöst und bei dieser Temperatur mit insgesamt 1,01 g (8.8 mmol) N-Hydroxysuccinimid und 2,11 g (2,0 mmol) der Titelverbindung aus Beispiel 29b), gelöst in 25 ml absolutem Dimethylsulfoxid, versetzt. Nach dem Abkühlen auf Raumtemperatur wird die Reaktionslösung mit 1,82 g (8,8 mmol) N,N'-Dicyclohexylcarbodiimid versetzt und 12 Stunden bei Raumtemperatur gerührt. Die erhaltene Suspension wird anschließend mit ausreichend Aceton bis zur vollständigen Fällung der oben genannten Titelverbindung versetzt, der Niederschlag abgesaugt, getrocknet, in Wasser aufgenommen , von unlöslichem Dicyclohexylharnstoff abfiltriert und das Filtrat über eine AMICON® YM-3 Ultrafiltrationsmembran (cut off : 3000 Da) entsalzt und von niedermolekularen Bestandteilen gereinigt. Das Retentat wird anschließend gefriergetrocknet.
Ausbeute: 3,29 g (72,2 % d. Th.; bezogen auf die eingesetzte Aminkomponente) als farbloses Lyophilisat.
H₂O-Gehalt (Karl-Fischer): 5,99 %.

| Elementaranalyse (berechnet auf wasserfreie Substanz): | | | | | | |
|---|---|---|---|---|---|---|
| ber. | C 36,84 | H 4,37 | N 9,82 | F 14,15 | Gd 19,63 | S 1,40 |
| gef. | C 36,87 | H 4,40 | N 9,82 | F 14,09 | Gd 19,59 | S 1,38 |

### Beispiel 30

### a) 3-(1-O-α-D-2,3,4,6-Tetra-O-benzyl-mannopyranose)-2-N-benzyloxycarbonyl-L-serinmethylester

In 500 ml trockenem Acetonitril werden 21,42 g (39,61 mmol) 2,3,4,6-Tetra-O-benzyl- α-Dmannopyranose (Darstellung gemäß: F. Kong et al., J. Carbohydr. Chem. ; 16;-6 ; 1997 ; 877-890 ) gelöst. Nach dem Abkühlen der Reaktionslösung auf 5°C tropft man bei dieser Temperatur unter Rühren langsam eine Lösung von 13,23 g (59,52 mmol) Trifluormethansulfonsäure-trimethylsilylester in 30 ml Acetonitril , gefolgt von einer Lösung aus 20,06 g (79,21 mmol) N-Benzyloxycarbonyl-L-serinmethylester (kommerziell erhältlichbei der Firma Bachem) in 50 ml Acetonitril , hinzu , wobei die Zutropfgeschwindigkeit so zu wählen ist, daß eine Innentemperatur von 10°C nicht überschritten wird. Nach einer Reaktionszeit von 15 Stunden bei Raumtemperatur wird die Reaktionslösung im Vakuum bis zur Trockne eingeengt. Der verbleibende Rückstand wird in 250 ml Essigsäureethylester aufgenommen und zweimal mit je 100 ml gesättigter Natriumhydrogencarbonatlösung sowie einmal mit 200 ml Wasser gewaschen. Nach dem Trocknen der organischen Phase über Natriumsulfat wird vom Salz abgesaugt und der Essigsäureethylester im Vakuum abgezogen. Der verbleibende ölige Rückstand wird an Kieselgel unter Verwendung von Essigsäureethylester / n- Hexan (1:5) als Eluent gereinigt. Ausbeute: 23,60 g (76,8 % d.Th.) der oben genannten Titelverbindung als farbloses Öl.

| Elementaranalyse: | | | |
|---|---|---|---|
| ber. | C 71,21 | H 6,37 | N 1,81 |
| gef. | C 71,19 | H 6,41 | N 1,79 |

### b) 3-(1-O-α-D-2,3,4,6-Tetra-O-benzyl-mannopyranose)-2-N-benzyloxycarbonyl-L-serin

In einem Gemisch bestehen aus 20 ml Methanol, 20 ml Wasser und 50 ml Tetrahydrofuran werden 10,0 g (12,90 mmol) der unter Beispiel 30a) hergestellten Verbindung gelöst. Man gibt dann 0,47 g (19,35 mmol) Lithiumhydroxid, gelöst in in 25 ml destilliertem Wasser, bei Raumtemperatur hinzu und rührt anschließend für 6 Stunden bei 60°C. Nach Kontrolle des Reaktionsverlaufes mittels Dünnschichtchromatographie (Eluent: Methylenchlorid / Methanol 10:1) ist die Verseifung des Methylesters aus Beispiel 30a) nach oben genannter Reaktionszeit bereits quantitativ erfolgt. Zum Zwecke der Aufarbeitung engt man die Produktlösung im Vakuum zur Trockne ein und nimmt den verbleibenden Rückstand in 250 ml Essigsäureethylester in der Wärme auf (ca. 60°C). Anschließend wird die so erhaltene Essigsäureethylesterphase zweimal mit jeweils 50 ml einer 15 %igen wässrigen Salzsäure gewaschen , sowie einmal mit 100 ml destilliertem Wasser. Die organische Phase wird über Magnesiumsulfat getrocknet, filtriert und im Vakuum zur Trockne eingedampft. Der Rückstand wird an Kieselgel chromatographiert (Laufmittel: n-Hexan/ Essigsäureethylester 5:1). Man erhält 8,40 g (85,7 % d.Th.) der Titelverbindung in Form eines farblosen Öls.

| Elementaranalyse: | | | |
|---|---|---|---|
| ber. | C 70,94 | H 6,22 | N 1,84 |
| gef. | C 70,97 | H 6,30 | N 1,78 |

### c) 3-(1-O-α-D-2,3,4,6-Tetra-O-benzyl-mannopyranose-2-N-benzyloxycarbonyl-L-serin-[1-(4-perfluoroctylsulfonyl)piperazin]-amid

Zu 13,86 g (24,40 mmol) 1- Perfluoroctylsulfonylpiperazin (hergestellt nach DE 19603033 ) , gelöst in einem Gemisch aus 150 ml Tetrahydrofuran und 15 ml Chloroform, werden bei 0°C und unter Stickstoffatmosphäre 20,57 g (27,0 mmol) der nach Beispiel 30b) dargestellten Carbonsäure, gelöst in 50 ml Tetrahydrofuran , tropfenweise hinzugegeben. Anschließend gibt man bei 0°C insgesamt 18,0 g (36,60 mmol) EEDQ [2-Ethoxy-1-ethoxycarbonyl-1,2-dihydrochinolin] portionsweise hinzu und läßt über Nacht bei Raumtemperatur rühren. Zum Zwecke der Aufarbeitung engt man die Reaktionslösung im Vakuum ein und chromatographiert das zurückbleibende, äußerst viskose Öl an Kieselgel unter Verwendung eines n-Hexan / Isopropanol (15:1) - Gemisches als Eluentsystem. Man erhält 17,0 g (79,6 % d. Th., bezogen auf eingesetztes prim.- Amin) der Titelverbindung in Form eines farblosenund viskosen Öls.

| Elementaranalyse: | | | | | |
|---|---|---|---|---|---|
| ber. | C 51,53 | H 4,23 | N 3,15 | F 25,65 | S 2,41 |
| gef. | C 51,48 | H 4,27 | N 3,10 | F 25,71 | S 2,35 |

### d) 3-(1-O-α-D-Mannopyranose)-L-serin-[1-(4-perfluoroctylsulfonyl)piperazin]-amid

In 200 ml Ethanol werden 15,0 g (11,41 mmol) der gemäß Beispiel 30c) dargestellten Verbindung gelöst und mit 1,5 g Pearlman-Katalysator (Pd 20 %, C) versetzt. Anschließend wird die Reaktionslösung solange bei Raumtemperatur unter einer Wasserstoffatmosphäre (1 atm) hydriert, bis keine Wasserstoffaufnahme mehr zu beobachten ist ( ca. 8 Stunden ).. Zum Zwecke der Aufarbeitung saugt vom Katalysator ab, wäscht gründlich mit Ethanol nach(zweimal mit je ca. 100 ml) und engt das produktenthaltende ethanolische Filtrat im Vakuum zur Trockene ein. Die Titelverbindung wird als stark viskoses und farbloses Öl erhalten.
Ausbeute: 8,79 g (94,0 % d. Th.).

| Elementaranalyse: | | | | | |
|---|---|---|---|---|---|
| ber. | C 30,78 | H 3,20 | N 5,13 | F 39,41 | S 3,91 |
| gef. | C 30,87 | H 3,14 | N 5,19 | F 39,50 | S 3,88 |

### e) 3-(1-O-α-D-Mannopyranose)-2-N-[1,4,7-tris(carboxylatomethyl)-10-(3-aza-4-oxo-5-methyl-5-yl-pentanoyl)-1,4,7,10-tetraazacyclododecan]-L-serin-[1-(4-perfluoroctylsulfonyl)-piperazin]-amid, Gd-Komplex

Eine gerührte Suspension von 5,7 g [9,06 mmol ; entsprechend 1,5 Molequivalenten bezüglich der eingesetzten Titelverbindung (primäres Amin) aus Beispiel 30d) ] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure in 75 ml absolutem Dimethylsulfoxid wird bei 70°C mit 0,68 g (15,9 mmol) Lithiumchlorid versetzt. Nach 30 minütigem Rühren bei 70°C wird die nun klare Reaktionslösung portionsweise mit insgesamt 1,83 g (15,9 mmol) N-Hydroxysuccinimid versetzt und das Reaktionsgemisch noch 1 Stunde bei 70 °C gehalten. Nach dem Abkühlen der Reaktionslösung auf 10°C wird mit 4,52 g (23,85 mmol) Dicyclohexylcarbodiimid versetzt und die Reaktionslösung noch 1 weitere Stunde bei 0°C, gefolgt von 12 Stunden bei 22°C, gerührt. Die so erhaltene Lösung des N-Hydroxysuccinimidesters des Gd-Komplexes der 10-(4-Carboxy-1-methyl-2-oxo-3-azabutyl)-1,4,7,10-tetraazacyclododecan-1,4,7-triessigsäure wird nun bei 22°C tropfenweise mit einer Lösung von 4,94 g (6,03 mmol) der Titelverbindung aus Beispiel 30d) , in 15 ml absolutem Dimethylsulfoxid , versetzt und weitere 12 Stunden bei Raumtemperatur nachgerührt. Zur Aufarbeitung wird die Reaktionslösung bei 22°C in ein Lösungsmittelgemisch , bestehend aus 250 ml Aceton und 250 ml 2-Propanol , langsam eingetropft, wobei sich die Titelverbindung nach 12 Stunden bei 10 °C vollständig als leicht gelblich gefärbtes Öl abgesetzt hat. Man dekantiert vorsichtig vom überstehenden Eluentgemisch ab und nimmt das ölige Produkt in 200 ml destilliertem Wasser auf, wobei dieses vollständig in Lösung geht, so daß eine leicht gelblich gefärbte wässrige Lösung der oben genannten Titelverbindung erhalten wird. Im Anschluß wird die wässrige Produktlösung zuerst über einen Membranfilter filtriert und danach , zum Zwecke des Entsalzens und der Abtrennung von niedermolekularen Bestandteilen , über eine YM3-Ultrafiltrationsmembran (AMICON ® : cut off : 3000 Da) dreimal ultrafiltriert. Das so erhaltene Retentat wird anschließend gefriergetrocknet.
Ausbeute: 8,63 g (80,2 % d. Th., bezogen auf die eingesetzte Titelverbindung aus Beispiel 30d) als farbloses Lyophilisat mit einem Wassergehalt von 7,65 %.

| Elementaranalyse (berechnet auf wasserfreie Substanz): | | | | | | |
|---|---|---|---|---|---|---|
| ber. | C 33,57 | H 3,80 | N 7,83 | F 22,57 | Gd 10,99 | S 2,24 |
| gef. | C 33,57 | H 3,76 | N 7,82 | F 22,63 | Gd 11,06 | S 2,18 |

### Beispiel 31

### a) 6-N-Benzyloxycarbonyl-2-N-[O-β-D-galactopyranosyl (1→4)-gluconosyl]-L-lysin-[1-(4-pertluoroctylsulfonyl)-piperazin]-amid

Zu einer gerührten Lösung von 4,98 g (6,0 mmol) der Titelverbindung aus Beispiel 1c) in 40 ml absolutem Dimethylsulfoxid wird bei Raumtemperatur eine Lösung von 13,3 g (37,2 mmol) O-β-D-Galactopyranosyl-(1 → 4)-D-glucono-1,5-lacton [ Lactobionolacton ; Darstellung gemäß : (a) Williams, T.J.; Plessas, N.R., Goldstein, I.J. Carbohydr. Res. 1978, 67, Cl. (b) Kobayashi, K.; Sumitomo, H.; Ina, Y. Polym. J. 1985, 17,567, (c) Hiromi Kitano,Katsuko Sohda, and Ayako Kosaka, Bioconjugate Chem. 1995, 6 131-134 ] in 40 ml absolutem Dimethylsulfoxid tropfenweise addiert. Die so erhaltene Reaktionslösung wird anschließend für 14 Stunden bei 40°C gerührt. Zur Aufarbeitung wird bei Raumtemperatur mit 500 ml absolutem 2-Propanol versetzt und der entstehende farblose Niederschlag mittels einer G4-Fritte abgesaugt und gut mit insgesamt 250 ml absolutem 2-Propanol nachgewaschen. Der so erhaltene Feststoff wird nun in 300 ml destilliertem Wasser gelöst und über eine YM3-Ultrafiltrationsmembran (AMICON® : cut off : 3000 Da) insgesamt dreimal ultrafiltriert. Durch den dreimaligen Ultrafiltrationsvorgang werden sowohl die Überschüsse an Lactobionolacton als auch ,möglicherweise noch vorhandene, niedermolekulare Bestandteile vom gewünschten Produkt abgetrennt. Der in der Ultrafiltrationsmembran verbleibende Rückstand wird im Anschluß vollständig in 300 ml destilliertem Wasser gelöst und gefriergetrocknet.
Ausbeute: 6,51 g (92,7 % d. Th.) als farbloses Lyophilisat
Wassergehalt: 10,03 %

| Elementaranalyse (berechnet auf wasserfreie Substanz): | | | | | |
|---|---|---|---|---|---|
| ber. | C 38,98 | H 4,05 | N 4,79 | F 27,58 | S 2,74 |
| gef. | C 39,04 | H 4,09 | N 4,82 | F 27,61 | S 2,71 |

### b) 2-N-[O-β-D-galactopyranosyl(1→4)-gluconosyl]-L-lysin-[1-(4-perfluoractylsulfonyl)-piperazin]-amid

In 100 ml Ethanol werden 5,0 g (4,27 mmol) der unter 31 a) hergestellten Verbindung gelöst, mit 0,5g Pearlman-Katalysator (Pd 20 %, C) versetzt und bis zur quantitativen Aufnahme von Wasserstoff bei 1 Atmosphäre Wasserstoffdruck hydriert. Man saugt vom Katalysator ab, wäscht mit Ethanol nach und engt im Vakuum zur Trockne ein. Die Titelverbindung wird als farbloses und , viskoses Öl erhalten.
Ausbeute: 4,36 g (98,5 % d. Th.).

| Elementaranalyse : | | | | | |
|---|---|---|---|---|---|
| ber. | C 34,76 | H 3,99 | N 5,40 | F 31,51 | S 3,09 |
| gef. | C 34,78 | H 4,04 | N 5,34 | F 31,51 | S 3,15 |

### c) 2-N-[O-β-D-galactopyranosyl (1→4)-gluconosyl]-6-N-[1,4,7-tris(carboxylatomethyl)-10-(3-aza-4-oxo-5-methyl-5-yl-pentanoyl)-1,4,7,10-tetraazacyclododecan]-L-lysin-[1-(4-perfluoroctylsulfonyl)-piperazin]-amid, Gd-Komplex

5,54 g [(8,8 mmol; 2,2 Molequivalente bezogen auf die eingesetzte Aminkomponente aus Beispiel 31b) ] des in der Patentanmeldung DE 197 28 954 C1 unter Beispiel 31h) beschriebenen Gd-Komplexes der 10-(4-Carboxy-1-methyl-2-oxo-3-azabutyl)-1,4,7,10-tetraazacyclododecan-1,4,7-triessigsäure und 0,37 g (8,8 mmol) wasserfreies Lithiumchlorid werden bei 40° C in 60 ml absolutem Dimethylsulfoxid unter Rühren gelöst und bei dieser Temperatur mit insgesamt 1,01 g (8,8 mmol) N-Hydroxysuccinimid und 3,85 g (4,0 mmol) der Titelverbindung aus Beispiel 31b), gelöst in 60ml absolutem Dimethylsulfoxid, versetzt. Nach dem Abkühlen auf Raumtemperatur wird die Reaktionslösung mit 1,82 g (8,8 mmol) N,N'-Dicyclohexylcarbodiimid versetzt und 12 Stunden bei Raumtemperatur gerührt. Die erhaltene Suspension wird anschließend mit ausreichend Aceton/2-Propanol (1:1) bis zur vollständigen Fällung der oben genannten Titelverbindung versetzt und der Niederschlag abgesaugt. Der so erhaltene Niederschlag wird im Anschluß in 300 ml Wasser aufgenommen und es wird vom unlöslichen Dicyclohexylharnstoff abfiltriert. Das Filtrat wird über eine AMICON® YM-3 Utrafiltrationsmembran (cut off : 3000Da) dreimal ultrafiltriert. Durch den durchgeführten dreimaligen Ultrafiltrationsvorgang werden sowohl die Überschüsse an Gd-Komplex als auch ,möglicherweise noch vorhandene, niedermolekulare Bestandteile vom gewünschten Produkt abgetrennt. Der in der Ultrafiltrationsmembran verbleibende Rückstand wird im Anschluß vollständig in 500 ml destilliertem Wasser gelöst und gefriergetrocknet.
Ausbeute: 4,64 g (70,4 %d.Th.) als farbloses Lyophilisat.
H₂O-Gehalt (Karl-Fischer): 10,08 %.

| Elementaranalyse (berechnet auf wasserfreie Substanz): | | | | | | |
|---|---|---|---|---|---|---|
| ber. | C 35,70 | H 4,22 | N 7,65 | F 19,59 | Gd 9,54 | S 1,95 |
| gef. | C 35,77 | H 4,17 | N 7,71 | F 19,61 | Gd 9,60 | S 1,99 |

### Beispiel 32

### a) 6-N-Benzyloxycarbonyl - 2-N-(2,3,4,5-pentahydroxy-hexanoyl)L-lysin-[1-(4-perfluoroctylsulfonyl)-piperazin)-amid

Zu einer Lösung aus 100,0 g (120,4 mol) der Titelverbindung aus Beispiel 1c), in 500 ml trockenem Tetrahydrofuran tropft man bei 50° C eine Lösung aus 21,45 g (120,4 mol) 5-Gluconolacton in 50 ml Tetrahydrofuran. Man rührt 3 Stunden bei 60° C und anschließend über Nacht bei Raumtemperatur. Man dampft im Vakuum zur Trockene ein und chromatographiert den Rückstand an Kieselgel.
(Laufmittel : Dichlormethan/Ethanol = 20 : 1).
Ausbeute: 98,37 g (82 % der Theorie ) eines zähen Öls.

| Elementaranalyse: | | | | | |
|---|---|---|---|---|---|
| ber. | C 38,10 | H 3,70 | F 32,02 | N 5,55 | S 3,18 |
| gef. | C 38,22 | H 3,7 9 | F 32,02 | N 5,42 | S 3,29 |

### b) 2-N-(2,3,4,5-pentahydroxy-hexanoyl)-L-lysin-1-[(4-perfluoroctylsulfonyl)-piperazin)-amid

100,9 g (100,0 mmol) der Titelverbindung aus Beispiel 32a) werden in 2000 ml Ethanol gelöst und es werden 10,0 g Palladium Katalysator (10% Pd / C ) hinzu gegeben. Man hydriert 12 Stunden bei Raumtemperatur. Man filtriert vom Katalysator ab und dampft das Filtrat im Vakuum zur Trockne ein.
Ausbeute : 87,46 g (quantitativ) eines farblosen Feststoffs.

| Elementaranalyse: | | | | | |
|---|---|---|---|---|---|
| ber. | C 32,96 | H 3,57 | N 6,41 | S 3,67 | F 36,93 |
| gef. | C 32,91 | H 3,72 | N 6,34 | S 3,50 | F 3678 |

### c) 6-N-[1,4,7-Tris(carboxylatomethyl) ]-1,4,7,10-tetraazacyclododecan-10-N-(pentanoyl-3-aza-4-oxo-5-methyl-5-yl)]-2-N-[1-O-α-D-carbonylmethyl-mannopyranose]-L-lysin-[1-(4-perfluoroctylsulfonyl)-piperazin]-amid,Gd-Komplex

50,0 g (54,55 mmol) der Titelverbindung aus Beispiel 1e), 6,28 g (54,55 mmol) N-Hydroxysuccinimid, 4,62 g (109,0 mol) Lithiumchlorid und 34,35 g (54,55 mol) 1,4,7-Tris (carboxylatomethyl)-10- (carboxy - 3-aza-4-oxo-5-methyl-pent-5-yl)-1,4,7,10-tetraazacyclododecan, Gd-Komplex, werden unter leichter Erwärmung in 400 ml Dimethylsulfoxid gelöst. Bei 10°C gibt man 16,88 g (81,8 mol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18 Laufmittel: Gradient aus Wasser / Ethanol / Acetonitril).
Ausbeute: 75,9 g (91,0 % der Theorie) eines farblosen Feststoffs.
Wassergehalt : 8,6 %.

| Elementaranalyse (auf wasserfreie Substanz berechnet): | | | | | | |
|---|---|---|---|---|---|---|
| ber. | C 35,34 | H,4,09 | N 8,24 | S 2,10 | F 21,12 | Gd 10,28 |
| gef. | C 35,28 | H 4,15 | N 8,19 | S 2,15 | F 21.03 | Gd 10,14 |

### Beispiel 33

### a) 6-N-Benzyloxycarbonyl - 2-N-(2,3,4,5-gentahydroxy-hexanoyl)L-lysin-[1-(4-perfluoroctylsulfonyl)-piperazin]-amid

Zu einer Lösung aus 100,0 g (120,4 mmol) der Titelverbindung aus Beispiel 1c), und 12,18 g (120,4 mmol) Triethylamin in 500 ml trockenem Tetrahydrofuran tropft man bei 50° C eine Lösung aus 21,45 g (120,4 mol) 5-Gluconolacton in 50 ml Tetrahydrofuran. Man rührt 3 Stunden bei 60° C und anschließend über Nacht bei Raumtemperatur. Dann gibt man 400 ml 5 % ige aqu.
Salzsäure dazu, rührt 5 Minuten bei Raumtemperatur, versetzt mit Natriumchlorid, trennt die organische Phase ab, trocknet sie über Magnesiumsulfat, dampft sie im Vakuum zur Trockene ein und chromatographiert den Rückstand an Kieselgel.
(Laufmittel : Dichlormethan/Ethanol = 20:1).
Ausbeute: 100,97 g (82 % der Theorie ) eines zähen Öls.

| Elementaranalyse: | | | | | |
|---|---|---|---|---|---|
| ber. | C 37,58 | H 3,45 | F 31,58 | N 5,48 | S 3,14 |
| gef. | C 37,72 | H 3,5 9 | F 31,72 | N 5,42 | S 3,29 |

### b) 2-N-(2,3,4,5-pentahydroxy-hexanoyl)-L-lysin-1-[(4-perfluoroctylsulfonyl)-piperazin]-amid

100,9 g (100,0 mmol) der Titelverbindung aus Beispiel 32a) werden in 2000 ml Ethanol gelöst und es werden 10,0 g Palladium Katalysator (10% Pd / C ) hinzu gegeben. Man hydriert 12 Stunden bei Raumtemperatur. Man filtriert vom Katalysator ab und dampft das Filtrat im Vakuum zur Trockne ein.
Ausbeute : 87,46 g (quantitativ) eines farblosen Feststoffs.

| Elementaranalyse: | | | | | |
|---|---|---|---|---|---|
| ber. | C 32,96 | H 3,57 | N 6,41 | S 3,67 | F 36,93 |
| gef. | C 32,91 | H 3,72 | N 6,34 | S 3,50 | F 3678 |

### c) 6-N-[1,4,7-Tris(carboxylatomethyl) ]-1,4,7,10-tetraazacyclododecan-10-N-(pentanoyl-3-aza-4-oxo-5-methyl-5-yl) ]-2-N-[1-O-α-D-carbonylmethyl-mannopyranose]-L-lysin-[1-(4-perfluoroctylsulfonyl)-piperazin]-amid,Gd-Komplex

50,0 g (54,55 mmol) der Titelverbindung aus Beispiel 1e), 6,28 g (54,55 mmol) N-Hydroxysuccinimid , 4,62 g (109,0 mol ) Lithiumchlorid und 34,35 g (54,55 mol) 1,4,7-Tris (carboxylatomethyl )-10- (carboxy - 3-aza-4-oxo-5-methyl-pent-5-yl )-1,4,7,10-tetraazacyclododecan, Gd-Komplex, werden unter leichter Erwärmung in 400 ml Dimethylsulfoxid gelöst. Bei 10°C gibt man 16,88 g (81,8 mol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18 Laufmittel: Gradient aus Wasser / Ethanol / Acetonitril).
Ausbeute: 75,9 g (91,0 % der Theorie) eines farblosen Feststoffs.
Wassergehalt : 8,6 %.

| Elementaranalyse (auf wasserfreie Substanz berechnet): | | | | | | |
|---|---|---|---|---|---|---|
| ber. | C 35,34 | H 4,09 | N 8,24 | S 2,10 | F 21,12 | Gd 10,28 |
| gef. | C 35,28 | H 4,15 | N 8,19 | S 2,15 | F 21.03 | Gd 10,14 |

Unter den Bedingungen des Beispiels 1 f wurde Mannose gegen Glucose bzw. Galactose ersetzt.

### Beispiel 34

### a) 6-N-Benzyloxycarbonyl-2-N-[1-0-α-D-carbonylmethyl-(2,3,4,6-tetra-O-benzyl glucopyranose]-L-lysin-[1-(4-perfluoroctylsulfonyl)-piperazin]-amid

Zu einer Lösung aus 100,0 g (120,4 mol) der Titelverbindung aus Beispiel 1c), 72,1 g (120,4 mol) 1-O-α-D-Carboxymethyl - 2,3,4,6-tetra-O-benzyl-glucopyranose und 13,86 g (120,4 mol) N-Hydroxysuccinimid, gelöst in 500 ml Dimethylformamid, gibt man bei 0°C 41,27g (200,0 mmol) N,N - Dicyclohexylcarbodiimid hinzu. Man rührt 3 Stunden bei 0°C und anschließend über Nacht bei Raumtemperatur. Man filtriert vom ausgefallenen Harnstoff ab, dampft das Filtrat im Vakuum zur Trockene ein und chromatographiert an Kieselgel (Laufmittel : Dichlormethan/Ethanol = 20 :1).
Ausbeute: 136,1 g (87 % der Theorie ) eines zähen Öls.

| Elementaranalyse: | | | | | |
|---|---|---|---|---|---|
| ber. | C 57,32 | H 4,89 | N 4,31 | F 24,86 | S 2,47 |
| gef. | C 57,48 | H 5,04 | N 4,20 | F 24,69 | S 2,38 |

### b) 2-N-[1-0-α-D-Carbonylmethylglucopyranose]-L-lysin-1-[(4-perfluoroctylsulfonyl)-piperazin]-amid

130,0 g (100,0 mmol) der Titelverbindung aus Beispiel 34a) werden in 2000 ml Ethanol gelöst und es werden 10,0 g Palladium Katalysator (10% Pd / C ) hinzu gegeben. Man hydriert 12 Stunden bei Raumtemperatur. Man filtriert vom Katalysator ab und dampft das Filtrat im Vakuum zur Trockne ein.
Ausbeute : 91,7 g (quantitativ) eines farblosen Feststoffs.

| Elementaranalyse: | | | | | |
|---|---|---|---|---|---|
| ber. | C 34,07 | H 3,63 | N 6,11 | S 3,50 | F 35,24 |
| gef. | C 33,92 | H 3,71 | N 6,02 | S 3,42 | F 35,33 |

### c) 6-N-[1,4,7-Tris (carboxylatomethyl) ]-1,4,7,10-tetraazacyclododecan-10-N-(pentanoyl-3-aza-4-oxo-5-methyl-5-yl) ]-2-N-[1-O-α-D-carbonylmethyl-glucopyranose]-L-lysin-[1-(4-perfluoroctylsulfonyl)-piperazin]-amid,Gd-Komplex

50,0 g (54,55 mmol) der Titelverbindung aus Beispiel 34b) , 6,28 g (54,55 mmol) N-Hydroxysuccinimid , 4,62 g (109,0 mol ) Lithiumchlorid und 34,35 g (54,55 mol) 1,4,7-Tris (carboxylatomethyl)-10- (carboxy - 3-aza-4-oxo-5-methyl-pent-5-yl)-1,4,7,10-tetraazacyclododecan, Gd-Komplex, werden unter leichter Erwärmung in 400 ml Dimethylsulfoxid gelöst. Bei 10°C gibt man 16,88 g (81,8 mol) N,N-Dicyclohexytcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18 Laufmittel: Gradient aus Wasser / Ethanol / Acetonitril).
Ausbeute: 75,9 g (91,0 % der Theorie) eines farblosen Feststoffs.
Wassergehalt : 8,6 %.

| Elementaranalyse (auf wasserfreie Substanz berechnet): | | | | | | |
|---|---|---|---|---|---|---|
| ber. | C 35,34 | H 4,09 | N 8,24 | S 2,10 | F 21,12 | Gd 10,28 |
| gef. | C 35,26 | H 4,18 | N 8,14 | S 2,158 | F 21.01 | Gd 10,13 |

### Beispiel 35

### a) 6-N-Benzyloxycarbonyl - 2-N-[1-0-α-D -carbonylmethyl-(2,3,4,6-tetra-O-benzylgalactopyranose]-L-lysin-[1-(4-perfluoroctylsulfonyl)-piperazin]-amid

Zu einer Lösung aus 50,0 g (60,2 mol) der Titelverbindung aus Beispiel 1c), 36,05 g (60,2 mmol) 1-O-α-D-Carboxymethyl - 2,3,4,6-tetra-O-benzyl-galactopyranose und 6,93 g (60,2 mmol) N-Hydroxysuccinimid, gelöst in 500 ml Dimethylformamid , gibt man bei 0°C 20,64 g (100,0 mmol ) N,N - Dicyclohexylcarbodiimid hinzu. Man rührt 3 Stunden bei 0°C und anschließend über Nacht bei Raumtemperatur. Man filtriert vom ausgefallenen Harnstoff ab, dampft das Filtrat im Vakuum zur Trockene ein und chromatographiert an Kieselgel. (Laufmittel: Dichlormethan/Ethanol = 20 :1).
Ausbeute: 68,1 g (87 % der Theorie ) eines zähen Öls.

| Elementaranalyse: | | | | | |
|---|---|---|---|---|---|
| ber. | C 57,32 | H 4,89 | N 4,31 | F 24,86 | S 2,47 |
| gef. | C 57,47 | H 5,05 | N 4,19 | F 24,72 | S 2,29 |

### b) 2-N-[1-0-α-D-Carbonylmethyl-galactopyranose]-L-lysin-1-[(4-perfluoroctylsulfonyl)-piperazin]-amid

65,0 g 50,0 mmol) der Titelverbindung aus Beispiel 35a) werden in 1000 ml Ethanol gelöst und es werden5,0 g Palladium Katalysator (10% Pd / C ) hinzu gegeben. Man hydriert 12 Stunden bei Raumtemperatur. Man filtriert vom Katalysator ab und dampft das Filtrat im Vakuum zur Trockne ein.
Ausbeute : 45,85 g (quantitativ) eines farblosen Feststoffs.

| Elementaranalyse: | | | | | |
|---|---|---|---|---|---|
| ber. | C 34,07 | H 3,63 | N 6,11 | S 3,50F | 35,24 |
| gef. | C 33,93 | H 3,74 | N 6,01 | S 3,39 | F 35,05 |

### c) 6-N-[1,4,7-Tris (carboxylatomethyl)]-1,4,7,10-tetraazacyclododecan-10-N-(pentanoyl-3-aza-4-oxo-5-methyl-5-yl)]-2-N-[1-O-α-D-carbonylmethyl-galactopyranose]-L-lysin-[1-(4-perfluoroctylsulfonyl)-piperazin]-amid,Gd-Komplex

50,0 g (54,55 mmol) der Titelverbindung aus Beispiel 35b) , 6,28 g (54,55 mmol) N-Hydroxysuccinimid, 4,62 g (109,0 mol) Lithiumchlorid und 34,35 g (54,55 mol) 1,4,7-Tris (carboxylatomethyl )-10- (carboxy - 3-aza-4-oxo-5-methyl-pent-5-yl )-1,4,7,10-tetraazacyclododecan, Gd-Komplex, werden unter leichter Erwärmung in 400 ml Dimethylsulfoxid gelöst. Bei 10°C gibt man 16,88 g (81,8 mol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18 Laufmittel: Gradient aus Wasser / Ethanol / Acetonitril).
Ausbeute: 37,95 g (91,0 % der Theorie) eines farblosen Feststoffs.
Wassergehalt : 8,6 %.

| Elementaranalyse (auf wasserfreie Substanz berechnet): | | | | | | |
|---|---|---|---|---|---|---|
| ber. | C 35,34 | H 4,09 | N 8,24 | S 2,10 | F 21,12 | Gd 10,28 |
| gef. | C 35,22 | H 4,17 | N 8,18 | S 2,19 | F 20.91 | Gd 10,12 |

### Beispiel 36

### a) N-Trifluoracetyl- L-glutaminsäure-mono-benzylester

100 g (421,5 mmol ) L-Glutaminsäure-mono-benzylester werden in einer Mischung aus 1000 ml Trifluoressigsäureethylester / 500 ml Ethanol gelöst und 24 Stunden bei Raumtemperatur gerührt. Man dampft zur Trockene ein und kristallisiert den Rückstand aus Diisopropylether.
Ausbeute: 140,47 g (96 % der Theorie) eines farblosen kristallienen Pulvers.

| Elementaranalyse: | | | | |
|---|---|---|---|---|
| ber. | C 50,46 | H 4,23 | F 17,10 | N 4,20 |
| gef. | C 51,35 | H 4,18 | F 17,03 | N 4,28 |

### b) 2-N-Trifluoacetyl-L-glutaminsäure-mono-benzylester-5-N-(methyl)-N-(2,3,4,5,6-pentahydroxyhexyl)-amid

Zu einer Lösung aus 24,9 g ( 24,08 mmol ) der Titelverbindung aus Beispiel 36a , 2x g (24,08 mmol ) N-Methylglucamin und 2,77 g (24,08 mmol ) N - Hydroxysuccinimid, gelöst in 150 ml Dimethylformamid, gibt man bei 0° C 8,25 g (40 mmol ) N,N-Dicyclohexylcabodiimid zu. Man rührt 3 Stunden bei 0°C, anschließend über Nacht bei Raumtemperatur. Man filtriert vom ausgefallenen Harnstoff ab, dampft das Filtrat im Vakuum zur Trockene ein und chomatographiert am Kieselgel (Laufmittel: = Dichlormethan / Ethanol = 20:1 ).
Ausbeute: 109,40 g ( 89% der Theorie ) eines zähen öls.

| Elementaranalyse : | | | | |
|---|---|---|---|---|
| ber. | C 51,43 | H 5,51 | F 13,56 | N 6,66 |
| gef. | C 51,22 | H 5,41 | F 13,40 | N 6,75 |

### c) N-Trifluoracetyl-L-glutaminsäure-N-(methyl)-N-(2,3,4,5,6-pentahydroxyhexyl)-amid

77,33g ( 15,15 mmol) der Titelverbindung aus Beispiel 36b werden in 500 ml Ethanol gelöst und 3 g Palladium-Katalysator (10 % Pd / C ) zugegeben. Man hydriert bei Raumtemperatur. Man filtriert vom Katalysator ab und dampft das Filtrat im Vakuum zur Trockene ein. Ausbeute: 43,0 g (quantitativ) eines farblosen Feststoffs.

| Elementaranalyse: | | | | |
|---|---|---|---|---|
| ber. | C 40,01 | H 5,19 | F 17,26 | N 8,48 |
| gef. | C 39,84 | H 5,13 | F 17,09 | N 8,68 |

### d) Trifluoracetyl-L-glutaminsäure-5-N-(methyl)-N-(2,3,4,5,6-pentahydroxyhexyl)-amid-[1-(4-perfluoroctylsulfonyl)-piperazin]-amidpiperazin]-amid

Zu 10,96g ( 33,2 mmol ) der Titelverbindung aus Beispiel 36c und 18,87g ( 33,2 mmol ) 1-Perfluoroctylsulfonyl - piperazin , (hergestellt nach DE 19603033 ) in 80 ml Tetrahydrofuran, gibt man bei 0° C 16,42 g ( 66,4 mmol ) EEDQ (2-Ethoxy-1,2-dihydrochinolin-1-carbonsäureethylester ) zu und rührt über Nacht bei Raumtemperatur. Man dampft im Vakuum zur Trockene ein und chromatographiert an Kieselgel (Laufmittel : Dichlormethan / Methanol =20:1)
Ausbeute: 28,67 g (92 % der Theorie ) eines farblosen Feststoffs.

| Elementaranalyse: | | | | | |
|---|---|---|---|---|---|
| ber. | C 39,61 | H 2,89 | F 35,66 | N 6,19 | S 3,54 |
| gef. | C 39,68 | H 2,74 | F 35,81 | N 6,13 | S 3,40 |

### e) L-Glutaminsäure-5-N-(methyl)-N-(2,3,4,5,6-pentahydroxyhexyl)-amid--[1-(4-perfluoroctylsulfonyl )- piperazin] - amid

In eine Lösung aus 28,36 g ( 30,22 mmol ) der Titelverbindung aus Beispiel 36d in 200 ml Ethanol, leitet man bei 0° C für eine Stunde Ammoniak-Gas ein. Man rührt anschließend 4 Stunden bei 0° C. Es wird zur Trockene eingedampft und der Rückstand aus Wasser ausgerührt. Man filtriert den Feststoff ab und trocknet im Vakuum (50° C ).
Ausbeute : 24,19 g ( 95 % der Theorie ) eines amorphen Feststoffs.

| Elementaranalyse: | | | | | |
|---|---|---|---|---|---|
| ber. | C 41,12 | H 2,89 | F 35,66 | N 6,19 | S 3,54 |
| gef. | C 41,15 | H 2,83 | F 35,78 | N 6,28 | S 3,71 |

### f) N-[1,4,7-Tris(carboxylatomethyl)-1,4,7,10- tetraazacyclododecan-10 -( pentanoyl- 3-aza-4- oxo- 5- methyl- 5-yl) ] -L-glutaminsäure-5-N-(methyl)-N-(2,3,4,5,6-pntahydroxyhexyl)-amid-5-[1-(4-perfluoroctylsulfonyl)-piperazin ]-amid, Gd-Komplex

20,43 g ( 24,25 mmol ) der Titelverbindung aus Beispiel 36e 2,79 g (24,25 mmol ) N-Hydroxysuccinimid, 2,12 g (50 mmol ) Lithiumchlorid und 15,27g (24,25 mmol) 1,4,7-Tris (carboxylatomethyl)-10-[ (3-aza- 4- oxo- 5- methyl- 5-yl) ]- pentansäure]-1,4,7,10- tetraazacyclododecan, Gd-Komplex werden unter leichter Erwärmung in 200 ml Dimethylsulfoxid gelöst. Bei 10° C gibt man 8,25 g (40 mmol ) N,NDicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chomatographie gereinigt ( Kieselgel RP-18, Laufmittel: Gradient aus Wasser / Ethanol / Acetonitril ).Ausbeute: 28,45 g (79 % der Theorie ) eines farblosen Feststoffs. Wassergehalt: 11,0 %

| Elementaranalyse ( auf wasserfreie Substanz berechnet ) | | | | | | |
|---|---|---|---|---|---|---|
| ber. | C 34,41 | H 3,83 | F 23,13 | N 9,03 | S 2,30 | Gd 11,26 |
| gef. | C 34,34 | H 3,98 | F 23,29 | N 9,19 | S 2,15 | Gd 11,07 |

### Beispiel 37

### a) 6-N-Benzyloxycarbonyl-2-N-[1-0-α-D-carbonylmethyl-(2,3,4 -tri O benzyl-glucuronsäure-benzylester]-L-lysin-[1-(4-perfluorocty)sulfonyl)-piperazin]-amid

Zu einer Lösung aus 100,0 g (120,4 mol) der Titelverbindung aus Beispiel 1c), 73,77 g (120,4 mol) 1-O-α-D-Carboxymethyl - 2,3,4-tri-O-benzyl-glucuronsäure-benzylester und 13,86 g (120,4 mol) N-Hydroxysuccinimid, gelöst in 500 ml Dimethylformamid , gibt man bei 0°C 41,27g (200,0 mmol ) N,N - Dicyclohexylcarbodiimid hinzu. Man rührt 3 Stunden bei 0°C und anschließend über Nacht bei Raumtemperatur. Man filtriert vom ausgefallenen Harnstoff ab, dampft das Filtrat im Vakuum zur Trockene ein und chromatographiert an Kieselgel. (Laufmittel : Dichlormethan/Ethanol = 20:1).
Ausbeute: 147,58 g (86 % der Theorie) eines zähen Öls.

| Elementaranalyse: | | | | | |
|---|---|---|---|---|---|
| ber. | C 52,25 | H 4,31 | N 3,93 | F 22,66 | S 2,45 |
| gef. | C 52,38 | H 4,17 | N 4,12 | F 22,78 | S 2,39 |

### b) 2-N-[1-0-α-D-Carbonylmethyl-glucuronsäure]-L-lysin-1-[(4-perfluoroctylsulfonyl)-piperazin]-amid

142,52 g (100,0 mmol) der Titelverbindung aus Beispiel 37a) werden in 2000 ml Ethanol gelöst und es werden 10,0 g Palladium Katalysator (10% Pd / C ) hinzu gegeben. Man hydriert 12 Stunden bei Raumtemperatur. Man filtriert vom Katalysator ab und dampft das Filtrat im Vakuum zur Trockne ein.
Ausbeute : 93,06 g (quantitativ) eines farblosen Feststoffs.

| Elementaranalyse: | | | | | |
|---|---|---|---|---|---|
| ber. | C 33,56 | H 3,36 | N 6,02 | S 3,45 | F 34,71 |
| gef. | C 33,31 | H 3,42 | N 6,04 | S 3,40 | F 35,51 |

### c) 6-N-[1,4,7-Tris (carboxylatomethyl) ]-1,4,7,10-tetraazacyclododecan-10-N-(pentanoyl-3-aza-4-oxo-5-methyl-5-yl)]-2-N-[1-O-α-D-carbonylmethyl-glucuronsäure]-L-lysin-[1-(4-perfluoroctylsulfonyl)-piperazin]-amid, Gd-Komplex, Natriumsalz

50,76 g (54,55 mmol) der Titelverbindung aus Beispiel 37b) , 6,28 g (54,55 mmol) N-Hydroxysuccinimid , 4,62 g (109,0 mol ) Lithiumchlorid und 34,35 g (54,55 mol) 1,4,7-Tris (carboxylatomethyl )-10- (carboxy - 3-aza-4-oxo-5-methyl-pent-5-yl )-1,4,7,10-tetraazacyclododecan, Gd-Komplex, werden unter leichter Erwärmung in 400 ml Dimethylsulfoxid gelöst. Bei 10°C gibt man 16,88 g (81,8 mol) N,N-Dicyctohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18 Laufmittel: Gradient aus Wasser / Ethanol / Acetonitril).
Ausbeute: 75,14,9 g (88,0 % der Theorie) eines farblosen Feststoffs.
Wassergehalt : 8,6 %.

| Elementaranalyse (auf wasserfreie Substanz berechnet): | | | | | | | |
|---|---|---|---|---|---|---|---|
| ber. | C 34,53 | H 3,80 | N 8,05 | Na 1,47 | S 2,05 | F 20,63 | Gd 10,05 |
| gef. | C 34,38 | H 3,95 | N 8,19 | Na 1,63 | S 2,15 | F 20.83 | Gd 10,14 |

### Beispiel 38

### a) 6-N-Benzyloxycarbonyl)-2--[2-(N-ethyl-N-perfluoroctylsulfonyl]-amino]-acetyl-L-lysin

31,82,0 g (113,5 mmol) 6-N-Benzyloxycarbonyl)-L-lysin und 66,42 g ( 113,5 mmol ) 2-(N-Ethyl-N- perfluoroctylsulfonyl)-aminoessigsäure (hergestellt nach DE 196 03 033 ) in 300 ml Tetrahydrofuran, gibt man bei 0°C 49,46 g (200,0 mmol ) EEDQ (2-Ethoxy-1,2-dihydrochinolin-1-carbonsäureethylester) hinzu und rührt über Nacht bei Raumtemperatur. Man dampft im Vakuum zur Trockene ein und chromatographiert an Kieselgel (Laufmittel :Dichlormethan /Methanol =20:1).
Ausbeute: 55,79 g (58 % der Theorie ) eines farblosen Feststoffs.

| Elementaranalyse: | | | | | |
|---|---|---|---|---|---|
| ber. | C 36.85 | H 3,09 | N 4,96 | F 38,11 | S 3,78 |
| gef. | C 36,85 | H 3,19 | N 4,87 | F 38,28 | S 3,95 |

### b) 6-N-Benzyloxycarbonyl - 2-N-[2-(N-ethyl-N-perfluoroctylsulfonyl)-amino]-acetyl--Llysin- N-methyl-N-(2,3,4,5,6-pentahydroxy-hexyl)-amid

Zu einer Lösung aus 51,02 g (60,2 mol) der Titelverbindung aus Beispiel 38a), 11,75 g (60,2 mol) N-methyl-glucamin und 6,93 g (60,2 mol) N-Hydroxysuccinimid, gelöst in 250 ml Dimethylformamid , gibt man bei 0°C 20,64g (100,0 mmol ) N,N -Dicyclohexylcarbodiimid hinzu. Man rührt 3 Stunden bei 0°C und anschließend über Nacht bei Raumtemperatur. Man filtriert vom ausgefallenen Harnstoff ab, dampft das Filtrat im Vakuum zur Trockene ein und chromatographiert an Kieselgel.
(Laufmittel: Dichlormethan/Ethanol = 20 :1).
Ausbeute: 53,05 g (86 % der Theorie) eines zähen Öls.

| Elementaranalyse: | | | | | |
|---|---|---|---|---|---|
| ber. | C 38,68 | H 4,03 | N 5,47 | F 31,52 | S 3,13 |
| gef. | C 38,49 | H 4,17 | N 5,32 | F 31,70 | S 3,29 |

### c) 2-N-[2-(N-ethyl-N-perfluoroctylsulfonyl)-amino]-acetyl-L-lysin-N-methyl-N-(2,3,4,5,6-pentahydroxy-hexyl)-amid

102,48g (100,0 mmol) der Titelverbindung aus Beispiel 38b) werden in 2000 ml Ethanol gelöst und es werden 10,0 g Palladium Katalysator (10% Pd / C ) hinzu gegeben. Man hydriert 12 Stunden bei Raumtemperatur. Man filtriert vom Katalysator ab und dampft das Filtrat im Vakuum zur Trockne ein.
Ausbeute : 89,06 g (quantitativ) eines farblosen Feststoffs.

| Elementaranalyse: | | | | | |
|---|---|---|---|---|---|
| ber. | C 33,72 | H 3,96 | N 6,29 | S 3,60 | F 36,26 |
| gef. | C 33,91 | H 3,82 | N 6,14 | S 3,47 | F 36,31 |

### d) 6-N-[1,4,7-Tris(carboxylatomethyl)]-1,4,7,10-tetraazacyclododecan-10-N-(pentanoyl-3-aza-4-oxo-5-methyl-5-yl)]-2-N-[2-(N-ethyl-N-perfluoroctylsulfonyl)-amino]-acetyl-Llysin-N-methyl-N-(2,3,4,5,6-pentahydroxy-hexyl)-amid, Gd-Komplex

48,58 g (54,55 mmol) der Titelverbindung aus Beispiel 38c) , 6,28 g (54,55 mmol) N-Hydroxysuccinimid , 4,62 g (109,0 mol ) Lithiumchlorid und 34,35 g (54,55 mol) 1,4,7-Tris (carboxylatomethyl)-10- (carboxy - 3-aza-4-oxo-5-methyl-pent-5-yl)-1,4,7,10-tetraazacyclododecan, Gd-Komplex, werden unter leichter Erwärmung in 400 ml Dimethylsulfoxid gelöst. Bei 10°C gibt man 16,88 g (81,8 mol) N,N-Dicyclohexylcarbodiimid zu und rührt anschließend über Nacht bei Raumtemperatur. Man gießt die Lösung in 3000 ml Aceton und rührt 10 Minuten. Der ausgefallene Feststoff wird abfiltriert und anschließend durch Chromatographie gereinigt (RP-18 Laufmittel: Gradient aus Wasser / Ethanol / Acetonitril)
Ausbeute: 73,27 g (89,4 % der Theorie) eines farblosen Feststoffs.
Wassergehalt : 8,6 %.

| Elementaranalyse (auf wasserfreie Substanz berechnet): | | | | | | |
|---|---|---|---|---|---|---|
| ber. | C 35,18 | H 4,23 | N 4,23 | S 2,13 | F 21,50 | Gd 10,47 |
| gef. | C 35,28 | H 4,15 | N 4,19 | S 2,18 | F 21.33 | Gd 10,61 |

### Beispiel 39

Organverteilung (einschließlich Tumor- und Lymphknotenanreicherung) nach intravenöser Gabe des erfindungsgemäßen Kontrastmittels aus Beispiel 1 in Prostatakarzinom-tragenden Ratten

Nach intravenöser Applikation von 225 µmol Gesamtgadolinium/kg KGW der Titelverbindung aus Beispiel 1 in Ratten (Cop-Inzucht, mit 12 Tage zuvor i.m. implantiertem Prostatakarzinom Dunning R3327 MAT-Lu) wurde 10 Minuten, 1 und 24 Stunden nach Applikation der Metallgehalt in verschiedenen Organen, im Tumor sowie in den Lymphknoten (gepoolt als mesenteriale und periphere Lymphknoten) bestimmt (MW ± SD, n=3).

| | Titelverbindung aus Beispiel 1 | | | | | |
|---|---|---|---|---|---|---|
| | Gd-Konzentration [µmol/l] | | | % Dosis pro Gesamtgewebe | | |
| | 10min p.i. | 1h p.i. | 24h p.i. | 10min p.i. | 1h p.i. | 24h p.i. |
| Leber | 387 ± 26 | 364 ± 8 | 746 ± 34 | 5,46 ± 0,16 | 5,81 ± 0,16 | 11,65 ± 0,97 |
| Milz | 548 ± 22 | 487 ± 25 | 645 ± 27 | 0,39 ± 0,03 | 0,39 ± 0,02 | 0,57 ± 0,03 |
| Pankreas | 229 ± 27 | 199 ± 30 | 130 ± 13 | 0,26 ± 0,05 | 0,21 ± 0,05 | 0,17 ± 0,02 |
| Niere | 208 ± 537 | 883 ± 94 | 1178 ± 139 | 5,02 ± 1,29 | 2,15 ± 0,23 | 2,97 ± 0,21 |
| | 1 | | | | | |
| Lunge | 837 ± 32 | 658 ± 29 | 370 ± 34 | 1,69 ± 0,06 | 1,38 ± 0,08 | 0,73 ± 0,04 |
| Herz | 438 ± 29 | 289 ± 24 | 131 ± 9 | 0,46 ± 0,01 | 0,31 ± 0,03 | 0,14 ± 0,02 |
| Gehirn | 47 ± 13 | 26 ± 5 | 14 ± 2 | 0,15 ± 0,03 | 0.08 ± 0,02 | 0,04 ± 0,00 |
| Muskel** | 99 ± 5 | 78 ± 1 | 36 ± 1 | 0,11 ± 0,03 | 0,09 ± 0,03 | 0,04 ± 0,00 |
| Tumor | 185 ± 36 | 184 ± 13 | 199 ± 19 | 0,28 ± 0,10 | 0,21 ± 0,02 | 0,31 ± 0,01 |
| Femur | 184 ± 4 | 127 ± 9 | 87 ± 6 | 0,65 ± 0,01 | 0,46 ± 0,03 | 0,31 ± 0,03 |
| mes. LK | 359 ± 72 | 697 ± 42 | 854 ± 135 | 0,11 ± 0,04 | 0,24 ± 0,02 | 0,32 ± 0,04 |
| periph. LK | 229 ± 15 | 436 ± 44 | 373 ± 24 | 0,10 ± 0,01 | 0,20 ± 0,03 | 0,18 ± 0,01 |
| Magen (entleert) | 231 ± 10 | 219 ± 46 | 138 ± 9 | 0,57 ± 0,04 | 0,54 ± 0,12 | 0,37 ± 0,06 |
| Darm (entleert) | 342 ± 16 | 409 ± 67 | 243 ± 22 | 2,91 ± 0,18 | 3,41 ± 1,02 | 2,14 ± 0,14 |
| Blut* | 166 ± 110 | 825 ± 67 | 214 ± 9 | 42,95 ± 2,59 | 21,47 ± 1,78 | 1,78 ± 0,03 |
| | 5 | | | | | |
| Restkörper **** | -- - -- | -- - -- | 225 ± 31 | - - - | -- - -- | 30,83 ± 4,05 |
| Harn 0-24 h | -- - -- | -- - -- | 94 ± 20 | - - - | -- - -- | 20,20 ± 4,41 |
| Faeces 0-24 h | -- - -- | -- - -- | 3128 ± 204 | -- - -- | -- - -- | 21,85 ± 1,46 |

| | | | | | | |
|---|---|---|---|---|---|---|
| * 58ml Blut/kg KGW | | | | | | |
| ** nur Gewebealiquot v. rechten Unterschenkelmuskel | | | | | | |
| ***Summe Organe 10 und 60 min p.i. ohne Restkörper **** Restkörper beinhaltet auch Restblut | | | | | | |

### Beispiel 40:

### Lymphknotendarstellung (MRT) nach intravenöser Gabe des erfindungsgemäßen Kontrastmittels aus Beispiel 1 in VX2-Tumortragenden Kaninchen

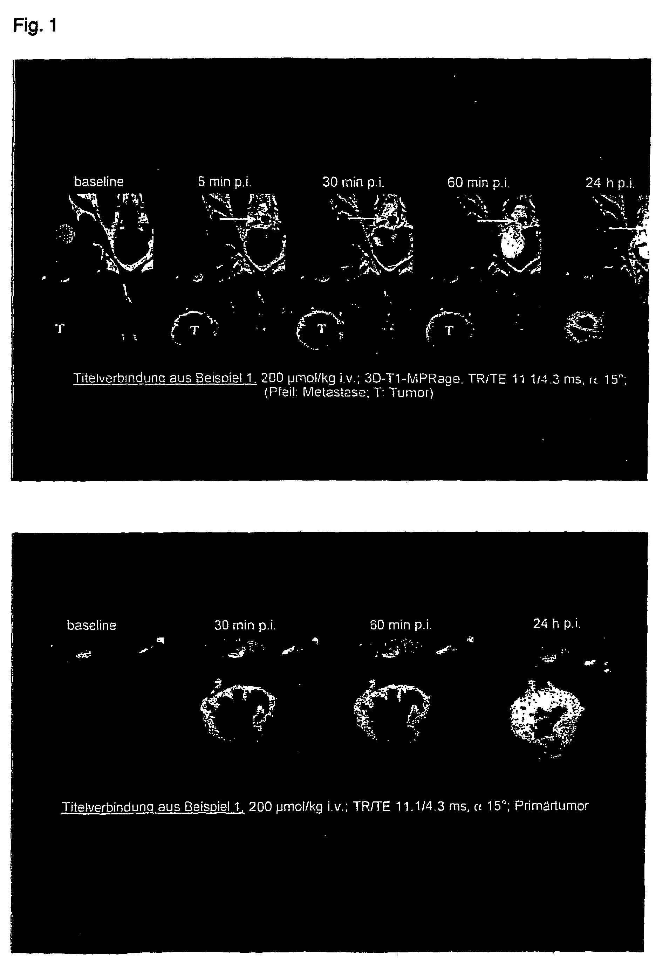

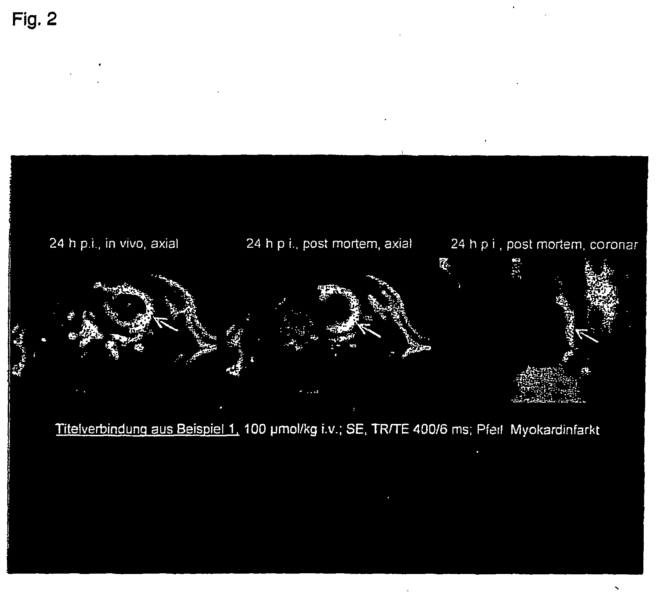

Die Abbildungen in Fig. 1 zeigen MR-Aufnahmen von iliakalen Lymphknoten präkontrast sowie bis 24 h nach intravenöser Applikation von 200µmol Gd/kg KGW der Titelverbindung aus Beispiel 1 in Kaninchen mit i.m. implantiertem VX2-Tumor. Die T₁gewichteten Gradientenecho-Aufnahmen (1.5 T; Sequenz: MPRange; TR 11.1 ms, TE 4.3 ms, α 15°) verdeutlichen den starken Signalanstieg im gesunden Lymphknotengewebe. Zonen ohne Signalanstieg innerhalb des Lymphknoten wurde als Metastasen diagnostiziert und histologisch (H/E-Färbung der Lymphknotenschnitte) bestätigt. Zu späten Zeitpunkten (24 h) nach Kontrastmittelapplikation konnte dagegen überraschenderweise eine Signalumkehr beobachtet werden. Der Signalanstieg im gesunden Lymphknotengewebe war reduziert, wohingegen die Metastase jetzt einen deutlichen Signalanstieg aufwies.
Überraschenderweise konnte bereits unmittelbar nach Applikation ein deutliches Enhancement des Primärtumors (besonders der Peripherie) beobachtet werden. Zu späteren Zeitpunkten (24 h p.i.) breitet sich dieses Enhancement auch Richtung Tumorzentrum aus.

### Beispiel 41:

### Infarkt-Darstellung (MRT) nach intravenöser Gabe des erfindungsgemäßen Kontrastmittels aus Beispiel 1 in Ratten

Die Abbildungen in Firg. 2 zeigen MR-Aufnahmen des Herzen (in vivo und post mortem) 24 h nach intravenöser Applikation von 100µmol Gd/kg KGW der Titelverbindung aus Beipiel 1 in Ratten mit akut induziertem Herzinfarkt. Die T₁-gewichteten Spin-Echo-Aufnahmen (1.5 T; TR: 400 ms, TE: 6 ms; NA: 4; Matrix: 128*128; Schichtdicke: 2.5 mm) verdeutlichen den starken Signalanstieg im Infarktareal. Die erfolgreiche Induktion eines akuten Myokardinfarkts wurde mittels NBT-Färbung bestätigt.

## Patentansprüche

1. Perfluoralkylhaltige Komplexe mit Zuckerresten der allgemeinen Formel I 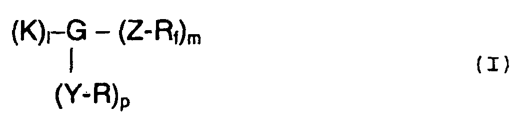 in der
R einen über die 1-OH- oder 1-SH-Position gebundenen Monosaccharidrest mit 5 oder 6 C-Atomen, dessen Desoxy- oder Thiozucker darstellt,
R_{f} eine perfluorierte, geradkettige oder verzweigte Kohlenstoffkette mit der Formel -CₙF₂ₙE ist, in der E ein endständiges Fluor-, Chlor-, Brom-, Jod- oder Wasserstoffatom darstellt und n für die Zahlen 4-30 steht,
K für einen Metallkomplex der allgemeinen Formel II steht, 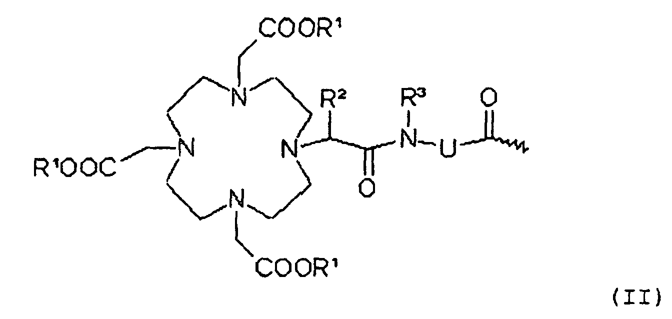
in der
R¹ ein Wasserstoffatom oder ein Metallionenäquivalent der Ordnungszahlen 21-29, 31-33, 37-39, 42-44, 49 oder 57-83 bedeutet, mit der Maßgabe, daß mindestens zwei R¹ für Metallionenäquivalente stehen
R² und R³ unabhängig voneinander Wasserstoff, C₁-C₇-Alkyl, Benzyl, Phenyl, -CH₂OH oder -CH₂OCH₃ darstellen und
U -C₆H₄-O-CH₂-ω-, -(CH₂)₁₋₅-ω, eine Phenylengruppe, -CH₂-NHCO-CH₂-CH(CH₂COOH)-C₆H₄-ω-, -C₆H₄-(OCH₂CH₂)₀₋₁-N(CH₂COOH)-CH₂-ω oder eine gegebenenfalls durch ein oder mehrere Sauerstoffatome, 1 bis 3-NHCO-, 1 bis 3 -CONH-gruppen unterbrochene und/oder mit 1 bis 3-(CH₂)₀₋₅COOH-Gruppen substituierte C₁-C₁₂-Alkylen- oder C₇-C₁₂-C₆H₄-O-Gruppe darstellt, wobei ω für die Bindungsstelle an -CO- steht,
oder
der allgemeinen Formel III 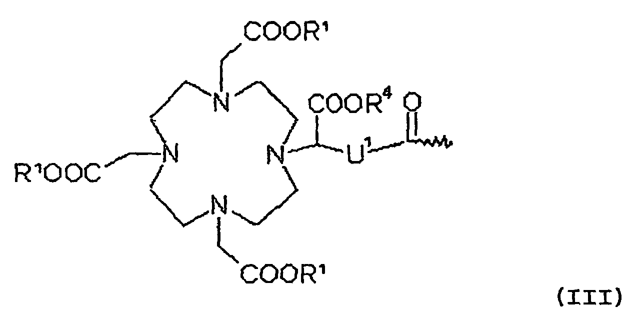 in der R¹ die oben genannte Bedeutung hat, R⁴ Wasserstoff oder ein unter R¹ genanntes Metallionenäquivalent darstellt und U¹ -C₆H₄-O-CH₂-ω- darstellt, wobei ω die Bindungsstelle an -CObedeutet
oder der allgemeinen Formel IV 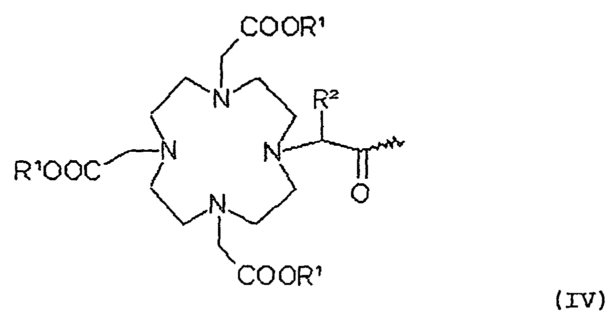 in der R¹ und R² die oben genannte Bedeutung haben
oder der allgemeinen Formel V A oder V B 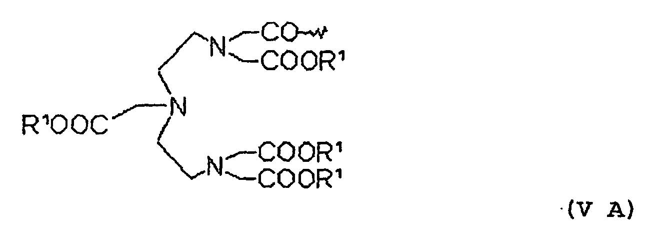 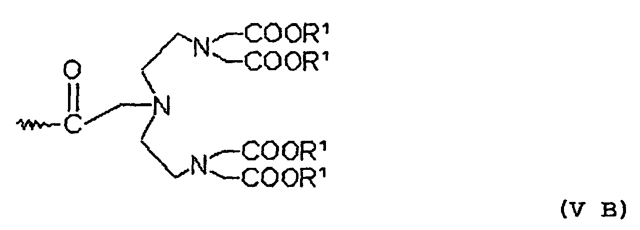 in der R¹ die oben genannte Bedeutung hat,
oder der allgemeinen Formel VI 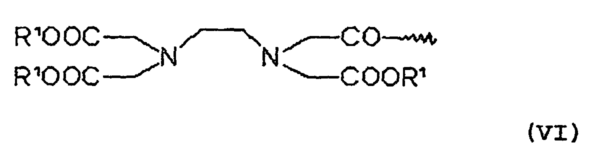 in der R¹ die oben genannte Bedeutung hat,
oder der allgemeinen Formel VII 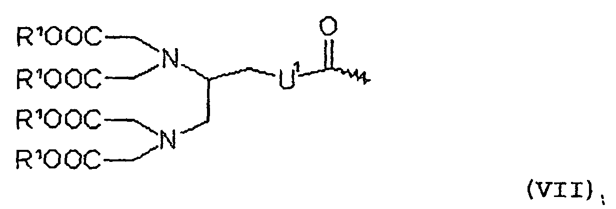 in der R¹ die oben genannte Bedeutung hat und
U¹ -C₆H₄-O-CH₂-ω- darstellt, wobei ω die Bindungsstelle an -CO- bedeutet
oder der allgemeinen Formel VIII 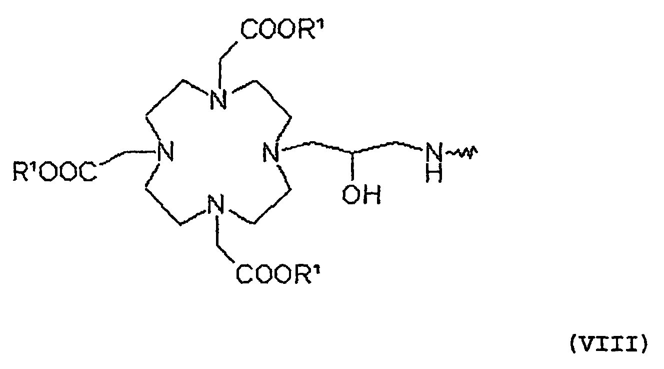 in der R¹ die oben genannte Bedeutung hat,
und im Rest K gegebenenfalls vorhandene freie Säuregruppen gegebenenfalls als Salze organischer und/oder anorganischer Basen oder Aminosäuren oder Aminosäureamide vorliegen können,
G für den Fall, daß K die Metallkomplexe II bis VII bedeutet, einen mindestens dreifach funktionalisierten Rest ausgewählt aus den nachfolgenden Resten a) bis j) darstellt 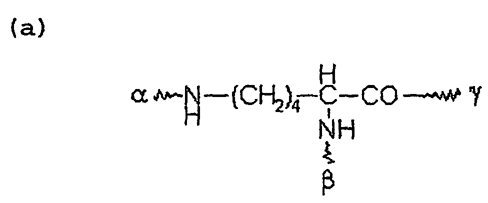 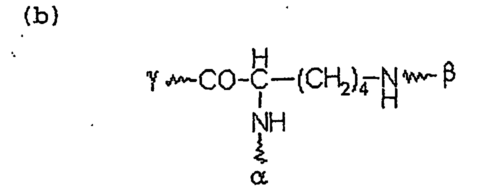 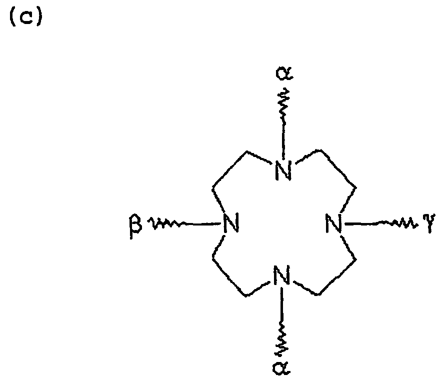 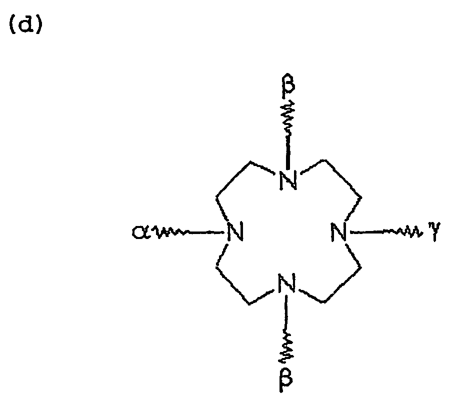 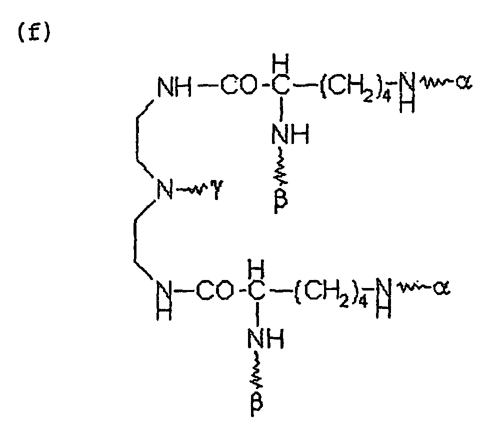 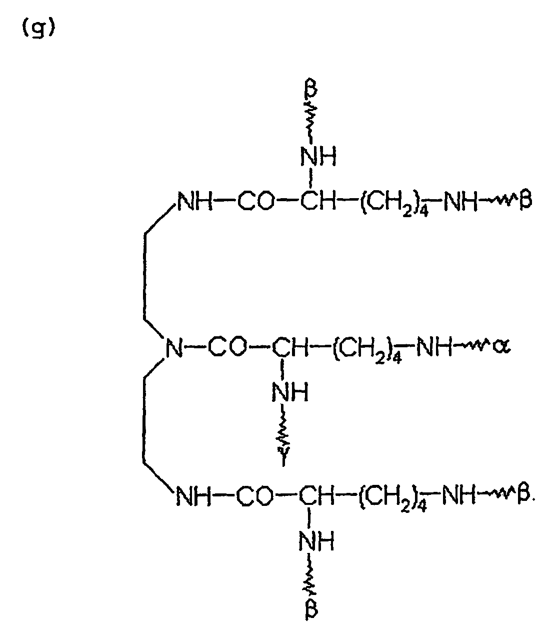 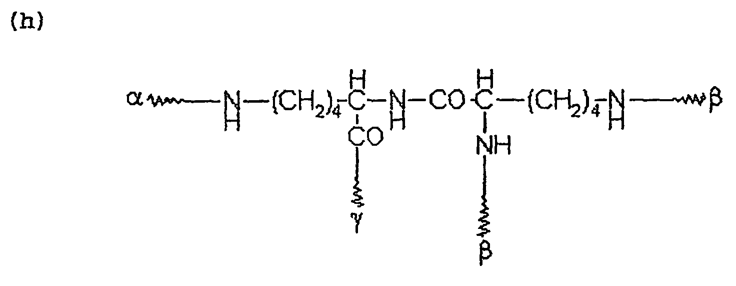 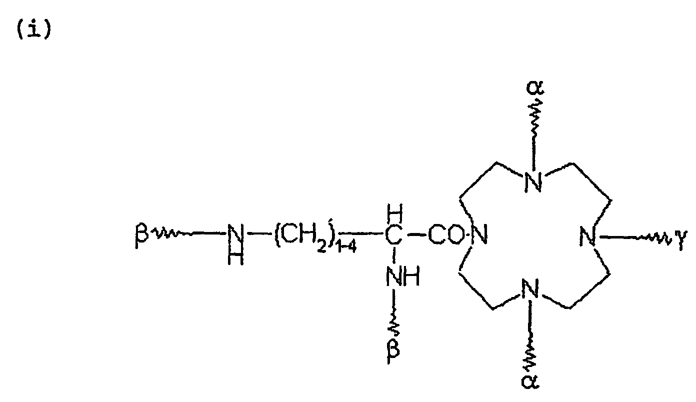 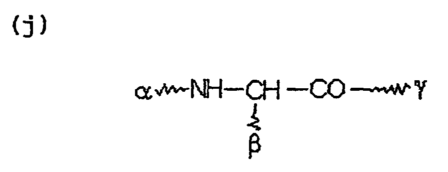
und
G für den Fall, daß K den Metallkomplex VIII bedeutet, einen mindestens dreifach funktionalisierten Rest ausgewählt aus k) oder l) darstellt, 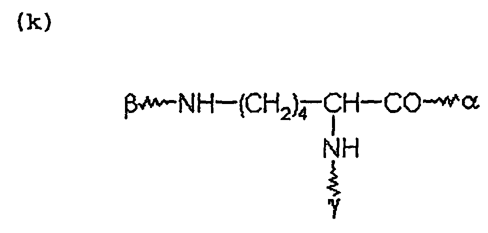 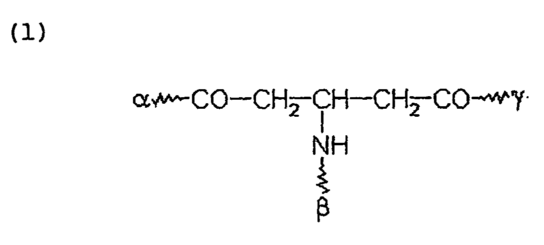
wobei α die Bindungsstelle von G an den Komplex K bedeutet, β die Bindungsstelle von G zum Rest Y ist und γ die Bindungsstelle von G zum Rest Z darstellt
Y -CH₂-, δ-(CH₂)ₙCO-β (wobei n=1-5 ist), δ-CH₂-CHOH-CO-β oder δ-CH(CHOH-CH₂OH)-CHOH-CHOH-CO-β bedeutet, wobei δ die Bindungsstelle zum Zuckerrest R darstellt und β die Bindungsstelle zum Rest G ist
Z für 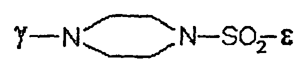 γ-COCH₂-N(C₂H₅)-SO₂-ε,
γ-COCH₂-O-(CH₂)₂-SO₂-ε, 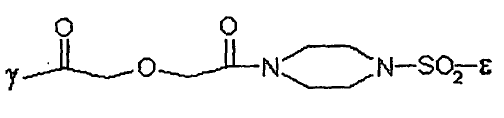 oder
y - NHCH₂CH₂-O-CH₂CH₂ - ε
steht, wobei γ die Bindungsstelle von Z zum Rest G darstellt und ε die Bindungsstelle von Z an den perfluorierten Rest R_{f} bedeutet
und
l, m unabhängig voneinander die ganzen Zahlen 1 oder 2 bedeuten und
p die ganzen Zahlen 1 bis 4 bedeutet.

2. Metallkomplexe nach Anspruch 1,
**dadurch gekennzeichnet, daß**
das Metallionenäquivalent R¹ ein Element der Ordnungszahlen 21-29, 39, 42, 44 oder 57-83 ist.

3. Metallkomplexe nach Anspruch 1,
**dadurch gekennzeichnet, daß**
das Metallionenäquivalent R¹ ein Element der Ordnungszahlen 27, 29, 31-33, 37-39, 43, 49, 62, 64, 70, 75 und 77 ist.

4. Metallkomplexe nach einem der Ansprüche 1 bis 3,
**dadurch gekennzeichnet, daß**
R einen Monosaccharidrest mit 5 bis 6 C-Atomen oder dessen Desoxy-Verbindung darstellt, vorzugsweise Glucose, Mannose oder Galactose.

5. Metallkomplexe nach einem der Ansprüche 1 bis 4,
**dadurch gekennzeichnet, daß**
K für einen Metallkomplex der allgemeinen Formel II steht.

6. Metallkomplexe nach Anspruch 5,
**dadurch gekennzeichnet, daß**
R² und R³ unabhängig voneinander Wasserstoff oder C₁-C₄-Alkyl bedeuten.

7. Metallkomplexe nach einem der Ansprüche 1 bis 6,
**dadurch gekennzeichnet, daß**
E in der Formel -CₙF₂ₙE ein Fluoratom bedeutet.

8. Metallkomplexe nach einem der Ansprüche 1 bis 7,
**dadurch gekennzeichnet, daß**
G in der allgemeinen Formel I den Lysinrest (a) oder (b) darstellt.

9. Metallkomplexe nach einem der Ansprüche 1 bis 8,
**dadurch gekennzeichnet, daß**
Z in der allgemeinen Formel I 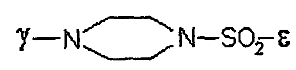 bedeutet, wobei γ die Bindungsstelle von Z zum Rest G darstellt und ε die Bindungsstelle von Z an den perfluorierten Rest R_{f} bedeutet.

10. Metallkomplexe nach einem der Ansprüche 1 bis 9,
**dadurch gekennzeichnet, daß**
Y in der allgemeinen Formel I δ-CH₂CO-β bedeutet, wobei δ die Bindungsstelle zum Zuckerrest R darstellt und β die Bindungsstelle zum Rest G darstellt.

11. Metallkomplexe nach einem der Ansprüche 1 bis 10,
**dadurch gekennzeichnet, daß**
U im Metallkomplex K -CH₂- oder -C₆H₄-O-CH₂-ω darstellt, wobei ω für die Bindungsstelle an -CO- steht.

12. Verwendung von Metallkomplexen nach Anspruch 2 zur Herstellung von Kontrastmitteln zur Anwendung in der NMRund Röntgendiagnostik.

13. Verwendung von Metallkomplexen nach Anspruch 12 zur Herstellung von Kontrastmitteln zum Infarkt- und Nekrose-Imaging.

14. Verwendung von Metallkomplexen nach Anspruch 3 zur Herstellung von Kontrastmitteln zur Anwendung in der Radiodiagnostik und Radiotherapie.

15. Verwendung von Metallkomplexen nach Anspruch 2 zur Herstellung von Kontrastmitteln für die Lymphographie zur Diagnose von Veränderungen des Lymphsystems.

16. Verwendung von Metallkomplexen nach Anspruch 2 zur Herstellung von Kontrastmitteln für die Anwendung in der indirekten Lymphographie.

17. Verwendung von Metallkomplexen nach Anspruch 2 zur Herstellung von Kontrastmitteln für die Anwendung in der i.v. Lymphographie.

18. Verwendung von Metallkomplexen nach Anspruch 2 zur Herstellung von Kontrastmitteln zur Darstellung des Vasalraumes.

19. Verwendung von Metallkomplexen nach Anspruch 2 zur Herstellung von Kontrastmitteln für das Tumorimaging.

20. Pharmazeutische Mittel enthaltend mindestens eine physiologisch verträgliche Verbindung nach den Ansprüchen 1 bis 11, gegebenenfalls mit den in. der Galenik üblichen Zusätzen.

21. Verfahren zur Herstellung von perfluoralkylhaltigen Komplexen mit Zuckerresten der allgemeinen Formel I 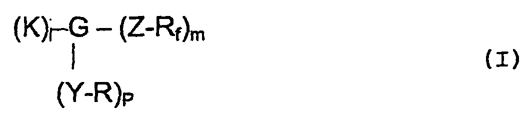 mit K in der Bedeutung eines Metallkomplexes der allgemeinen Formeln II bis VII gemäß Anspruch 1, G in der Bedeutung a) bis j) gemäß Anspruch 1, und Y, Z, R, R_{f}, m, p und 1 in der Bedeutung gemäß Anspruch 1,
**dadurch gekennzeichnet, daß**
man in an sich bekannter Weise eine Carbonsäüre der allgemeinen Formel IIa 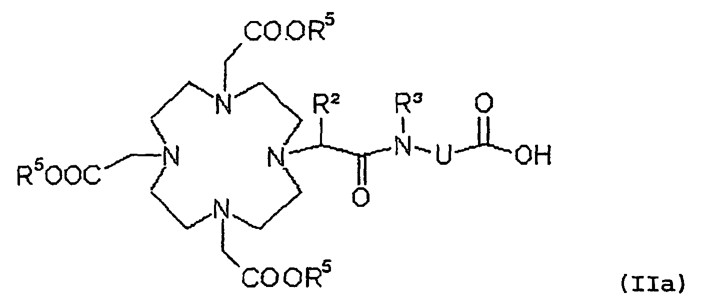 worin R⁵ ein Metallionenäquivalent der Ordnungszahlen 21-29, 31-33, 37-39, 42-44, 49 oder 57-83 oder eine Carboxylschutzgruppe bedeutet, und R², R³ und U die genannte Bedeutung haben
oder eine Carbonsäure der allgemeinen Formel IIIa 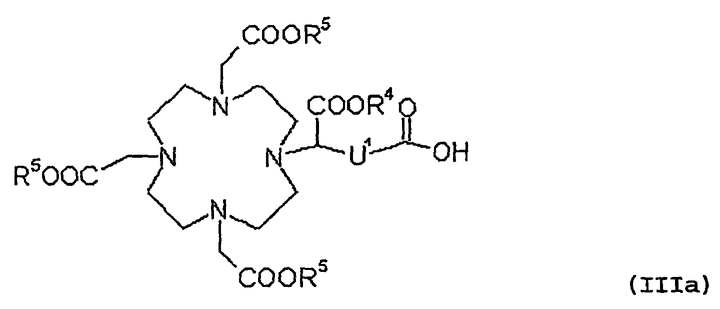 worin R⁴, R⁵ und U¹ die genannte Bedeutung haben
oder eine Carbonsäure der allgemeinen Formel IVa 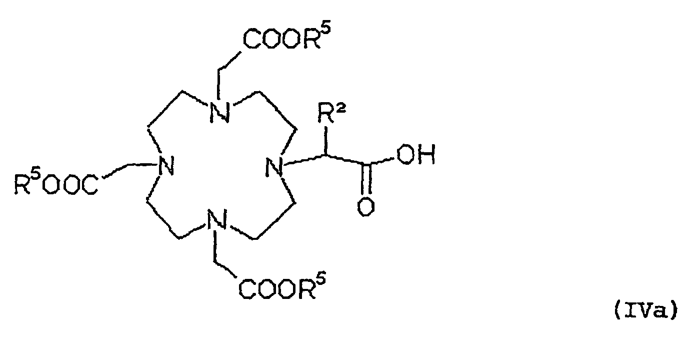 worin R⁵ und R² die genannte Bedeutung haben
oder eine Carbonsäure der allgemeinen Formel Va oder Vb 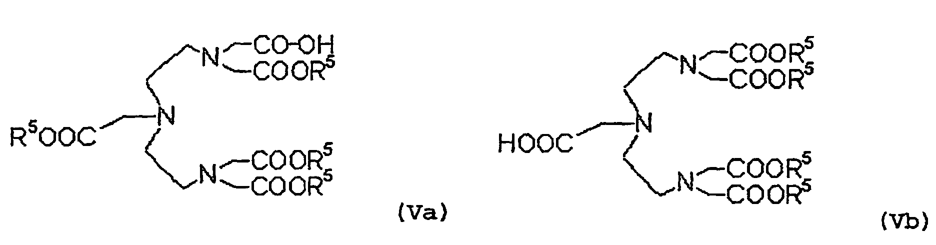 worin R⁵ die genannte Bedeutung hat
oder eine Carbonsäure der allgemeinen Formel VIa 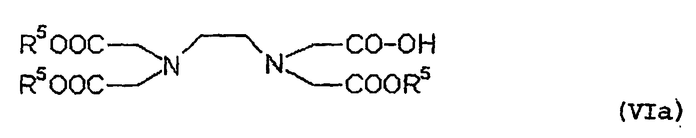 worin R⁵ die genannte Bedeutung hat
oder eine Carbonsäure der allgemeinen Formel VIIa 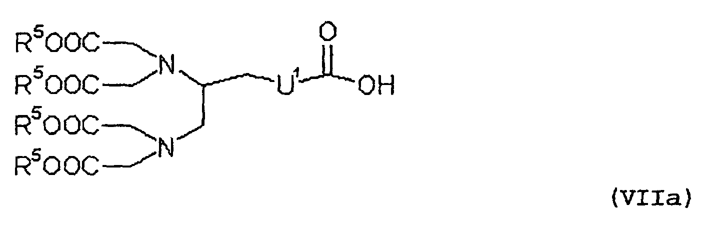 worin R⁵ und U¹ die genannten Bedeutungen haben,
in gegebenenfalls aktivierter Form mit einem Amin der allgemeinen Formel IX 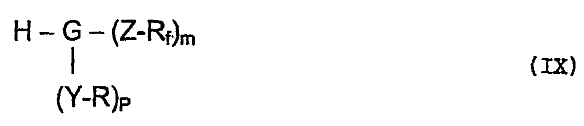 in der G, R, R_{f}, Y, Z, m und p die oben im Anspruch angegebene Bedeutung haben, in einer Kupplungsreaktion und gegebenenfalls nachfolgender Abspaltung gegebenenfalls vorhandener Schutzgruppen zu einem Metallkomplex der allgemeinen Formel I umsetzt
oder
wenn R⁵ die Bedeutung einer Schutzgruppe hat, nach Abspaltung dieser Schutzgruppen in einem Folgeschritt in an sich bekannter Weise mit mindestens einem Metalloxid oder Metallsalz eines Elementes der Ordnungszahlen 21-29, 31-33, 37-39, 42-44, 49 oder 57-83 umsetzt, und anschließend, falls gewünscht, gegebenenfalls vorhandene acide Wasserstoffatome durch Kationen anorganischer und/oder organischer Basen, Aminosäuren oder Aminosäureamide substituiert.

22. Verfahren zur Herstellung von perfluoralkylhaltigen Komplexen mit Zuckerresten der allgemeinen Formel I 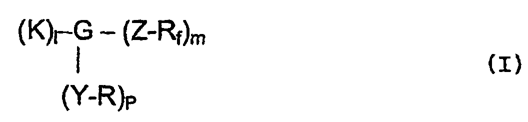 mit K in der Bedeutung eines Metallkomplexes der allgemeinen Formel VIII gemäß Anspruch 1, G in der Bedeutung k) oder 1) gemäß Anspruch 1 und Y, Z, R, R_{f}, m, p, 1 in der Bedeutung gemäß Anspruch 1,
**dadurch gekennzeichnet, daß**
man in an sich bekannter Weise ein Amin der allgemeinen Formel VIIIa 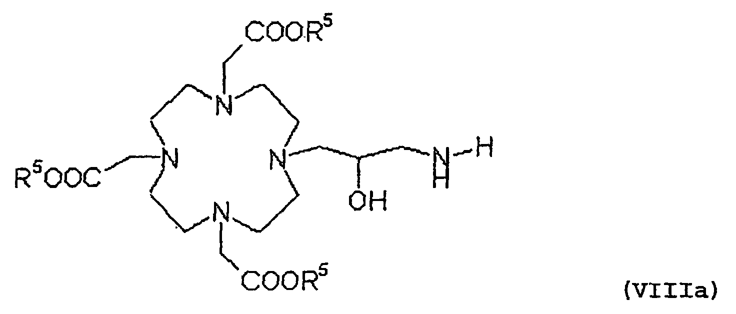 worin R⁵ ein Metallionenäquivalent der Ordnungszahlen 21-29, 31-33, 37-39, 42-44, 49 oder 57-83 oder eine Carboxylschutzgruppe bedeutet,
mit einer, gegebenenfalls aktivierten, Carbonsäure der allgemeinen Formel X 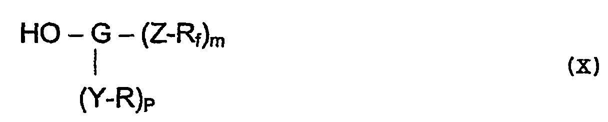 worin G, R, R_{f}, Y, Z, m und p die oben im Anspruch angegebenen Bedeutungen haben,
in einer Kupplungsreaktion und gegebenenfalls nachfolgender Abspaltung gegebenenfalls vorhandener Schutzgruppen zu einem Metallkomplex der allgemeinen Formel I umsetzt
oder
wenn R⁵ die Bedeutung einer Schutzgruppe hat, nach Abspaltung dieser Schutzgruppen in einem Folgeschritt in an sich bekannter Weise mit mindestens einem Metalloxid oder Metallsalz eines Elementes der Ordnungszahlen 21-29, 31-33, 37-39, 42-44, 49 oder 57-83 umsetzt, und anschließend, falls gewünscht, gegebenenfalls vorhandene acide Wasserstoffatome durch Kationen anorganischer und/oder organischer Basen, Aminosäuren oder Aminosäureamide substituiert.

## Claims

1. Perfluoroalkyl-containing complexes with sugar residues of the general formula I 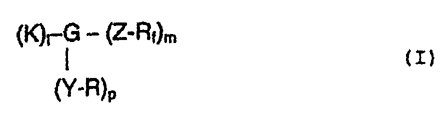 where
R is a monosaccharide residue of 5 or 6 carbon atoms which is attached via the 1-OH or 1-SH position or is its deoxy- or thiosugar,
R_{f} is a perfluorinated straight-chain or branched hydrocarbon chain having the formula -CₙF₂ₙE, where E is a terminal fluorine, chlorine, bromine, iodine or hydrogen atom and n is from 4 to 30,
K is a metal complex of the general formula II 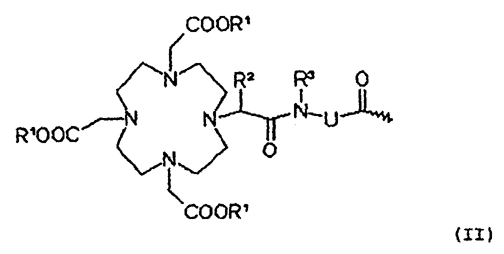
where
R¹ is a hydrogen atom or a metal ion equivalent of atomic numbers 21-29, 31-33, 37-39, 42-44, 49 or 57-83, with the proviso that at least two R¹'s are metal ion equivalents,
R² and R³ are independently hydrogen, C ₁-C₇ alkyl, benzyl, phenyl, -CH₂OH or -CH₂OCH₃ and
U is -C₆H₄-O-CH₂-ω-, -(CH₂)₁₋₅-ω, a phenylene group, -CH₂-NHCO-CH₂-CH(CH₂COOH)-C₆H₄-ω-, -C₆H₄-(OCH₂CH₂)₀₋₁-N(CH₂COOH)-CH₂-ω or is a C₁-C₁₂ alkylene or C₇-C₁₂-C₆H₄-O- group which is optionally interrupted by one or more oxygen atoms, 1 to 3 -NHCO-, 1 to 3 -CONH- groups and/or substituted by 1 to 3 -(CH₂)₀₋₅COOH groups, where ω denotes the site of attachment to -CO-,
or
of the general formula III 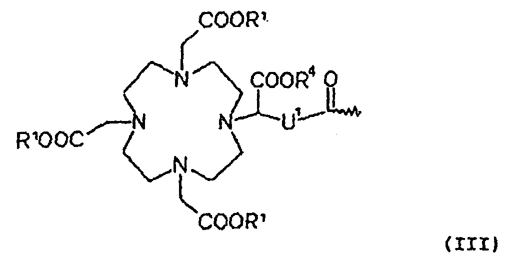 where R¹ is as defined above, R⁴ is hydrogen or a metal ion equivalent identified under R ¹ and U ¹ is -C₆H₄-O-CH₂-ω-, where ω denotes the site of attachment to -CO-,
or of the general formula IV 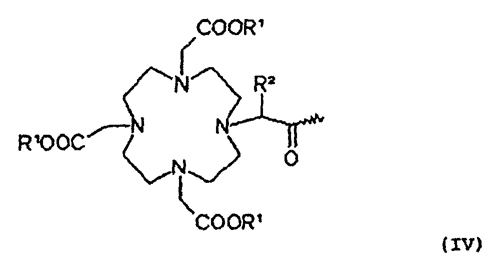 where R¹ and R² are each as defined above,
or of the general formula V A or V B 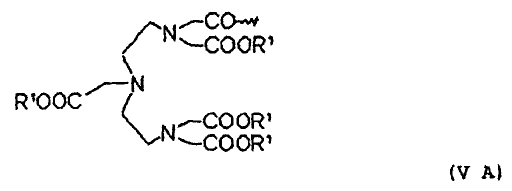 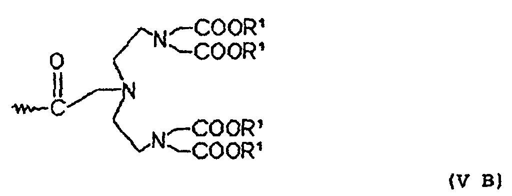 where R¹ is as defined above,
or of the general formula VI 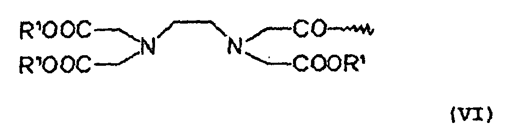 where R¹ is as defined above,
or of the general formula VII 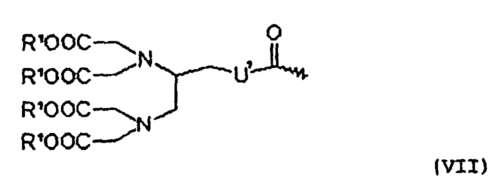 where R¹ is as defined above and
U¹ is -C ₆H₄-O-CH₂-ω-, where ω denotes the site of attachment to -CO-,
or of the general formula VIII 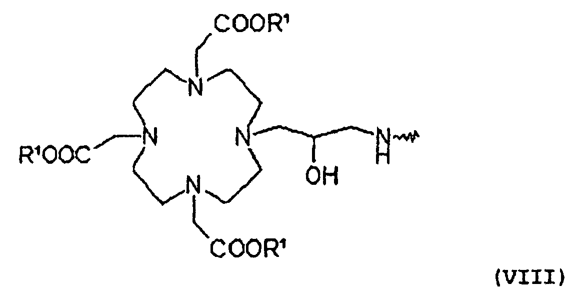 where R¹ is as defined above,
and free acid groups optionally present in the K radical can optionally be present as salts of organic and/or inorganic bases or amino acids or amino acid amides,
G in the event that K represents the metal complexes II to VII is an at least triply functionalized radical selected from the following radicals a) to j) 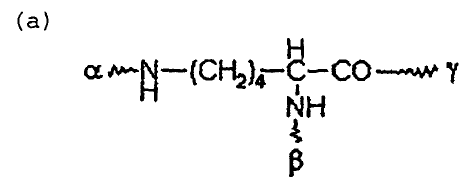 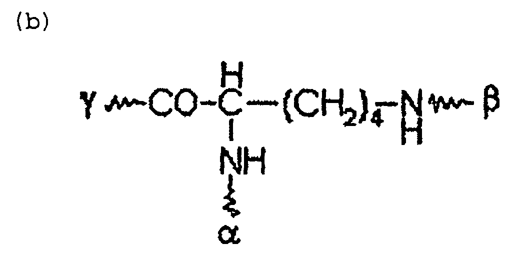 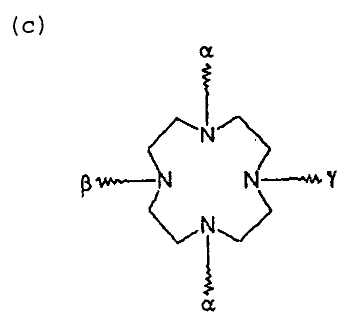 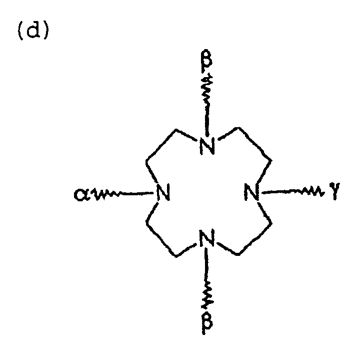 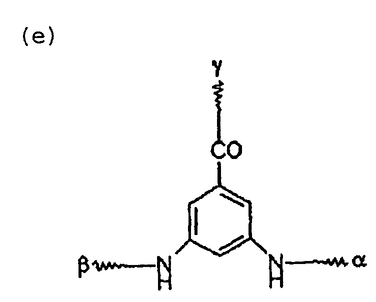 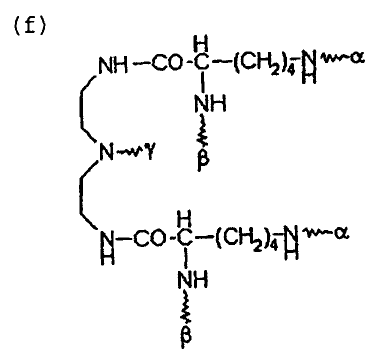 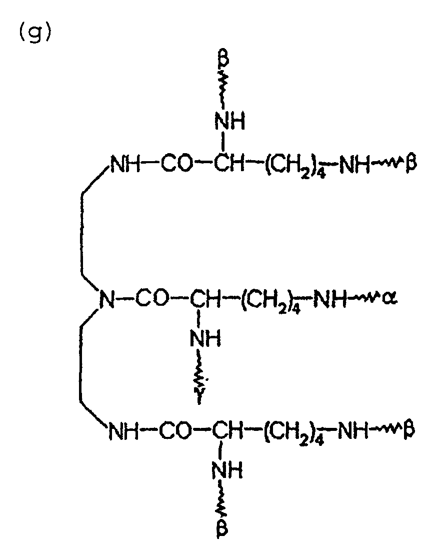 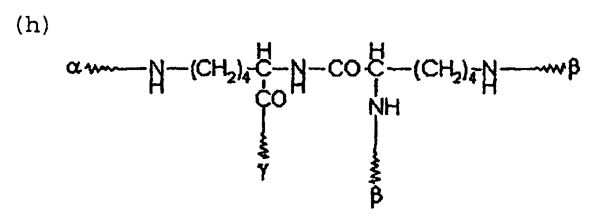 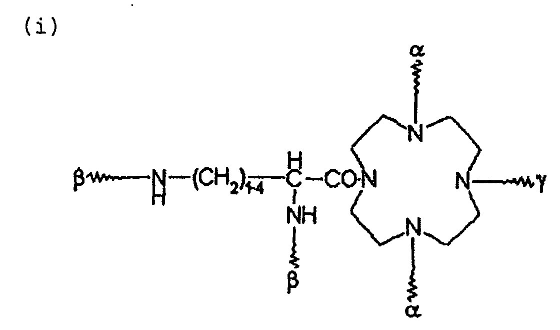 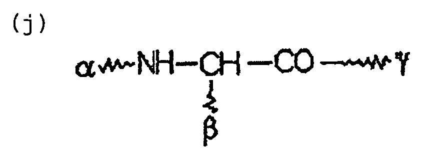
and
G in the event that K represents the metal complex VIII is an at least triply functionalized radical selected from k) or 1) 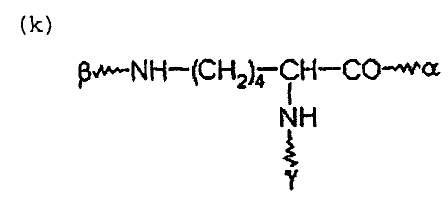 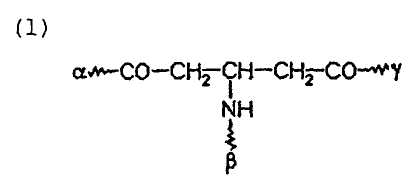
where α denotes the site of attachment of G to the complex K, β denotes the site of attachment of G to the radical Y and γ denotes the site of attachment of G to the radical Z,
Y is -CH ₂-, δ-(CH₂)ₙCO-β ( where n is = 1-5), δ-CH₂-CHOH-CO-β or δ-CH(CHOH-CH₂OH)-CHOH-CHOH-CO-β, where δ denotes the site of attachment to the sugar residue R and β denotes the site of attachment to the radical G,
Z is 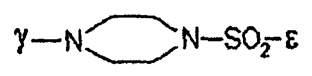 γ-COCH₂-N(C₂H₅)-SO₂-ε
γ-COCH₂-O-(CH₂)₂-SO₂-ε 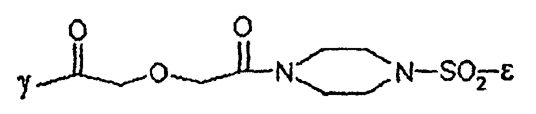 or
γ-NHCH₂CH₂-O-CH₂CH₂-ε
where γ denotes the site of attachment of Z to the radical G and ε denotes the site of attachment of Z to the perfluorinated radical R_{f},
and
l and m are independently integers 1 or 2 and
p is the integers 1 to 4.

2. Metal complexes according to Claim 1, **characterized in that** the metal ion equivalent R ¹ is an element of atomic numbers 21-29, 39, 42, 44 or 57-83.

3. Metal complexes according to Claim 1, **characterized in that** the metal ion equivalent R ¹ is an element of atomic numbers 27, 29, 31-33, 37-39, 43, 49, 62, 64, 70, 75 and 77.

4. Metal complexes according to any one of Claims 1 to 3, **characterized in that** R is a monosaccharide residue having 5 to 6 carbon atoms or its deoxy compound, preferably glucose, mannose or galactose.

5. Metal complexes according to any one of Claims 1 to 4, **characterized in that** K is a metal complex of the general formula II.

6. Metal complexes according to Claim 5, **characterized in that** R² and R³ are independently hydrogen or C₁-C₄ alkyl.

7. Metal complexes according to any one of Claims 1 to 6, **characterized in that** E in the formula -CₙF₂ₙE is a fluorine atom.

8. Metal complexes according to any one of Claims 1 to 7, **characterized in that** G in the general formula I is the lysine residue (a) or (b).

9. Metal complexes according to any one of Claims 1 to 8, **characterized in that** Z in the general formula I is 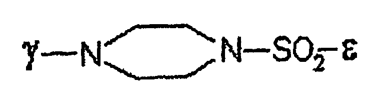 where γ denotes the site of attachment of Z to the radical G and ε denotes the site of attachment of Z to the perfluorinated radical R_{f}.

10. Metal complexes according to any one of Claims 1 to 9, **characterized in that** Y in the general formula I is δ-CH₂CO-β, where δ denotes the site of attachment to the sugar residue R and β denotes the site of attachment to the radical G.

11. Metal complexes according to any one of Claims 1 to 10, **characterized in that** U in the metal complex K is -CH₂- or -C₆H₄-O-CH₂-ω, where ω denotes the site of attachment to -CO-.

12. Use of metal complexes according to Claim 2 for manufacturing contrast media for use in NMR and x -ray diagnostics.

13. Use of metal complexes ac cording to Claim 12 for manufacturing contrast media for infarction and necrosis imaging.

14. Use of metal complexes according to Claim 3 for manufacturing contrast media for use in radiodiagnostics and radiotherapy.

15. Use of metal complexes according to Claim 2 for manufacturing contrast media for lymphography for diagnosing changes in the lymphatic system.

16. Use of metal complexes according to Claim 2 for manufacturing contrast media for use in indirect lymphography.

17. Use of metal complexes acc ording to Claim 2 for manufacturing contrast media for use in intravenous lymphography.

18. Use of metal complexes according to Claim 2 for manufacturing contrast media for visualizing the vascular space.

19. Use of metal complexes according to Claim 2 f or manufacturing contrast media for tumour imaging.

20. Pharmaceutical compositions comprising at least one physiologically compatible compound according to Claims 1 to 11 with or without customary pharmaceutical additives.

21. Process for preparing perf luoroalkyl-containing complexes with sugar residues of the general formula I 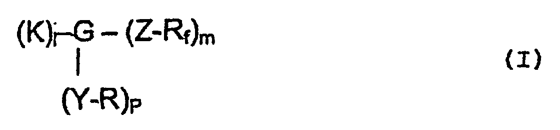 where K has the meaning of a metal complex of the general formulae II to VII according to Claim 1, G has the meaning a) to j) according to Claim 1 and Y, Z, R, R_{f}, m, p and 1 are each as defined according to Claim 1, **characterized in that** it comprises reacting in a conventional manner a carboxylic acid of the general formula IIa 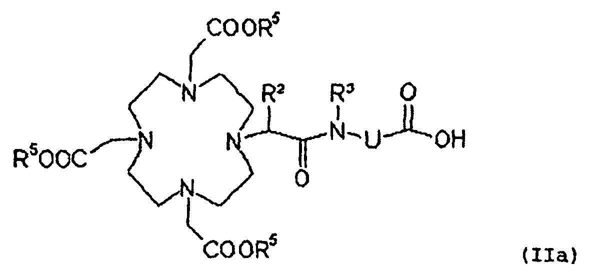 where R⁵ is a metal ion equivalent of atomic numbers 21-29, 31-33, 37-39, 42-44, 49 or 57 -83 or a carboxyl-protecting group and R ², R³ and U are each as defined above
or a carboxylic acid of the general formula IIIa 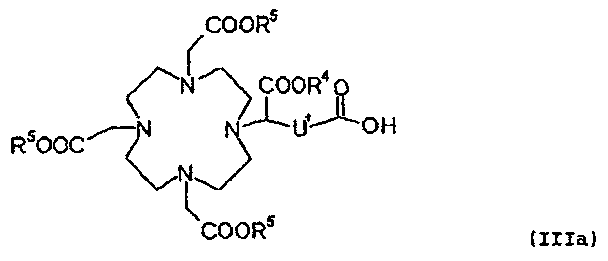 where R⁴, R⁵ and U¹ are each as defined above
or a carboxylic acid of the general formula IVa 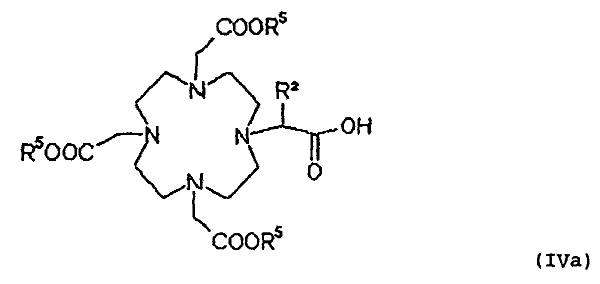 where R⁵ and R² are each as defined above
or a carboxylic acid of the general formula Va or Vb 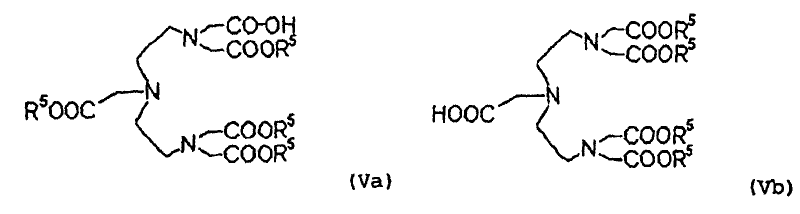 where R⁵ is as defined above
or a carboxylic acid of the general formula VIa 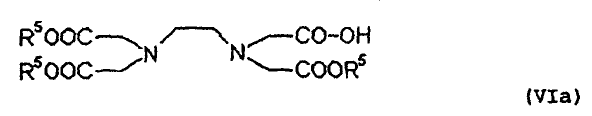 where R⁵ is as defined above
or a carboxylic acid of the general formula VIIa 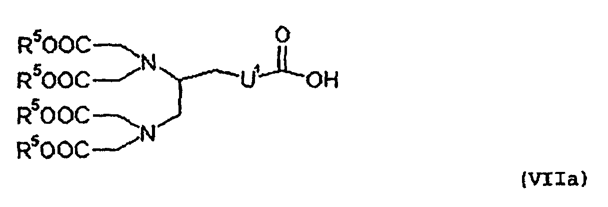 where R⁵ and U¹ are each as defined above,
in optionally activated form with an amine of the general formula IX 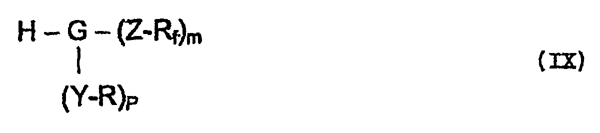 where G, R, R_{f}, Y, Z, m and p are each as defined above in the claim, in a coupling reaction and if appropriate subsequent detachment of any protecting groups to form a metal complex of the general formula I
or
when R⁵ is a protecting group, after detachment of these protecting groups, a subsequent step of reacting in a conventional manner with at least one metal oxide or metal salt of an element of atomic numbers 21-29, 31-33, 37-39, 42-44, 49 or 57-83 and subsequently, if desired, replacing any acidic hydrogen atoms by cations of inorganic and/or organic bases, amino acids or amino acid amides.

22. Process for preparing perfluoroalkyl-containing complexes with sugar residues of the general formula I 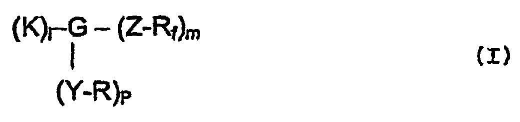 where K has the meaning of a metal complex of the general formula VIII according to Claim 1, G has the meaning k) or 1) according to Claim 1 and Y, Z, R, R _{f}, m, p, 1 are each as defined in Claim 1,
**characterized in that** it comprises reacting in a conventional manner an amine of the general formula VIIIa 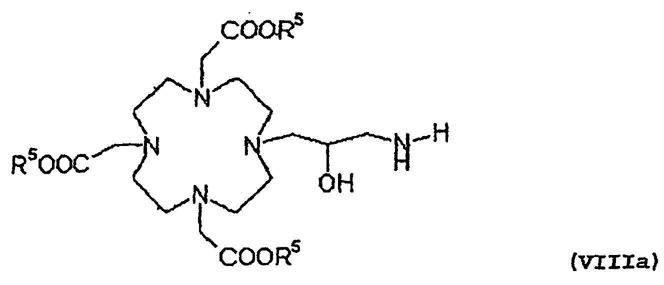 where R⁵ is a metal ion equivalent of atomic numbers 21-29, 31-33, 37-39, 42-44, 49 or 57-83 or a carboxyl-protecting group, with an, optionally activated, carboxylic acid of the general formula X 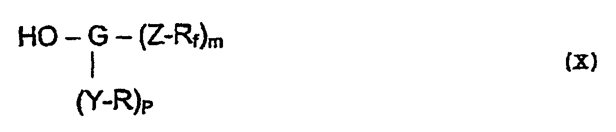 where G, R, R_{f}, Y, Z, m and p are each as defined above in the claim,
in a coupling reaction and if appropriate subsequent detachment of any protecting groups to form a metal complex of the general formula I
or
when R⁵ is a protecting group, after detachment of these protecting groups, a subsequent step of reacting in a conventional manner with at least one metal oxide or metal salt of an element of atomic numbers 21-29, 31-33, 37-39, 42-44, 49 or 57-83 and subsequently, if desired, replacing any acidic hydrogen atoms by cations of inorganic and/or organic bases, amino acids or amino acid amides.

## Revendications

1. Complexes contenant perfluoroalkyle avec des radicaux de sucre de formule générale I 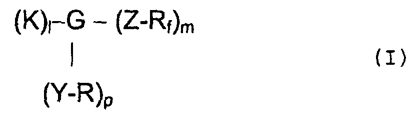 dans laquelle
R représente un radical monosaccharide lié via la position 1-OH ou 1-SH, comprenant 5 ou 6 atomes de carbone, son sucre désoxy ou thio,
R_{f} représente une chaîne carbonée perfluorée, linéaire ou ramifiée de formule -CₙF₂ₙE, dans laquelle E représente un atome de fluor, de chlore, de brome, d'iode ou d'hydrogène en position terminale et n représente les nombres 4-30,
K représente un complexe métallique de formule générale II, 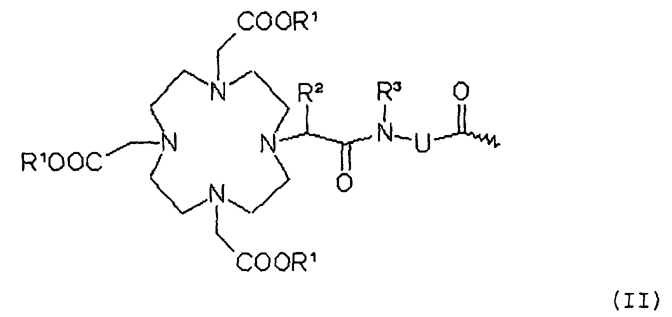
dans laquelle
R¹ signifie un atome d 'hydrogène ou un équivalent d'ion métallique des nombres atomiques 21-29, 31-33, 37-39, 42-44, 49 ou 57-83,
à condition qu'au moins deux R ¹ représentent des équivalents d'ion métallique
R² et R³ représentent, indépendamment 1 'un de l'autre, hydrogène, alkyle en C ₁ à C ₇, benzyle, phényle, -CH₂OH ou -CH₂OCH₃ et
U représente -C₆H₄-O-CH₂-ω-, - (CH₂)₁₋₅-ω, un groupe phénylène, -CH₂-NHCO-CH₂-CH(CH₂COOH)-C₆H₄-ω-, -C₆H₄- (OCH₂CH₂)₀₋₁-N (CH₂COOH) -CH₂-ω ou un groupe alkylène en C₁ à C₁₂ ou C₇-C₁₂-C₆H₄-O- qui est le cas échéant interrompu par un ou plusieurs atomes d'oxygène, 1 à 3 groupes -NHCO-, 1 à 3 groupes -CONH- et/ou substitué par 1 à 3 groupes -(CH₂)₀₋₅COOH-, où ω représente le site de liaison sur -CO-,
ou
de formule générale III 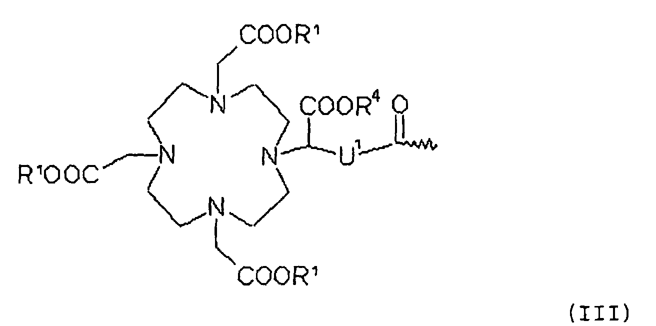 dans laquelle R¹ a la signification susmentionnée, R⁴ représente hydrogène ou un équivalent d'ion métallique mentionné sous R¹ et U¹ représente -C₆H₄-O-CH₂-ω-, ω signifiant le site de liaison sur -CO-
ou de formule générale IV 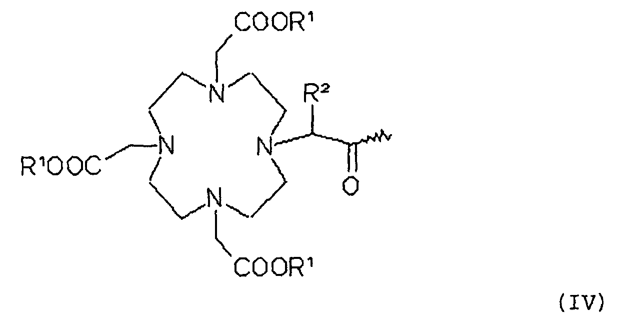 dans laquelle R ¹ et R ² ont la signification susmentionnée
ou de formule générale V A ou V B 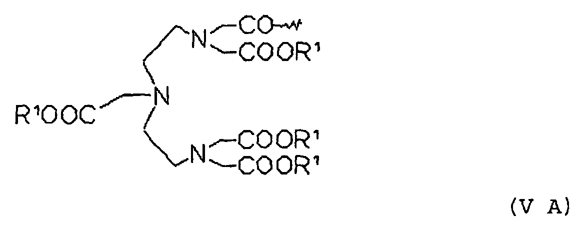 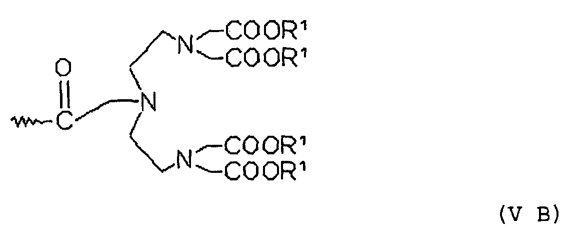 dans laquelle R¹ a la signification susmentionnée,
ou de formule générale VI 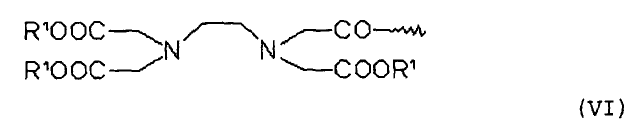 dans laquelle R¹ a la signification susmentionnée,
ou de formule générale VII 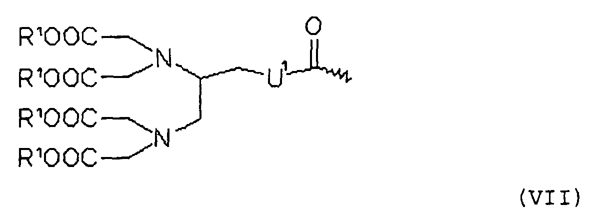 dans laquelle R¹ a la signification susmentionnée et
U¹ représente -C₆H₄-O-CH₂-ω-, ω signifiant le site de liaison sur -CO-
ou de formule générale VIII 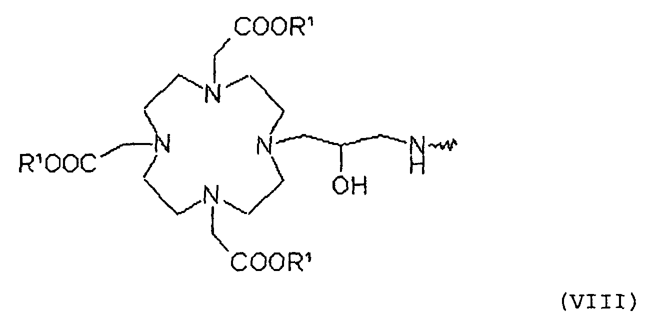 dans laquelle R¹ a la signification susmentionnée,
et les groupes acides libres le cas échéant présents dans le radical K pouvant le cas échéant se trouver sous forme de sels de bases organiques et/ou inorganiques ou d'aminoacides ou d'amides d'aminoacides,
G représente, pour le cas où K représente les complexes métalliques II à VII, un radical au moins trifonctionnalisé choisi parmi les radicaux suivants a) à j) 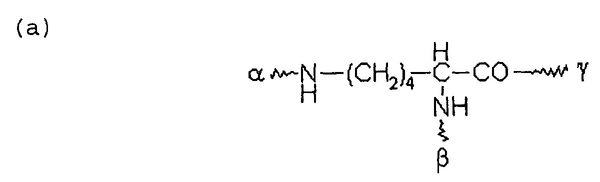 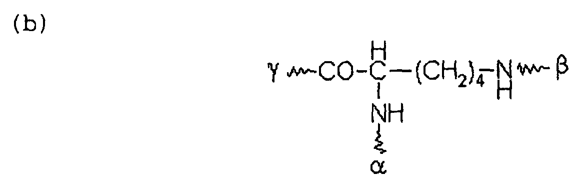 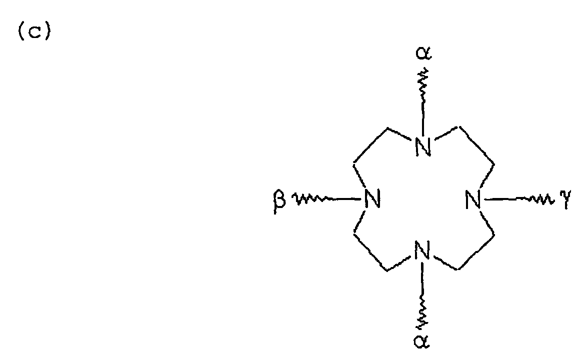 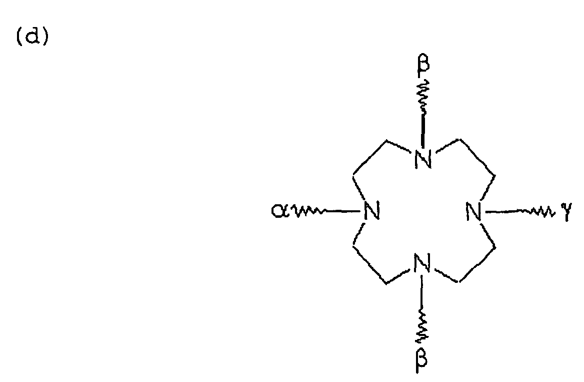 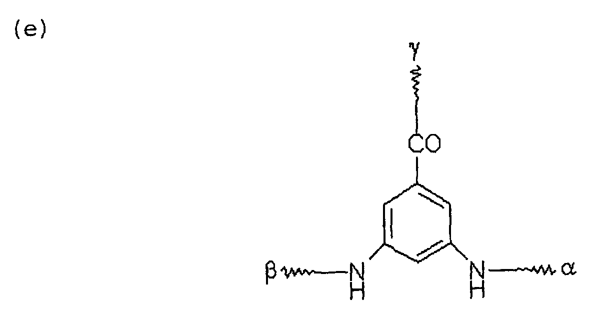 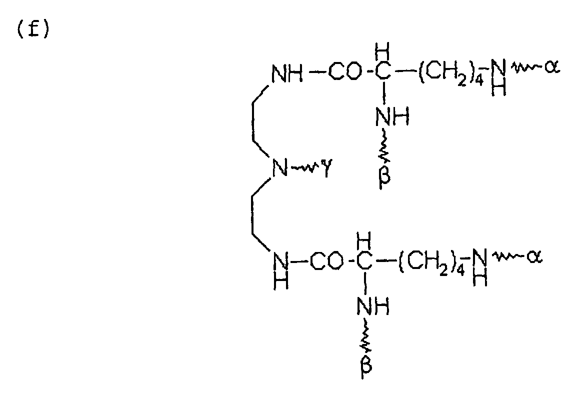 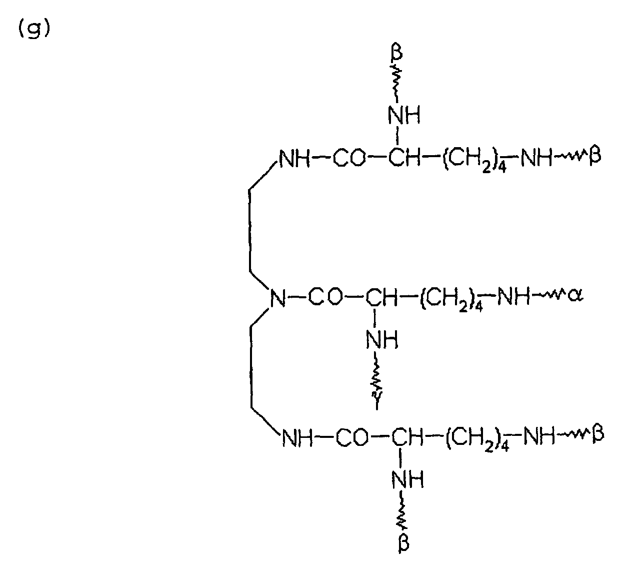 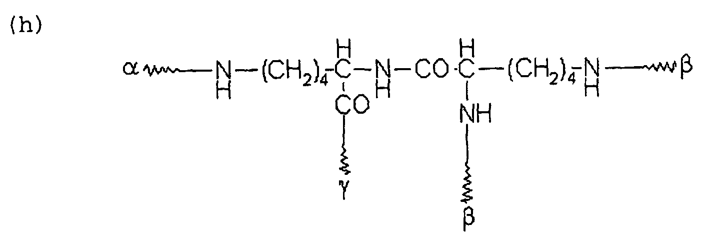 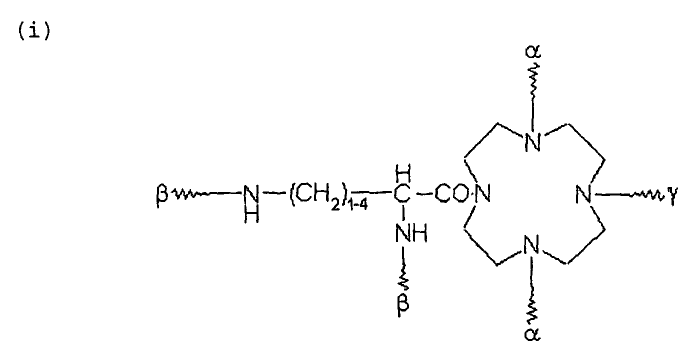 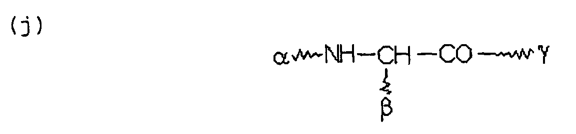 et
G représente, pour le cas où K représente le complexe métallique VIII, un radical au moins trifonctionnalisé choisi parmi k) ou 1), 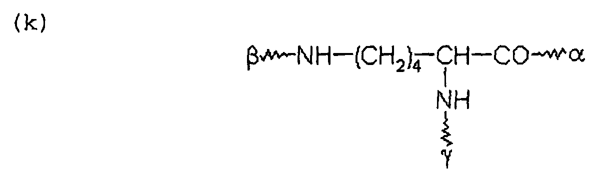 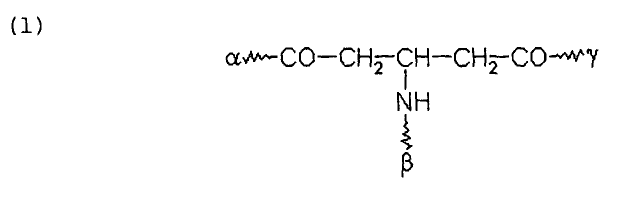 α signifiant le site de liaison de G sur le complexe K, β signifiant le site de liaison de G sur le radical Y et γ représentant le site de liaison de G sur le radical Z
Y signifie -CH₂-, δ-(CH₂)ₙCO-β (où n=1-5), δ-CH₂-CHOH-CO-β ou δ-CH(CHOH-CH₂OH)-CHOH-CHOH-CO-β, δ représentant le site de liaison dans le radical de sucre R et β étant le site de liaison au radical G
Z représente 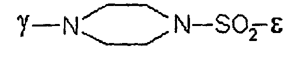 γ-COCH₂-N(C₂H₅)-SO₂-ε,
γ-COCH₂-O-(CH₂)₂-SO₂-ε, 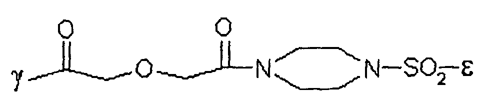 ou
γ - NHCH₂CH₂-O-CH₂CH₂ - ε,
γ représentant le site de liaison de Z au radical G et ε signifiant le site de liaison de Z sur le radical perfluoré R_{f}
et
l, m signifient indépendamment l'un de l'autre les nombres entiers 1 ou 2 et
p signifie les nombres entiers 1 à 4.

2. Complexes métalliques selon la revendication 1,
**caractérisés en ce que** l'équivalent d'ion métallique R¹ est un élément des nombres atomiques 21-29, 39, 42, 44 ou 57-83.

3. Complexes métalliques selon la revendication 1,
**caractérisés en ce que** l'équivalent d'ion métallique R¹ est un élément des nombres atomiques 27, 29, 31-33, 37-39, 43, 49, 62, 64, 70, 75 et 77.

4. Complexes métalliques selon l 'une quelconque des revendications 1 à 3,
**caractérisés en ce que** R représente un radical monosaccharide comprenant 5 à 6 atomes de carbone ou son composé désoxy, de préférence le glucose, le mannose ou le galactose.

5. Complexes métalliques selon l'une quelconque des revendications 1 à 4,
**caractérisés en ce que** K représente un complexe métallique de formule générale II.

6. Complexes métalliques selon la revendication 5,
**caractérisés en ce que** R ² et R ³ représentent, indépendamment l'un de l'autre, hydrogène ou un radical alkyle en C₁ à C₄.

7. Complexes métalliques selon l 'une quelconque des revendications 1 à 6,
**caractérisés en ce que** E dans la formule -C ₙF₂ₙE signifie un atome de fluor.

8. Complexes métalliques selon l 'une quelconque des revendications 1 à 7,
**caractérisés en ce que** G dans la formule générale I représente le radical lysine (a) ou (b).

9. Complexes métalliques selon l 'une quelconque des revendications 1 à 8,
**caractérisés en ce que** Z dans la formule générale I signifie 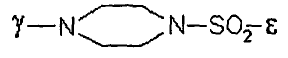 γ représentant le site de liaison de Z au radical G et ε représentant le site de liaison de Z au radical perfluoré R_{f}.

10. Complexes métalliques selon l'une quelconque des revendications 1 à 9,
**caractérisés en ce que** Y dans la formule générale I signifie δ-CH₂CO-β, δ représentant le site de liaison au radical de sucre R et β représentant le site de liaison au radical G.

11. Complexes métalliques selon l 'une quelconque des revendications 1 à 10,
**caractérisés en ce que** U dans le complexe métallique K représente -CH ₂- ou -C ₆H₄-O-CH₂-ω, ω représentant le site de liaison sur -CO-.

12. Utilisation de complexes métalliques selon la revendication 2 pour la préparation d'agents de contraste destinés à une utilisation dans le diagnostic par RMN et rayons X.

13. Utilisation de complexes métalliques selon la revendication 12 pour la préparation d'agents de contraste destinés à l'imagerie de l'infarctus et de la nécrose.

14. Utilisation de complexes métalliques selon la revendication 3 pour la préparation d'agents de contraste destinés à une utilisation dans le radio-diagnostic et la radiothérapie.

15. Utilisation de complexes métalliques selon la revendication 2 pour la préparation d'agents de contraste destinés à la lymphographie en vue du diagnostic de modifications du système lymphatique.

16. Utilisation de complexes métalliques selon la revendication 2 pour la préparation d'agents de contraste destinés à une utilisation dans la lymphographie indirecte.

17. Utilisation de complexes métalliques selon la revendication 2 pour la préparation d'agents de contraste destinés à une utilisation dans la lymphographie intraveineuse.

18. Utilisation de complexes métalliques selon la revendication 2 pour la préparation d'agents de contraste destinés à la représentation de l'espace vasculaire.

19. Utilisation de complexes métalliques selon la revendication 2 pour la préparation d'agents de contraste destinés à l'imagerie de tumeurs.

20. Agents pharmaceutiques contenant au moins un composé physiologiquement acceptable selon les revendications 1 à 11, le cas échéant avec les additifs usuels en galénique.

21. Procédé pour la préparation de complexes contenant perfluoroalkyle avec des radicaux de sucre de formule générale I 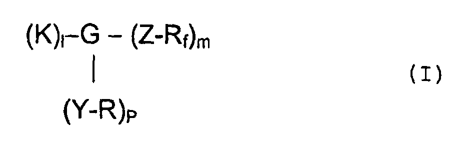 avec K ayant la signification d'un complexe métallique de formules générales II à VII selon la revendication 1, G ayant la signification a) à j) selon la revendication 1, et Y, Z, R, R_{f}, m, p et 1 ayant la signification selon la revendication 1 **caractérisé en ce qu'**on transforme de manière connue en soi un acide carboxylique de formule générale IIa 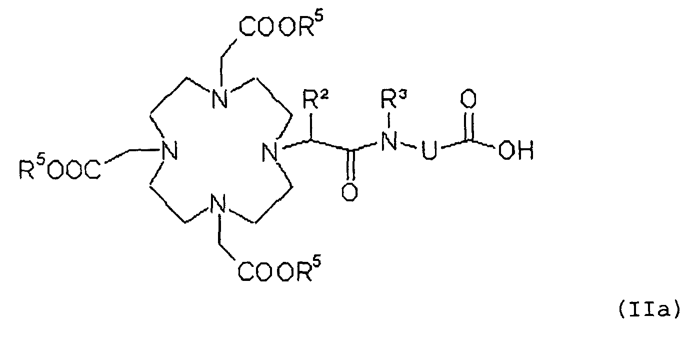 dans laquelle R ⁵ signifie un équivalent d'ion métallique des nombres atomiques 21-29, 31-33, 37-39, 42-44, 49 ou 57-83 ou un groupe de protection de la fonction carboxyle et R ², R³ et U ont la signification mentionnée
ou un acide carboxylique de formule générale IIIa 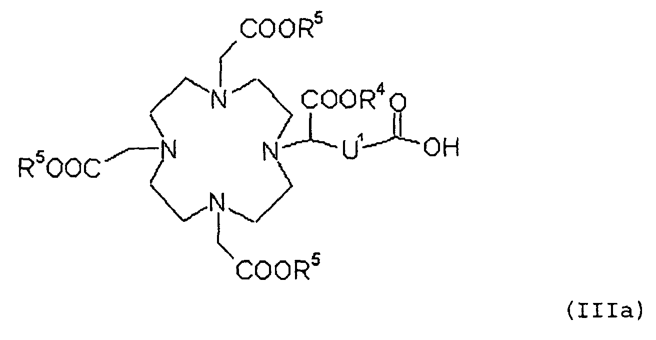 dans laquelle R ⁴, R ⁵ et U ¹ ont la signification mentionnée
ou un acide carboxylique de formule générale IVa 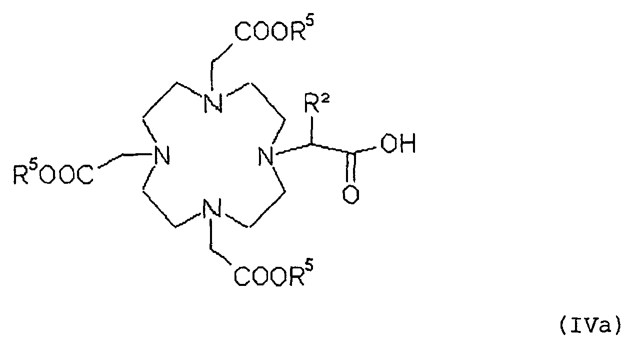 dans laquelle R ⁵ et R ² ont la signification mentionnée
ou un acide carboxylique de formule générale Va ou Vb 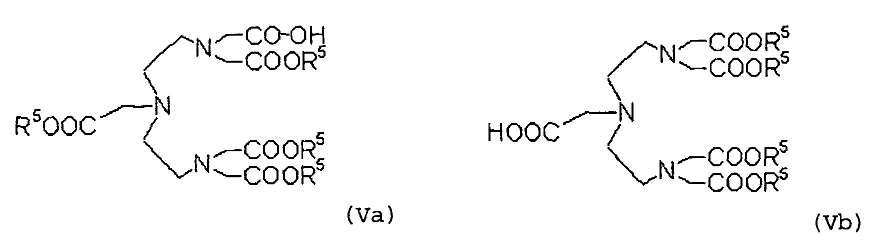 dans laquelle R⁵ a la signification mentionnée
ou un acide carboxylique de formule générale VIa 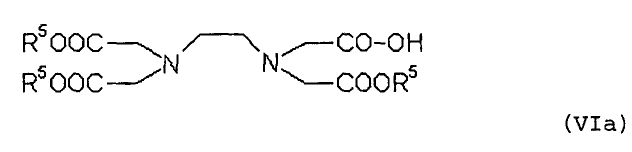 dans laquelle R⁵ a la signification mentionnée
ou un acide carboxylique de formule générale VIIa 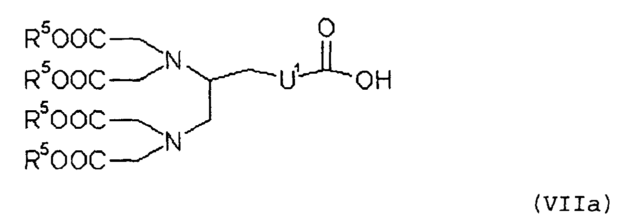 dans laquelle R ⁵ et U ¹ ont les significations mentionnées,
sous forme éventuellement activée avec une amine de formule générale IX 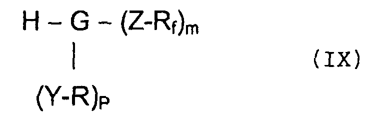 dans laquelle G, R, R _{f}, Y, Z, m et p ont la signification indiquée ci-dessus dans la revendication, dans une réaction de couplage et le cas échéant avec une dissociation consécutive des groupes de protection éventuellement présents, en un complexe métallique de formule générale I
ou
lorsque R ⁵ a la signification d'un groupe de protection, on transforme, après la dissociation de ces groupes de protection, dans une étape consécutive de manière connue en soi avec au moins un oxyde de métal ou un sel de métal d'un élément des nombres atomiques 21-29, 31-33, 37-39, 42-44, 49 ou 57-83 et on substitue ensuite, si souhaité, les atomes d'hydrogène acides le cas échéant présents par des cations de bases inorganiques et/ou organiques, des aminoacides ou des amides d'aminoacides.

22. Procédé pour la préparation de complexes contenant perfluoroalkyle comprenant des radicaux de sucre de formule générale I 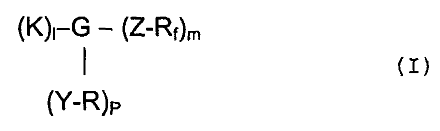 K ayant la signification d'un complexe métallique de formule générale VIII selon la revendication 1, G ayant la signification k) ou 1) selon la revendication 1 et Y, Z, R, R _{f}, m, p, 1 ayant la signification selon la revendication 1,
**caractérisé en ce qu'**on transforme de manière connue en soi une amine de formule générale VIIIa 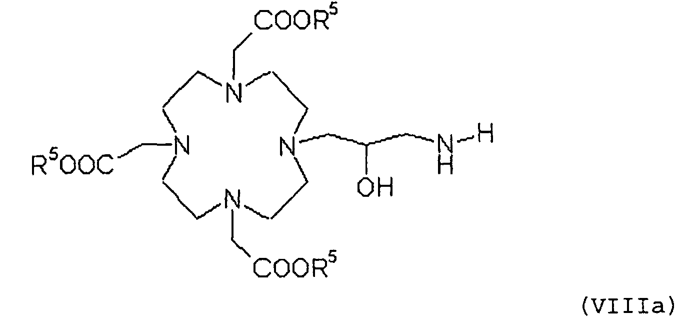 dans laquelle R ⁵ signifie un équivalent d'ion métallique des nombres atomiques 21-29, 31-33, 37-39, 42-44, 49 ou 57-83 ou un groupe de protection de la fonction carboxyle,
avec un acide carboxylique le cas échéant activé de formule générale X 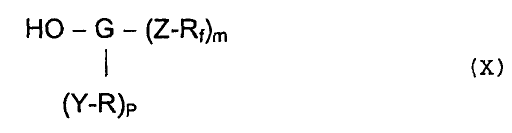 dans laquelle G, R, Rf, Y, Z, m et p ont les significations indiquées ci-dessus dans la revendication, dans une réaction de couplage et le cas échéant avec une dissociation consécutive de groupes de protection éventuellement présents en un complexe métallique de formule générale I
ou
lorsque R ⁵ a la signification d'un groupe de protection, après dissociation de ces groupes de protection dans une étape consécutive de manière connue en soi avec au moins un oxyde de métal ou un sel de métal d'un élément des nombres atomiques 21-29, 31-33, 37-39, 42-44, 49 ou 57-83 et on substitue ensuite, si souhaité, les atomes d'hydrogène acides le cas échéant présents par des cations de bases inorganiques et/ou organiques, des aminoacides ou des amides d'aminoacides.
